# EUROPÄISCHE PATENTANMELDUNG

(11) **EP 2 295 436 A1**
(43) Veröffentlichungstag der Anmeldung: **16.03.2011**
(21) Anmeldenummer: 10182939.8
(22) Anmeldetag: 31.10.1998
(51) Int. Cl.: C07D 487/04, A61K 31/53

(54) **2-Phenyl-substituierte Imidazotriazinone als Phosphodiesterase V Inhibitoren**

(30) Priorität: 12.11.1997 DE 19750085; 23.03.1998 DE 19812462; 04.09.1998 DE 19840289
(62) Teilanmeldung aus: 01123321.0
(71) Anmelder: Bayer Schering Pharma AG, 13353 Berlin (DE)
(72) Erfinder: Niewöhner, Maria, 42929 Wermelskirchen (DE); Es-Sayed, Mazen, Dr., 69011 Lyon (FR); Haning, Helmut, Dr., 42327 Wuppertal (DE); Schenke, Thomas, Dr., 51469 Bergisch Gladbach (DE); Schlemmer, Karl-Heinz, Dr., 42113 Wuppertal (DE); Keldenich, Jörg, Dr., 42113 Wuppertal (DE); Bischoff, Erwin, Dr., 42115 Wuppertal (DE); Perzborn, Elisabeth, Dr., 42327 Wuppertal (DE); Dembowsky, Klaus, Dr., 81249 München (DE); Serno, Peter, Dr., 51467 Bergisch Gladbach (DE); Nowakowski, Marc, Dr., 42111 Wuppertal (DE)

(57) **Zusammenfassung**

Die 2-Phenyl-substituierten Imidazotriazinone mit kurzen, unverzweigten Alkylresten in der 9-Position werden aus den entsprechenden 2-Phenyl-imidazotriazinonen durch Chlorsulfonierung und anschließender Umsetzung mit den Aminen hergestellt. Die Verbindungen hemmen cGMP-metabolisierende Phosphodiesterasen und eignen sich als Wirkstoffe in Arzneimitteln, zur Behandlung von cardiovaskulären und cerebrovaskulären Erkrankungen und/oder Erkrankungen des Urogenitalsystems, insbesondere zur Behandlung der erektilen Dysfunktion.

## Beschreibung

Die vorliegende Erfindung betrifft 2-Phenyl-substituierte Imidazotriazinone, Verfahren zu ihrer Herstellung und ihre Verwendung als Arzneimittel, insbesondere als Inhibitoren cGMP-metabolisierender Phosphodiesterasen.

In der Offenlegungsschrift DE 28 11 780 sind Imidazotriazine als Bronchodilatoren mit spasmolytischer Aktivität und Hemmaktivität gegen cyclisches Adenosin-monophosphat metabolisierende Phosphodiesterasen (cAMP-PDE's, Nomenklatur nach Beavo: PDE-111 und PDE-IV) beschrieben. Eine Hemmwirkung gegen cyclisches Guanosin-monophosphat metabolisierende Phosphodiesterasen (cGMP-PDE's, Nomenklatur nach Beavo und Reifsnyder (Trends in Pharmacol. Sci. 11,150-155, 1990) PDE-I, PDE-II und PDE-V) ist nicht beschrieben. Es werden keine Verbindungen beansprucht, die eine Sulfonamidgruppe im Arylrest in der 2-Position enthalten. Weiterhin werden Imidazotriazinone in FR 22 13 058, CH 59 46 71, DE 22 55 172, DE 23 64 076 und EP 000 9384 beschrieben, die in der 2-Position keinen substituierten Arylrest besitzen, und ebenfalls als Bronchodilatatoren mit cAMP-PDE inhibitorischer Wirkung beschrieben werden.

In WO 94/28902 werden Pyrazolopyrimidinone beschrieben, die sich für die Behandlung von Impotenz eignen.

Die erfindungsgemäßen Verbindungen sind potente Inhibitoren von entweder einer oder mehrerer der cyclisches Guanosin 3',5'-monophosphat metabolisierenden Phosphodiesterasen (cGMP-PDE's). Entsprechend der Nomenklatur von Beavo und Reifsnyder (Trends in Pharmacol. Sci. 11, 150-155, 1990) handelt es sich um die Phosphodiesterase Isoenzyme PDE-I, PDE-II und PDE-V.

Ein Anstieg der cGMP-Konzentration kann zu heilsamen, antiaggregatorischen, antithrombotischen, antiproliferativen, antivasospastischen, vasodilatierenden, natriuretischen und diuretischen Effekten führen. Es kann die Kurz- oder Langzeitmodulation der vaskulären und kardialen Inotropie, den Herzrhythmus und die kardiale Erregungsleitung beeinflussen (J.C. Stoclet, T. Keravis, N. Komas and C. Kugnier, Exp. Opin. Invest. Drugs (1995), 4 (11), 1081 -1100).

Die vorliegende Erfindung betrifft jetzt 2-Phenyl-substituierte Imidazotriazinone der allgemeinen Formel (I) 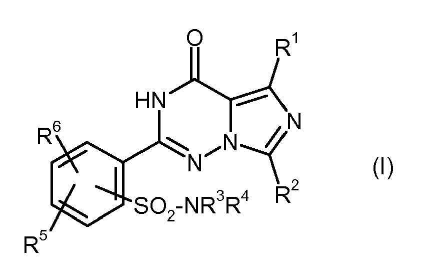 in welcher
- R¹: für Wasserstoff oder geradkettiges oder verzweigtes Alkyl mit bis zu 4 Kohlenstoffatomen steht,
- R²: für geradkettiges Alkyl mit bis zu 4 Kohlenstoffatomen steht,
- R³ und R⁴: gleich oder verschieden sind und für Wasserstoff oder für geradkettiges oder verzweigtes Alkenyl oder Alkoxy mit jeweils bis zu 8 Kohlenstoffatomen stehen, oder
für eine geradkettige oder verzweigte Alkylkette mit bis zu 10 Kohlenstoffatomen stehen, die gegebenenfalls durch ein Sauerstoffatom unterbrochen ist, und die gegebenenfalls ein- bis mehrfach, gleich oder verschieden durch Trifluormethyl, Trifluormethoxy, Hydroxy, Halogen, Carboxyl, Benzyloxycarbonyl, geradkettiges oder verzweigtes Alkoxycarbonyl mit bis zu 6 Kohlenstoffatomen und/oder durch Reste der Formeln -SO₃H, -(A)ₐ-NR⁷R⁸, -O-CO-NR^{7'}R^{8'}, -S(O)_{b}-R⁹, -P(O)(OR¹⁰(OR¹¹), 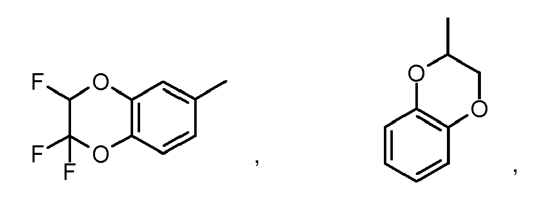 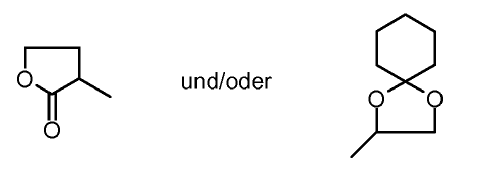
substituiert ist,
worin
- a und b: gleich oder verschieden sind und eine Zahl 0 oder 1 bedeuten,
- A: einen Rest CO oder SO₂ bedeutet,
- R⁷, R^{7'}, R⁸ und R^{8'}: gleich oder verschieden sind und Wasserstoff bedeuten, oder
Cycloalkyl mit 3 bis 8 Kohlenstoffatomen, Aryl mit 6 bis 10 Kohlenstoffatomen, einen 5- bis 6-gliedrigen ungesättigten, partiell ungesättigten oder gesättigten, gegebenenfalls benzokondensierten Heterocyclus, mit bis zu 3 Heteroatomen aus der Reihe S, N und/oder O bedeuten, wobei die oben aufgeführten Ringsysteme gegebenenfalls ein- bis mehrfach, gleich oder verschieden durch Hydroxy, Nitro, Trifluormethyl, Trifluormethoxy, Carboxyl, Halogen, geradkettiges oder verzweigtes Alkoxy oder Alkoxycarbonyl mit jeweils bis zu 6 Kohlenstoffatomen oder durch eine Gruppe der Formel -(SO₂)_{c}-NR¹²R¹³ substituiert sind,
worin
c eine Zahl 0 oder 1 bedeutet,
R¹² und R¹³ gleich oder verschieden sind und Wasserstoff oder geradkettiges oder verzweigtes Alkyl mit bis zu 5 Kohlenstoffatomen bedeuten, oder
- R⁷, R^{7'}, R⁸ und R^{8'}: geradkettiges oder verzweigtes Alkoxy mit bis zu 6 Kohlenstoffatomen bedeuten, oder geradkettiges oder verzweigtes Alkyl mit bis zu 8 Kohlenstoffatomen bedeuten, das gegebenenfalls ein- oder mehrfach, gleich oder verschieden durch Hydroxy, Halogen, Aryl mit 6 bis 10 Kohlenstoffatomen, geradkettiges oder verzweigtes Alkoxy oder Alkoxycarbonyl mit jeweils bis zu 6 Kohlenstoffatomen oder durch eine Gruppe der Formel -(CO)_{d}-NR¹⁴R¹⁵ substituiert ist,
worin
R¹⁴ und R¹⁵ gleich oder verschieden sind und Wasserstoff oder geradkettiges oder verzweigtes Alkyl mit bis zu 4 Kohlenstoffatomen bedeuten,
und
- d: eine Zahl 0 oder 1 bedeutet,
oder
- R⁷ und R⁸ und/oder R^{7'} und R^{8'}: gemeinsam mit dem Stickstoffatom einen 5-bis 7-gliedrigen, gesättigten Heterocyclus bilden, der gegebenenfalls noch ein weiteres Heteroatom aus der Reihe S oder O oder einen Rest der Formel -NR¹⁶ enthalten kann,
worin
R¹⁶ Wasserstoff, Aryl mit 6 bis 10 Kohlenstoffatomen, Benzyl, einen 5- bis 7-gliedrigen aromatischen oder gesättigten Heterocyclus mit bis zu 3 Heteroatomen aus der Reihe S, N und/oder O bedeutet, der gegebenenfalls durch Methyl substituiert ist, oder geradkettiges oder verzweigtes Alkyl mit bis zu 6 Kohlenstoffatomen bedeutet, das gegebenenfalls durch Hydroxy substituiert ist,
- R⁹: Aryl mit 6 bis 10 Kohlenstoffatomen bedeutet, oder geradkettiges oder verzweigtes Alkyl mit bis zu 4 Kohlenstoffatomen bedeutet,
- R¹⁰ und R¹¹: gleich oder verschieden sind und Wasserstoff oder geradkettiges oder verzweigtes Alkyl mit bis zu 4 Kohlenstoffatomen bedeuten,
und/oder die oben unter R³/R⁴ aufgeführte Alkylkette gegebenenfalls durch Cycloalkyl mit 3 bis 8 Kohlenstoffatomen, Aryl mit 6 bis 10 Kohlenstoffatomen oder durch einen 5- bis 7-gliedrigen, partiell ungesättigten, gesättigten oder ungesättigten, gegebenenfalls benzokondensierten Heterocyclus, der bis zu 4 Heteroatome aus der Reihe S, N; O oder einen Rest der Formel -NR¹⁷ enthalten kann, substituiert ist,
worin
- R¹⁷: Wasserstoff, Hydroxy, Formyl, Trifluormethyl, geradkettiges oder verzweigtes Acyl oder Alkoxy mit jeweils bis zu 4 Kohlenstoffatomen bedeutet,
oder geradkettiges oder verzweigtes Alkyl mit bis zu 6 Kohlenstoffatomen bedeutet, das gegebenenfalls ein- bis mehrfach, gleich oder verschieden durch Hydroxy, oder geradkettiges oder verzweigtes Alkoxy mit bis zu 6 Kohlenstoffatomen substituiert ist,
und wobei Aryl und der Heterocyclus gegebenenfalls ein- bis mehrfach, gleich oder verschieden durch Nitro, Halogen, -SO₃H, geradkettiges oder verzweigtes Alkyl oder Alkoxy mit jeweils bis zu 6 Kohlenstoffatomen, Hydroxy, Trifluormethyl, Trifluormethoxy und/oder durch einen Rest der Formel -SO₂NR¹⁸R¹⁹ substituiert sind,
worin
- R¹⁸ und R¹⁹: gleich oder verschieden sind und Wasserstoff oder geradkettiges oder verzweigtes Alkyl mit bis zu 6 Kohlenstoffatomen bedeuten,
und/oder
R³ oder R⁴ für eine Gruppe der Formel -NR²⁰R²¹ steht,
worin
- R²⁰ und R²¹: die oben angegebene Bedeutung von R¹⁸ und R¹⁹ haben und mit dieser gleich oder verschieden sind,
und/oder
R³ oder R⁴ für Adamantyl stehen, oder
für Reste der Formeln 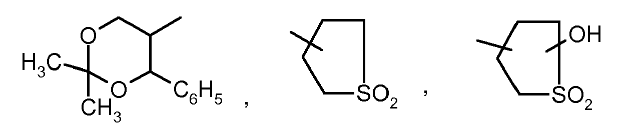 oder 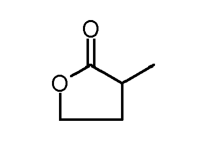 stehen,
oder für Cycloalkyl mit 3 bis 8 Kohlenstoffatomen, Aryl mit 6 bis 10 Kohlenstoffatomen oder für einen 5- bis 7-gliedrigen partiell ungesättigten, gesättigten oder ungesättigten, gegebenenfalls benzokondensierten Heterocyclus stehen, der bis zu 4 Heteroatome aus der Reihe S, N; O oder einen Rest der Formel -NR²² enthalten kann,
worin
- R²²: die oben angegebene Bedeutung von R¹⁶ hat und mit dieser gleich oder verschieden ist, oder Carboxyl, Formyl oder geradkettiges oder verzweigtes Acyl mit bis zu 5 Kohlenstoffatomen bedeutet,
und wobei Cycloalkyl, Aryl und/oder der Heterocyclus gegebenenfalls ein- bis mehrfach, gleich oder verschieden durch Halogen, Triazolyl, Trifluormethyl, Trifluormethoxy, Carboxyl, geradkettiges oder verzweigtes Acyl oder Alkoxycarbonyl mit jeweils bis zu 6 Kohlenstoffatomen, Nitro und/oder durch Gruppen der Formeln -SO₃H, -OR²³, (SO₂)ₑNR²⁴R²⁵, -P(O)(OR²⁶)(OR²⁷) substituiert sind,
worin
- e: eine Zahl 0 oder 1 bedeutet,
- R²³: einen Rest der Formel 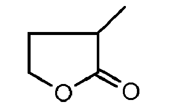 bedeutet, oder
Cycloalkyl mit 3 bis 7 Kohlenstoffatomen bedeutet, oder Wasserstoff oder geradkettiges oder verzweigtes Alkyl mit bis zu 4 Kohlenstoffatomen bedeutet, das gegebenenfalls durch Cycloalkyl mit 3 bis 7 Kohlenstoffatomen, Benzyloxy, Tetrahydropyranyl, Tetrahydrofuranyl, geradkettiges oder verzweigtes Alkoxy oder Alkoxycarbonyl mit jeweils bis zu 6 Kohlenstoffatomen, Carboxyl, Benzyloxycarbonyl oder Phenyl substituiert ist, das seinerseits ein- bis mehrfach, gleich oder verschieden durch geradkettiges oder verzweigtes Alkoxy mit bis zu 4 Kohlenstoffatomen, Hydroxy oder Halogen substituiert sein kann,
und/oder Alkyl gegebenenfalls durch Reste der Formeln -CO-NR²⁸R²⁹ oder -CO-R³⁰ substituiert ist,
worin
R²⁸ und R²⁹ gleich oder verschieden sind und Wasserstoff oder geradkettiges oder verzweigtes Alkyl mit bis zu 8 Kohlenstoffatomen bedeuten, oder
R²⁸ und R²⁹ gemeinsam mit dem Stickstoffatom einen 5- bis 7-gliedrigen gesättigten Heterocyclus bilden, der gegebenenfalls ein weiteres Heteroatom aus der Reihe S oder O enthalten kann,
und
R³⁰ Phenyl oder Adamantyl bedeutet,
- R²⁴ und R²⁵: die oben angegebene Bedeutung von R¹⁸ und R¹⁹ haben und mit dieser gleich oder verschieden sind,
- R²⁶ und R²⁷: die oben angegebene Bedeutung von R¹⁰ und R¹¹ haben und mit dieser gleich oder verschieden sind
und/oder Cycloalkyl, Aryl und/oder der Heterocyclus gegebenenfalls durch geradkettiges oder verzweigtes Alkyl mit bis zu 6 Kohlenstoffatomen substituiert sind, das gegebenenfalls durch Hydroxy, Carboxyl, durch einen 5- bis 7-gliedrigen Heterocyclus mit bis zu 3 Heteroatomen aus der Reihe S, N und/oder O oder durch Gruppen der Formel -SO₂-R³¹, P(O)(OR³²)(OR³³) oder -NR³⁴R³⁵ substituiert ist,
worin
- R³¹: Wasserstoff bedeutet oder die oben angegebene Bedeutung von R⁹ hat und mit dieser gleich oder verschieden ist,
- R³² und R³³: die oben angegebene Bedeutung von R¹⁰ und R¹¹ haben und mit dieser gleich oder verschieden sind,
- R³⁴ und R³⁵: gleich oder verschieden sind und Wasserstoff oder geradkettiges oder verzweigtes Alkyl mit bis zu 6 Kohlenstoffatomen bedeuten, das gegebenenfalls durch Hydroxy oder geradkettiges oder verzweigtes Alkoxy mit bis zu 4 Kohlenstoffatomen substituiert ist, oder
- R³⁴ und R³⁵: gemeinsam mit dem Stickstoffatom einen 5- bis 6-gliedrigen gesättigten Heterocyclus bilden, der ein weiteres Heteroatom aus der Reihe S oder O oder einen Rest der Formel -NR³⁶ enthalten kann,
worin
R³⁶ Wasserstoff, Hydroxy, geradkettiges oder verzweigtes Alkoxycarbonyl mit bis zu 7 Kohlenstoffatomen oder geradkettiges oder verzweigtes Alkyl mit bis zu 5 Kohlenstoffatomen bedeutet, das gegebenenfalls durch Hydroxy substituiert ist,
oder
- R³ und R⁴: gemeinsam mit dem Stickstoffatom einen 5- bis 7-gliedrigen, ungesättigten oder gesättigten oder partiell ungesättigten, gegebenenfalls benzokondensierten Heterocyclus bilden, der gegebenenfalls bis zu 3 Heteroatome aus der Reihe S, N, O oder einen Rest der Formel -NR³⁷ enthalten kann,
worin
R³⁷ Wasserstoff, Hydroxy, Formyl, Trifluormethyl, geradkettiges oder verzweigtes Acyl, Alkoxy oder Alkoxycarbonyl mit jeweils bis zu 4 Kohlenstoffatomen bedeutet, oder geradkettiges oder verzweigtes Alkyl mit bis zu 6 Kohlenstoffatomen bedeutet, das gegebenenfalls ein- bis mehrfach, gleich oder verschieden durch Hydroxy, Trifluormethyl, Carboxyl, geradkettiges oder verzweigtes Alkoxy oder Alkoxycarbonyl mit jeweils bis zu 6 Kohlenstoffatomen oder durch Gruppen der Formel -(D)_{f-}NR³⁸R³⁹, -CO-(CH₂)_{g}-O-CO-R⁴⁰, -CO-(CH₂)ₕ-OR⁴¹ oder -P(O)(OR⁴²)(OR⁴³) substituiert ist, worin
g und h gleich oder verschieden sind und eine Zahl 1, 2, 3 oder 4 bedeuten,
und
f eine Zahl 0 oder 1 bedeutet,
D eine Gruppe der Formel -CO oder -SO₂ bedeutet,
R³⁸ und R³⁹ gleich oder verschieden sind und die oben angegebene Bedeutung von R⁷ und R⁸ haben,
R⁴⁰ geradkettiges oder verzweigtes Alkyl mit bis zu 6 Kohlenstoffatomen bedeutet,
R⁴¹ geradkettiges oder verzweigtes Alkyl mit bis zu 6 Kohlenstoffatomen bedeutet,
R⁴² und R⁴³ gleich oder verschieden sind und Wasserstoff oder geradkettiges oder verzweigtes Alkyl mit bis zu 4 Kohlenstoffatomen bedeuten, oder
- R³⁷: einen Rest der Formel -(CO)ᵢ-E bedeutet,
worin
i eine Zahl 0 oder 1 bedeutet,
- E: Cycloalkyl mit 3 bis 7 Kohlenstoffatomen oder Benzyl bedeutet,
Aryl mit 6 bis 10 Kohlenstoffatomen oder einen 5- bis 6-gliedrigen aromatischen Heterocyclus mit bis zu 4 Heteroatomen aus der Reihe S, N und/oder O bedeutet, wobei die oben aufgeführten Ringsysteme gegebenenfalls ein- bis mehrfach, gleich oder verschieden durch Nitro, Halogen, -SO₃H, geradkettiges oder verzweigtes Alkoxy mit bis zu 6 Kohlenstoffatomen, Hydroxy, Trifluormethyl, Trifluormethoxy oder durch einen Rest der Formel -SO₂-NR⁴⁴R⁴⁵, substituiert sind,
worin
R⁴⁴ und R⁴⁵ die oben angegebene Bedeutung von R¹⁸ und R¹⁹ haben und mit dieser gleich oder verschieden sind,
oder
- E: Reste der Formeln 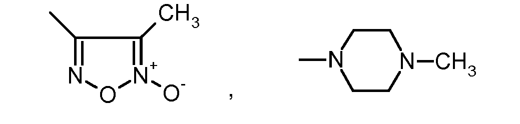 oder 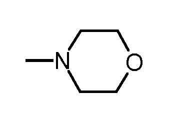 bedeutet,
und der unter R³ und R⁴ aufgeführte, gemeinsam mit dem Stickstoffatom gebildete Heterocyclus, gegebenenfalls ein- bis mehrfach, gleich oder verschieden, gegebenenfalls auch geminal, durch Hydroxy, Formyl, Carboxyl, geradkettiges oder verzweigtes Acyl oder Alkoxycarbonyl mit bis jeweils zu 6 Kohlenstoffatomen, Nitro und Gruppen der Formeln -P(O)(OR⁴⁶)(OR⁴⁷), 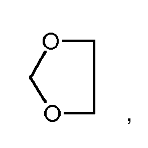 =NR⁴⁸ oder -(CO)ⱼNR⁴⁹R⁵⁰
substituiert ist,
worin
- R⁴⁶ und R⁴⁷: die oben angegebene Bedeutung von R¹⁰ und R¹¹ haben und mit dieser gleich oder verschieden sind,
- R⁴⁸: Hydroxy oder geradkettiges oder verzweigtes Alkoxy mit bis zu 4 Kohlenstoffatomen bedeutet,
- j: eine Zahl 0 oder 1 bedeutet,
und
- R⁴⁹ und R⁵⁰: gleich oder verschieden sind und die oben angegebene Bedeutung von R¹⁴ und R¹⁵ haben,
und/oder der unter R³ und R⁴ aufgeführte, gemeinsam mit dem Stickstoffatom gebildete Heterocyclus, gegebenenfalls durch geradkettiges oder verzweigtes Alkyl mit bis zu 6 Kohlenstoffatomen substituiert ist, das gegebenenfalls einbis mehrfach, gleich oder verschieden durch Hydroxy, Halogen, Carboxyl, Cycloalkyl oder Cycloalkyloxy mit jeweils 3 bis 8 Kohlenstoffatomen, geradkettiges oder verzweigtes Alkoxy oder Alkoxycarbonyl mit jeweils bis zu 6 Kohlenstoffatomen oder durch einen Rest der Formel -SO₃H, -NR⁵¹R⁵² oder P(O)OR⁵³OR⁵⁴ substituiert ist,
worin
- R⁵¹ und R⁵²: gleich oder verschieden sind und Wasserstoff, Phenyl, Carboxyl, Benzyl oder geradkettiges oder verzweigtes Alkyl oder Alkoxy mit jeweils bis zu 6 Kohlenstoffatomen bedeuten,
- R⁵³ und R⁵⁴: gleich oder verschieden sind und die oben angegebene Bedeutung von R¹⁰ und R¹¹ haben,
und/oder das Alkyl gegebenenfalls durch Aryl mit 6 bis 10 Kohlenstoffatomen substituiert ist, das seinerseits ein- bis mehrfach, gleich oder verschieden durch Halogen, Hydroxy, geradkettiges oder verzweigtes Alkoxy mit bis zu 6 Kohlenstoffatomen, oder durch eine Gruppe der Formel -NR^{51'}R^{52'} substituiert sein kann,
worin
- R^{51'} und R^{52'}: die oben angegebene Bedeutung von R⁵¹ und R⁵² haben und mit dieser gleich oder verschieden sind,
und/oder der unter R³ und R⁴ aufgeführte, gemeinsam mit dem Stickstoffatom gebildete Heterocyclus, gegebenenfalls durch Aryl mit 6 bis 10 Kohlenstoffatomen oder durch einen 5- bis 7-gliedrigen, gesättigen, partiell ungesättigten oder ungesättigten Heterocyclus mit bis zu 3 Heteratomen aus der Reihe S, N und/oder O , gegebenenfalls auch über eine N-Funktion verknüpft, substituiert ist, wobei die Ringsysteme ihrerseits durch Hydroxy oder durch geradkettiges oder verzweigtes Alkyl oder Alkoxy mit jeweils bis zu 6 Kohlenstoffatomen substituiert sein können,
oder
- R³ und R⁴: gemeinsam mit dem Stickstoffatom Reste der Formeln 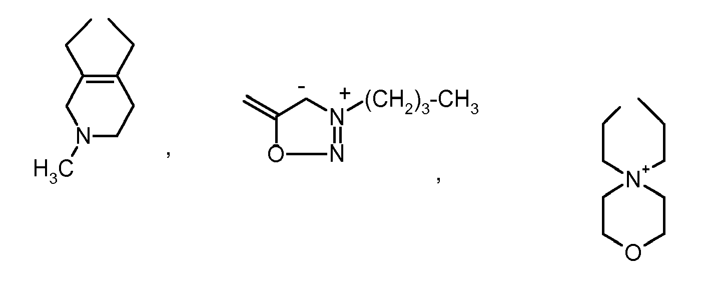 oder 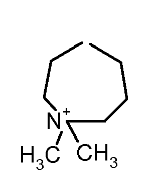 bilden,
- R⁵ und R⁶: gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen,
und deren Salze, Hydrate, N-Oxide und isomere Formen.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Heterocyclus, gegebenenfalls benzokondensiert, steht im Rahmen der Erfindung im allgemeinen für einen gesättigten, partiell ungesättigten oder ungesättigten 5- bis 7-gliedrigen Heterocyclus, der bis zu 4 Heteroatome aus der Reihe S, N und/oder O enthalten kann. Beispielsweise seien genannt: Azepin, Diazepin, Indolyl, Isochinolyl, Chinolyl, Benzo[b]thiophen, Benzo[b]furanyl, Pyridyl, Thienyl, Tetrahydrofuranyl, Tetrahydropyranyl, Furyl, Pyrrolyl, Thiazolyl, Triazolyl, Tetrazolyl, Isoxazolyl, Imidazolyl, Morpholinyl, Thiomorpholinyl, Pyrrolidinyl, Piperazinyl, N-Methylpiperazinyl oder Piperidinyl. Bevorzugt sind Chinolyl, Furyl, Pyridyl, Thienyl, Piperidinyl, Pyrrolidinyl, Piperazinyl, Azepin, Diazepin, Thiazolyl, Triazolyl, Tetrazolyl, Tetrahydrofuranyl, Tetrahydropyranyl, Morpholinyl und Thiomorpholinyl.

Ein geradkettiger oder verzweigter Acylrest mit 1 bis 6 Kohlenstoffatomen steht im Rahmen der Erfindung beispielsweise für Acetyl, Ethylcarbonyl, Propylcarbonyl, Isopropylcarbonyl, Butylcarbonyl, Isobutylcarbonyl, Pentylcarbonyl und Hexylcarbonyl. Bevorzugt ist ein geradkettiger oder verzweigter Acylrest mit 1 bis 4 Kohlenstoffatomen. Besonders bevorzugt sind Acetyl und Ethylcarbonyl.

Ein geradkettiger oder verzweigterAlkoxyrest mit 1 bis 6 bzw. 1 bis 4 Kohlenstoffatomen steht im Rahmen der Erfindung für Methoxy, Ethoxy, n-Propoxy, Isopropoxy, tert.Butoxy, n-Pentoxy und n-Hexoxy. Bevorzugt ist ein geradkettiger oder verzweigter Alkoxyrest mit 1 bis 6, 1 bis 4 bzw. 1 bis 3 Kohlenstoffatomen. Besonders bevorzugt ist ein geradkettiger oder verzweigter Alkoxyrest mit 1 bis 3 Kohlenstoffatomen.

Ein geradkettiger oder verzweigter Alkoxycarbonylrest mit 1 bis 6 Kohlenstoffatomen steht im Rahmen der Erfindung beispielsweise für Methoxycarbonyl, Ethoxycarbonyl, n-Propoxycarbonyl, Isopropoxycarbonyl und tert.Butoxycarbonyl. Bevorzugt ist ein geradkettiger oder verzweigter Alkoxycarbonylrest mit 1 bis 4 Kohlenstoffatomen. Besonders bevorzugt ist ein geradkettiger oder verzweigter Alkoxycarbonylrest mit 1 bis 3 Kohlenstoffatomen.

Ein geradkettiger oder verzweigter Alkylrest mit 1 bis 4, 1 bis 6, 1 bis 8 und 1 - 10 Kohlenstoffatomen steht im Rahmen der Erfindung beispielsweise für Methyl, Ethyl, n-Propyl, Isopropyl, tert.Butyl, n-Pentyl, n-Hexyl, n-Heptyl, n-Octyl, n-Nonyl und n-Decyl. Bevorzugt sind geradkettige oder verzweigte Alkylreste mit 1 bis 3, 1 bis 4 bzw. 1 bis 8 Kohlenstoffatomen. Besonders bevorzugt sind geradkettige oder verzweigte Alkylreste mit 1 bis 4 bzw. 1 bis 3 Kohlenstoffatomen.

Geradkettiges Alkyl mit bis zu 4 Kohlenstoffatomen steht im Rahmen der Erfindung beispielsweise für Methyl, Ethyl, n-Propyl und n-Butyl.

(C₆-C₁₀-Aryl steht im allgemeinen für einen aromatischen Rest mit 6 bis 10 Kohlenstoffatomen. Bevorzugte Arylreste sind Phenyl und Naphthyl.

Cycloalkyl mit 3 bis 8 bzw. 3 bis 7 Kohlenstoffatomen steht im Rahmen der Erfindung beispielsweise für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl. Cycloalkyloxy mit 3 bis 8 Kohlenstoffatomen steht im Rahmen der Erfindung für Cyclopropyloxy, Cyclopentyloxy, Cyclobutyloxy, Cyclohexyloxy, Cycloheptyloxy oder Cyclooctyloxy. Bevorzugt seien genannt: Cyclopropyloxy, Cyclopentyloxy und Cyclohexyloxy.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Ein 5- bis 6-gliedriger bzw. 7-gliedriger gesättigter Heterocyclus, der ein weiteres Heteroatom aus der Reihe S, N und/oder O enthalten kann steht im Rahmen der Erfindung und in Abhängigkeit der oben aufgeführten Substituenten beispielsweise für Morpholinyl, Piperidinyl, Piperazinyl, Tetrahydropyranyl oder Tetrahydrofuranyl. Bevorzugt sind Morpholinyl, Tetrahydropyranyl, Piperidinyl und Piperazinyl.

Ein 5- bis 6-gliedriger aromatischer Heterocyclus mit bis zu 3 oder 4 Heteroatomen aus der Reihe S, O und/oder N steht im Rahmen der Erfindung beispielsweise für Pyridyl, Pyrimidyl, Pyridazinyl, Thienyl, Furyl, Pyrrolyl, Thiazolyl, Oxazolyl oder Imidazolyl. Bevorzugt sind Pyridyl, Pyrimidyl, Pyridazinyl, Furyl und Thiazolyl.

Ein 5- bis 6-gliedriger ungesättigter, partiell ungesättigter und gesättigter Heterocyclus, der bis zu 3 bzw. 4 Heteroatome aus der Reihe S, O und/oder N enthalten kann, steht im Rahmen der Erfindung beispielsweise für Pyridyl, Pyrimidyl, Pyridazinyl, Thienyl, Furyl, Pyrrolyl, Thiazolyl, Oxazolyl, Imidazolyl, Piperidinyl, Piperazinyl oder Morpholinyl. Bevorzugt sind Pyridyl, Pyrimidyl, Piperazinyl, Pyridazinyl, Morpholinyl, Furyl und Thiazolyl.

Die erfindungsgemäßen Verbindungen, insbesondere die Salze, können auch als Hydrate vorliegen. Im Rahmen der Erfindung werden unter Hydraten solche Verbindungen verstanden, die im Kristall Wasser enthalten. Solche Verbindungen können ein oder mehrere, typischerweise 1 bis 5, Äquivalente Wasser enthalten. Hydrate lassen sich beispielsweise herstellen, indem man die betreffende Verbindung aus Wasser oder einem wasserhaltigen Lösungsmittel kristallisiert.

Bevorzugt sind erfindungsgemäße Verbindungen der allgemeinen Formel (I),
in welcher
- R¹: für geradkettiges oder verzweigtes Alkyl mit bis zu 3 Kohlenstoffatomen steht,
- R²: für geradkettiges Alkyl mit bis zu 3 Kohlenstoffatomen steht,
- R³ und R⁴: gleich oder verschieden sind und für Wasserstoff oder für geradkettiges oder verzweigtes Alkenyl oder Alkoxy mit jeweils bis zu 6 Kohlenstoffatomen stehen, oder
für eine geradkettige oder verzweigte Alkylkette mit bis zu 8 Kohlenstoffatomen stehen, die gegebenenfalls durch ein Sauerstoffatom unterbrochen ist, und die gegebenenfalls ein- bis dreifach, gleich oder verschieden durch Hydroxy, Fluor, Chlor, Carboxyl, Benzyloxycarbonyl, geradkettiges oder verzweigtes Alkoxycarbonyl mit bis zu 5 Kohlenstoffatomen und/oder durch Reste der Formeln -SO₃H, -(A)ₐ-NR⁷R⁸, -O-CO-NR^{7'}R^{8'}, -S(O)_{b}-R⁹, -P(O)(OR¹⁰)(OR¹¹), 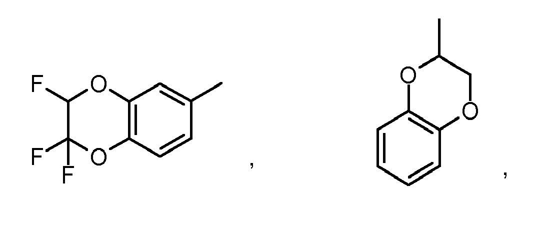 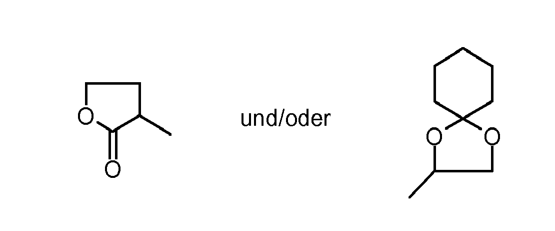 substituiert ist,
worin
- a und b: gleich oder verschieden sind und eine Zahl 0 oder 1 bedeuten,
- A: einen Rest CO oder SO₂ bedeutet,
- R⁷, R^{7'}, R⁸ und R^{8'}: gleich oder verschieden sind und Wasserstoff bedeuten, oder
Cyclopropyl, Cyclopentyl, Cyclohexyl, Cycloheptyl, Phenyl, Piperidinyl und Pyridyl bedeuten, wobei die oben aufgeführten Ringsysteme gegebenenfalls ein- bis dreifach, gleich oder verschieden durch Hydroxy, Nitro, Trifluormethyl, Trifluormethoxy, Carboxyl, Fluor, Chlor, geradkettiges oder verzweigtes Alkoxy oder Alkoxycarbonyl mit jeweils bis zu 4 Kohlenstoffatomen oder durch eine Gruppe der Formel -(SO₂)_{c}-NR¹²R¹³ substituiert sind,
worin
c eine Zahl 0 oder 1 bedeutet,
R¹² und R¹³ gleich oder verschieden sind und Wasserstoff oder geradkettiges oder verzweigtes Alkyl mit bis zu 4 Kohlenstoffatomen bedeuten, oder
- R⁷, R^{7'}, R⁸ und R^{8'}: geradkettiges oder verzweigtes Alkoxy mit bis zu 3 Kohlenstoffatomen bedeuten, oder geradkettiges oder verzweigtes Alkyl mit bis zu 7 Kohlenstoffatomen bedeuten, das gegebenenfalls ein- oder mehrfach, gleich oder verschieden durch Hydroxy, Fluor, Chlor, Phenyl, geradkettiges oder verzweigtes Alkoxy oder Alkoxycarbonyl mit jeweils bis zu 4 Kohlenstoffatomen oder durch eine Gruppe der Formel -(CO)_{d}-NR¹⁴R¹⁵ substituiert ist,
worin
R¹⁴ und R¹⁵ gleich oder verschieden sind und Wasserstoff oder geradkettiges oder verzweigtes Alkyl mit bis zu 3 Kohlenstoffatomen bedeuten,
und
d eine Zahl 0 oder 1 bedeutet, oder
- R⁷ und R⁸ und/oder R^{7'} und R^{8'}: gemeinsam mit dem Stickstoffatom einen Pyrrolidinyl-, Morpholinyl-, Piperidinyl- oder Triazolylring oder Reste der Formeln 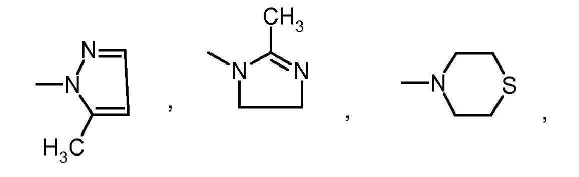 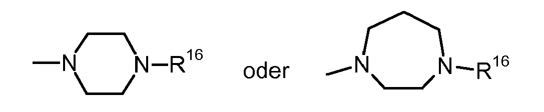 bilden,
worin
R¹⁶ Wasserstoff, Phenyl, Benzyl, Morpholinyl, Pyrrolidinyl, Piperidinyl, Piperazinyl oder N-Methylpiperazinyl bedeutet, oder geradkettiges oder verzweigtes Alkyl mit bis zu 5 Kohlenstoffatomen bedeutet, das gegebenenfalls durch Hydroxy substituiert ist,
- R⁹: geradkettiges oder verzweigtes Alkyl mit bis zu 3 Kohlenstoffatomen bedeutet,
- R¹⁰ und R¹¹: gleich oder verschieden sind und Wasserstoff oder geradkettiges oder verzweigtes Alkyl mit bis zu 3 Kohlenstoffatomen bedeuten, und/oder die unter R³/R⁴ aufgeführte Alkylkette gegebenenfalls durch Cyclopropyl, Cyclopentyl, Cyclohexyl, Cycloheptyl, Phenyl, Pyridyl, Chinolyl, Pyrrolidinyl, Pyrimidyl, Morpholinyl, Furyl, Piperidinyl, Tetrahydrofuranyl oder durch Reste der Formeln 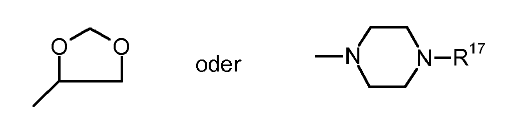 substituiert ist,
worin
R¹⁷ Wasserstoff, Hydroxy, Formyl, Trifluormethyl, geradkettiges oder verzweigtes Acyl oder Alkoxy mit jeweils bis zu 3 Kohlenstoffatomen bedeutet, oder geradkettiges oder verzweigtes Alkyl mit bis zu 4 Kohlenstoffatomen bedeutet, das gegebenenfalls ein- bis dreifach gleich oder verschieden durch Hydroxy, oder geradkettiges oder verzweigtes Alkoxy mit bis zu 4 Kohlenstoffatomen substituiert ist,
und wobei Phenyl und die Heterocyclen gegebenenfalls ein- bis dreifach, gleich oder verschieden durch Nitro, Fluor, Chlor, -SO₃H, geradkettiges oder verzweigtes Alkyl oder Alkoxy mit jeweils bis zu 4 Kohlenstoffatomen, Hydroxy und/oder durch einen Rest der Formel -SO₂-NR¹⁸R¹⁹ substituiert sind,
worin
R¹⁸ und R¹⁹ gleich oder verschieden sind und Wasserstoff oder geradkettiges oder verzweigtes Alkyl mit bis zu 4 Kohlenstoffatomen bedeuten,
und/oder
- R³ oder R⁴: für eine Gruppe der Formel -NR²⁰R²¹ steht,
worin
R²⁰ und R²¹ die oben angegebene Bedeutung von R¹⁸ und R¹⁹ haben und mit dieser gleich oder verschieden sind, und/oder
- R³ oder R⁴: für Adamantyl stehen, oder
für Reste der Formeln 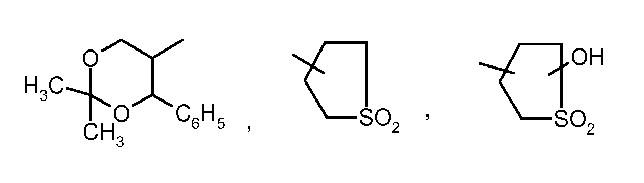 oder 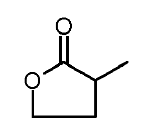 stehen,
oder für Cyclopentyl, Cyclohexyl, Cycloheptyl, Phenyl, Morpholinyl, Oxazolyl, Thiazolyl, Chinolyl, Isoxazolyl, Pyridyl, Tetrahydrofuranyl, Tetrahydropyranyl oder für Reste der Formeln 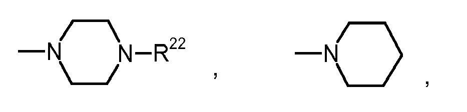 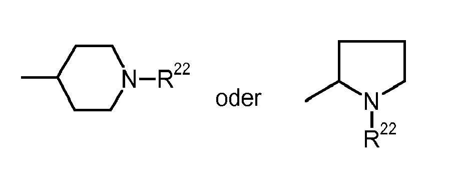 stehen,
worin
R²² die oben angegbene Bedeutung von R¹⁶ hat und mit dieser gleich oder verschieden ist, oder Carboxyl, Formyl oder geradkettiges oder verzweigtes Acyl mit bis zu 3 Kohlenstoffatomen bedeutet,
und wobei Cycloalkyl, Phenyl und/oder die Heterocyclen gegebenenfalls einbis dreifach, gleich oder verschieden durch Fluor, Chlor, Triazolyl, Trifluormethyl, Trifluormethoxy, Carboxyl, geradkettiges oder verzweigtes Acyl oder Alkoxycarbonyl mit jeweils bis zu 5 Kohlenstoffatomen, Nitro und/oder durch Gruppen der Formeln -SO₃H, -OR²³, (SO₂)ₑNR²⁴R²⁵, -P(O)(OR²⁶)(OR²⁷) substituiert sind,
worin
e eine Zahl 0 oder 1 bedeutet,
R²³ einen Rest der Formel 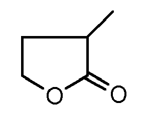 bedeutet, oder Cyclopropyl, Cyclopentyl, Cyclobutyl, Cyclohexyl oder Cycloheptyl bedeutet, Wasserstoff oder geradkettiges oder verzweigtes Alkyl mit bis zu 4 Kohlenstoffatomen bedeutet, das gegebenenfalls durch Cyclopropyl, Cyclopentyl, Cyclohexyl, Benzyloxy, Tetrahydropyranyl, Tetrahydrofuranyl, geradkettiges oder verzweigtes Alkoxy oder Alkoxycarbonyl mit jeweils bis zu 4 Kohlenstoffatomen, Benzyloxycarbonyl oder Phenyl substituiert ist, das seinerseits ein- bis mehrfach, gleich oder verschieden durch geradkettiges oder verzweigtes Alkoxy mit bis zu 3 Kohlenstoffatomen, Hydroxy, Fluor oder Chlor substituiert sein kann,
und/oder Alkyl gegebenenfalls durch Reste der Formeln -CO-NR²⁸R²⁹ oder -CO-R³⁰ substituiert ist,
worin
R²⁸ und R²⁹ gleich oder verschieden sind und Wasserstoff oder geradkettiges oder verzweigtes Alkyl mit bis zu 5 Kohlenstoffatomen bedeuten, oder
R²⁸ und R²⁹ gemeinsam mit dem Stickstoffatom einen Morpholinyl-, Pyrrolidinyl- oder Piperidinylring bilden,
und
R³⁰ Phenyl oder Adamantyl bedeutet,
- R²⁴ und R²⁵: die oben angegebene Bedeutung von R¹⁸ und R¹⁹ haben und mit dieser gleich oder verschieden sind,
- R²⁶ und R²⁷: die oben angegebene Bedeutung von R¹⁰ und R¹¹ haben und mit dieser gleich oder verschieden sind
und/oder Cycloalkyl, Phenyl und/oder die Heterocyclen gegebenenfalls durch geradkettiges oder verzweigtes Alkyl mit bis zu 4 Kohlenstoffatomen substituiert sind, das gegebenenfalls durch Hydroxy, Carboxyl, Pyridyl, Pyrimidyl, Pyrrolidinyl, Piperidinyl, Tetrahydrofuranyl, Triazolyl oder durch Gruppen der Formel -SO₂-R³¹, P(O)(OR³²)(OR³³) oder -NR³⁴R³⁵ substituiert ist,
worin
- R³¹: die oben angegebene Bedeutung von R⁹ hat und mit dieser gleich oder verschieden ist,
- R³² und R³³: die oben angegebene Bedeutung von R¹⁰ und R¹¹ haben und mit dieser gleich oder verschieden sind,
- R³⁴ und R³⁵: gleich oder verschieden sind und Wasserstoff oder geradkettiges oder verzweigtes Alkyl mit bis zu 5 Kohlenstoffatomen bedeuten, das gegebenenfalls durch Hydroxy oder geradkettiges oder verzweigtes Alkoxy mit bis zu 3 Kohlenstoffatomen substituiert ist, oder
- R³⁴ und R³⁵: gemeinsam mit dem Stickstoffatom einen Morpholinyl-, Triazolyl-oder Thiomorpholinylring oder einen Rest der Formel 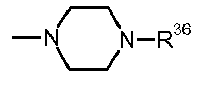 bilden,
worin
R³⁶ Wasserstoff, Hydroxy, geradkettiges oder verzweigtes Alkoxycarbonyl mit bis zu 5 Kohlenstoffatomen oder geradkettiges oder verzweigtes Alkyl mit bis zu 4 Kohlenstoffatomen bedeutet, das gegebenenfalls durch Hydroxy substituiert ist,
oder
- R³ und R⁴: gemeinsam mit dem Stickstoffatom einen Morpholinyl-, Thiomorpholinyl-, Pyrrolidinyl-, Piperidinylring oder einen einen Rest der Formel 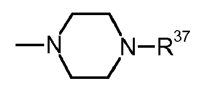 bilden,
worin
R³⁷ Wasserstoff, Hydroxy, Formyl, Trifluormethyl, geradkettiges oder verzweigtes Acyl, Alkoxy oder Alkoxycarbonyl mit jeweils bis zu 4 Kohlenstoffatomen bedeutet,
oder geradkettiges oder verzweigtes Alkyl mit bis zu 5 Kohlenstoffatomen bedeutet, das gegebenenfalls ein- bis dreifach, gleich oder verschieden durch Hydroxy, Trifluormethyl, Carboxyl, geradkettiges oder verzweigtes Alkoxy oder Alkoxycarbonyl mit jeweils bis zu 4 Kohlen- stoffatomen oder durch Gruppen der Formel -(D)_{f-}NR³⁸R³⁹, -CO-(CH₂)_{g}-O-CO-R⁴⁰, -CO-(CH₂)ₕ-OR⁴¹ oder -P(O)(OR⁴²)(OR⁴³) substituiert ist,
worin
g und h gleich oder verschieden sind und eine Zahl 1, 2 oder 3 bedeuten,
und
f eine Zahl 0 oder 1 bedeutet,
D eine Gruppe der Formel -CO oder -SO₂ bedeutet,
R³⁸ und R³⁹ gleich oder verschieden sind und die oben angegebene Bedeutung von R⁷ und R⁸ haben,
R⁴⁰ geradkettiges oder verzweigtes Alkyl mit bis zu 4 Kohlenstoffatomen bedeutet,
R⁴¹ geradkettiges oder verzweigtes Alkyl mit bis zu 4 Kohlenstoffatomen bedeutet,
R⁴² und R⁴³ gleich oder verschieden sind und Wasserstoff oder geradkettiges oder verzweigtes Alkyl mit bis zu 3 Kohlenstoffatomen bedeuten,
oder
- R³⁷: einen Rest der Formel -(CO)ᵢ-E bedeutet,
worin
i eine Zahl 0 oder 1 bedeutet,
E Cyclopentyl, Cyclohexyl, Cycloheptyl, Benzyl, Phenyl, Pyridyl, Pyrimidyl oder Furyl bedeutet, wobei die oben aufgeführten Ringsysteme gegebenenfalls ein- bis zweifach, gleich oder verschieden durch Nitro, Fluor, Chlor, -SO₃H, geradkettiges oder verzweigtes Alkoxy mit bis zu 4 Kohlenstoffatomen, Hydroxy, Trifluormethyl, Trifluormethoxy oder durch einen Rest der Formel -SO²-NR⁴⁴R⁴⁵, substituiert sind,
worin
R⁴⁴ und R⁴⁵ die oben angegebene Bedeutung von R¹⁸ und R¹⁹ haben und mit dieser gleich oder verschieden sind, oder
E Reste der Formeln 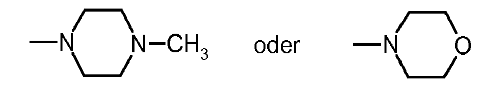 bedeutet, und die unter R³ und R⁴ aufgeführten, gemeinsam mit dem Stickstoffatom gebildeten Heterocyclen, gegebenenfalls ein- bis dreifach, gleich oder verschieden, gegebenenfalls auch geminal, durch Hydroxy, Formyl, Carboxyl, geradkettiges oder verzweigtes Acyl oder Alkoxycarbonyl mit bis jeweils zu 5 Kohlenstoffatomen, Nitro und Gruppen der Formeln -P(O)(OR⁴⁶)(OR⁴⁷), 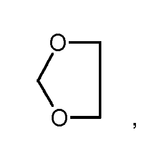 =NR⁴⁸ oder -(CO)ⱼNR⁴⁹R⁵⁰
substituiert sind,
worin
- R⁴⁶ und R⁴⁷: die oben angegebene Bedeutung von R¹⁰ und R¹¹ haben und mit dieser gleich oder verschieden sind,
- R⁴⁸: Hydroxy oder geradkettiges oder verzweigtes Alkoxy mit bis zu 3 Kohlenstoffatomen bedeutet,
- j: eine Zahl 0 oder 1 bedeutet,
und
- R⁴⁹ und R⁵⁰: gleich oder verschieden sind und die oben angegebene Bedeutung von R¹⁴ und R¹⁵ haben,
und/oder die unter R³ und R⁴ aufgeführten gemeinsam mit dem Stickstoffatom gebildeten Heterocyclen gegebenenfalls durch geradkettiges oder verzweigtes Alkyl mit bis zu 5 Kohlenstoffatomen substituiert sind, das gegebenenfalls einbis mehrfach, gleich oder verschieden durch Hydroxy, Fluor, Chlor, Carboxyl, Cyclopropyl, Cyclopentyl, Cyclohexyl, Cycloheptyl, geradkettiges oder verzweigtes Alkoxy oder Alkoxycarbonyl mit jeweils bis zu 4 Kohlenstoffatomen oder durch einen Rest der Formel -SO₃H, -NR⁵¹R⁵² oder P(O)OR⁵³OR⁵⁴ substituiert ist,
worin
- R⁵¹ und R⁵²: gleich oder verschieden sind und Wasserstoff, Phenyl, Carboxyl, Benzyl oder geradkettiges oder verzweigtes Alkyl oder Alkoxy mit jeweils bis zu 4 Kohlenstoffatomen bedeuten,
- R⁵³ und R⁵⁴: gleich oder verschieden sind und die oben angegebene Bedeutung von R¹⁰ und R¹¹ haben,
und/oder das Alkyl gegebenenfalls durch Phenyl substituiert ist, das seinerseits ein- bis dreifach, gleich oder verschieden durch Fluor, Chlor, Hydroxy, geradkettiges oder verzweigtes Alkoxy mit bis zu 4 Kohlenstoffatomen, oder durch eine Gruppe der Formel -NR^{51'}R^{52'} substituiert sein kann,
worin
- R^{51'} und R^{52'}: die oben angegebene Bedeutung von R⁵¹ und R⁵² haben und mit dieser gleich oder verschieden sind,
und/oder die unter R³ und R⁴ aufgeführten, gemeinsam mit dem Stickstoffatom gebildeten Heterocyclen, gegebenenfalls durch Phenyl, Pyridyl, Piperidinyl, Pyrrolidinyl oder Terazolyl, gegebenenfalls auch über eine N-Funktion verknüpft, substituiert sind, wobei die Ringsysteme ihrerseits durch Hydroxy oder durch geradkettiges oder verzweigtes Alkyl oder Alkoxy mit jeweils bis zu 5 Kohlenstoffatomen substituiert sein können,
oder
- R³ und R⁴: gemeinsam mit dem Stickstoffatom Reste der Formeln 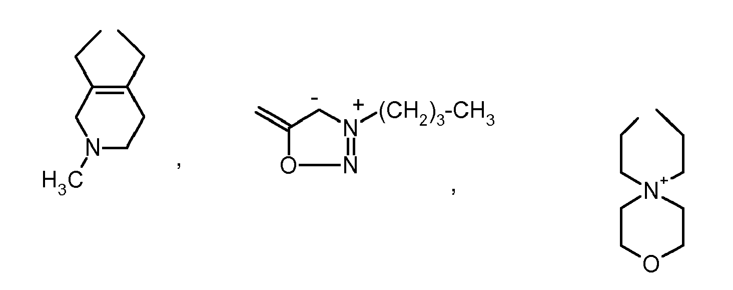 oder 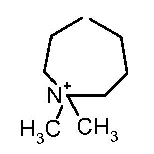 bilden,
- R⁵ und R⁶: gleich oder verschieden sind und für Wasserstoff, Hydroxy oder für geradkettiges oder verzweigtes Alkoxy mit bis zu 4 Kohlenstoffatomen stehen, und deren Salze, N-Oxide, Hydrate und isomere Formen. Besonders bevorzugt sind erfindungsgemäße Verbindungen der allgemeinen Formel (I),
in welcher
- R¹: für geradkettiges oder verzweigtes Alkyl mit bis zu 3 Kohlenstoffatomen steht,
- R²: für geradkettiges Alkyl mit bis zu 3 Kohlenstoffatomen steht,
- R³ und R⁴: gleich oder verschieden sind und für Wasserstoff oder für geradkettiges oder verzweigtes Alkenyl oder Alkoxy mit jeweils bis zu 4 Kohlenstoffatomen stehen, oder
für eine geradkettige oder verzweigte Alkylkette mit bis zu 6 Kohlenstoffatomen stehen, die gegebenenfalls durch ein Sauerstoffatom unterbrochen ist, und die gegebenenfalls ein- bis dreifach, gleich oder verschieden durch Hydroxy, Fluor, Chlor, Carboxyl, geradkettiges oder verzweigtes Alkoxycarbonyl mit bis zu 4 Kohlenstoffatomen und/oder durch Reste der Formeln -SO₃H, -(A)ₐ-NR⁷R⁸, -O-CO-NR^{7'}R^{8'}, -S(O)_{b}-R⁹, -P(O)(OR¹⁰)(OR¹¹), 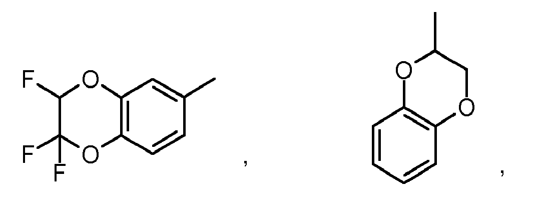 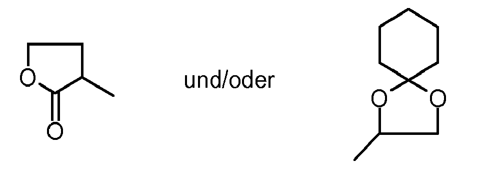
substituiert ist,
worin
a und b gleich oder verschieden sind und eine Zahl 0 oder 1 bedeuten,
- A: einen Rest CO oder SO₂ bedeutet,
- R⁷, R^{7'}, R⁸ und R^{8'}: gleich oder verschieden sind und Wasserstoff bedeuten, oder
Cyclopentyl, Cyclohexyl, Cycloheptyl, Phenyl, Piperidinyl und Pyridyl bedeuten, wobei die oben aufgeführten Ringsysteme gegebenenfalls ein- bis zweifach, gleich oder verschieden durch Hydroxy, Nitro, Carboxyl, Fluor, Chlor, geradkettiges oder verzweigtes Alkoxy oder Alkoxycarbonyl mit jeweils bis zu 3 Kohlenstoffatomen oder durch eine Gruppe der Formel -(SO₂)_{c}-NR¹²R¹³ substituiert sind,
worin
c eine Zahl 0 oder 1 bedeutet,
R¹² und R¹³ gleich oder verschieden sind und Wasserstoff oder geradkettiges oder verzweigtes Alkyl mit bis zu 3 Kohlenstoffatomen bedeuten,
oder
- R⁷ R^{7'}, R⁸ und R^{8'}: Methoxy bedeuten, oder geradkettiges oder verzweigtes Alkyl mit bis zu 6 Kohlenstoffatomen bedeuten, das gegebenenfalls ein- oder zweifach, gleich oder verschieden durch Hydroxy, Fluor, Chlor, Phenyl, geradkettiges oder verzweigtes Alkoxy oder Alkoxycarbonyl mit jeweils bis zu 3 Kohlenstoffatomen oder durch eine Gruppe der Formel -(CO)_{d}-NR¹⁴R¹⁵ substitutiert ist,
worin
R¹⁴ und R¹⁵ gleich oder verschieden sind und Wasserstoff, Methyl oder Ethyl bedeuten,
und
d eine Zahl 0 oder 1 bedeutet,
oder
- R⁷ und R⁸ und/oder R^{7'} und R^{8'}: gemeinsam mit dem Stickstoffatom einen Morpholinyl-, Piperidinyl- oder Triazolylring oder Reste der Formeln 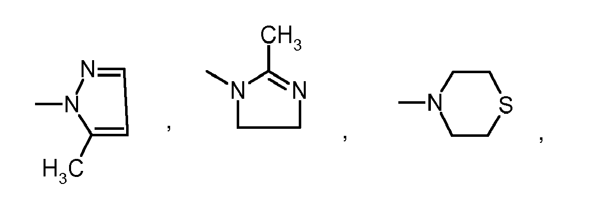 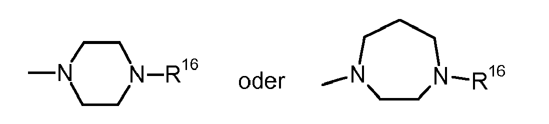 bilden,
worin
R¹⁶ Wasserstoff, Phenyl, Benzyl, Morpholinyl, Pyrrolidinyl, Piperidinyl, Piperazinyl oder N-Methylpiperazinyl bedeutet, oder geradkettiges oder verzweigtes Alkyl mit bis zu 3 Kohlenstoffatomen bedeutet, das gegebenenfalls durch Hydroxy substituiert ist,
R⁹ Methyl bedeutet,
R¹⁰ und R¹¹ gleich oder verschieden sind und Wasserstoff, Methyl oder Ethyl bedeuten,
und/oder die unter R³/R⁴ aufgeführte Alkylkette gegebenenfalls durch Cyclopropyl, Cyclopentyl, Cyclohexyl, Cycloheptyl, Morpholinyl, Furyl, Tetrahydrofuranyl oder durch Reste der Formeln 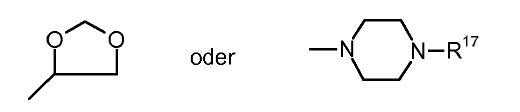 substituiert ist,
worin
- R¹⁷: Wasserstoff, Hydroxy, Formyl, Acetyl oder Alkoxy mit bis zu 3 Kohlenstoffatomen bedeutet, oder geradkettiges oder verzweigtes Alkyl mit bis zu 3 Kohlenstoffatomen bedeutet, das gegebenenfalls ein- bis zweifach gleich oder verschieden durch Hydroxy oder geradkettiges oder verzweigtes Alkoxy mit bis zu 3 Kohlenstoffatomen substituiert ist, und wobei Phenyl und die Heterocyclen gegebenenfalls ein- bis dreifach, gleich oder verschieden durch Fluor, Chlor, -SO₃H, geradkettiges oder verzweigtes Alkyl oder Alkoxy mit jeweils bis zu 3 Kohlenstoffatomen, Hydroxy und/oder durch einen Rest der Formel -SO₂-NR¹⁸R¹⁹ substituiert sind,
worin
- R¹⁸ und R¹⁹: gleich oder verschieden sind und Wasserstoff oder geradkettiges oder verzweigtes Alkyl mit bis zu 3 Kohlenstoffatomen bedeuten,
und/oder
R³ oder R⁴ für eine Gruppe der Formel -NR²⁰R²¹ steht,
worin
- R²⁰ und R²¹: die oben angegebene Bedeutung von R¹⁸ und R¹⁹ haben und mit dieser gleich oder verschieden sind,
und/oder
R³ oder R⁴ für Adamantyl stehen, oder
für Reste der Formeln 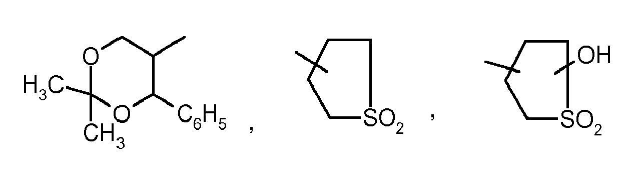 oder 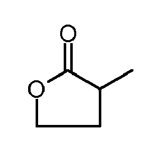 stehen,
oder für Cyclopentyl, Cyclohexyl, Cycloheptyl, Phenyl, Morpholinyl, Oxazolyl, Thiazolyl, Chinolyl, Isoxazolyl, Pyridyl, Tetrahydrofuranyl, Tetrahydropyranyl oder für Reste der Formeln 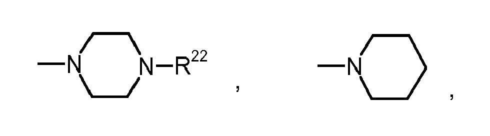 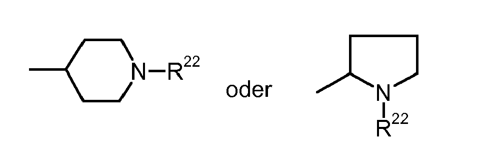 stehen,
worin
- R²²: die oben angegbene Bedeutung von R¹⁶ hat und mit dieser gleich oder verschieden ist, oder Formyl oder Acetyl bedeutet,
und wobei Cycloalkyl, Phenyl und/oder die Heterocyclen gegebenenfalls einbis zweifach, gleich oder verschieden durch Fluor, Chlor, Triazolyl, Carboxyl, geradkettiges oder verzweigtes Acyl oder Alkoxycarbonyl mit jeweils bis zu 4 Kohlenstoffatomen, Nitro und/oder durch Gruppen der Formeln -SO₃H, -OR²³, (SO₂)ₑNR²⁴R²⁵, -P(O)(OR²⁶)(OR²⁷) substituiert sind,
worin
e eine Zahl 0 oder 1 bedeutet,
R²³ einen Rest der Formel 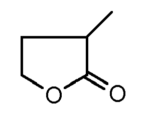 bedeutet, oder
Cyclopropyl, Cyclopentyl, Cyclobutyl oder Cyclohexyl bedeutet, Wasserstoff oder geradkettiges oder verzweigtes Alkyl mit bis zu 3 Kohlenstoffatomen bedeutet, das gegebenenfalls durch Cyclopropyl, Cyclohexyl, Benzyloxy, Tetrahydropyranyl, geradkettiges oder verzweigtes Alkoxy oder Alkoxycarbonyl mit jeweils bis zu 3 Kohlenstoffatomen, Benzyloxycarbonyl oder Phenyl substituiert ist, das seinerseits ein- bis zweifach, gleich oder verschieden durch Methoxy, Hydroxy, Fluor oder Chlor substituiert sein kann,
und/oder Alkyl gegebenenfalls durch Reste der Formeln -CO-NR²⁸R²⁹ oder -CO-R³⁰ substituiert ist,
worin
R²⁸ und R²⁹ gleich oder verschieden sind und Wasserstoff oder geradkettiges oder verzweigtes Alkyl mit bis zu 4 Kohlenstoffatomen bedeuten, oder
R²⁸ und R²⁹ gemeinsam mit dem Stickstoffatom einen Morpholinyl-, Pyrrolidinyl- oder Piperidinylring bilden,
und
R³⁰ Phenyl oder Adamantyl bedeutet,
R²⁴ und R²⁵ die oben angegebene Bedeutung von R¹⁸ und R¹⁹ haben und mit dieser gleich oder verschieden sind,
R²⁶ und R²⁷ die oben angegebene Bedeutung von R¹⁰ und R¹¹ haben und mit dieser gleich oder verschieden sind und/oder Cycloalkyl, Phenyl und/oder die Heterocyclen gegebenenfalls durch geradkettiges oder verzweigtes Alkyl mit bis zu 3 Kohlenstoffatomen substituiert sind, das gegebenenfalls durch Hydroxy, Carboxyl, Pyridyl, Pyrimidyl, Pyrrolidinyl, Piperidinyl, Tetrahydrofuranyl, Triazolyl oder durch Gruppen der Formel -SO₂-R³¹, P(O)(OR³²)(OR³³) oder -NR³⁴R³⁵ substituiert ist,
worin
R³¹ Methyl bedeutet,
R³² und R³³ die oben angegebene Bedeutung von R¹⁰ und R¹¹ haben und mit dieser gleich oder verschieden sind,
R³⁴ und R³⁵ gleich oder verschieden sind und Wasserstoff oder geradkettiges oder verzweigtes Alkyl mit bis zu 3 Kohlenstoffatomen bedeuten, das gegebenenfalls durch Hydroxy oder Methoxy substituiert ist, oder
R³⁴ und R³⁵ gemeinsam mit dem Stickstoffatom einen Morpholinyl-, Triazolyloder Thiomorpholinylring oder einen Rest der Formel 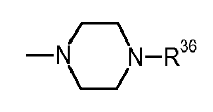 bilden,
worin
R³⁶ Wasserstoff, Hydroxy, geradkettiges oder verzweigtes Alkoxycarbonyl mit bis zu 3 Kohlenstoffatomen oder geradkettiges oder verzweigtes Alkyl mit bis zu 3 Kohlenstoffatomen bedeutet, das gegebenenfalls durch Hydroxy substituiert ist, oder
- R³ und R⁴: gemeinsam mit dem Stickstoffatom einen Morpholinyl-, Thiomorpholinyl-, Pyrrolidinyl-, Piperidinylring oder einen einen Rest der Formel 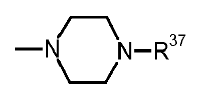 bilden,
worin
R³⁷ Wasserstoff, Hydroxy, Formyl, geradkettiges oder verzweigtes Acyl, Alkoxy oder Alkoxycarbonyl mit jeweils bis zu 3 Kohlenstoffatomen bedeutet, oder geradkettiges oder verzweigtes Alkyl mit bis zu 4 Kohlenstoffatomen bedeutet, das gegebenenfalls ein- bis zweifach, gleich oder verschieden durch Hydroxy, geradkettiges oder verzweigtes Alkoxy oder Alkoxycarbonyl mit jeweils bis zu 3 Kohlenstoffatomen oder durch Gruppen der Formel -(D)_{f-}NR³⁸R³⁹, -CO-(CH₂)_{g}-O-CO-R⁴⁰, -CO-(CH₂)ₕ-OR⁴¹ oder -P(O)(OR⁴²)(OR⁴³) substituiert ist,
worin
g und h gleich oder verschieden sind und eine Zahl 1 oder 2 bedeuten,
und
f eine Zahl 0 oder 1 bedeutet,
D eine Gruppe der Formel -CO oder -SO₂ bedeutet,
R³⁸ und R³⁹ gleich oder verschieden sind und die oben angegebene Bedeutung von R⁷ und R⁸ haben,
R⁴⁰ geradkettiges oder verzweigtes Alkyl mit bis zu 3 Kohlenstoffatomen bedeuten,
R⁴¹ geradkettiges oder verzweigtes Alkyl mit bis zu 3 Kohlenstoffatomen bedeutet,
R⁴² und R⁴³ gleich oder verschieden sind und Wasserstoff, Methyl oder Ethyl bedeuten, oder
- R³⁷: einen Rest der Formel -(CO)ᵢ-E bedeutet,
worin
i eine Zahl 0 oder 1 bedeutet,
E Cyclopentyl, Benzyl, Phenyl, Pyridyl, Pyrimidyl oder Furyl bedeutet, wobei die oben aufgeführten Ringsysteme gegebenenfalls ein- bis zweifach, gleich oder verschieden durch Nitro, Fluor, Chlor, -SO₃H, geradkettiges oder verzweigtes Alkoxy mit bis zu 3 Kohlenstoffatomen, Hydroxy oder durch einen Rest der Formel -SO₂-NR⁴⁴R⁴⁵, substituiert sind,
worin
R⁴⁴ und R⁴⁵ die oben angegebene Bedeutung von R¹⁸ und R¹⁹ haben und mit dieser gleich oder verschieden sind, oder
E Reste der Formeln 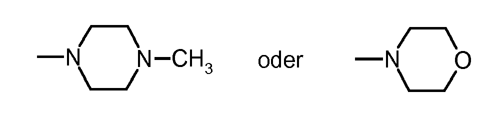 bedeutet, und die unter R³ und R⁴ aufgeführten, gemeinsam mit dem Stickstoffatom, gebildeten Heterocyclen, gegebenenfalls ein- bis dreifach, gleich oder verschieden, gegebenenfalls auch geminal, durch Hydroxy, Formyl, Carboxyl, geradkettiges oder verzweigtes Acyl oder Alkoxycarbonyl mit bis jeweils zu 3 Kohlenstoffatomen oder Gruppen der Formeln -P(O)(OR⁴⁶)(OR⁴⁷), 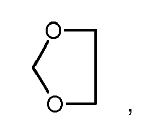 =NR⁴⁸ oder -(CO)ⱼNR⁴⁹R⁵⁰
substituiert sind,
worin
R⁴⁶ und R⁴⁷ die oben angegebene Bedeutung von R¹⁰ und R¹¹ haben und mit dieser gleich oder verschieden sind,
R⁴⁸ Hydroxy oder Methoxy bedeutet,
- j: eine Zahl 0 oder 1 bedeutet,
und
- R⁴⁹ und R⁵⁰: gleich oder verschieden sind und die oben angegebene Bedeutung von R¹⁴ und R¹⁵ haben,
und/oder die unter R³ und R⁴ aufgeführten, gemeinsam mit dem Stickstoffatom gebildeten Heterocyclen gegebenenfalls durch geradkettiges oder verzweigtes Alkyl mit bis zu 4 Kohlenstoffatomen substituiert sind, das gegebenenfalls ein- bis dreifach, gleich oder verschieden durch Hydroxy, Fluor, Chlor, Carboxyl, Cyclopropyl, Cycloheptyl, geradkettiges oder verzweigtes Alkoxy oder Alkoxycarbonyl mit jeweils bis zu 3 Kohlenstoffatomen oder durch einen Rest der Formel -SO₃H, -NR⁵¹R⁵² oder P(O)OR⁵³OR⁵⁴ substituiert ist,
worin
- R⁵¹ und R⁵²: gleich oder verschieden sind und Wasserstoff, Phenyl, Carboxyl, Benzyl oder geradkettiges oder verzweigtes Alkyl oder Alkoxy mit jeweils bis zu 3 Kohlenstoffatomen bedeuten,
- R⁵³ und R⁵⁴: gleich oder verschieden sind und die oben angegebene Bedeutung von R¹⁰ und R¹¹ haben,
und/oder das Alkyl gegebenenfalls durch Phenyl substituiert ist, das seinerseits ein- bis zweifach, gleich oder verschieden durch Fluor, Chlor, Hydroxy, Methoxy oder durch eine Gruppe der Formel -NR^{51'}R^{52'} substituiert sein kann,
worin
- R^{51'} und R^{52'}: die oben angegebene Bedeutung von R⁵¹ und R⁵² haben und mit dieser gleich oder verschieden sind,
und/oder die unter R³ und R⁴ aufgeführten, gemeinsam mit dem Stickstoffatom gebildeten Heterocyclen, gegebenenfalls durch Phenyl, Pyridyl, Piperidinyl, Pyrrolidinyl oder Tetrazolyl, gegebenenfalls auch über eine N-Funktion verknüpft, substituiert sind, wobei die Ringsysteme ihrerseits durch Hydroxy oder durch geradkettiges oder verzweigtes Alkyl oder Alkoxy mit jeweils bis zu 3 Kohlenstoffatomen substituiert sein können ,
oder
- R³ und R⁴: gemeinsam mit dem Stickstoffatom Reste der Formeln 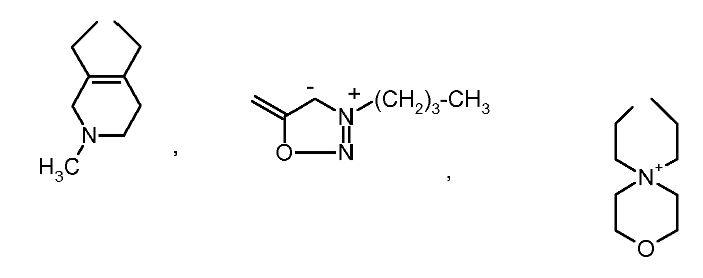 oder 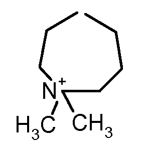 bilden,
- R⁵ und R⁶: gleich oder verschieden sind und für Wasserstoff, Hydroxy oder für geradkettiges oder verzweigtes Alkoxy mit bis zu 3 Kohlenstoffatomen stehen, und deren Salze, N-Oxide, Hydrate und isomere Formen.

Ganz besonders bevorzugt sind Verbindungen der allgemeinen Formel (I),
in welcher
- R¹: für Methyl oder Ethyl steht,
- R²: für Ethyl oder Propyl steht,
- R³ und R⁴: gleich oder verschieden sind und für eine geradkettige oder verzweigte Alkylkette mit bis zu 5 Kohlenstoffatomen stehen, die gegebenenfalls bis zu zweifach gleich oder verschieden durch Hydroxy oder Methoxy substituiert ist,
oder
- R³ und R⁴: gemeinsam mit dem Stickstoffatom einen Piperidinyl-, Morpholinyl-, Thiomorpholinylring oder einen Rest der Formel 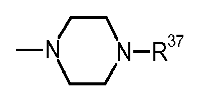 bilden,
worin
R³⁷ Wasserstoff, Formyl, geradkettiges oder verzweigtes Acyl oder Alkoxycarbonyl mit jeweils bis zu 3 Kohlenstoffatomen bedeutet, oder geradkettiges oder verzweigtes Alkyl mit bis zu 3 Kohlenstoffatomen bedeutet, das gegebenenfalls ein- bis zweifach, gleich oder verschieden durch Hydroxy, Carboxyl, geradkettiges oder verzweigtes Alkoxy oder Alkoxycarbonyl mit jeweils bis zu 3 Kohlenstoffatomen oder durch Gruppen der Formeln -(D)_{f}-NR³⁸R³⁹ oder -P(O)(OR⁴²)(OR⁴³) substituiert ist,
worin
f eine Zahl 0 oder 1 bedeutet,
D eine Gruppe der Formel -CO bedeutet,
R³⁸ und R³⁹ gleich oder verschieden sind und Wasserstoff oder Methyl bedeuten,
R⁴² und R⁴³ * gleich oder verschieden sind und Wasserstoff, Methyl oder Ethyl bedeuten, oder
- R³⁷: Cyclopentyl bedeutet, und die unter R³ und R⁴ aufgeführten, gemeinsam mit dem Stickstoffatom gebildeten Heterocyclen, gegebenenfalls ein- bis zweifach, gleich oder verschieden, gegebenenfalls auch geminal, durch Hydroxy, Formyl, Carboxyl, geradkettiges oder verzweigtes Acyl oder Alkoxycarbonyl mit bis jeweils zu 3 Kohlenstoffatomen oder Gruppen der Formeln -P(O)(OR⁴⁶)(OR⁴⁷) oder -(CO)ᵢNR⁴⁹R⁵⁰ substituiert sind,
worin
- R⁴⁶ und R⁴⁷: gleich oder verschieden sind und Wasserstoff, Methyl oder Ethyl bedeuten,
- j: eine Zahl 0 oder 1 bedeutet,
und
R⁴⁹ und R⁵⁰ gleich oder verschieden sind und Wasserstoff oder Methyl bedeuten
und/oder die unter R³ und R⁴ aufgeführten, gemeinsam mit dem Stickstoffatom gebildeten Heterocyclen, gegebenenfalls durch geradkettiges oder verzweigtes Alkyl mit bis zu 3 Kohlenstoffatomen substituiert sind, das gegebenenfalls ein- bis zweifach, gleich oder verschieden durch Hydroxy, Carboxyl oder durch einen Rest der Formel P(O)OR⁵³OR⁵⁴ substituiert ist,
worin
R⁵³ und R⁵⁴ gleich oder verschieden sind und Wasserstoff, Methyl oder Ethyl bedeuten,
und/oder die unter R³ und R⁴ aufgeführten, gemeinsam mit dem Stickstoffatom gebildeten Heterocyclen, gegebenenfalls durch über N-verknüpftes Piperidinyl oder Pyrrolidinyl substituiert sind,
- R⁵: für Wasserstoff steht,
und
- R⁶: für Ethoxy oder Propoxy steht,
und deren Salze, Hydrate, N-Oxide und isomere Formen.

Ebenso sind solche erfindungsgemäßen Verbindungen der allgemeinen Formel (I) ganz besonders bevorzugt, in denen R⁵ für Wasserstoff steht und die Reste R⁶ und -SO₂NR³R⁴ in para-Position zueinander am Phenylring stehen.

Insbesonders bevorzugte Verbindungen sind in der Tabelle A aufgeführt.

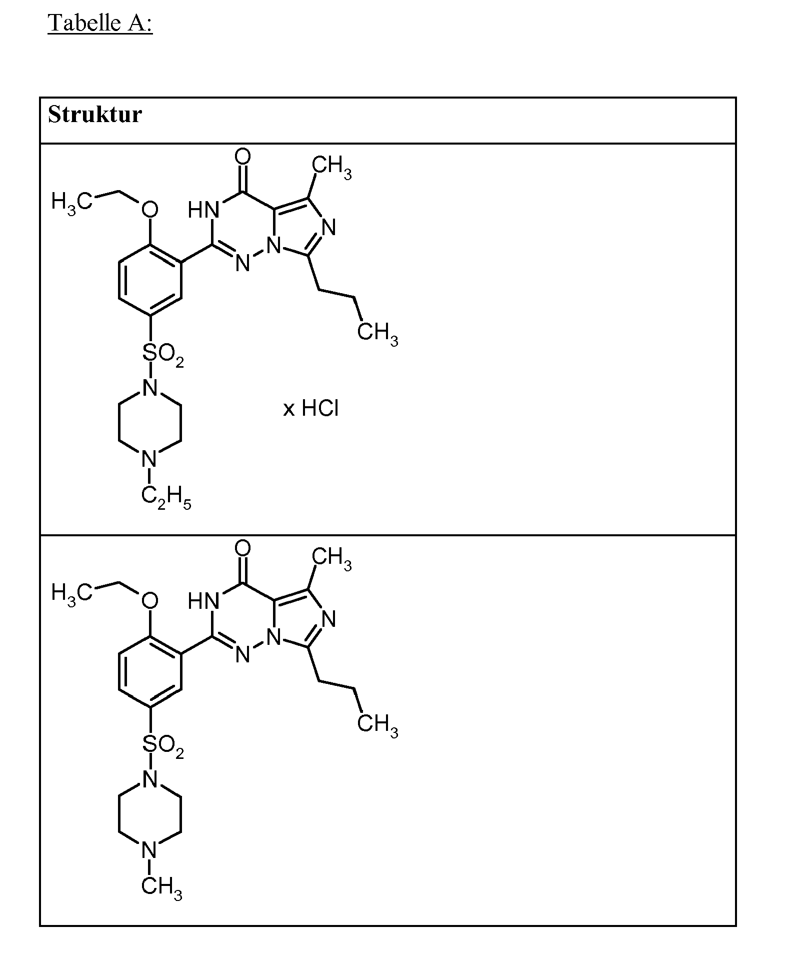

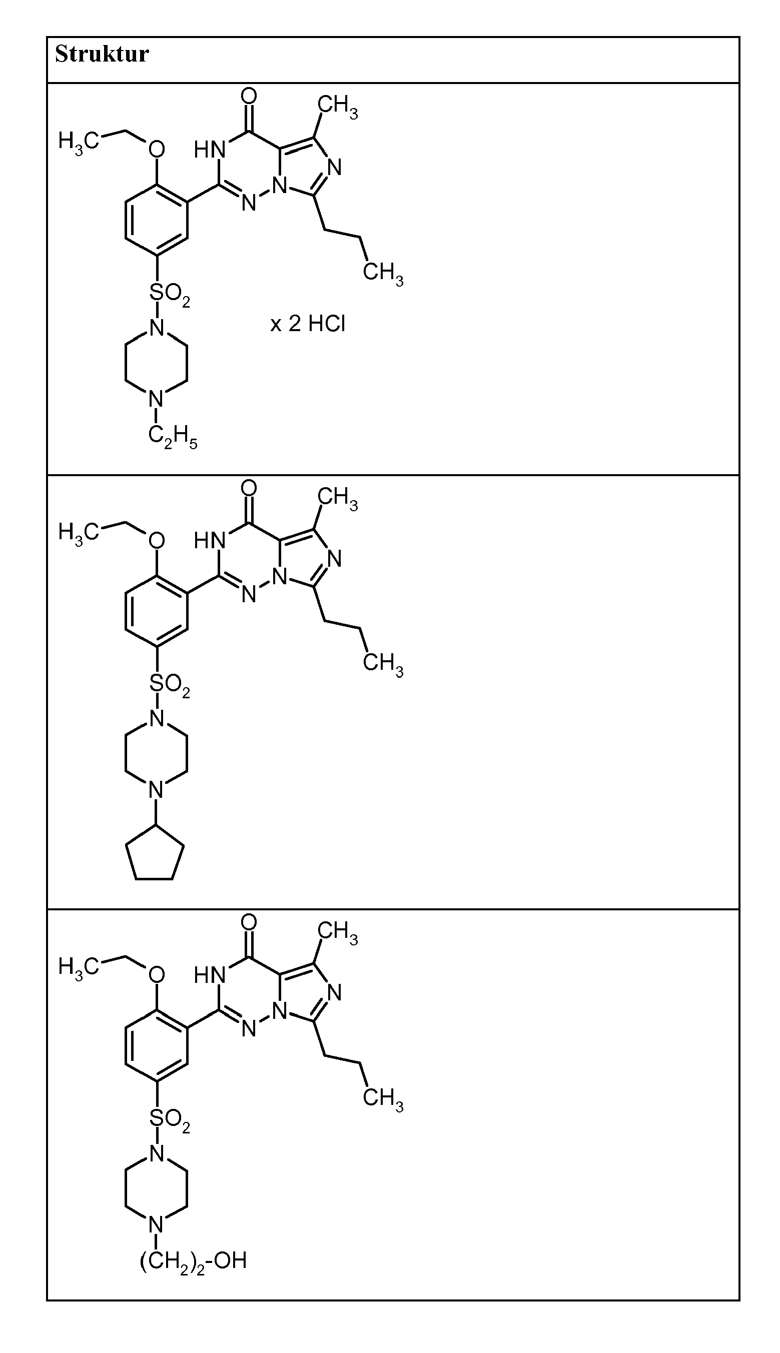

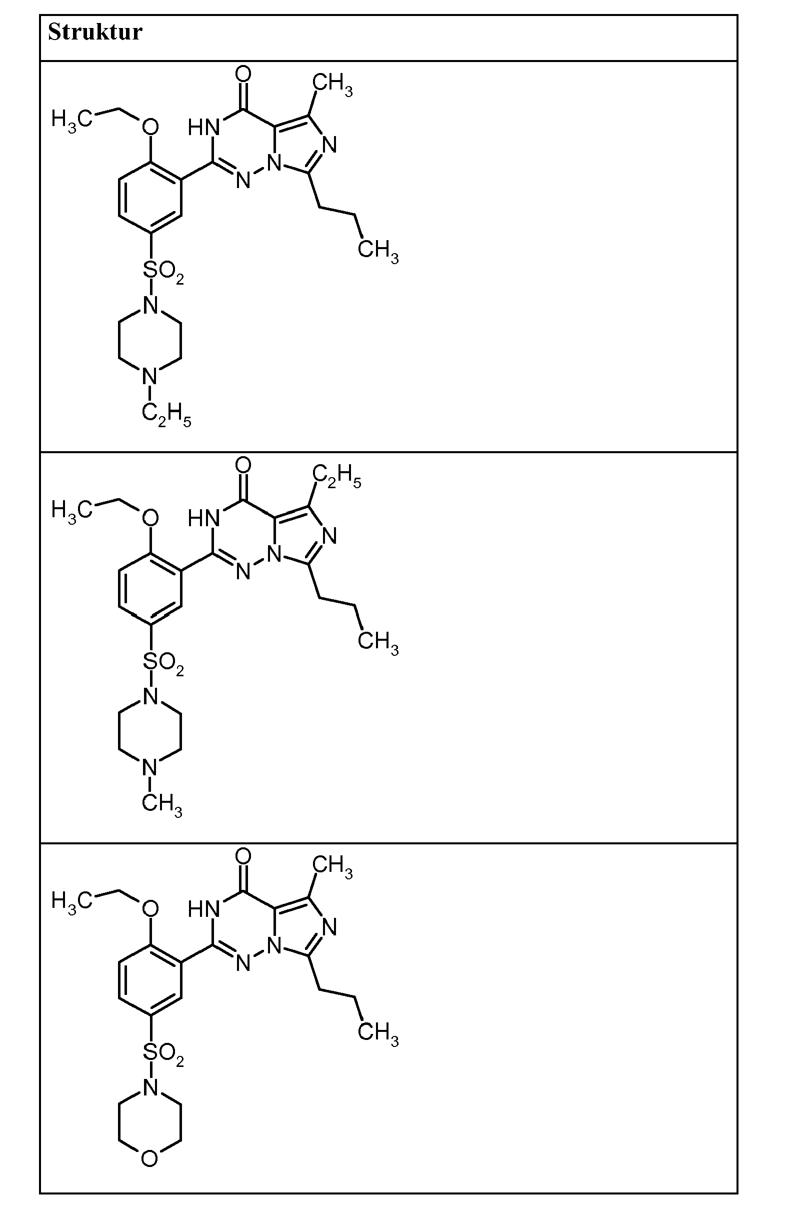

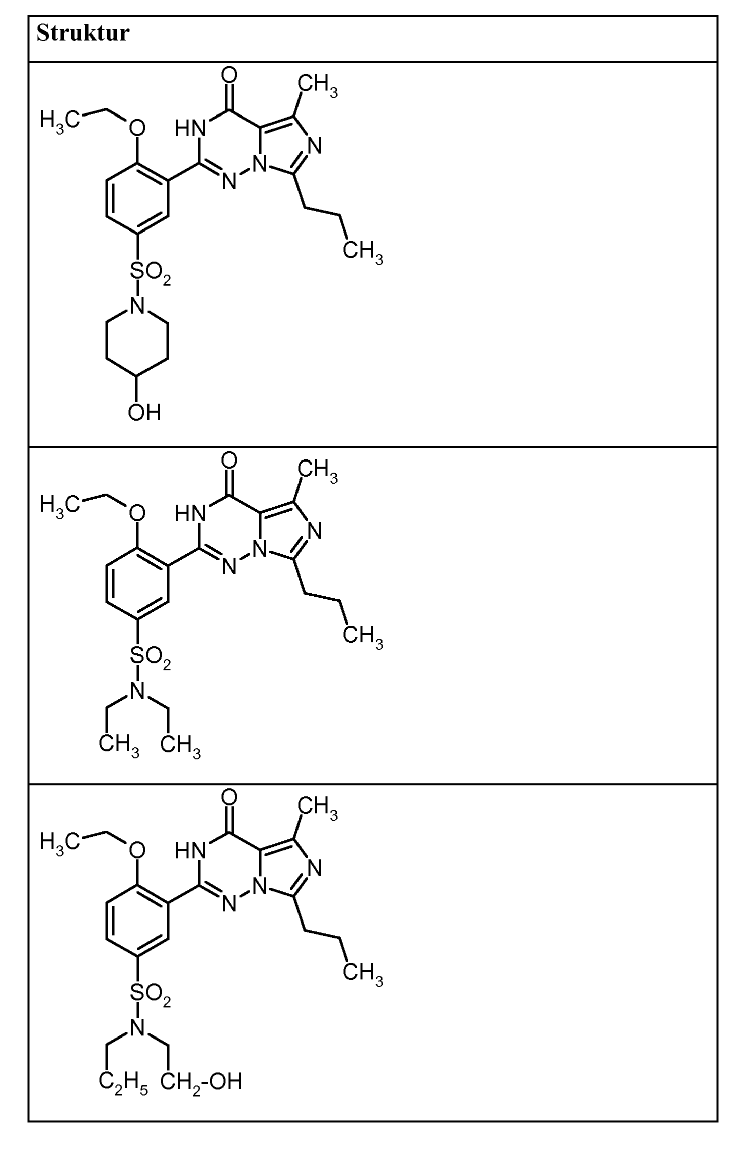

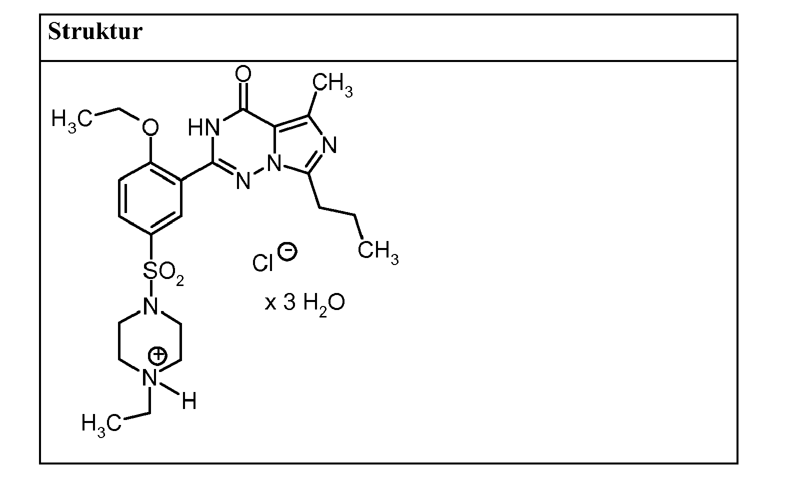

Außerdem wurde ein Verfahren zur Herstellung der erfindungsgemäßen Verbindungen der allgemeinen Formel (I) gefunden, **dadurch gekennzeichnet, daß** man zunächst Verbindungen der allgemeinen Formel (II) 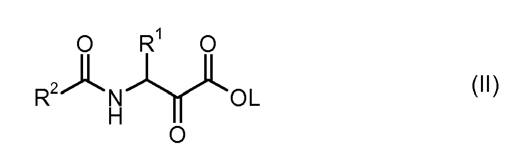 in welcher
R¹ und R² die oben angegebene Bedeutung haben
und
L für geradkettiges oder verzweigtes Alkyl mit bis zu 4 Kohlenstoffatomen steht,
mit Verbindungen der allgemeinen Formel (III) 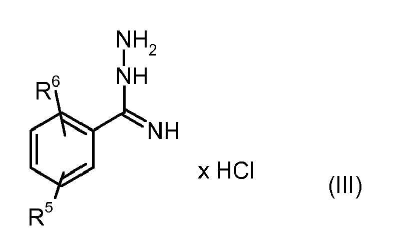 in welcher
R⁵ und R⁶ die oben angegebene Bedeutung haben,
in einer Zweistufenreaktion in den Systemen Ethanol und Phosphoroxytrichlorid /Dichlorethan in die Verbindungen der allgemeinen Formel (IV) 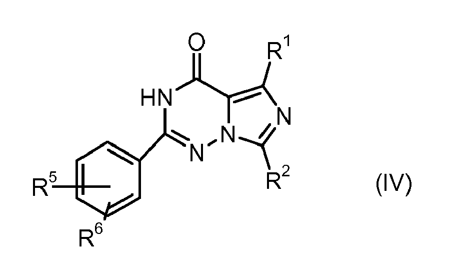 in welcher
R¹, R², R⁵ und R⁶ die oben angegebene Bedeutung haben,
überführt, in einem weiteren Schritt mit Chlorsulfonsäure zu den Verbindungen der allgemeinen Formel (V) 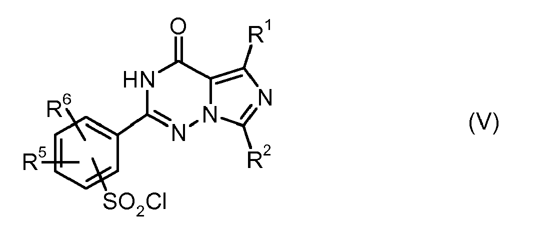 in welcher
R¹, R², R⁵ und R⁶ die oben angegebene Bedeutung haben,
umsetzt und abschließend mit Aminen der allgemeinen Formel (VI)

HN³R⁴ (VI)

in welcher
R³ und R⁴ die oben angegebene Bedeutung haben,
in inerten Lösemitteln umsetzt.
Das erfindungsgemäße Verfahren kann durch folgendes Formelschema beispielhaft erläutert werden:

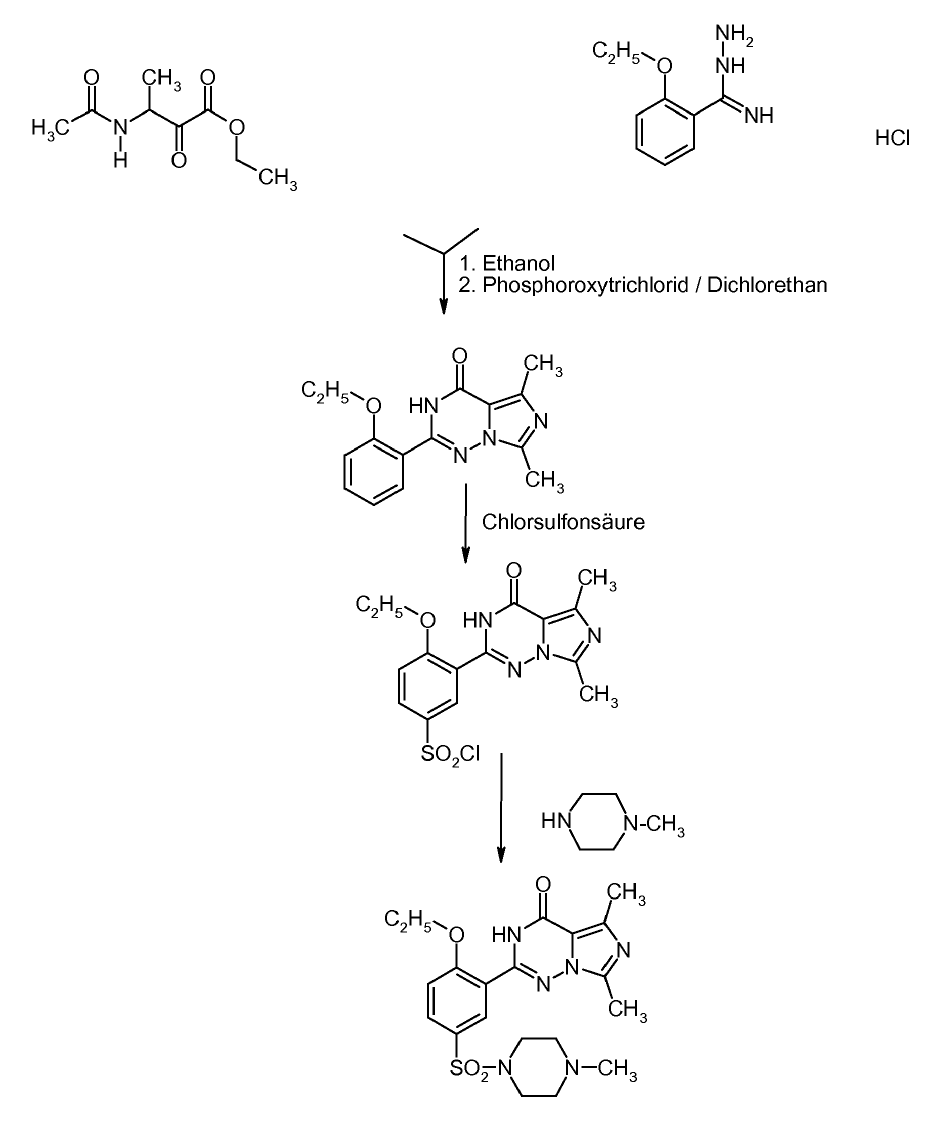

Als Lösemittel für die einzelnen Schritte eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist für den ersten Schritt Ethanol und für den zweiten Schritt Dichlorethan..

Die Reaktionstemperatur kann im allgemeinen in einem größeren Bereich variiert werden. Im allgemeinen arbeitet man in einem Bereich von -20°C bis 200°C, bevorzugt von 0°C bis 70°C.

Die erfindungsgemäßen Verfahrensschritte werden im allgemeinen bei Normaldruck durchgeführt. Es ist aber auch möglich, bei Überdruck oder bei Unterdruck durchzuführen (z.B. in einem Bereich von 0,5 bis 5 bar).

Die Umsetzung zu den Verbindungen der allgemeinen Formel (V) erfolgt in einem Temperaturbereich von 0°C bis Raumtemperatur und Normaldruck.

Die Umsetzung mit den Aminen der allgemeinen Formel (VI) erfolgt in einem der oben aufgeführten chlorierten Kohlenwasserstoffe, vorzugsweise in Dichlormethan.

Die Reaktionstemperatur kann im allgemeinen in einem größeren Bereich variiert werden. Im allgemeinen arbeitet man in einem Bereich von -20°C bis 200°C, bevorzugt von 0°C bis Raumtemperatur.

Die Umsetzung wird im allgemeinen bei Normaldruck durchgeführt. Es ist aber auch möglich, bei Überdruck oder bei Unterdruck durchzuführen (z.B. in einem Bereich von 0,5 bis 5 bar).

Die Verbindungen der allgemeinen Formel (II) sind teilweise bekannt oder neu und können dann hergestellt werden, indem man

Verbindungen der allgemeinen Formel (VII)

R²-CO-T (VII)

in welcher
- R²: die oben angegebene Bedeutung hat
und
- T: für Halogen, vorzugsweise für Chlor steht,
zunächst durch Umsetzung mit Verbindungen der allgemeinen Formel (VIII) 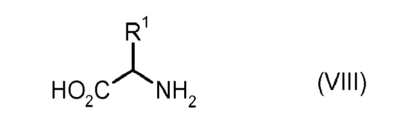 in welcher
- R¹: die oben angegebene Bedeutung hat
in inerten Lösemitteln, gegebenenfalls in Anwesenheit einer Base und Trimethylsilylchlorid in die Verbindungen der allgemeinen Formel (IX) 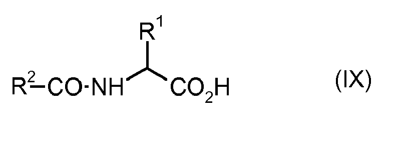 in welcher
R¹ und R² die oben angegebene Bedeutung haben,
überführt und abschließend mit der Verbindung der Formel (X) 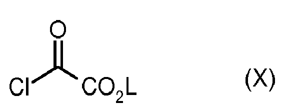 worin L die oben angegebene Bedeutung hat,
in inerten Lösemitteln, gegebenenfalls in Anwesenheit einer Base umsetzt.

Als Lösemittel für die einzelnen Schritte des Verfahrens eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist für den ersten Schritt Dichlormethan und für den zweiten Schritt ein Gemisch aus Tetrahydrofuran und Pyridin.

Als Basen eignen sich im allgemeinen Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Triethylamin, Pyridin und/oder Dimethylaminopyridin.

Die Base wird im allgemeinen in einer Menge von 1 mol bis 4 mol, bevorzugt von 1,2 mol bis 3 mol jeweils bezogen auf 1 mol der Verbindung der Formel (X) eingesetzt.

Die Reaktionstemperatur kann im allgemeinen in einem größeren Bereich variiert werden. Im allgemeinen arbeitet man in einem Bereich von -20°C bis 200°C, bevorzugt von 0°C bis 100°C.

Die Verbindungen der allgemeinen Formeln (VII), (VIII), (IX) und (X) sind an sich bekannt oder nach üblichen Methoden herstellbar.

Die Verbindungen der allgemeinen Formel (III) können hergestellt werden, indem man Verbindungen der allgemeinen Formel (XI) 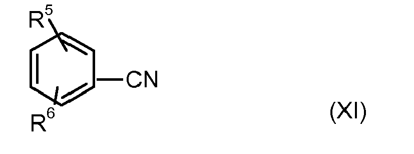 in welcher
R⁵ und R⁶ die oben angegebene Bedeutung haben,
mit Ammoniumchlorid in Toluol und in Anwesenheit von Trimethylaluminium in Hexan in einem Temperaturbereich von -20°C bis Raumtemperatur, vorzugsweise bei 0°C und Normaldruck umsetzt und das entstehende Amidin, gegebenenfalls in situ, mit Hydrazin-hydrat umsetzt.

Die Verbindungen der allgemeinen Formel (XI) sind an sich bekannt oder nach üblichen Methoden herstellbar.

Die Verbindungen der allgemeinen Formel (IV) sind teilweise bekannt oder neu und können dann nach bekannten Methoden [vgl. David R. Marshall, Chemistry and Industry, 2 May 1983, 331-335] hergestellt werden.

Die Verbindungen der allgemeinen Formel (V) sind an sich neu, können aber aus den Verbindungen der allgemeinen Formel (IV) nach der Publikation Organikum, VEB Deutscher Verlag der Wissenschaften, Berlin 1974, Seite 338 - 339, hergestellt werden.

Die erfindungsgemäßen Verbindungen der allgemeinen Formel (I) zeigen ein nicht vorhersehbares, wertvolles pharmakologisches Wirkspektrum.

Sie inhibieren entweder eine oder mehrere der c-GMP metabolisierenden Phosphodiesterasen (PDE I, PDE II und PDE V). Dies führt zu einem Anstieg von c-GMP. Die differenzierte Expression der Phosphodiesterasen in verschiedenen Zellen, Geweben und Organen, ebenso wie die differenzierte subzelluläre Lokalisation dieser Enzyme, ermöglichen in Verbindung mit den erfindungsgemäßen selektiven Inhibitoren, eine selektive Adressierung der verschiedenen von cGMP regulierten Vorgänge.

Außerdem verstärken die erfindungsgemäßen Verbindungen die Wirkung von Substanzen, wie beispielsweise EDRF (Endothelium derived relaxing factor), ANP (atrial natriuretic peptide), von Nitrovasodilatoren und allen anderen Substanzen, die auf eine andere Art als Phosphodiesterase-Inhibitoren die cGMP-Konzentration erhöhen.

Sie können daher in Arzneimitteln zur Behandlung von cardiovaskulären Erkrankungen wie beispielsweise zur Behandlung des Bluthochdrucks, neuronaler Hypertonie, stabiler und instabiler Angina, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, Angina pectoris, periphere Durchblutungsstörungen, Verhinderung von Restenosen nach Thrombolysetherapie, percutaner transluminaler Angioplastie (PTA), percutan transluminalen Koronarangioplastien (PTCA) und Bypass eingesetzt werden. Weiterhin können sie auch Bedeutung für cerebrovaskuläre Erkrankungen haben. Die relaxierende Wirkung auf glatte Muskulatur macht sie geeignet für die Behandlung von Erkrankungen des Urogenitalsystems wie Prostatahypertrophie, Inkontinenz sowie insbesondere zur Behandlung der erektilen Dysfunktion und der weiblichen sexuellen Dysfunktion.

### Aktivität der Phosphordiesterasen (PDE's)

Die c-GMP stimulierbare PDE II, die c-GMP hemmbare PDE III und die cAMP spezifische PDE IV wurden entweder aus Schweine- oder Rinderherzmyokard isoliert. Die Ca²⁺-Calmodulin stimulierbare PDE I wurde aus Schweineaorta, Schweinehirn oder bevorzugt aus Rinderaorta isoliert. Die c-GMP spezifische PDE V wurde aus Schweinedünndarm, Schweineaorta, humanen Blutplättchen und bevorzugt aus Rinderaorta gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990) und C. Lugman et al. Biochemical Pharmacology Vol. 35 1743-1751 (1986).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca 50% des Substrates umgesetzt werden. Um die cGMP stimulierbare PDE II zu testen, wird als Substrat ³HcAMP verwendet und dem Ansatz 10⁻⁶ mol/l nicht markiertes cGMP zugesetzt. Um die Ca²⁺-Calmodulinabhängige PDE I zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflußscintillationszähler quantitativ bestimmt. Es wird die Substanzkonzentration gemessen, bei der die Reaktionsgeschwindigkeit um 50% vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt. Für die Aktivitätsbestimmung der PDEII wurde der [³H] cAMP SPA assay verwendet, wobei dem Reaktionsansatz 10⁻⁶ M cGMP zur Aktivierung des Enzyms zugegeben wurde. Für die Messung der PDEI wurden Calmodulin 10⁻⁷ M und CaCl₂ 1µM zum Reaktionsansatz zugegeben. Die PDEV wurde mit dem [³H] cGMP SPA assay gemessen.

### Inhibition der Phosphodiesterasen in vitro

| **Bsp.-Nr.** | **PDE I** | **PDE II** | **PDE V** |
|---|---|---|---|
| | **IC₅₀ [nM]** | **IC₅₀ [nM]** | **IC₅₀ [nM]** |
| 16 | 300 | >1000 | 2 |
| 19 | 200 | >1000 | 2 |
| 20 | 200 | >1000 | 2 |
| 26 | 100 | >1000 | 1 |
| 27 | 200 | >1000 | 3 |
| 32 | 100 | >1000 | 4 |
| 260 | 300 | >1000 | 10 |
| 275 | 50 | >1000 | 3 |
| 338 | 200 | >1000 | 5 |

Grundsätzlich führt die Inhibition einer oder mehrerer Phosphodiesterasen dieses Typs zu einer Erhöhung der cGMP-Konzentration. Dadurch sind die Verbindungen interessant für alle Therapien, in denen eine Erhöhung der cGMP-Konzentration als heilsam angenommen werden kann.

Die Untersuchung der cardiovaskulären Wirkungen wurden an SH-Ratten und Hunden durchgeführt. Die Substanzen wurden intravenös oder oral appliziert.

Die Untersuchung auf erektionsauslösende Wirkung wurde am wachen Kaninchen durchgeführt [Naganuma H, Egashira T, Fuji J, Clinical and Experimental Pharmacology and Physiology 20, 177-183 (1993)]. Die Substanzen wurden intravenös, oral oder parenteral appliziert.

Die neuen Wirkstoffe sowie ihre physiologisch unbedenklichen Salze (z.Bsp. Hydrochloride, Maleinate oder Lactate) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, z.Bsp.perlingual, buccal, intravenös, nasal, rektal oder inhalativ.

Für die Anwendung beim Menschen werden bei oraler Administration Dosierungen von 0,001 bis 50 mg/kg vorzugsweise 0,01 mg/kg - 20 mg/kg sinnvollerweise verabreicht. Bei parenteraler Administration, wie z.B. über Schleimhäute nasal, buccal, inhalativ, ist eine Dosierung von 0,001 mg/kg - 0,5 mg/kg sinnvoll.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der oben genannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Die erfindungsgemäßen Verbindungen sind auch zur Anwendung in der Tiermedizin geeignet. Für Anwendungen in der Tiermedizin können die Verbindungen oder ihre nicht toxischen Salze in einer geeigneten Formulierung in Übereinstimmung mit den allgemeinen tiermedizinischen Praxen verabreicht werden. Der Tierarzt kann die Art der Anwendung und die Dosierung nach Art des zu behandelnden Tieres festlegen.

### Ausgangsverbindungen

### Beispiel 1A

### 2-Butyrylaminopropionsäure

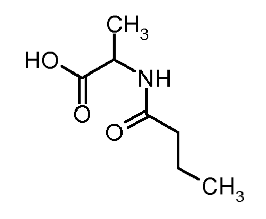

22,27 g (250 mmol) D,L-Alanin und 55,66g (550 mmol) Triethylamin werden in 250 ml Dichlormethan gelöst und die Lösung auf 0°C abgekühlt. 59,75 g (550 mmol) Trimethylsilylchlorid werden zugetropft und die Lösung 1 Stunde bei Raumtemperatur und eine Stunde bei 40°C gerührt. Nach dem Abkühlen auf -10°C werden 26,64 g (250 mmol) Buttersäurechlorid zugetropft und die resultierende Mischung 2 Stunden bei -10°C und eine Stunde bei Raumtemperatur gerührt.

Unter Eiskühlung werden 125 ml Wasser zugetropft und die Reaktionsmischung 15 Minuten bei Raumtemperatur gerührt. Die wäßrige Phase wird bis zur Trockene eingedampft, der Rückstand mit Aceton verrieben und die Mutterlauge abgesaugt. Nach dem Entfernen des Lösungsmittels wird der Rückstand chromatographiert. Das erhaltene Produkt wird in 3N Natronlauge gelöst und die resultierende Lösung bis zur Trockene eingedampft. Es wird mit konz. HCl aufgenommen und wieder bis zur Trockene eingedampft. Es wird mit Aceton verrührt, vom ausgefallenen Feststoff abgesaugt und das Lösungsmittel im Vakuum entfernt. Man erhält 28,2 g (71 %) eines zähen Öls, das nach einiger Zeit kristallisiert.

200 MHz ¹H-NMR (DMSO-d6): 0.84, t, 3H; 1.22, d, 3H; 1.50, hex, 2H; 2.07, t, 2H; 4.20, quin., 1H; 8.09, d, 1H.

### Beispiel 2A

### 2-Butyrylamino-buttersäure

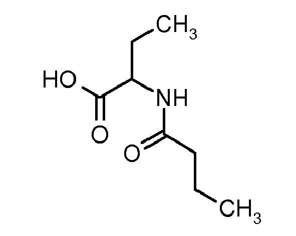

25,78 g 2-Aminobuttersäure (250 mmol) und 55,66 g (550 mmol) Triethylamin werden in 250 ml Dichlormethan gelöst und die Lösung auf 0°C abgekühlt. 59,75 g (550 mmol) Trimethylsilylchlorid werden zugetroft und die Lösung 1 Stunde bei Raumtemperatur und eine Stunde bei 40°C gerührt. Nach dem Abkühlen auf -10°C werden 26,64g (250 mmol) Buttersäurechlorid zugetropft und die resultierende Mischung 2 Stunden bei -10°C und eine Stunde bei Raumtemperatur gerührt.

Unter Eiskühlung werden 125 ml Wasser zugetropft und die Reaktionsmischung 15 Minuten bei Raumtemperatur gerührt. Die organische Phase wird mit Natronlauge versetzt und das organische Lösungsmittel im Vakuum entfernt. Nach dem Ansäuern wird der ausgefallene Feststoff 1 mal mit Wasser und 2 mal mit Petrolether verrührt und im Vakuum bei 45°C getrocknet. 29,1 g (67 %) farbloser Feststoff.

200 MHz ¹H-NMR (DMSO-d6):0.88, 2t, 6H; 1.51, quart., 2H, 1.65, m, 2H, 2.09, t, 2H, 4.10, m, 1H; 8.01, d, 1H; 12.25, s,m 1H.

### Beispiel 3A

### 2-Ethoxybenzonitril

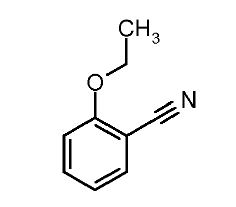

25 g (210 mmol) 2-Hydroxybenzonitril werden mit 87 g Kaliumcarbonat und 34,3 g (314,8 mmol) Ethylbromid in 500 ml Aceton über Nacht refluxiert. Es wird vom Feststoff abfiltriert, das Lösungsmittel im Vakuum entfernt und der Rückstand im Vakuum destilliert. Man erhält 30,0 g (97 %) einer farblosen Flüssigkeit.

200 MHz ¹H-NMR (DMSO-d6): 1.48, t, 3H; 4.15, quart., 2H; 6.99, dt, 2H; 7.51, dt, 2H.

### Beispiel 4A

### 2-Ethoxybenzamidinhydrochlorid

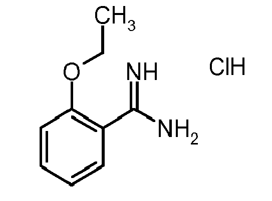

21,4 g (400 mmol) Ammoniumchlorid werden in 375 ml Toluol suspendiert und die Suspension auf 0°C abgekühlt. 200 ml einer 2M Lösung von Trimethylaluminium in Hexan werden zugetropft und die Mischung bis zur beendeten Gasentwicklung bei Raumtemperatur gerührt. Nach Zugabe von 29,44 g (200 mmol) 2-Ethoxybenzonitril wird die Reaktionsmischung über Nacht bei 80°C (Bad) gerührt.

Die abgekühlte Reaktionsmischung wird unter Eiskühlung zu einer Suspension aus 100 g Kieselgel und 950 ml Chloroform gegeben und die Mischung 30 Minuten bei Raumtemperatur gerührt. Es wird abgesaugt und mit der gleichen Menge Methanol nachgewaschen. Die Mutterlauge wird eingedampft, der erhaltene Rückstand mit einer Mischung aus Dichlormethan und Methanol (9:1) verrührt, der Feststoff abgesaugt und die Mutterlauge eingedampft. Man erhält 30,4 g (76 %) farblosen Feststoff.

200 MHz ¹H-NMR (DMSO-d6): 1.36, t, 3H; 4.12, quart., 2H; 7.10, t, 1H; 7.21, d, 1H; 7.52, m, 2H; 9.30, s, breit, 4H.

### Beispiel 5A

### 2-Propoxybenzonitril

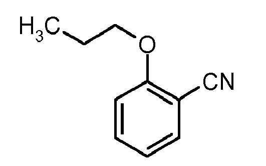

75 g (630 ml) 2-Hydroxybenzonitril werden mit 174 g (1,26 mol) Kaliumcarbonat und 232,2 g (1,89 mol) Ethylbromid in 1 1 Aceton über Nacht refluxiert. Es wird vom Feststoff abfiltriert, das Lösemittel im Vakuum entfernt und der Rückstand im Vakuum destilliert.
Kp.: 89°C (0,7 mbar)
Ausbeute: 95,1 g (93,7%)

### Beispiel 6A

### 2-Propoxybenzamidin-hydrochlorid

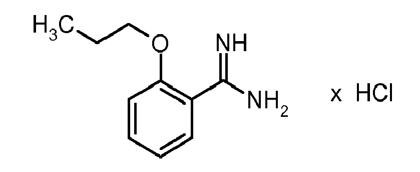

21,41 g (400 mmol) Ammoniumchlorid werden in 400 ml Toluol suspendiert und auf 0-5°C gekühlt. 200 ml einer 2 M Lösung von Triethylaluminium in Hexan werden zugetropft und die Mischung bis zur beendeten Gasentwicklung bei Raumtemperatur gerührt. Nach Zugabe von 32,2 g (200 mmol) 2-Propoxybenzonitril wird die Reaktionsmischung über Nacht bei 80°C (Bad) gerührt. Die abgekühlte Reaktionsmischung wird unter Eiskühlung zu einer Suspension aus 300 g Kieselgel und 2,85 1 eisgekühltem Chloroform gegeben und 30 Minuten gerührt. Es wird abgesaugt und mit der gleichen Menge Methanol nachgewaschen. Das Lösemittel wird im Vakuum abdestilliert, der Rückstand in 500 ml einer Mischung aus Dichlormethan und Methanol (9:1) verrührt, der Feststoff abfiltriert und die Mutterlauge eingedampft. Der Rückstand wird mit Petrolether verrührt und abgesaugt. Man erhält 22,3 g (52 %) Produkt.

¹H-NMR (200 MHz, CD₃OD): 1,05 (3H); 1,85 (sex, 2H); 4,1 (A, 2H); 7,0 - 7,2 (m, 2H); 7,5 - 7,65 (m, 2H).

### Beispiel 7A

### 2-Ethoxy-4-methoxybenzonitril

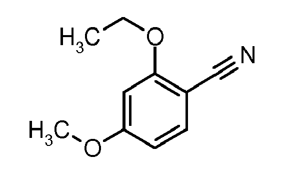

30,0 g (201 mmol) 2-Hydroxy-4-methoxybenzonitril werden mit 83,4 g Kaliumcarbonat (603 mmol) und 32,88 g (301 mmol) Bromethan 18 Stunden in 550 ml Aceton refluxiert. Nach Filtration wird das Lösungsmittel im Vakuum entfernt und der Rückstand durch Chromatographie an Kieselgel (Cyclohexan:Ethylacetat=10:1) gereinigt: 35,9 g Öl
R_{f}=0.37 (Cyclohexan:Ethylacetat=3:1)

200 MHz ¹H-NMR (CDCl₃): 1.48, t, 3H; 3.85, s, 3H; 4.12, quart., 2H; 6.46, m, 2H; 7.48, d, 1H.

### Beispiel 8A

### 2-Ethoxy-4-methoxybenzamidinhydrochlorid

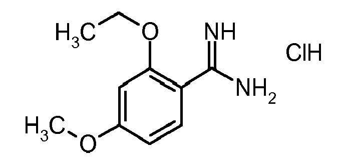

6,98 g (130 mmol) Ammoniumchlorid werden in 150 ml Toluol suspendiert und die Suspension auf 0°C abgekühlt. 70 ml einer 2M Lösung von Trimethylaluminium in Hexan werden zugetropft und die Mischung bis zur beendeten Gasentwicklung bei Raumtemperatur gerührt. Nach Zugabe von 11,56 g (65 mmol) 2-Ethoxy-4-methoxy-benzonitril wird die Reaktionsmischung über Nacht bei 80°C (Bad) gerührt.

Die abgekühlte Reaktionsmischung wird unter Eiskühlung zu einer Suspension aus 100 g Kieselgel und 950 ml Dichlormethan gegeben und die Mischung 30 Minuten bei Raumtemperatur gerührt. Es wird abgesaugt und mit der gleichen Menge Methanol nachgewaschen. Die Mutterlauge wird eingedampft, der erhaltene Rückstand mit einer Mischung aus Dichlormethan und Methanol (9:1) verrührt, der Feststoff abgesaugt und die Mutterlauge eingedampft. Der Rückstand wird mit Petrolether verrührt und abgesaugt. Man erhält 7,95 g (50 %) Feststoff.

200 MHz ¹H-NMR (DMSO-d6): 1.36, t, 3H; 3.84, s, 3H; 4.15, quart., 2H; 6.71, m, 2H; 7.53, d, 1H, 8.91, s, breit, 3H.

### Beispiel 9A

### 2-(2-Ethoxyphenyl)-5,7-dimethyl-3H-imidazo[5,1-f][1,2,4]triazin-4-on

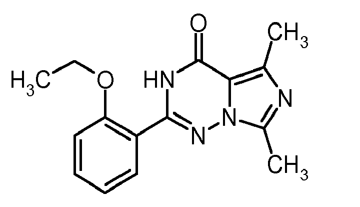

Man legt 24,4 g (0,186 mol) N-Acetyl-D,L-Alanin in 200 ml absolutem Tetrahydrofuran vor und setzt 45 ml absolutes Pyridin und 0,5 g 4-Dimethylaminopyridin hinzu. Man erhitzt zum Rückfluß und tropft 51,85 g (0,372 mol) Oxalsäuremonoethylesterchlorid hinzu. Man erhitzt weitere 90 Minuten unter Rückfluß, kühlt ab, gießt auf Eiswasser, extrahiert dreimal mit Essigsäureethylester. Man trocknet die organische Phase über Natriumsulfat, engt ein und nimmt in 62,5 ml Methanol auf. Man setzt 9 g Natriumhydrogencarbonat hinzu, rührt 2,5 Stunden unter Rückfluß und filtriert.

Zu einer Lösung von 38,26 g (190,65 mmol) 2-Ethoxy-4-methoxybenzamidinhydrochlorid in 250 ml Methanol tropft man unter Eiskühlung 9,54 g (190,65 mmol) Hydrazinhydrat zu und rührt die resultierende Suspension noch 30 Minuten bei Raumtemperatur. Zu dieser Reaktionsmischung gibt man die oben beschriebene methanolische Lösung und rührt 4 Stunden bei 70°C Badtemperatur. Nach Filtration wird eingedampft, der Rückstand zwischen Dichlormethan und Wasser verteilt, die organische Phase über Natriumsulfat getrocknet und das Lösungsmittel im Vakuum entfernt.

Der Rückstand wird in 250 ml 1,2-Dichlorethan aufgenommen, 32,1 ml (348 mmol) Phosphoroxychlorid zugetropft und zwei Stunden unter Rückfluß erhitzt. Man kühlt ab, engt ein, nimmt in wenig Methylenchlorid auf, versetzt mit Diethylether und saugt den Feststoff ab. Man chromatografiert an Kieselgel (Methylenchlorid/Methanol 95:5), engt die Lösung ein und verrührt den kristallinen Rückstand mit Diethylether. Ausbeute: 8,1g (14,9% der Theorie) 200 MHz ¹H-NMR (CDCl₃): 1,58, t, 3H; 2,62, s, 3H; 2,68, s, 3H; 4,25, q, 2H; 7,04, d, 1H; 7,12, t, 1H; 7,5, dt, 1H; 8,19, dd, 1H; 10,02, s, 1H.

### Beispiel 10A

### 2-(2-Ethoxy-phenyl)-5-methyl-7-propyl-3H-imidazo[5,1-f][1,2,4]triazin-4-on

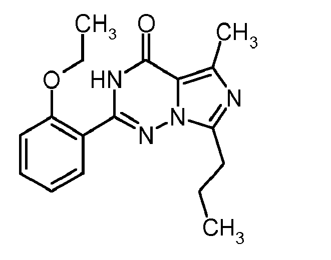

7,16 g (45 mmol) 2-Butyrylamino-propionsäure werden mit 10,67 g Pyridin in 45 ml THF gelöst und nach Zugabe einer Spatelspitze DMAP zum Rückfluß erhitzt. 12,29 g (90 mmol) Oxalsäure-ethylesterchlorid werden langsam zugetropft und die Reaktionsmischung wird 3 Stunden refluxiert. Es wird auf Eiswasser gegossen, dreimal mit Ethylacetat extrahiert, über Natriumsulfat getrocknet und einrotiert. Der Rückstand wird in 15 ml Ethanol aufgenommen und mit 2,15 g Natriumhydrogencarbonat 2,5 Stunden refluxiert. Die abgekühlte Lösung wird filtriert.

Zu einer Lösung von 9,03 g (45 mmol) 2-Ethoxybenzamidinhydrochlorid in 45 ml Ethanol tropft man unter Eiskühlung 2,25 g (45 mmol) Hydrazinhydrat zu und rührt die resultierende Suspension noch 10 Minuten bei Raumtemperatur. Zu dieser Reaktionsmischung gibt man die oben beschriebene ethanolische Lösung und rührt 4 Stunden bei 70°C Badtemperatur. Nach Filtration wird eingedampft, der Rückstand zwischen Dichlormethan und Wasser verteilt, die organische Phase über Natriumsulfat getrocknet und das Lösungsmittel im Vakuum entfernt.

Dieser Rückstand wird in 60 ml 1,2-Dichlorethan gelöst und nach Zugabe von 7,5 ml Phosphoroxychlorid 2 Stunden refluxiert. Es wird mit Dichlormethan verdünnt und durch Zugabe von Natriumhydrogencarbonatlösung und festem Natriumhydrogencarbonat neutralisiert. Die organische Phase wird getrocknet und das Lösungsmittel im Vakuum entfernt. Chromatographie mit Ethylacetat und Kristallisation ergeben 4,00 g (28 %) farblosen Feststoff, R_{f}=0,42 (Dichlormethan/Methanol=95:5)

200 MHz ¹H-NMR (CDCl₃): 1.02, t, 3H; 1.56, t, 3H; 1.89, hex, 2H; 2.67, s, 3H; 3.00, t, 2H; 4.26, quart., 2H; 7.05, m, 2H; 7.50, dt, 1H; 8.17, dd, 1H; 10.00, s, 1H.

### Beispiel 11A

### 2-(2-Propoxy-phenyl)-5-methyl-7-propyl-3H-imidazo[5,1-f][1,2,4]triazin-4-on

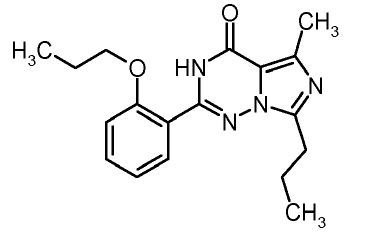

7,16 g (45 mmol) 2-Butyrylaminopropionsäure werden mit 10,67 g Pyridin in 45 ml Tetrahydrofuran gelöst und nach Zugabe einer Spatelspitze Dimethylaminopyridin zum Rückfluß erhitzt. 12,29 g (90 mmol) Oxalsäureethylesterchlorid werden langsam zugetropft und die Reaktionsmischung wird 3 Stunden refluxiert. Es wird auf Eiswasser gegossen, dreimal mit Ethylacetat extrahiert, über Natriumsulfat getrocknet und einrotiert. Der Rückstand wird in 15 ml Ethanol aufgenommen und mit 2,15 g Natriumhydrogencarbonat 2,5 Stunden refluxiert. Die abgekühlte Lösung wird filtriert.

Zu einer Lösung von 9,66 g (45 mmol) 2-Propoxybenzamidinhydrochlorid in 45 ml Ethanol tropft man unter Eiskühlung 2,25 g (45 mmol) Hydrazinhydrat zu und rührt die resultierende Suspension noch 10 Minuten bei Raumtemperatur. Zu dieser Reaktionsmischung gibt man die oben beschriebene ethanolische Lösung und rührt 4 Stunden bei 70°C Badtemperatur. Nach Filtration wird eingedampft, der Rückstand zwischen Dichlormethan und Wasser verteilt, die organische Phase über Natriumsulfat getrocknet und das Lösungsmittel im Vakuum entfernt.

Dieser Rückstand wird in 60 ml 1,2-Dichlorethan gelöst und nach Zugabe von 7,5 ml Phosphoroxychlorid 2 Stunden refluxiert. Es wird mit Dichlormethan verdünnt und durch Zugabe von Natriumhydrogencarbonatlösung und festem Natriumhydrogencarbonat neutralisiert. Die organische Phase wird getrocknet und das Lösungsmittel im Vakuum entfernt. Kristallisation aus Ethylacetat ergeben 2,85 g (19,1 %) eines gelben Feststoffs, chromatographische Reinigung der Mutterlauge ergibt weitere 1,25 g (8,4 %) des Produktes. R_{f}=0,45 (Dichlormethan/Methanol=95:5)

200 MHz ¹H-NMR (CDCl₃): 1.03, t, 3H; 1.15, t, 3H; 1.92, m, 4H; 2.67, s, 3H; 3.01, t, 2H; 4.17, t., 2H; 7.09, m, 2H; 7.50, dt, 1H; 8.17, dd, 1H; 10.02, s, 1H.

### Beispiel 12A

### 2-(2-Ethoxy-4-methoxyphenyl)-5-methyl-7-propyl-3H-imidazo[5,1-f][1,2,4]triazin-4-on

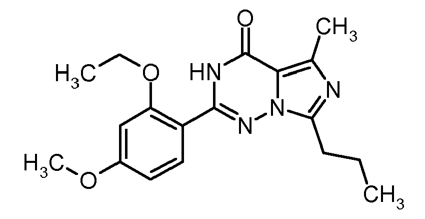

5,50 g (34,8 mmol) 2-Butyrylaminopropionsäure werden mit 8,19 g Pyridin in 35 ml Tetrahydrofuran gelöst und nach Zugabe einer Spatelspitze Dimethylaminopyridin zum Rückfluß erhitzt. 9,43 g (69 mmol) Oxalsäureethylesterchlorid werden langsam zugetropft und die Reaktionsmischung wird 3 Stunden refluxiert. Es wird auf Eiswasser gegossen, dreimal mit Ethylacetat extrahiert, über Natriumsulfat getrocknet und einrotiert. Der Rückstand wird in 11 ml Methanol aufgenommen und mit 1,65 g Natriumhydrogencarbonat 2,5 Stunden refluxiert. Die abgekühlte Lösung wird filtriert.

Zu einer Lösung von 7,95 g (34,5 mmol) 2-Ethoxy-4-methoxybenzamidinhydrochlorid in 35 ml Ethanol tropft man unter Eiskühlung 1,73 g (34,5 mmol) Hydrazinhydrat zu und rührt die resultierende Suspension noch 30 Minuten bei Raumtemperatur. Zu dieser Reaktionsmischung gibt man die oben beschriebene methanolische Lösung und rührt 4 Stunden bei 70°C Badtemperatur. Nach Filtration wird eingedampft, der Rückstand zwischen Dichlormethan und Wasser verteilt, die organische Phase über Natriumsulfat getrocknet und das Lösungsmittel im Vakuum entfernt.

Dieser Rückstand wird in 46 ml 1,2-Dichlorethan gelöst und nach Zugabe von 5,74 ml Phosphoroxychlorid 2 Stunden refluxiert. Es wird mit Dichlormethan verdünnt und durch Zugabe von Natriumhydrogencarbonatlösung und festem Natriumhydrogencarbonat neutralisiert. Die organische Phase wird getrocknet und das Lösungsmittel im Vakuum entfernt. Chromatographie (Dichlormethan:Methanol=50:1) ergibt 0,31 g (2,5 %) eines Feststoffs.
R_{f}=0,46 (Dichlormethan:Methanol=20: 1)

200 MHz ¹H-NMR (CDCl₃): 1.03, t, 3H; 1.58, t, 3H; 1.88, m, 2H; 2.62, s, 3H; 2.98, t, 2H; 3.89, s, 3H; 4.25, quart., 2H; 6.54, d, 1H, 6.67, dd, 1H; 8.14, d, 1H; 9.54, s, 1H.

### Beispiel 13A

### 2-(2-Ethoxyphenyl)-5-ethyl-7-propyl-3H-imdazo[5,1-f][1,2,4]triazin-4-on

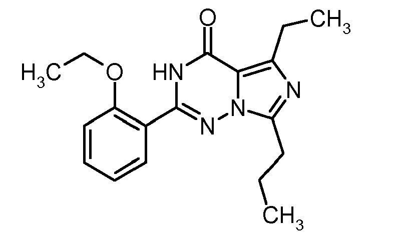

29,06 g (167,8 mmol) 2-Butyrylaminobuttersäure werden mit 39,76 g Pyridin in 170 ml Tetrahydrofuran gelöst und nach Zugabe einer Spatelspitze Dimethylaminopyridin zum Rückfluß erhitzt. 45,81 g (335,5 mmol) Oxalsäureethylesterchlorid werden langsam zugetropft und die Reaktionsmischung wird 3 Stunden refluxiert. Es wird auf Eiswasser gegossen, dreimal mit Ethylacetat extrahiert, über Natriumsulfat getrocknet und einrotiert. Der Rückstand wird in 15 ml Methanol aufgenommen und die Hälfte der Lösung mit 7,96 g Natriumhydrogencarbonat 2,5 Stunden refluxiert. Die abgekühlte Lösung wird filtriert.

Zu einer Lösung von 16,83 g (83,9 mmol) 2-Ethoxybenzoesäureamidin Hydrochlorid in 85 ml Ethanol tropft man unter Eiskühlung 4,20 g (83,9 mmol) Hydrazinhydrat zu und rührt die resultierende Suspension noch 10 Minuten bei Raumtemperatur. Zu dieser Reaktionsmischung gibt man die oben beschriebene methanolische Lösung und rührt 4 Stunden bei 70°C Badtemperatur. Nach Filtration wird eingedampft, der Rückstand zwischen Dichlormethan und Wasser verteilt, die organische Phase über Natriumsulfat getrocknet und das Lösungsmittel im Vakuum entfernt.

Dieser Rückstand wird in 112 ml 1,2-Dichlorethan gelöst und nach Zugabe von 14 ml Phosphoroxychlorid 2 Stunden refluxiert. Es wird mit Dichlormethan verdünnt und durch Zugabe von Natriumhydrogencarbonatlösung und festem Natriumhydrogencarbonat neutralisiert. Die organische Phase wird getrocknet und das Lösungsmittel im Vakuum entfernt. Chromatographie (Dichlormethan:Methanol=50:1) ergibt 3,69 g (12,4 %) farblosen Feststoff, R_{f}=0,46 (Dichlormethan:Methanol=20:1)

200 MHz ¹H-NMR (CDCl₃): 1.32, t, 3H; 1.57, t, 3H; 1.94, m, 8H; 3.03, quart., 2H; 3.64, quin., 1H; 4.27, quart., 2H; 7.06, d, 1H; 7.12, t, 1H; 7.50, dt, 1H, 8.16, dd, 1H; 9.91, s, 1H.

### Beispiel 14A

### 4-Ethoxy-3-(5,7-dimethyl-4-oxo-3,4-dihydroimidazo[5,1-f][1,2,4]triazin-2-yl)-benzolsulfon-säurechlorid

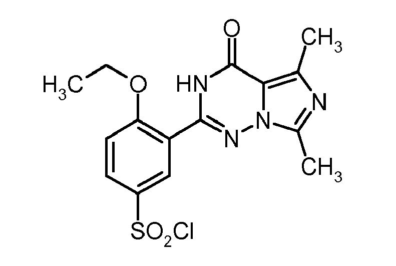

Man legt 7,25 g (25,5 mmol) 2-(2-Ethoxyphenyl)-5,7-dimethyl-3*H*-imidazo[5,1-f][1,2,4]-triazin-4-on vor und setzt unter Eiskühlung 26,74 g (0,23 mol) Chlorsulfonsäure hinzu. Man rührt über Nacht bei Raumtemperatur, gießt auf Eiswasser, saugt die Kristalle ab und trocknet sie im Vakuumexsikkator.
Ausbeute: 9,5 g (97 % der Theorie)

200 MHz ¹H-NMR (d⁶-DMSO): 1,32, t, 3H; 2,63, s, 3H; 2,73, s, 3H; 4,13, q, 2H; 7,15, d, 1H; 7,77, m, 2H; 12,5, s, 1H;

### Beispiel 15A

### 4-Ethoxy-3-(5-methyl-4-oxo-7-propyl-3,4-dihydro-imidazo[5,1-f][1,2,4]triazin-2-yl)-benzolsulfonsäurechlorid

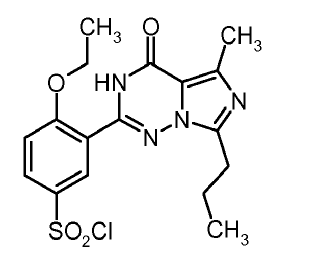

2,00 g (6,4 mmol) 2-(2-Ethoxy-phenyl)-5-methyl-7-propyl-3*H-*imidazo[5,1-f][1,2,4]-triazin-4-on werden langsam zu 3,83 ml Chlorsulfonsäure bei 0°C gegeben. Die Reaktionsmischung wird bei Raumtemperatur über Nacht gerührt, auf Eiswasser gegossen und mit Dichlormethan extrahiert. Man erhält 2,40 g (91 %) farblosen Schaum.

200 MHz ¹H-NMR (CDCl₃): 1.03, t, 3H; 1.61, t, 2H; 1.92, hex, 2H; 2.67, s, 3H; 3.10, t, 2H; 4.42, quart., 2H; 7.27, t, 1H; 8.20, dd, 1H; 8.67, d, 1H; 10.18, s, 1H.

### Beispiel 16A

### 4-Propoxy-3-(5-methyl-4-oxo-7-propyl-3,4-dihydro-imidazo[5,1-f][1,2,4]triazin-2-yl)-benzolsulfonsäurechlorid

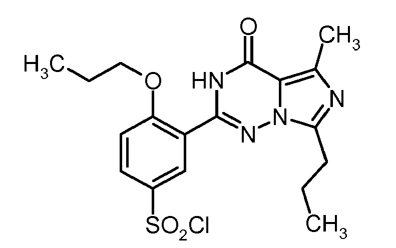

2,80 g (8 , 6 mmol) 2-(2-Propoxy-phenyl)-5-methyl-7-propyl-3*H*-imidazo[5,1-f][1,2,4]triazin-4-on werden langsam zu 5,13 ml Chlorsulfonsäure bei 0°C gegeben. Die Reaktionsmischung wird bei Raumtemperatur über Nacht gerührt, auf Eiswasser gegossen und mit Dichlormethan extrahiert. Man erhält 3,50 g (96 %) farblosen Schaum.
R_{f}=0.49 (Dichlormethan/Methanol=95:5)

200 MHz ¹H-NMR (CDCl₃): 1.03, 2t, 6H; 1.95, m, 4H; 2.81, s, 3H; 3.22, t, 2H; 4.11, t., 2H; 7.09, m, 1H; 8.06, dd, 1H; 8.21 m, 1H; 12.0, s, 1H.

### Beispiel 17A

### 4-Ethoxy-2-methoxy-5-(5-methyl-4-oxo-7-propyl-3,4-dihydroimidazo [5,1-f][1,2,4]triazin-2-yl)-benzolsulfonsäurechlorid

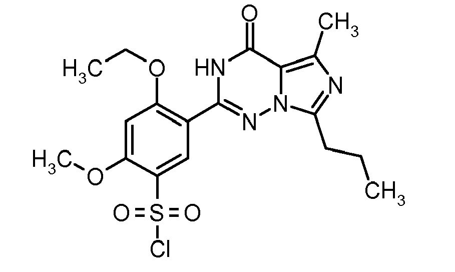

0,31 g (0,9 mmol) 2-(2-Ethoxy-4-methoxyphenyl)-5-methyl-7-propyl-3*H*-imidazo-[5,1-*f*]-[1,2,4]triazin-4-on werden langsam zu 0,54 ml Chlorsulfonsäure bei 0°C gegeben. Die Reaktionsmischung wird bei Raumtemperatur über Nacht gerührt, auf Eiswasser gegossen und mit Dichlormethan extrahiert. Man erhält 0,355 g (89 %) farblosen Schaum.
R_{f}=0,50 (Dichlormethan/Methanol=20:1)

200 MHz ¹H-NMR (CDCl₃): 1.05, t, 3H; 1.66, t, 3H; 1.95, m, 2H; 2.61, s, 3H, 3.11, t, 2H; 4.15, s, 3H; 4.40, quart., 2H; 6.65, s, 1H, 8.72, s, 1H; 9.75, s, 1H.

### Beispiel 18A

### 4-Ethoxy-3-(5-ethyl-4-oxo-7-propyl-3,4-dihydroimidazo[5,1-f][1,2,4]triazin-2-yl)-benzol-sulfonsäurechlorid

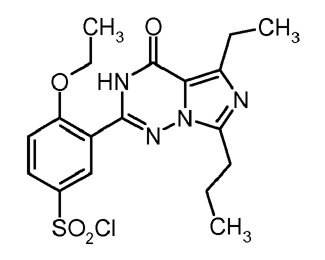

1,70 g (5,21 mmol) 2-(2-Ethoxy-phenyl)-5-ethyl-7-propyl-3*H*-imidazo[5,1-f][1,2,4]-triazin-4-on werden langsam zu 3,12 ml Chlorsulfonsäure bei 0°C gegeben. Die Reaktionsmischung wird bei Raumtemperatur über Nacht gerührt, auf Eiswasser gegossen und mit Dichlormethan extrahiert. Man erhält 2,10 g (94 %) farblosen Schaum.

400 MHz ¹H-NMR (CDCl₃): 1.03, t, 3H; 1.35, t, 3H; 1.62, t, 3H; 1.92, sex., 2H; 3.07, quart., 2H; 3.12, t, 2H; 4.42, quart., 2H; 7.38, d, 1H; 8.19, dd, 1H; 8.70, d, 1H; 10.08, s, breit, 1H.

### Beispiel 19A

### (4-Piperidinylmethyl)-phosphonsäurediethylester

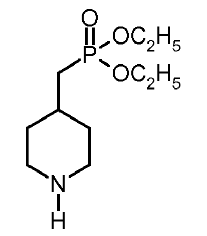

Man legt 2,11 g (528 mmol) 60%iges Natriumhydrid in 50 ml absolutem Tetrahydrofuran vor und tropft 15,7 g (52,8 mmol) Methandiphosphonsäurediethylester hinzu. Man rührt noch 30 Minuten bei Raumtemperatur und tropft dann 10,1 g (52,8 mmol) 1-Benzyl-4-piperidon hinzu. Man rührt eine Stunde bei Raumtemperatur und eine Stunde unter Rückfluß, engt ein, versetzt mit Wasser, extrahiert dreimal mit Dichlormethan, trocknet über Natriumsulfat und engt ein. Der Rückstand wird in 50 ml Ethanol an 1,7 g 10%iger Palladium-Aktivkohle bei Raumtemperatur und 3 bar hydriert. Man saugt den Katalysator ab und engt das Filtrat ein.
Ausbeute: 12,5 g (100% d.Th.)

400 MHz, ¹H-NMR (CDCl₃): 1,13, m, 2H; 1,32, t, 6H; 1,69, dd, 2H; 1,74 - 1,95, m, 4H; 2,62, dt, 2H; 3,05, m, 2H; 4,1, m, 4H.

### Beispiel 20A

### 5-Methyl-4-furoxancarbaldehyd

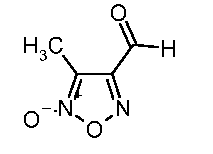

40 g (571 mmol) Crotonaldehyd werden in 80 ml Essigsäure gelöst und bei 0°C mit einer Lösung von 137 g (1,99 mol) Natriumnitrit in 300 ml Wasser tropfenweise versetzt. Man rührt 2 Stunden bei Raumtemperatur. Es wird mit 800 ml Wasser verdünnt und 3 mal mit Dichlormethan extrahiert. Nach Trocknen der organischen Phase erhält man durch Chromatographie (Cyclohexan/Ethylacetat) 13,8 g (18,9 %) 5-Methyl-4-furoxancarbaldehyd.

200 MHz ¹H-NMR (CDCl₃):2.39, s, 3H; 10.10, s, 1H.

### Beispiel 21A

### 5-Methyl-4-furoxancarbonsäurechlorid

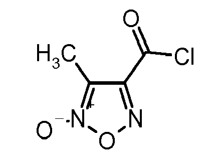

13,5 g (105 mmol) 5-Methyl-4-furoxancarbaldehyd werden in 200 ml Aceton gelöst und bei 0°C tropfenweise mit einer Lösung von 16,86 g (168 mmol) Chromtrioxid in 120 ml einer 2.2M Schwefelsäure versetzt. Man rührt 2 Stunden bei 10-15°C und bei Raumtemperatur über Nacht. Unter Kühlung werden 100 ml Isopropanol zugetropft und nach 30 Minuten das Lösungsmittel im Vakuum entfernt. Die wäßrige Phase wird 3 mal mit Ether extrahiert, die organische Phase über Magnesiumsulfat getrocknet und das Lösungsmittel im Vakuum entfernt. Der Rückstand wird in 1M Natriumhydroxidlösung gelöst und die Lösung 3 mal mit Ether extrahiert. Die wäßrige Phase wird sauer gestellt und 3 mal mit Ether extrahiert. Die organische Phase wird getrocknet und das Lösungmittel im Vakuum entfernt. Der Rückstand wird mit Petrolether verrührt und abgesaugt.

6,92 g des Rückstandes werden mit 10ml Thionylchlorid in 20 ml Dichlormethan 6 Stunden refluxiert. Es wird mit Toluol verdünnt, filtriert und einrotiert. Der Rückstand wird wiederum in Dichlormethan aufgenommen, mit 10 ml Thionylchlorid versetzt und 48 Stunden refluxiert. Das Lösungsmittel wird im Vakuum entfernt und der Rückstand im Vakuum destilliert. Man erhält 2,00 g (25 %) farblose Kristalle.

200 MHz ¹H-NMR (CDCl₃): 2.41, s.

### Beispiel 22A

### 1-(5-Methyl-4-furoxancarbonyl)-4-tert-butyl-oxycarbonyl-piperazin

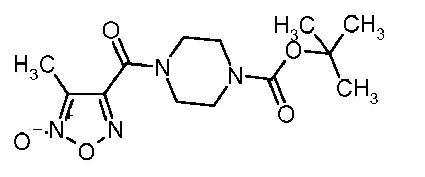

2,75 g (14,7 mmol) Boc-Piperazin werden mit 1,49 g Triethylamin in 20 ml Dichlormethan gelöst und bei 0°C portionsweise mit 2,00 g (12,3 mmol) 5-Methyl-4-furoxan-carbonsäurechlorid versetzt. Es wird 30 Minuten bei 0°C und 2 Stunden bei Raumtemperatur gerührt, mit Dichlormethan verdünnt und mit Wasser gewaschen. Das Lösungsmittel wird im Vakuum entfernt und der Rückstand durch Chromatographie (Cyclohexan/Ethylacetat) gereinigt. Man erhält 3,33 g (87 %) 1-(5-Methyl-4-furoxan-carbonyl)-4-tert-butyl-oxycarbonyl-piperazin.

200 MHz ¹H-NMR(CDCl₃):1.50, s, 9H; 2.30, s, 3H; 3.55, m, 4H; 3.78, m, 2H; 3.87, m, 2H.

### Beispiel 23A

### 1-(5-Methyl-4-furoxancarbonyl)-piperazin Trifluoracetat

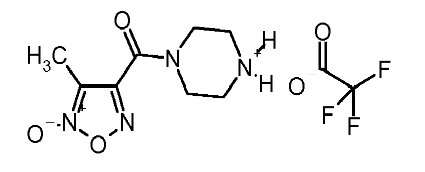

3,12 g (10 mmol) 1-(5-Methyl-4-furoxancarbonyl)-4-tert-butyl-oxycarbonyl-piperazin werden in 20 ml Dichlormethan gelöst und bei 0°C mit 2 ml Trifluoressigsäure versetzt. Man läßt auf Raumtemperatur aufwärmen und rührt 72 Stunden. Nach Zugabe von 10 ml Ether wird der Niederschlag abgesaugt und getrocknet. Man erhält 2,47 g (83 %) 1-(5-Methyl-4-furoxancarbonyl)-piperazin Trifluoracetat.

200 MHz ¹H-NMR (DMSO-d₆): 2.18, s, 3H; 3.18, m, 2H; 3.25, m, 2H; 3.83, m, 2H; 3.90, m, 2H; 8.89, s, breit, 2H.

### Herstellungsbeispiele

### Beispiel 1

2-[2-Ethoxy-5-(4-methyl-piperazin-1-sulfnyl)-phenyl]-5,7-dimethyl-3*H*-imdazo[5,1-f]-[1,2,4]triazin-4-on

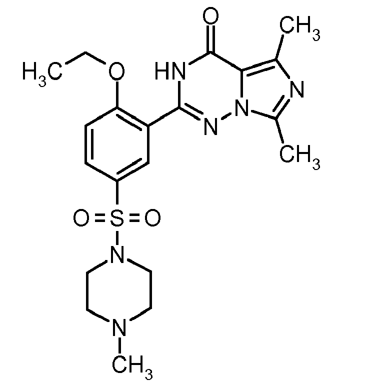

0,1 g (0,26 mmol) 4-Ethoxy-3-(5,7-dimethyl-4-oxo-3,4-dihydroimidazo[5,1-f][1,2,4]-triazin-2-yl)-benzolsulfonsäurechlorid werden in 10 ml Dichlormethan gelöst und auf 0°C gekühlt. Nach Zugabe einer Spatelspitze DMAP werden 80 mg (0,784 mmol) N-Methylpiperazin zugegeben und die Reaktionsmischung über Nacht bei Raumtemperatur gerührt. Es wird mit Dichlormethan verdünnt, die organische Phase mit Ammoniumchloridlösung gewaschen, über Natriumsulfat getrocknet und das Lösungsmittel im Vakuum entfernt. Man chromatografiert an Kieselgel (Dichlormethan/Methanol 9:1).
Ausbeute: 40 mg (34,5 % der Theorie)
Massenspektrum: 447 (M+H); 284; 256; 224;

### Beispiel 2

### 2-[2-Ethoxy-5-(4-hydroxyethylpiperazine-1-sulfnyl)-phenyl]-5,7-dimethyl-3H-imidazo[5,1-f]-[1,2,4]triazin-4-on

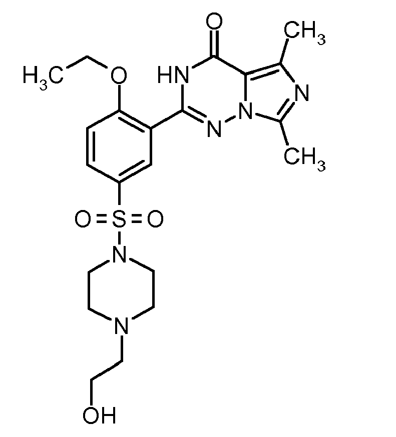

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäurechlorid und 100 mg (0,784 mmol) 4-Hydroxypiperazin 45 mg (36,1 % der Theorie) 2-[2-Ethoxy-5-(4-hydroxy-ethylpiperazin-1-sulfonyl)-phenyl]-5,7-dimethyl-3*H*-imidazo-[5,1-f]-[1,2,4]triazin-4-on.
Massenspektrum: 477 (M+H); 284; 256; 239.

### Beispiel 3

### 2-[2-Ethoxy-5-(4-hydroxypiperidine-1-sulfonyl)-phenyl]-5,7-dimethyl-3H-imidazo[5,1-f]-[1,2,4]triazin-4-on

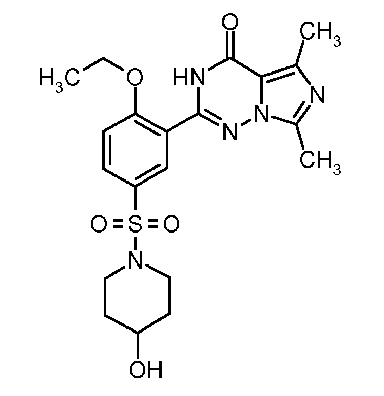

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 80 mg (0,784 mmol) 4-Hydroxypiperidin 35 mg (29,8 % der Theorie) 2-[2-Ethoxy-5-(4-hydroxy-piperidin-1-sulfonyl)-phenyl]-5,7-dimethyl-3*H*-imidazo[5,1-f]-[1,2,4]triazin-4-on.

200 MHz ¹H-NMR (CDCl₃): 1,61, t, 3H; 1,69, m, 2H; 1,94, m, 2H; 2,67, s, 3H; 2,70, s, 3H; 3,02, m, 2H; 3,30, m, 2H; 3,84, m, 1H; 4,37, q, 2H; 7,18, d, 1H; 7,90, dd, 1H; 8,52, d, 1H; 9,73, s, 1H.

### Beispiel 4

### 2-[2-Ethoxy-5-(4-hydroxymethylpiperidm-1-sulfonyl)-phenyl]-5,7-dimethyl-3H-imidazo[5,1-f][1,2,4]triazin-4-on

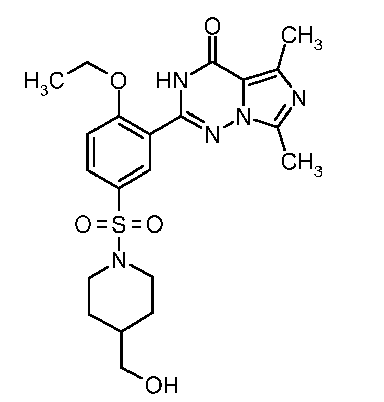

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäurechlorid und 90 mg (0,784 mmol) 4-Hydroxymethylpiperidin 22 mg (18 % der Theorie) 2-[2-Ethoxy-5-(4-hydroxy-methylpiperidin-1-sulfonyl)-phenyl]-5,7-dimethyl-3*H-*imid-azo[5,1-f][1,2,4]triazin-4-on.

200 MHz ¹H-NMR (CDCl₃):1,38, dt, 2H; 1,62, t, 3H; 1,82, dd, 2H; 2,35, dt, 2H; 2,78, s, 3H; 2,84, s, 3H; 3,5, d, 2H; 3,87, d, 2H; 4,39, q, 2H; 7,21, d, 1H; 7,95, dd, 1H; 8,51, d, 1H; 10,03, bs, 1H.

### Beispiel 5

### 2-[2-Ethoxy-5-(3-hydroxypyrrolidin-1-sulfonyl)-phenyl]-5,7-dimethyl-3H-imidazo[5,1-f]-[1,2,4]triazin-4-on

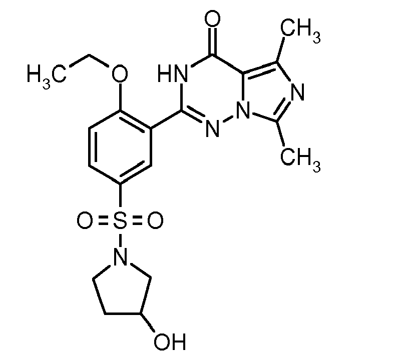

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 70 mg (0,784 mmol) 3-Hydroxypyrrolidin 13 mg (11,1 % der Theorie) 2-[2-Ethoxy-5-(3-hydroxy-pyrrolidin-1-sulfonyl)-phenyl]-5,7-dimethyl-3*H*-imidazo-[5,1-f][1,2,4]triazin-4-on.
Massenspektrum: 434 (M+H)

### Beispiel 6

### 4-Ethoxy-N-ethyl-N-(2-hydroxyethyl)-3-(5,7-dimethyl-4-oxo-3,4-dihydro-imidazo[5,1-f]-[1,2,4]triazin-2-yl)benzolsulfonamid

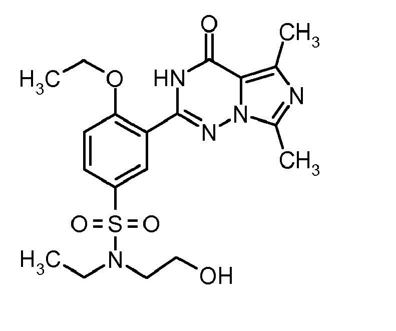

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 70 mg (0,784 mmol) 2-(Ethylamino)-ethanol 23 mg (20,1 % der Theorie) 4-Ethoxy-N-ethyl-N-(2-hydroxyethyl)-3-(5,7-dimethyl-4-oxo-3,4-dihydroimidazo-[5,1-f][1,2,4]triazin-2-yl)-benzol-sulfonamid.

200 MHz ¹H-NMR (CDCl₃): 1,2, t, 3H; 1,6, t, 3H; 2,17, bs, 1H; 2,69, s, 3H; 2,75, s, 3H; 3,33, m, 4H; 3,8, t, 2H; 4,36, q, 2H; 7,18, d, 1H; 7,99, dd, 1H; 8,6, d, 1H; 9,84, bs,1H.

### Beispiel 7

### N,N-Diethyl-4-ethoxy-3-(5,7-dimethyl-4-oxo-3,4-dihydroimidazo[5,1-f][1,2,4]triazin-2-yl)-benzolsulfonamid

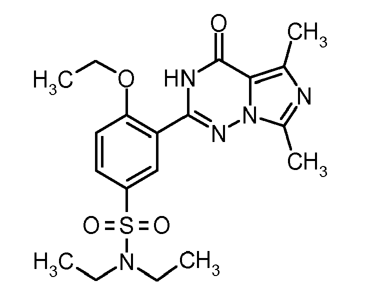

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 60 mg (0,784 mmol) Diethylamin 21 mg (18,6 % der Theorie) N,N-Diethyl-4-ethoxy-3-(5,7-dimethyl-4-oxo-3,4-dihydroimidazo[5,1-f][1,2,4]triazin-2-yl)-benzolsulfonamid.

200 MHz ¹H-NMR (CDCl₃): 1,18, t, 6H; 1,61, t, 3H; 2,68, s, 3H; 2,72, s, 3H; 3,29, q, 4H; 4,35, q, 2H; 7,15, d, 1H; 7,95, dd, 1H; 8,58, d, 1H; 9,8, bs, 1H.

### Beispiel 8

### 2-[2-Ethoxy-5-(4-(2-pyrimidinyl)-piperazin-1-sulfonyl)-phenyl]-5,7-dimethyl-3H-imidazo-[5,1-f][1,2,4]triazin-4-on

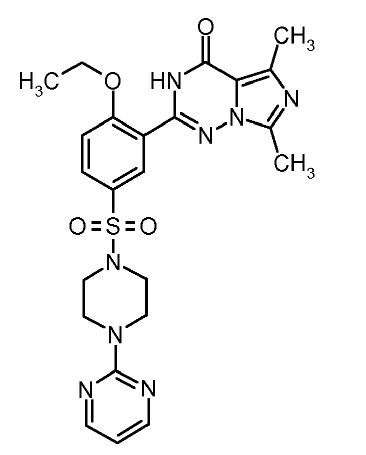

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 130 mg (0,784 mmol) 1-(2-Pyrimidinyl)-piperazin 38 mg (28,2% der Theorie) 2-[2-Ethoxy-5-(4-(2-pyrimidinyl)-piperazin-1-sulfonyl)-phenyl]-5,7-dimethyl-3*H-*imidazo-[5,1-f][1,2,4]triazin-4-on.

200 MHz ¹H-NMR (CDCl₃): 1,6, t, 3H; 2,68, s, 3H; 2,72, s, 3H; 3,12, t, 4H; 3,96, t, 4H; 4,34, q, 2H; 6,5, t, 1H; 7,18, d, 1H; 7,9, dd, 1H; 8,28, d, 2H; 8,51, d, 1H; 9,7, bs, 1H;

### Beispiel 9

### 2-[2-Ethoxy-5-(morpholin-4-sulfonyl)-phenyl]-5,7-dimethyl-3H-imidazo[5,1-f][1,2,4]triazin-4-on

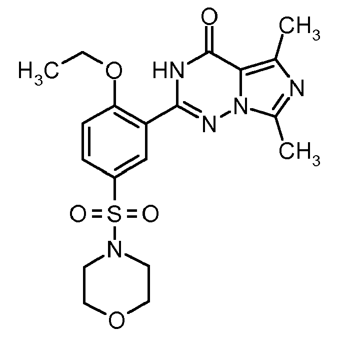

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 70 mg (0,784 mmol) Morpholin 28 mg (24,2% der Theorie) 2-[2-Ethoxy-5-(morpholin-4-sulfonyl)-phenyl]-5,7-dimethyl-3*H*-imidazo[5,1-f][1,2,4]triazin-4-on.

200 MHz ¹H-NMR (CDCl₃): 1,53, t, 3H; 2,69, s, 3H; 2,72, s, 3H; 3,06, t, 4H; 3,77, t, 4H; 4,39, q, 2H; 7,2, d, 1H; 7,91, dd, 1H; 8,51, d, 1H; 9,78, bs, 1H.

### Beispiel 10

### 2-[2-Ethoxy-5-(1,4-dioxa-6-azaspiro[4.4]nonan-6-sulfonyl)-phenyl]-5,7-dimethyl-3H imidazo[5,1-f][1,2,4]triazin-4-on

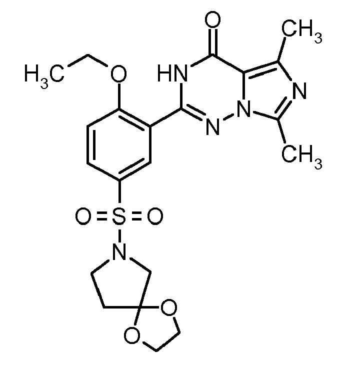

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 100 mg (0,784 mmol) 1,4-Dioxa-6-azaspiro[4.4]nonan 45 mg (35,3% der Theorie) 2-[2-Ethoxy-5-(1,4-dioxa-6-azaspiro[4.4]nonan-6-sulfonyl)-phenyl]-5,7-dimethyl-3*H*-imidazo[5,1-f][1,2,4]tri-azin-4-on.

200 MHz ¹H-NMR (CDCl₃): 1,58, t, 3H; 2,02, t, 2H; 2,61, s, 3H; 2,65, s, 3H; 3,32, s, 2H; 3,41, t, 2H; 3,88, m, 4H; 4,34, q, 2H; 7,17, d, 1H; 7,92, dd, 1H; 8,51, d, 1H; 9,92, bs, 1H.

### Beispiel 11

### N,N-Bis-(2-Methoxyethyl)-4-ethoxy-3-(5,7-dimethyl-4-oxo-3,4-dihydroimidazo[5,1-f]-[1,2,4]triazin-2-yl)-benzolsulfonamid

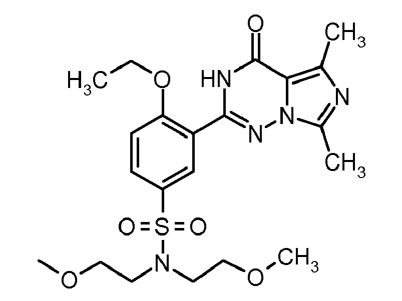

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 100 mg (0,784 mmol) Bis-(2-Methoxyethyl)-amin 37 mg (27,5% der Theorie) N,N-Bis-(2-Methoxy-ethyl)-4-ethoxy-3-(5,7-dimethyl-4-oxo-3,4-dihydro-imidazo[5,1-f][1,2,4]triazin-2-yl)-benzol-sulfonamid.

200 MHz ¹H-NMR (CDCl₃):1,58, t, 3H; 2,61, s, 3H; 2,64, s, 3H; 3,3, s, 6H; 3,46, t, 4H; 3,56, t, 4H; 4,32, q, 2H; 7,12, d, 1H; 7,95, dd, 1H; 8,51, d, 1H; 9,9, bs, 1H.

### Beispiel 12

### N-(3-Isoxazolyl)-4-ethoxy-3-(5,7-dimethyl-4-oxo-3,4-dihydroimidazo[5,1-f] [1,2,4]triazin-2-yl)-benzolsulfonamid

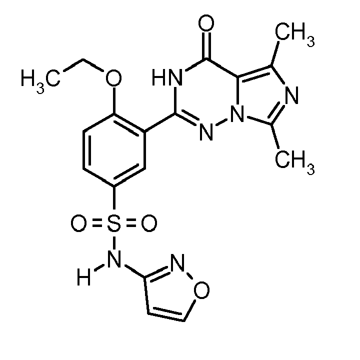

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 70 mg (0,784 mmol) 3-Aminoisoxazol 20 mg (17,2 % der Theorie) N-(3-isoxazolyl)-4-ethoxy-3-(5,7-dimethyl-4-oxo-3,4-dihydro-imidazo[5,1-f][1,2,4]-triazin-2-yl)benzolsulfonamid.

200 MHz ¹H-NMR (CDCl₃): 1,6, t, 3H; 2,73, s, 3H; 2,81, s, 3H; 4,35, q, 2H; 6,6, d, 1H; 7,14, d, 1H; 8,05, dd, 1H; 8,27, d, 1H; 8,63, d, 1H; 9,61, bs, 1H.

### Beispiel 13

### 2-[2-Ethoxy-5-(2-t-butoxycarbonylaminomethylmorpholin-4-sulfonyl)-phenyl]-5,7-dimethyl-3H-imidazo[5,1-f][1,2,4]triazin-4-on

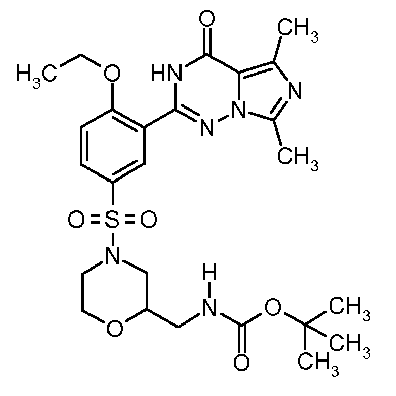

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 170 mg (0,784 mmol) 2-t-Butoxycarbonylaminomethylmorpholin 64 mg (42,2 % der Theorie) 2-[2-Ethoxy-5-(2-t-butoxycarbonylaminomethylmorpholin-4-sulfonyl)-phenyl]-5,7-dimethyl-3*H*-imidazo[5,1-f][1,2,4]triazin-4-on. Massenspektrum: 563 (M+H)

### Beispiel 14

### 2-[2-Ethoxy-5-(4-phenylpiperazin-1-sulfonyl)-phenyl]-5,7-dimethyl-3H-imdazo[5,1-f]-[1,2,4]triazin-4-on

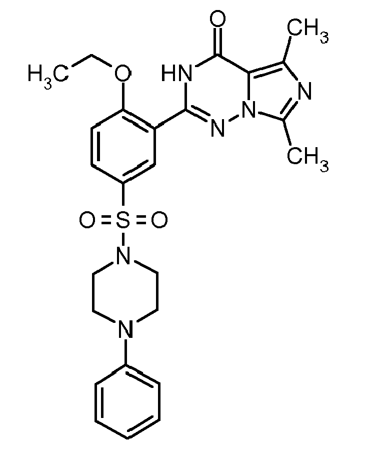

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 130 mg (0,784 mmol) 1-Phenylpiperazin, 38 mg (28,3 % der Theorie) 2-[2-Ethoxy-5-(4-phenylpiperazin-1-sulfonyl)-phenyl]-5,7-dimethyl-3*H*-imidazo[5,1-f][1,2,4]triazin-4-on

200 MHz ¹H-NMR (CDCl₃):1,62, t, 3H; 2,72, s, 3H; 2,77, s, 3H; 3,25, m, 8H; 4,38, q, 2H; 6,92, m, 2H; 7,02, d, 1H; 7,18-7,37, m, 3H; 7,94, dd, 1H; 8,55, m, 1H; 9,79, bs, 1H.

### Beispiel 15

### 2-[2-Ethoxy-5-(3-hydroxy-3-methoxymethylpyrrolidin-1-sulfonyl)-phenyl]-5,7-dimethyl-3H-imidazo[5,1-f][1,2,4]triazin-4-on

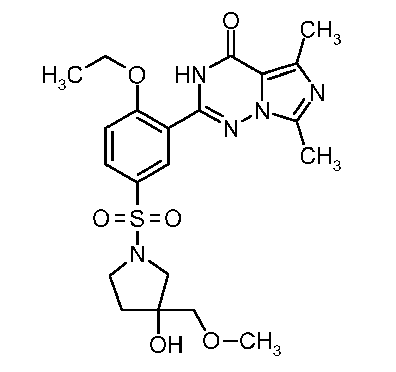

Auf analoge Weise erhält man ausgehend von 100 mg (0,261 mmol) 4-Ethoxy-3-(5,7-dimethyl-4-oxo-3,4-dihydroimidazo[5,1-f][1,2,4]triazin-2-yl)-benzolsulfonsäure-chlorid und 100 mg (0,784 mmol) 3-Hydroxy-3-methoxymethylpyrrolidin 30 mg (23,5 % der Theorie) 2-[2-Ethoxy-5-(3-hydroxy-3-methoxymethylpyrrolidin-1-sulfonyl)-phenyl]-5,7-dimethyl-3*H*-imidazo[5,1-f][1,2,4]triazin-4-on.
Massenspektrum: 478 (M+H)

### Beispiel 16

### 2-[2-Ethoxy-5-(4-methyl-piperazin-1-sulfonyl)-phenyl]-5-methyl-7-propyl-3H-imidazo[5,1-f][1,2,4]triazin-4-on

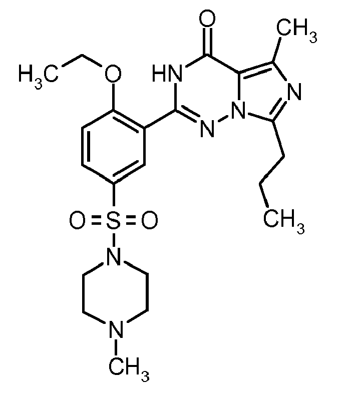

1,23 g (3 mmol) 4-Ethoxy-3-(5-methyl-4-oxo-7-propyl-3,4-dihydro-imidazo[5,1-f]-[1,2,4]triazin-2-yl)-benzolsulfonsäurechlorid werden in 40 ml Dichlormethan gelöst und auf 0°C gekühlt. Nach Zugabe einer Spatelspitze DMAP werden 0,90 g (9,00 mmol) N-Methylpiperazin zugegeben und die Reaktionsmischung über Nacht bei Raumtemperatur gerührt. Es wird mit Dichlormethan verdünnt, die organische Phase zweimal mit Wasser gewaschen, über Natriumsulfat getrocknet und das Lösungsmittel im Vakuum entfernt. Kristallisation aus Ether ergibt 1,25 g (88 %) farblosen Feststoff.

200 MHz ¹H-NMR (CDCl₃): 1.01, t, 3H; 1.59, t, 3H; 1.88, hex, 2H; 2.29, s, 3H; 2.51, m, 4H; 2.63, s, 3H; 3.00, t, 2H; 3.08, m, 4H; 4.33, quart., 2H, 7.17, d, ,1H; 7.88, dd, 1H; 8.44, d, 1H; 9.75, s, 1H.

### Beispiel 17

### 2-[2-Ethoxy-5-(4-methyl-piperazin-1-sulfonyl)-phenyl]-5-methyl-7-propyl-3H-imidazo[5,1-f][1,2,4]triazin-4-on Lactat

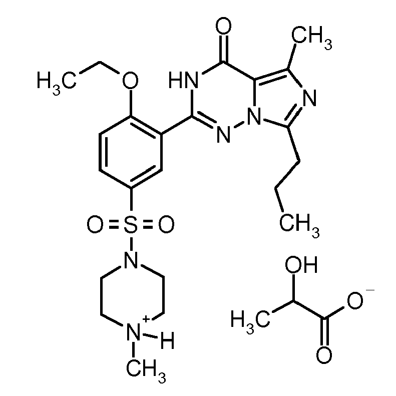

100 mg (0,211 mmol) 2-[2-Ethoxy-5-(4-methyl-piperazin-1-sulfonyl)-phenyl]-5-methyl-7-propyl-3*H*-imidazo[5,1-f][1,2,4]triazin-4-on werden in 5 ml Ether suspendiert und mit 20 mg einer 85%igen Lösung von Milchsäure in Wasser versetzt. Man rührt 10 Minuten bei Raumtemperatur und dampft bis zur Trockene ein. Es wird mit Ether verrieben und abgesaugt. Man erhält 110 mg (92 %) 2-[2-Ethoxy-5-(4-methylpiperazin-1-sulfonyl)-phenyl]-5-methyl-7-propyl-3*H*-imidazo[5,1-f][1,2,4]triazin-4-on Lactat.

200 MHz ¹H-NMR (DMSO-d₆): 0.92, t, 3H; 1.22, d, 3H; 1.31, t, 3H; 1.74, m, 1H; 2.15, s, 3H; 2.38, m, 4H; 2.81, t, 2H; 2.91, m, 4H; 4.05, quart., 1H; 4.21, quart., 2H; 7.40, d, 1H; 7.85, m, 2H; 11.71, s, breit, 1H.

### Beispiel 18

### 2-[2-Ethoxy-5-(4-methyl-piperazin-1-sulfonyl)-phenyl]-5-methyl-7-propyl-3H-imidazo[5,1-f][1,2,4]triazin-4-on Hydrochlorid

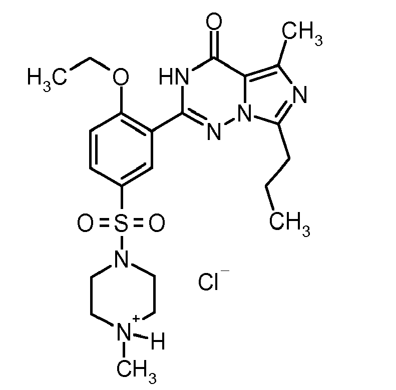

100 mg (0,211 mmol) 2-[2-Ethoxy-5-(4-methyl-piperazin-1-sulfonyl)-phenyl]-5-methyl-7-propyl-3*H*-imidazo[5,1-f][1,2,4]triazin-4-on werden in 5 ml Diethylether suspendiert, mit 0,23 ml einer 1M Lösung von HCl in Ether versetzt und 15 Minuten bei Raumtemperatur gerührt. Das Lösungsmittel wird im Vakuum entfernt. Man erhält 107 mg (97 %) 2-[2-Ethoxy-5-(4-methyl-piperazin-1-sulfonyl)-phenyl]-5-methyl-7-propyl-3*H*-imidazo[5,1-f][1,2,4]triazin-4-on Hydrochlorid.

200 MHz ¹H-NMR (DMSO-d₆): 0.93, t, 3H; 1.35, t, 3H; 1.75, sex., 2H; 2.72, s, 3H; 2.86, m, 4H; 3.15, m, 2H; 3.45, m, 2H; 3.81, m, 2H; 4.25, quart., 2H; 7.45, d, 1H; 7.95, m, 2H; 11.39, s, 1H; 11.90, s, 1H.

### Beispiel 19

### 2-[2-Ethoxy-5-(4-ethyl-piperazin-1-sulfonyl)-phenyl]-5-methyl-7-propyl-3H-imidazo[5,1-f][1,2,4]triazin-4-on

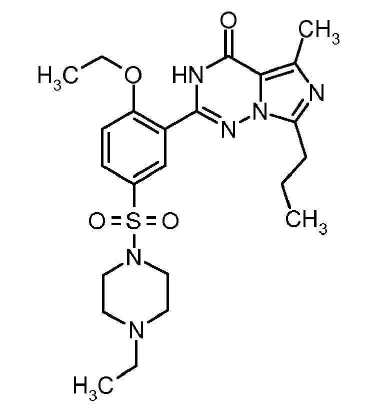

470 mg (1,14 mmol) 4-Ethoxy-3-(5-methyl-4-oxo-7-propyl-3,4-dihydro-imidazo[5,1-f][1,2,4]triazin-2-yl)-benzolsulfonsäurechlorid werden in 20 ml Dichlormethan gelöst und auf 0°C gekühlt. Es werden 390 mg (3,42 mmol) N-Ethylpiperazin zugegeben und die Reaktionsmischung über Nacht bei Raumtemperatur gerührt. Es wird mit Dichlormethan verdünnt, die organische Phase zweimal mit Wasser gewaschen, über Natriumsulfat getrocknet und das Lösungsmittel im Vakuum entfernt. Kristallisation aus Ether ergibt 370 mg (66 %) farblosen Feststoff.

400 MHz ¹H-NMR (CDCl₃): 1.01, t, 3H; 1.59, t, 3H; 1.88, hex, 2H; 2.42, quart., 2H; 2.56, m, 4H; 2.63, s, 3H; 3.00, t, 2H; 3.10, m, 4H; 4.33, quart., 2H, 7.17, d, ,1H; 7.88, dd, 1H; 8.44, d, 1H; 9.75, s, 1H.

### Beispiel 20

### 2-[2-Ethoxy-5-(4-ethyl-piperazin-1-sulfonyl)-phenyl]-5-methyl-7-propyl-3H-imidazo[5,1-f][1,2,4]triazin-4-on Hydrochlorid

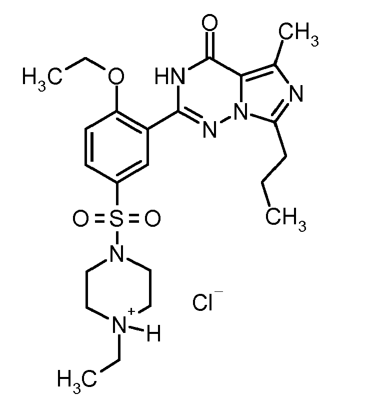

0,35 g (0,712 mmol) 2-[2-Ethoxy-5-(4-ethyl-piperazin-1-sulfonyl)-phenyl]-5-methyl-7-propyl-3*H*-imidazo[5,1-f][1,2,4]triazin-4-on werden in 8 ml Ether suspendiert und soviel Dichlormethan zugegeben, bis eine homogene Lösung entsteht. Man gibt 0,8 ml einer 1M Lösung von HCl in Ether zu, rührt 20 Minuten bei Raumtemperatur und saugt ab. Man erhält 372 mg (99 %) 2-[2-Ethoxy-5-(4-ethyl-piperazin-1-sulfonyl)-phenyl]-5-methyl-7-propyl-3*H*-imidazo[5,1-f][1,2,4]triazin-4-on Hydrochlorid.

200 MHz ¹H-NMR (DMSO-d₆):0.96, t, 3H; 1.22, t, 3H; 1.36, t, 3H; 1.82, sex., 2H; 2.61, s, 3H; 2.88, m, 2H; 3.08, m, 6H; 3.50, m, 2H; 3.70, m, 2H; 4.25, quart., 2H; 7.48, d, 1H; 7.95, m, 2H; 11.42, s, 1H; 12.45, s, 1H.

### Beispiel 21

### 2-[2-Ethoxy-5-(4-methyl-1-amino-piperazin-1-sulfonyl)-phenyl]-5-methyl-7-propyl-3H-imidazo[5,1-f][1,2,4]triazin-4-on

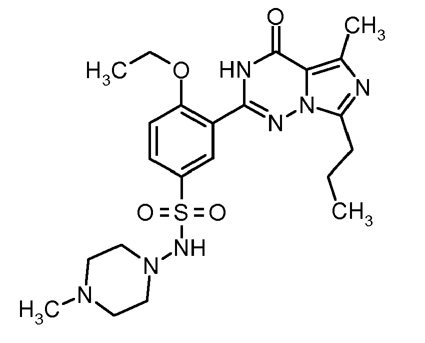

auf analoge Weise erhält man ausgehend von 0,04 g (0,097 mmol) 4-Ethoxy-3-(5-methyl-4-oxo-7-propyl-3,4-dihydro-imidazo[5,1-f][1,2,4]triazin-2-yl)-benzolsulfonsäurechlorid und 0,03 g (0,29 mmol) 1-Amino-4-methylpiperazin 40 mg (83 %) 2-[2-Ethoxy-5-(4-methyl-1-amino-piperazin-1-sulfonyl)-phenyl]-5-methyl-7-propyl-3*H-*imidazo[5,1-f][1,2,4]triazin-4-on.
R_{f}=0.09 (Dichlormethan/Methanol=19:1)

200 MHz ¹H-NMR (CDCl₃): 1.02, t, 3H; 1.59, t, 3H; 1.90, sex., 2H; 2.22, s, 3H; 2.40, m, 4H; 2.62, s, 3H; 2.71, m, 4H; 3.00, m, 2H; 4.32, quart., 2H; 7.14, d, 1H; 8.05, dd, 1H; 8.60, d, 1H.

### Beispiel 22

### 2-[2-Ethoxy-5-(4-hydroxyethyl-1-amino-piperazin-1-sulfonyl)-phenyl]-5-methyl-7-propyl-3H-imidazo[5,1-f][1,2,4]triazin-4-on

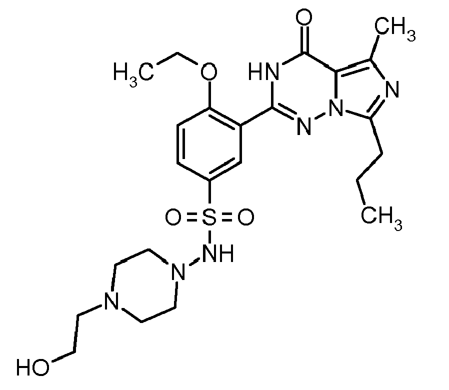

auf analoge Weise erhält man ausgehend von 0,04 g (0,097 mmol) 4-Ethoxy-3-(5-methyl-4-oxo-7-propyl-3,4-dihydro-imidazo[5,1-f][1,2,4]triazin-2-yl)-benzolsulfonsäurechlorid und 0,04 g (0,29 mmol) 1-Amino-4-hydroxyethylpiperazin 46 mg (91 %) 2-[2-Ethoxy-5-(4-hydroxyethyl-1-amino-piperazin-1-sulfonyl)-phenyl]-5-methyl-7-propyl-3*H*-imidazo[5,1-f][1,2,4]triazin-4-on.
R_{f}=0.08 (Dichlormethan/Methanol=19:1)

200 MHz ¹H-NMR (CDCl₃): 1.02, t, 3H; 1.59, t, 3H; 1.90, sex., 2H; 2.49, m, 6H; 2.62, s, 3H; 2.71, m, 4H; 3.00, t, 2H; 3.55, t, 2H; 4.31, quart., 2H; 7.14, d, 1H; 8.05, dd, 1H; 8.60, d, 1H.

### Beispiel 23

### N,N-Bishydroxyethylaminoethyl-4-ethoxy-3-(5-methyl-4-oxo-7-propyl-3,4-dihydroimidazo[5,1-f][1,2,4]triazin-2-yl)benzolsulfonamid

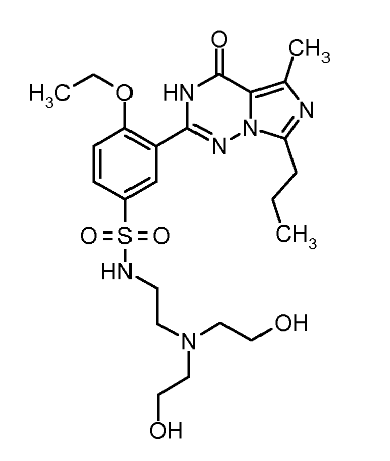

auf analoge Weise erhält man ausgehend von 0,04 g (0,097 mmol) 4-Ethoxy-3-(5-methyl-4-oxo-7-propyl-3,4-dihydro-imidazo[5,1-f][1,2,4]triazin-2-yl)-benzolsulfonsäurechlorid und 0,043 g (0,29 mmol) N,N-Bishydroxyethylamino-ethylamin 46 mg (91 %) N , N-Bishydroxyethylaminoethyl-4-ethoxy-3-(5-methyl-4-oxo-7-propyl-3,4-dihydro-imidazo[5,1-f][1,2,4]triazin-2-yl)benzolsulfonamid.

200 MHz ¹H-NMR (CDCl₃): 1.02, t, 3H; 1.53, t, 3H; 1.70, m, 2H; 1.86, sex., 2H; 2.9, m, 9H; 2.95, t, 2H; 3.09, t, 2H; 3.65, t, 4H; 4.28, quart., 2H; 7.14, d, 1H; 7.95, dd, 1H; 8.35, d, 1H.

### Beispiel 24

### 2-[2-Ethoxy-5-(4-dimethoxyphosphorylmethyl-piperazin-1-sulfonyl)-phenyl]-5-methyl-7-propyl-3H-imidazo[5,1-f][1,2,4]triazin-4-on

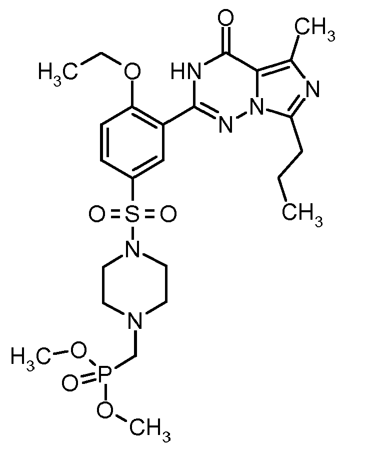

auf analoge Weise erhält man ausgehend von 0,4 g (0,97 mmol) 4-Ethoxy-3-(5-methyl-4-oxo-7-propyl-3,4-dihydro-imidazo[5,1-f][1,2,4]triazin-2-yl)-benzolsulfonsäurechlorid, 390 mg Triethylamin und 0,86 g (2,99 mmol) 4-Dimethoxyphosphorylmethyl-piperazin Trifluoracetat 321 mg (5 3 %) 2-[2-Ethoxy-5-(4-dimethoxyphosphorylmethyl-piperazin-1-sulfonyl)-phenyl]-5-methyl-7-propyl-3*H*-imidazo[5,1-f][1,2,4]triazin-4-on
R_{f}=0.4 (Dichlormethan/Methanol=20: 1)

200 MHz ¹H-NMR (CDCl₃): 1.02, t, 3H; 1.60, t, 3H; 1.88, sex., 2H; 2.62, s, 3H; 2.75, m, 4H; 3.02, t, 2H; 3.11, m, 4H; 3.70, s, 3H; 3.75, s, 3H; 4.35, quart., 2H; 5.30, s, 2H; 7.18, d, 1H; 7.88, dd, 1H; 8.45, d, 1H; 9.71, s, 1H.

### Beispiel 25

### 2-[2-Ethoxy-5-(4-diethoxyphosphorylmethyl-piperidin-1-sulfonyl)-phenyl]-5-methyl-7-propyl-3H-imidazo[5,1-f][1,2,4]triazin-4-on

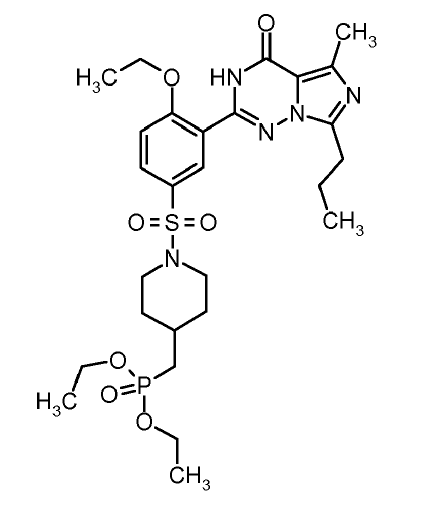

auf analoge Weise erhält man ausgehend von 0,4 g (0,97 mmol) 4-Ethoxy-3-(5-methyl-4-oxo-7-propyl-3,4-dihydro-imidazo[5,1-f][1,2,4]triazin-2-yl)-benzolsulfonsäurechlorid und 0,86 g (3,7 mmol) 4-Diethoxyphosphorylmethyl-piperidin 366 mg (49 %) 2-[2-Ethoxy-5-(4-diethoxyphosphorylmethyl-piperidin-1-sulfonyl)-phenyl]-5-methyl-7-propyl-3*H*-imidazo[5,1-f][1,2,4]triazin-4-on
R_{f}=0.4 (Dichlormethan/Methanol=20: 1)

200 MHz ¹H-NMR (DMSO-d₆): 0.92, t, 3H; 1.20, t, 6H; 1.35, t, 3H; 1.75, m, 7H; 2.25, m, 2H; 2.82, t, 2H; 3.61, d, 2H; 3.95, quin., 4H; 4.21, quart., 2H; 7.38, d, 1H; 7.87, m, 2H; 11.70, s, 1H.

### Beispiel 26

### 2-[2-Ethoxy-5-(4-hydroxy-piperidin-1-sulfonyl)-phenyl]-5-methyl-7-propyl-3H-imidazo[5,1-f][1,2,4]triazin-4-on

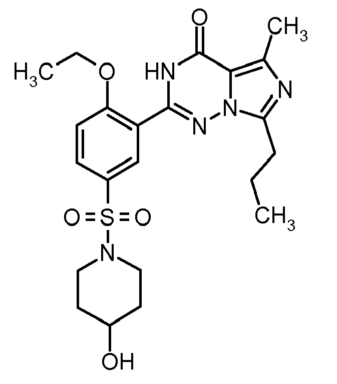

auf analoge Weise erhält man ausgehend von 531 mg (1,29 mmol) 4-Ethoxy-3-(5-methyl-4-oxo-7-propyl-3,4-dihydro-imidazo[5,1-f][1,2,4]triazin-2-yl)-benzolsulfonsäurechlorid und 393 mg (3,88 mmol) 4-Hydroxypiperidin 400 mg (64 %) 2-[2-Ethoxy-5-(4-hydroxy-piperidin-1-sulfonyl)-phenyl]-5-methyl-7-propyl-3*H*-imidazo[5,1-f][1,2,4]triazin-4-on

200 MHz ¹H-NMR (DMSO-d6): 0.941, t, 3H; 1.32, t, 3H; 1.45, m, 2H; 1.71, m, 4H; 2.48, s, 3H; 2.82, m, 4H; 3.11,m, 2H; 3.55, m, 1H; 4.20, quart., 2H; 4.72, d, 1H, 7.39, d,1H; 7.87, m, 2H; 11.70, s, 1H.

### Beispiel 27

### 2-{2-Ethoxy-5-[4-(2-hydroxy-ethyl)-piperazin-1-sulfonyl]-phenyl}-5-methyl-7-propyl-3H-imidazo[5,1-f][1,2,4]triazin-4-on

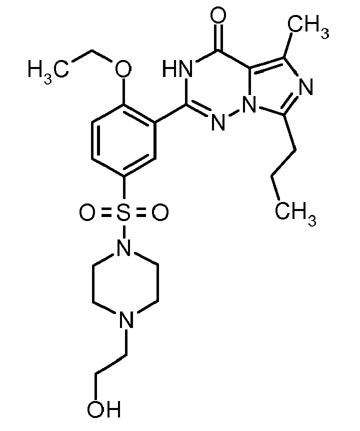

auf analoge Weise erhält man ausgehend von 411 mg (1 mmol) 4-Ethoxy-3-(5-methyl-4-oxo-7-propyl-3,4-dihydro-imidazo[5,1-f][1,2,4]triazin-2-yl)-benzolsulfonsäurechlorid und 391 mg (3 mmol) 4-Hydroxyethylpiperazin 380 mg (75 %) 2-{2-Ethoxy-5-[4-(2-hydroxy-ethyl)-piperazin-1-sulfonyl]-phenyl}-5-methyl-7-propyl-3*H-*imidazo[5,1-f][1,2,4]triazin-4-on
R_{f}=0.198 (Dichlormethan/Methanol=95:5)

200 MHz ¹H-NMR (CDCl₃):1.02, t, 3H; 1.61, t, 3H; 1.87, hex., 3H; 2.60, m, 7H; 3.00, t, 2H; 3.10, m, 4H; 3.60, t, 2H; 4.36, quart., 2H; 7.18, d, 1H, 7.89, dd, 1H, 8.47, d, 1H, 9.71, s, 1H.

### Beispiel 28

### 2-{2-Ethoxy-5-[4-(2-hydroxy-ethyl)-piperazin-1-sulfonyl]-phenyl}-5-methyl-7-propyl-3H-imidazo[5,1-f][1,2,4]triazin-4-on Hydrochlorid

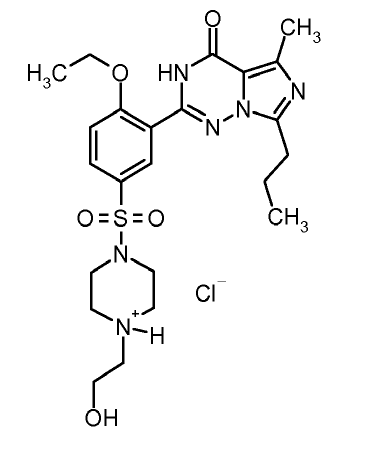

200 m g (0,39 mmol) 2-{2-Ethoxy-5-[4-(2-hydroxy-ethyl)-piperazin-1-sulfonyl]-phenyl}-5-methyl-7-propyl-3*H*-imidazo[5,1-f][1,2,4]triazin-4-on werden in Ether suspendiert, mit 2 ml einer 1M Lösung von HCl in Ether versetzt und 20 Minuten bei Raumtemperatur gerührt. Nach Entfernen des Lösungsmittels erhält man 209 mg (100 %) 2-{2-Ethoxy-5-[4-(2-hydroxy-ethyl)-piperazin-1-sulfonyl]-phenyl}-5-methyl-7-propyl-3*H*-imidazo[5,1-f][1,2,4]triazin-4-on Hydrochlorid.

200 MHz ¹H-NMR (DMSO-d6): 0.96, t, 3H; 1.35, t, 3H; 1.70, sex., 2H; 2.59, s, 3H; 2.85, t, 2H; 2.99, t, 2H; 3.18, m, 4H; 3.59, d, 2H; 3.75, m, 4H; 4.25, quart., 2H; 7.49, d, 1H; 7.95, m, 2H; 10.62, s, 1H; 12.31, s, 1H.

### Beispiel 29

### 2-{2-Ethoxy-5-[4-(3-hydroxy-propyl)-piperazin-1-sulfonyl]-phenyl}-5-methyl-7-propyl-3H-imidazo[5,1-f][1,2,4]triazin-4-on

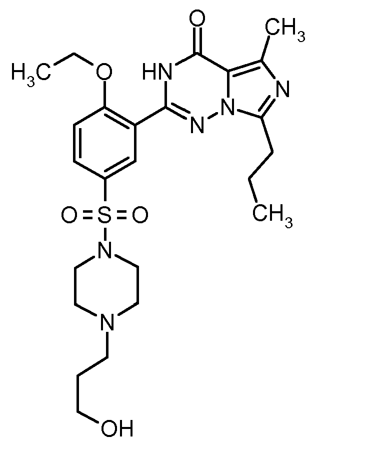

auf analoge Weise erhält man ausgehend von 150 mg (0,37 mmol) 4-Ethoxy-3-(5-methyl-4-oxo-7-propyl-3,4-dihydro-imidazo[5,1-f][1,2,4]triazin-2-yl)-benzolsulfonsäurechlorid und 158 mg (1,09 mmol) 4-(3-Hydroxypropyl)-piperazin 167 mg (83 %) 2-{2-Ethoxy-5-[4-(3-hydroxy-propyl)-piperazin-1-sulfonyl]-phenyl}-5-methyl-7-propyl-3*H*-imidazo[5,1-f][1,2,4]triazin-4-on
R_{f}=0.52 (Dichlormethan/Methanol=10: 1)

200 MHz ¹H-NMR (CDCl₃):1.02, t, 3H; 1.61, t, 3H; 1.70, m, 5; 2.62 m, 8H; 3.00, t, 2H; 3.10, m, 4H; 3.72, t, 2H; 4.36, quart., 2H; 7.18, d, 1H, 7.89, dd, 1H, 8.47, d, 1H, 9.71, s, 1H.

### Beispiel 30

### N-Allyl-4-ethoxy-N-(2-hydroxy-ethyl)-3-(5-methyl-4-oxo-7-propyl-3,4-dihydroimidazo[5,1-f][1,2,4]triazin-2-yl)benzolsulfonamid

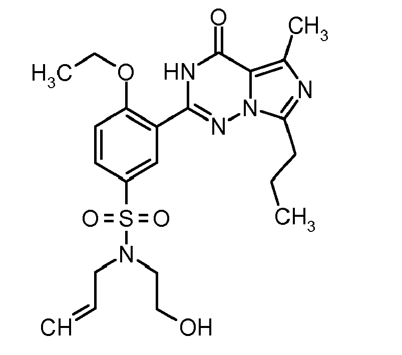

auf analoge Weise erhält man ausgehend von 420 mg (1,02 mmol) (1 mmol) 4-Ethoxy-3-(5-methyl-4-oxo-7-propyl-3,4-dihydro-imidazo[5,1-f][1,2,4]triazin-2-yl)-benzolsulfonsäurechlorid und 300 mg (3 mmol) Allylhydroxyethylamin 400 mg (82 %) N-Allyl-4-ethoxy-N-(2-hydroxy-ethyl)-3-(5-methyl-4-oxo-7-propyl-3,4-dihydro-imidazo[5,1-f][1,2,4]triazin-2-yl)benzolsulfonamid
R_{f}=0.345 (Dichlormethan/Methanol=95:5)

200 MHz ¹H-NMR (CDCl₃):1.02, t, 3H; 1.61, t, 3H; 1.90, m, 2H; 2.22, s, breit, 1H; 2.62, s, 3H; 2.99, t, 2H; 3.31, t, 2H; 3.78, t, 2H; 3.92, d, 2H; 4.37, quart., 2H; 5.23, m, 2H; 5.71, m, 1H; 7.15, d, 1H; 7.98, dd, 1H; 8.56, d, 1H; 9.66, s, 1H.

### Beispiel 31

### N-Ethyl-4-ethoxy-N-(2-hydroxy-ethyl)-3-(5-methyl-4-oxo-7-propyl-3,4-dihydroimidazo[5,1-f][1,2,4]triazin-2-yl)benzolsulfonamid

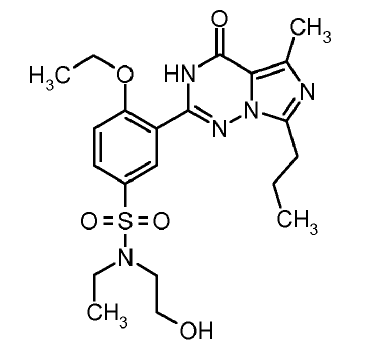

auf analoge Weise erhält man ausgehend von 411 mg (1,0 mmol) 4-Ethoxy-3-(5-methyl-4-oxo-7-propyl-3,4-dihydro-imidazo[5,1-f][1,2,4]triazin-2-yl)-benzolsulfonsäurechlorid und 267 mg (3 mmol) Ethylhydroxyethylamin 325 mg (70 %) N-Ethyl-4-ethoxy-N-(2-hydroxy-ethyl)-3-(5-methyl-4-oxo-7-propyl-3,4-dihydro-imidazo[5,1-f][1,2,4]triazin-2-yl)benzolsulfonamid
R_{f}=0.29 (Dichlormethan/Methanol=95:5)

200 MHz ¹H-NMR (CDCl₃):1.02, t, 3H; 1.20, t, 3H; 1.61, t, 3H; 1.88, sex., 2H; 2.30, s, breit, 1H; 2.62, s, 3H; 2.99, t, 2H; 3.32, m, 4H; 3.78, t, 2H; 3.80, m, 2H; 4.37, quart., 2H; 7.15, d, 1H; 7.98, dd, 1H; 8.56, d, 1H; 9.70, s, 1H.

### Beispiel 32

### N,N-Diethyl-4-ethoxy-3-(5-methyl-4-oxo-7-propyl-3,4-dihydro-imidazo[5,1-f][1,2,4]triazin-2-yl)benzolsulfonamid

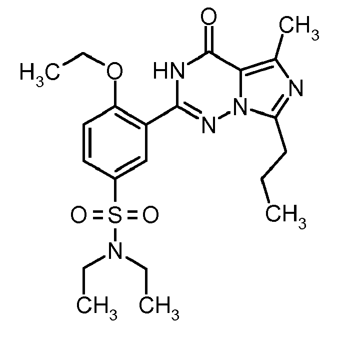

auf analoge Weise erhält man ausgehend von 400 mg (0,97 mmol) 4-Ethoxy-3-(5-methyl-4-oxo-7-propyl-3,4-dihydro-imidazo[5,1-f][1,2,4]triazin-2-yl)-benzolsulfonsäurechlorid und 210 mg (2,92 mmol) Diethylamin 398 mg (89 %) N,N-Diethyl-4-ethoxy-3-(5-methyl-4-oxo-7-propyl-3,4-dihydro-imidazo[5,1-f][1,2,4]triazin-2-yl)benzolsulfonamid
R_{f}=0.49 (Dichlormethan/Methanol=20: 1)

200 MHz ¹H-NMR (CDCl₃):1.02, t, 3H; 1.20, t, 6H; 1.49, t, 1.61, t, 3H; 1.88, sex., 2H; 2.30, s, breit, 1H; 2.62, s, 3H; 2.99, t, 2H; 3.32, m, 4H; 3.78, t, 2H; 3.80, m, 2H; 4.37, quart., 2H; 7.15, d, 1H; 7.98, dd, 1H; 8.56, d, 1H; 9.70, s, 1H.

### Beispiel 33

### N-(2-methoxyethyl)-3-(5-methyl-4-oxo-7-propyl-3,4-dihydro-imidazo[5,1-f][1,2,4]triazin-2-yl)-4-ethoxy-benzolsulfonsäureamid

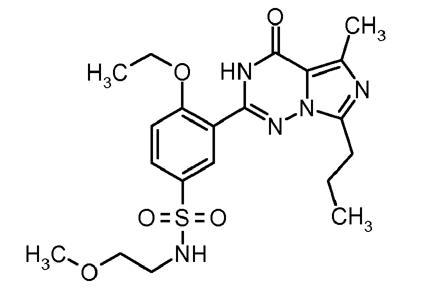

auf analoge Weise erhält man ausgehend von 1,23 g (3 mmol) 4-Ethoxy-3-(5-methyl-4-oxo-7-propyl-3,4-dihydro-imidazo[5,1-f][1,2,4]triazin-2-yl)-benzolsulfonsäurechlorid und 680 mg (9 mmol) 2-Methoxyethylamin 900 mg (67 %) N-(2-methoxyethyl)-3-(5-methyl-4-oxo-7-propyl-3,4-dihydro-imidazo[5,1-f][1,2,4]triazin-2-yl)-4-ethoxy-benzolsulfonsäureamid
R_{f}=0.25 (Dichlormethan/Methanol=95:5)

400 MHz ¹H-NMR (CDCl₃): 1.01, t, 3H, 1.58, t, 3H; 1.88, sex., 2H; 2.62, s, 3H; 3.01, t, 2H; 3.18, quart., 2H; 3.30, s, 3H; 3.45, t, 2H; 4.32, quart., 2H; 5.12, t, 1H; 7.13, d, 1H, 7.97, dd, 1H, 8.53, d, 1H; 9.82, s, 1H.

### Beispiel 34

### N-(2-N,N-dimethylethyl)-3-(5-methyl-4-oxo-7-propyl-3,4-dihydro-imidazo[5,1-f][1,2,4]triazin-2-yl)-4-ethoxy-benzolsulfonsäureamid

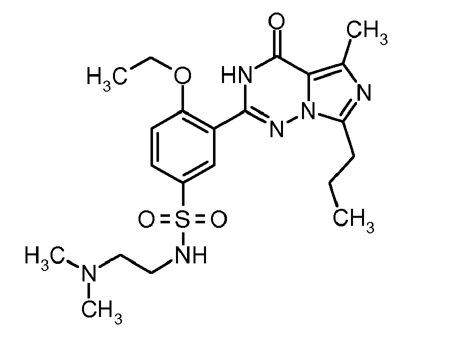

auf analoge Weise erhält man ausgehend von 210 mg (0,49 mmol) 4-Ethoxy-3-(5-methyl-4-oxo-7-propyl-3,4-dihydro-imidazo[5,1-f][1,2,4]triazin-2-yl)-benzolsulfonsäurechlorid und 130 mg (9 mmol) 2-N,N-Dimethylethylamin 150 mg (59 %) N-(2-N,N-dimethylethyl)-3-(5-methyl-4-oxo-7-propyl-3,4-dihydro-imidazo[5,1-f][1,2,4]-triazin-2-yl)-4-ethoxy-benzolsulfonsäureamid

200 MHz ¹H-NMR (CDCl₃): 1.01, t, 3H, 1.62, m, 4H; 1.88, sex., 2H; 2.11, s, 6H; 2.39, t, 2H; 2.63, s, 3H; 3.01, m, 3H; 4.38, quart., 2H; 7.13, d, 1H, 7.97, dd, 1H, 8.53, d, 1H; 9.82, s, 1H.

### Beispiel 35

### N-[3-(1-morpholino)propyl]-3-(5-methyl-4-oxo-7-propyl-3,4-dihydro-imidazo[5,1-f][1,2,4]triazin-2-yl)-4-ethoxy-benzolsulfonsäureamid

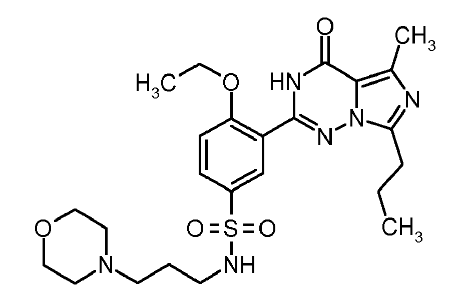

auf analoge Weise erhält man ausgehend von 1,23 g (3 mmol) 4-Ethoxy-3-(5-methyl-4-oxo-7-propyl-3,4-dihydro-imidazo[5,1-f][1,2,4]triazin-2-yl)-benzolsulfonsäurechlorid und 1,3 g (9 mmol) 3-(1-Morpholino)-propylamin 1,38 g (88 %) N-[3-(1-morpholino)propyl]-3-(5-methyl-4-oxo-7-propyl-3,4-dihydro-imidazo[5,1-f][1,2,4]-triazin-2-yl)-4-ethoxy-benzolsulfonsäureamid
R_{f}=0.23 (Dichlormethan/Methanol=95:5)

200 MHz ¹H-NMR (CDCl₃): 1.01, t, 3H, 1.58, t, 3H; 1.72, m, 2H; 1.88, sex., 2H; 2.46, m, 6H; 2.62, s, 3H; 3.01, t, 2H; 3.15, t, 2H; 3.71, t, 4H; 4.32, quart., 2H; 7.13, d, 1H, 7.97, dd, 1H, 8.53, d, 1H; 9.79, s, 1H.

### Beispiel 36

### N-{3-[1-(4-methyl)piperazino]-propyl}-3-(5-methyl-4-oxo-7-propyl-3,4-dihydroimidazo[5,1-f][1,2,4]triazin-2-yl)-4-ethoxy-benzolsulfonsäureamid

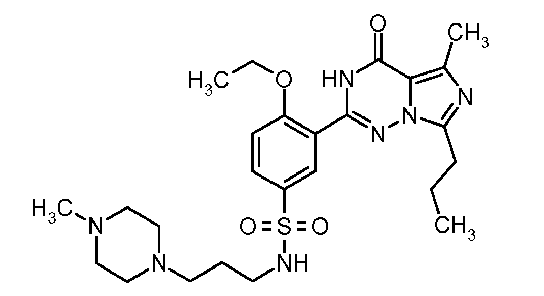

auf analoge Weise erhält man ausgehend von 0,04 g (0,097 mmol) 4-Ethoxy-3-(5-methyl-4-oxo-7-propyl-3,4-dihydro-imidazo[5,1-f][1,2,4]triazin-2-yl)-benzolsulfonsäurechlorid und 0,05 g (0,29 mmol) 3-[1-(4-Methyl-)piperazino]-propylamin 0,04 g (77 % ) N-{3-[1-(4-methyl)piperazino]-propyl}-3-(5-methyl-4-oxo-7-propyl-3,4-dihydro-imidazo[5,1-f][1,2,4]triazin-2-yl)-4-ethoxy-benzolsulfonsäureamid
R_{f}=0.11 (Dichlormethan/Methanol=95:5)

200 MHz ¹H-NMR (CDCl₃): 1.01, t, 3H, 1.55, t, 3H;1.68, m, 2H; 1.88, sex., 2H; 2.27, s, 3H; 2.45, m, 8H; 2.62, s, 3H; 2.98, m, 3H; 3.10, t, 2H; 3.46, s, 1H; 4.30, quart., 2H; 7.13, d, 1H, 7.97, dd, 1H, 8.53, d, 1H.

### Beispiel 37

### 2-{2-Ethoxy-5-[4-(2-methoxy-ethyl)-piperazin-1-sulfonyl]-phenyl}-5-methyl-7-propyl-3H-imidazo[5,1-f][1,2,4]triazin-4-on

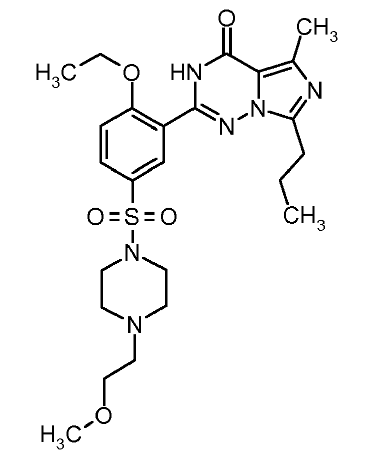

auf analoge Weise erhält man ausgehend von 40 mg (0,097 mmol) 4-Ethoxy-3-(5-methyl-4-oxo-7-propyl-3,4-dihydro-imidazo[5,1-f][1,2,4]triazin-2-yl)-benzolsulfonsäurechlorid und 40 mg (0,29 mmol) 4-Methoxyethylpiperazin 50mg (99%) 2-{2-Ethoxy-5-[4-(2-methoxy-ethyl)-piperazin-1-sulfonyl]-phenyl}-5-methyl-7-propyl-3*H-*imidazo[5,1-f][1,2,4]triazin-4-on
R_{f}=0.27 (Dichlormethan/Methanol=95:5)

200 MHz ¹H-NMR (CDCl₃):1.02, t, 3H; 1.61, t, 3H; 1.87, hex., 3H; 2.60, m, 9H; 2.97, t, 2H; 3.10, m, 4H; 3.60, s, 3H; 3.46, t, 2H; 4.36, quart., 2H; 7.18, d, 1H, 7.89, dd, 1H, 8.47, d, 1H, 9.71, s, 1H.

### Beispiel 38

### 2-{2-Ethoxy-5-[4-(2-N,N-dimethyl-ethyl)-piperazin-1-sulfonyl]-phenyl}-5-methyl-7-propyl-3H-imidazo[5,1-f][1,2,4]triazin-4-on

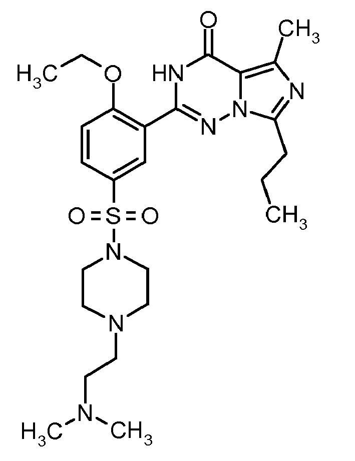

auf analoge Weise erhält man ausgehend von 40 mg (0,097 mmol) 4-Ethoxy-3-(5-methyl-4-oxo-7-propyl-3,4-dihydro-imidazo[5,1-f][1,2,4]triazin-2-yl)-benzolsulfonsäurechlorid und 50 mg (0,29 mmol) 4-(2-N,N-dimethyl)-ethylpiperazin 50 mg (99 % ) 2-{2-Ethoxy-5-[4-(2-N,N-dimethyl-ethyl)-piperazin-1-sulfonyl]-phenyl}-5-methyl-7-propyl-3*H*-imidazo[5,1-f][1,2,4]triazin-4-on
R_{f}=0.11 (Dichlormethan/Methanol=95:5)

200 MHz ¹H-NMR (CDCl₃):1.02, t, 3H; 1.61, t, 3H; 1.87, hex., 3H; 2.20, s, 6H; 2.42, m, 4H; 2.58, m, 4H; 2.63, s, 3H; 2.99, m, 3H; 3.10, m, 4H; 4.36, quart., 2H; 7.18, d, 1H, 7.89, dd, 1H, 8.47, d, 1H, 9.71, s, 1H.

### Beispiel 39

### 2-{2-Ethoxy-5-[4-(3-N,N-dimethyl-propyl)-piperazin-1-sulfonyl]-phenyl}-5-methyl-7-propyl-3H-imidazo[5,1-f][1,2,4]triazin-4-on

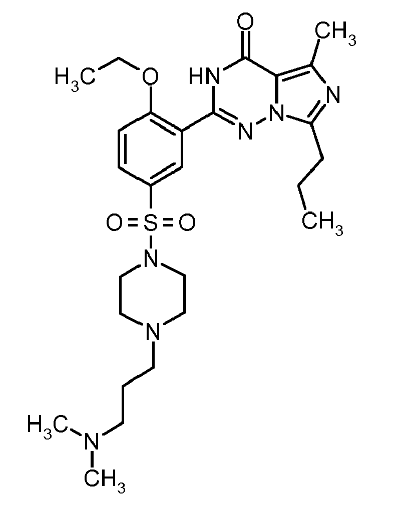

auf analoge Weise erhält man ausgehend von 100 mg (0,243 mmol) 4-Ethoxy-3-(5-methyl-4-oxo-7-propyl-3,4-dihydro-imidazo[5,1-f][1,2,4]triazin-2-yl)-benzolsulfonsäurechlorid und 130 mg (0,73 mmol) 4-(3-N,N-dimethyl)-propylpiperazin 72 mg (54 % ) 2-{2-Ethoxy-5-[4-(3-N,N-dimethyl-propyl)-piperazin-1-sulfonyl]-phenyl}-5-methyl-7-propyl-3*H*-imidazo[5,1-f][1,2,4]triazin-4-on
R_{f}=0.08 (Dichlormethan/Methanol=95:5)

200 MHz ¹H-NMR (CDCl₃):1.02, t, 3H; 1.61, t, 3H; 1.87, sex., 3H; 2.20, s, 6H; 2.25, m, 2H; 2.38, t, 2H; 2.52, m, 4H; 2.63, s, 3H; 2.99, m, 6H; 4.33, quart., 2H; 7.18, d, 1H, 7.89, dd, 1H, 8.47, d, 1H, 9.71, s, 1H.

### Beispiel 40

### 2-[2-Ethoxy-5-(4-dioxolano-piperidin-1-sulfonyl)-phenyl]-5-methyl-7-propyl-3H-imidazo[5,1-f][1,2,4]triazin-4-on

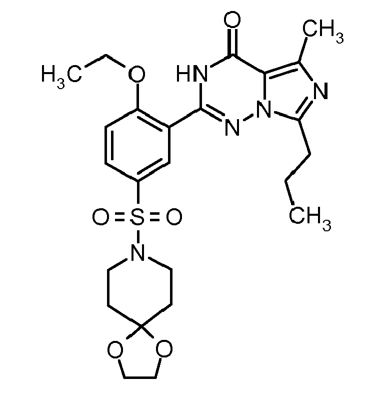

auf analoge Weise erhält man ausgehend von 100 mg (0,243 mmol) 4-Ethoxy-3-(5-methyl-4-oxo-7-propyl-3,4-dihydro-imidazo[5,1-f][1,2,4]triazin-2-yl)-benzolsulfonsäurechlorid und 100 mg (0,73 mmol) 4-Dioxolanopiperidin 111 mg (88 %) 2-[2-Ethoxy-5-(4-dioxolano-piperidin-1-sulfonyl)-phenyl]-5-methyl-7-propyl-3*H*-imidazo[5,1-f][1,2,4]triazin-4-on

200 MHz ¹H-NMR (CDCl₃):1.02, t, 3H; 1.61, t, 3H; 1.80, m, 6H; 2.63, s, 3H; 2.99, t, 2H; 3.20, m, 4H; 3.90, s, 4H; 4.33, quart., 2H; 7.18, d, 1H, 7.89, dd, 1H, 8.47, d, 1H, 9.71, s, 1H.

### Beispiel 41

### 2-[2-Ethoxy-5-(4-(5-methyl-4-furoxancarbonyl)-piperazin-1-sulfonyl)-phenyl]-5-methyl-7-propyl-3H-imidazo[5,1-f][1,2,4]triazin-4-on

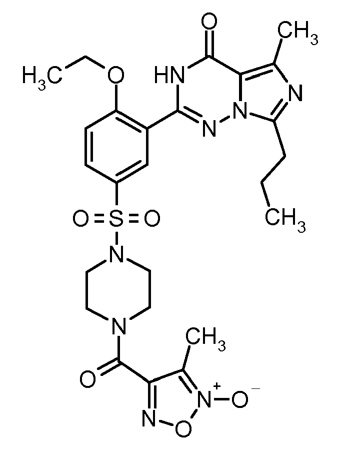

410 mg (1,0 mmol) 4-Ethoxy-3-(5-methyl-4-oxo-7-propyl-3,4-dihydro-imidazo[5,1-f][1,2,4]triazin-2-yl)-benzolsulfonsäurechlorid werden in 10 ml Dichlormethan gelöst und auf 0°C gekühlt. Es werden 590 mg (2,00 mmol) 1-(5-Methyl-4-furoxancarbonyl)-piperazin Trifluoracetat und 400 mg Triethylamin zugegeben und die Reaktionsmischung über Nacht bei Raumtemperatur gerührt. Es wird mit Dichlormethan verdünnt, die organische Phase mit Ammoniumchloridlösung, 1M Salzsäure und Wasser gewaschen, über Natriumsulfat getrocknet und das Lösungsmittel im Vakuum entfernt. Kristallisation aus Ether ergibt 448 mg (74 %) farblosen Feststoff.

200 MHz ¹H-NMR (CDCl₃): 1.01, t, 3H; 1.59, t, 3H; 1.88, hex, 2H; 2.25, s, 3H; 2.63, s, 3H; 3.00, t, 2H; 3.20, m, 4H; 3.90, m, 2H; 4.02, m, 2H; 4.33, quart., 2H, 7.19, d, 1H; 7.89, dd, 1H; 8.48, d, 1H; 9.57, s, 1H.

### Beispiel 42

### 2-{2-Ethoxy-5-[4-acetyl-piperazin-1-sulfonyl]-phenyl}-5-methyl-7-propyl-3H-imidazo[5,1-f][1,2,4]triazin-4-on

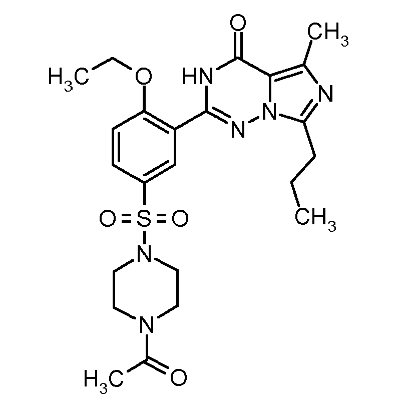

auf analoge Weise erhält man ausgehend von 40 mg (0,097 mmol) 4-Ethoxy-3-(5-methyl-4-oxo-7-propyl-3,4-dihydro-imidazo[5,1-f][1,2,4]triazin-2-yl)-benzolsulfonsäurechlorid und 40 mg (0,29 mmol) N-Acetylpiperazin 9 mg (18 %) 2-{2-Ethoxy-5-[4-acetyl-piperazin-1-sulfonyl]-phenyl}-5-methyl-7-propyl-3*H*-imidazo[5,1-f][1,2,4]-triazin-4-on
R_{f}=0.34 (Dichlormethan/Methanol=95:5)

200 MHz ¹H-NMR (CDCl₃):1.02, t, 3H; 1.61, t, 3H; 1.87, sex., 3H; 2.05, s, 3H; 2.63, s, 3H; 3.00, m, 6H; 3.59, m, 2H; 3.72, m, 2H; 4.33, quart., 2H; 7.18, d, 1H, 7.89, dd, 1H, 8.47, d, 1H, 9.71, s, 1H.

### Beispiel 43

### 2-{2-Ethoxy-5-[4-formyl-piperazin-1-sulfonyl]-phenyl}-5-methyl-7-propyl-3H-imidazo[5,1-f][1,2,4]triazin-4-on

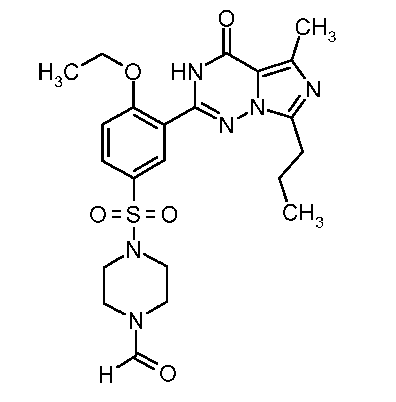

auf analoge Weise erhält man ausgehend von 40 mg (0,097 mmol) 4-Ethoxy-3-(5-methyl-4-oxo-7-propyl-3,4-dihydro-imidazo[5,1-f][1,2,4]triazin-2-yl)-benzolsulfonsäurechlorid und 30 mg (0,29 mmol) N-Formylpiperazin 35 mg (73 %) 2-{2-Ethoxy-5-[4-formyl-piperazin-1-sulfonyl]-phenyl}-5-methyl-7-propyl-3*H*-imidazo[5,1-f][1,2,4]triazin-4-on
R_{f}=0.29 (Dichlormethan/Methanol=95:5)

200 MHz ¹H-NMR (CDCl₃):1.02, t, 3H; 1.61, t, 3H; 1.87, sex., 3H; 2.05, s, 3H; 2.63, s, 3H; 3.00, m, 6H; 3.50, m, 2H; 3.69, m, 2H; 4.33, quart., 2H; 7.18, d, 1H, 7.89, dd, 1H; 8.00, s, 1H; 8.47, d, 1H, 9.71, s, 1H.

### Beispiel 44

### 2-[2-Ethoxy-5-(3-butylsydnonimin)-1-sulfonyl)-phenyl]-5-methyl-7-propyl-3H-imidazo[5,1-f][1,2,4]triazin-4-on

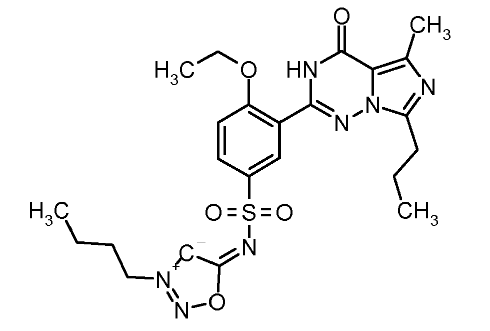

110 mg (0,6 mmol) 3-Butylsydnoniminhydrochorid werden in 2,5 ml Pyridin gelöst und auf 0°C gekühlt. Es werden 210 mg (0,5 mmol) 4-Ethoxy-3-(5-methyl-4-oxo-7-propyl-3,4-dihydro-imidazo[5,1-f][1,2,4]triazin-2-yl)-benzolsulfonsäurechlorid zugegeben und die Reaktionsmischung wird 2 Stunden bei 0°C und über Nacht bei Raumtemperatur gerührt. Es wird mit Dichlormethan verdünnt, die organische Phase mit Wasser gewaschen, über Natriumsulfat getrocknet und das Lösungsmittel im Vakuum entfernt. Durch Chromatographie (Dichlormethan/Methanol) erhält man 16 mg (6 %) 2-[2-Ethoxy-5-(3-butylsydnonimin)-1-sulfonyl)-phenyl]-5-methyl-7-propyl-3*H-*imidazo[5,1-f][1,2,4]triazin-4-on.
R_{f}=0.41 (Dichlormethan/Methanol=95:5)

200 MHz ¹H-NMR (CDCl₃): 1.01, 2t, 6H; 1.47, sex., 2H; 1.55, t, 3H; 1.88, m, 2H; 2.04, quin., 2H; 2.62, s, 3H; 2.98, t, 2H; 4.29, quart., 2H; 4.41, t, 2H; 7.08, d, 1H; 7.56, s, 1H; 7.98, dd, 1H; 8.58, d, 1H; 9.79, s, breit, 1H.

### Beispiel 45

### 5-Methyl-2-[5-(4-methyl-piperazin-1-sulfonyl)-2-propoxy-phenyl]-7-propyl-3H-imidazo[5,1-f][1,2,4]triazin-4-on

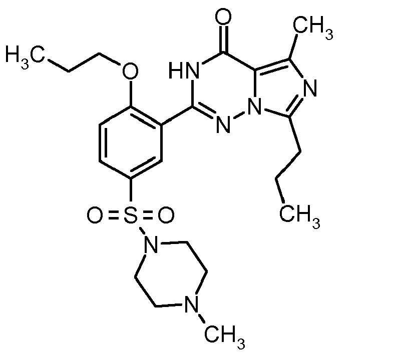

0,85 g (2 mmol) 4-Propoxy-3-(5-methyl-4-oxo-7-propyl-3,4-dihydro-imidazo[5,1-f][1,2,4]-triazin-2-yl)-benzolsulfonsäurechlorid werden in 20 ml Dichlormethan gelöst und auf 0°C gekühlt. Nach Zugabe einer Spatelspitze DMAP werden 0,60 g (6,00 mmol) N-Methylpiperazin zugegeben und die Reaktionsmischung über Nacht bei Raumtemperatur gerührt. Es wird mit Dichlormethan verdünnt, die organische Phase mit Ammoniumchloridlösung gewaschen, über Natriumsulfat getrocknet und das Lösungsmittel im Vakuum entfernt. Kristallisation aus Ether ergibt 0,80 g (77 %) farblosen Feststoff.
R_{f}=0.233 (Dichlormethan/Methanol=95:5)

200 MHz ¹H-NMR (CDCl₃): 1.00, t, 3H; 1.15, t, 3H; 1.87, hex, 2H; 1.99, hex., 2H; 2.30, s, 3H; 2.52, m, 4H; 2.62, s, 3H; 2.99, t, 2H; 3.10, m, 4H; 4.21, t, 2H; 7.17, d, 1H; 7.87, dd, 1h, 8.48, d, 1H, 9.70, s, 1H.

### Beispiel 46

### 5-Methyl-2-[5-(4-methyl-piperazin-1-sulfonyl)-2-propoxy-phenyl]-7-propyl-3H-imidazo[5,1-f][1,2,4]triazin-4-on Hydrochlorid

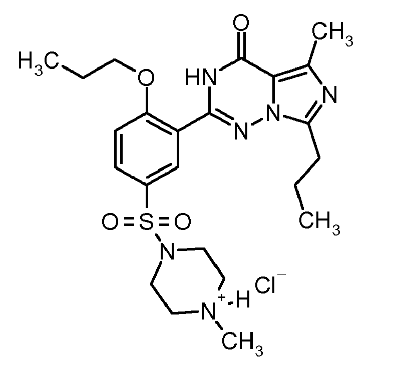

22 m g (0,045 mmol) 5-Methyl-2-[5-(4-methyl-piperazin-1-sulfonyl)-2-propoxy-phenyl]-7-propyl-3*H*-imidazo[5,1-f][1,2,4]triazin-4-on werden in 2 ml Ether und 1 ml Dichlormethan gelöst und mit 0,1 ml einer 1M Lösung von HCl in Ether versetzt. Der ausgefallene Niederschlag wird nach 20 Minuten abgesaugt und getrocknet.

200 MHz ¹H-NMR (CDCl₃): 0.95, t, 3H; 1.75, m, 2H; 2.56, s, 3H; 2.75, m, 4H; 2.97, t, 2H; 3.15, m, 2H; 3.44, m, 2H; 3.81, m, 2H; 4.15, t, 2H; 7.47, d, 1H; 7.95, m, 2H; 11.12, s, 1H; 12.22, s, 1H.

### Beispiel 47

### 2-[5-(4-Hydroxypiperidin-1-sulfonyl)-2-propoxy-phenyl]-5-methyl-7-propyl-3H-imidazo[5,1-f][1,2,4]triazin-4-on

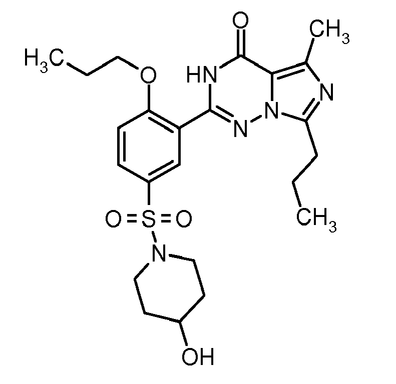

auf analoge Weise erhält man ausgehend von 850 mg (2 mmol) 4-Propoxy-3-(5-methyl-4-oxo-7-propyl-3,4-dihydro-imidazo[5,1-f][1,2,4]triazin-2-yl)-benzolsulfonsäurechlorid und 610 mg (6 mmol) 4-Hydroxypiperidin 736 mg (75 %) 2-[5-(4-Hydroxypiperidin-1-sulfonyl)-2-propoxy-phenyl]-5-methyl-7-propyl-3*H*-imidazo[5,1-f][1,2,4]triazin-4-on
R_{f}=0.07 (Dichlormethan/Methanol=95:5)

200 MHz ¹H-NMR (CDCl₃): 1.01, t, 3H; 1.16, t, 3H; 1.80, m, 9H; 2.65, s, 3H; 3.00, m, 4H; 3.32, m, 2H; 3.85,m, 1H; 4.22, t., 2H; 7.17, d,1H; 7.89, dd, 1H; 8.50, d, 1H; 11.70, s, 1H.

### Beispiel 48

### 2-[5-(4-Hydroxymethylpiperidin-1-sulfonyl)-2-propoxy-phenyl]-5-methyl-7-propyl-3H-imidazo[5,1-f][1,2,4]triazin-4-on

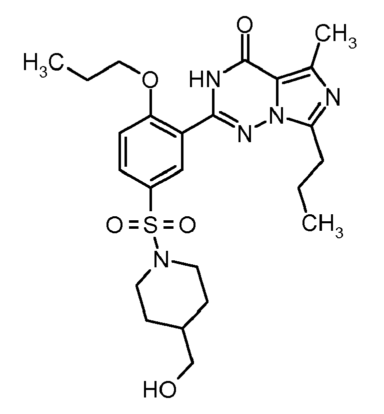

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 35 mg (0,3 mmol) 4-Hydroxymethylpiperidin 41 mg (82 %) 2-[5-(4-Hydroxymethylpiperidin-1-sulfonyl)-2-propoxy-phenyl]-5-methyl-7-propyl-3*H-*imidazo[5,1-f][1,2,4]triazin-4-on
R_{f}=0.52 (Dichlormethan/Methanol=9:1)

200 MHz ¹H-NMR (CDCl₃): 1.001, t, 3H; 1.16, t, 3H; 1.60, m, 4H; 1.82, m, 5H; 2.31, t, 2H, 2.62, s, 3H, 2.98, t, 2H,; 3.48, d, 2H; 3.85, d, 2H; 4.21, t, 2H; 7.,17, d, 1H; 7.88, dd, 1H, 8.45, d, 1H; 9. 71, s, 1H.

### Beispiel 49

### 2-{5-[4-(2-hydroxyethyl)-piperazin-1-sulfonyl]-2-propoxy-phenyl}-5-methyl-7-propyl-3H-imidazo[5,1-f][1,2,4]triazin-4-on

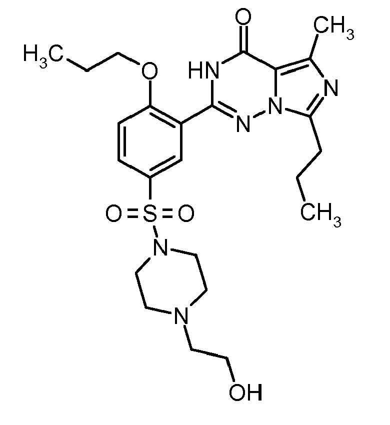

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 39 mg (0,3 mmol) 4-Hydroxyethylpiperazin 50 mg (96 %) 2-{5-[4-(2-hydroxyethyl)-piperazin-1-sulfonyl]-2-propoxy-phenyl}-5-methyl-7-propyl-3*H-*imidazo[5,1-f][1,2,4]triazin-4-on
R_{f}=0.43 (Dichlormethan/Methanol=9:1)

200 MHz ¹H-NMR (CDCl₃): 1.01, t, 3H; 1.15, t, 3H, 1.88, m, 2H, 2.00, m, 2H, 2.62, m, 9H, 3.00, t, 2H, 3.07, m, 4H, 3.58, t, 2H, 4.23, t, 2H; 7.19, d, 1H; 7.88, dd, 1H, 8.43, d, 1H, 9.85, s, 1H.

### Beispiel 50

### N-(1,1-Dioxotetrahydro-1λ⁶-thiophen-3-yl)-3-(5-methyl-4-oxo-7-propyl-3,4-dihydro-imidazo-[5,1-f][1,2,4]triazin-2-yl)-4-propoxy-benzolsulfonsäureamid

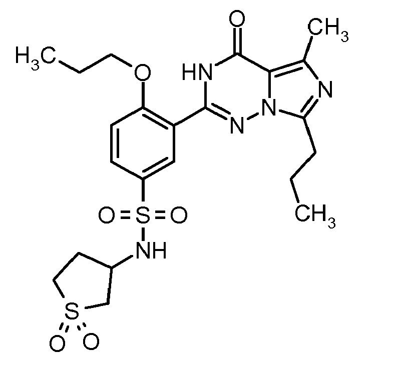

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 41 mg (0,3 mmol) 2-Aminosulfolan 8 mg (14 %) N-(1,1-Dioxo-tetrahydro-1λ⁶-thiophen-3-yl)-3-(5-methyl-4-oxo-7-propyl-3,4-dihydro-imidazo-[5,1-f][1,2,4]triazin-2-yl)-4-propoxy-benzolsulfonsäureamid
R_{f}=0.49 (Dichlormethan/Methanol=9: 1)

200 MHz ¹H-NMR (CDCl₃): 1.01, t, 3H, 1.15, t, 3H, 1.85, m, 2H; 1.99, m, 2H; 2.30, m, 1H; 2.50, m, 1H; 2.62, s, 3H; 2.95, m, 4H; 3.21, m, 1H; 4.20, m, 3H; 5.98, s, 1H; 7.18, d, 1H, 7.98, dd, 1H; 8.51,d, 1H, 9.71, s, 1H.

### Beispiel 51

### N-(2-Dimethylaminoethyl)-N-methyl-3-(5-methyl-4-oxo-7-propyl-3,4-dihydro-imidazo[5,1-f][1,2,4]triazin-2-yl)-4-propoxy-benzolsulfonsäureamid

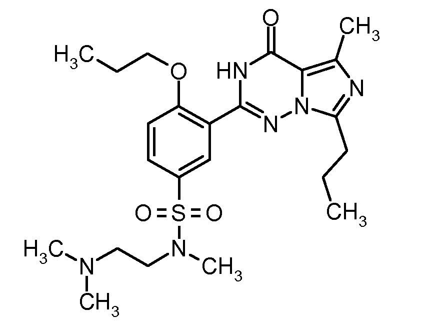

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 31 mg (0,3 mmol) 1,1,4-Trimethyldiaminoethan 39 mg (79 %) N-(2-Dimethylaminoethyl)-N-methyl-3-(5-methyl-4-oxo-7-propyl-3,4-dihydro-imidazo[5,1-f][1,2,4]triazin-2-yl)-4-propoxy-benzolsulfonsäureamid
R_{f}=0.28 (Dichlormethan/Methanol=9:1)

200 MHz ¹H-NMR (CDCl₃): 1.01, t, 3H, 1.15, t, 3H, 1.88, m, 2H; 2.01, m, 2H; 2.25, s, 6H; 2.50, t, 2H; 2.62, s, 3H; 2.82, s, 3H; 3.01, t, 2H; 3.18, t, 2H; 4.21, t, 2H; 7.16, d, 1H, 7.91, dd, 1H, 8.50, d, 1H; 9.70, s, 1H.

### Beispiel 52

### 3-(5-Methyl-4-oxo-7-propyl-3,4-dihydro-imidazo[5,1-f][1,2,4]triazin-2-yl)-N-(3-morpholin-4-yl-propyl)-4-propoxy-benzolsulfonsäureamid

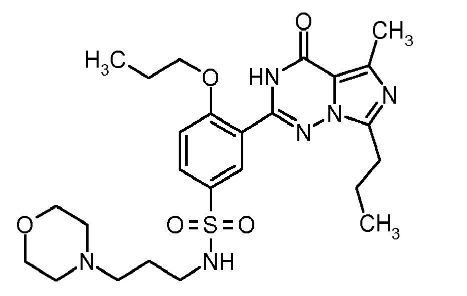

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 43 mg (0,3 mmol) 1-(3-Aminopropyl)-morpholin 52 mg (97 %) 3-(5-Methyl-4-oxo-7-propyl-3,4-dihydro-imidazo[5,1-f][1,2,4]triazin-2-yl)-N-(3-morpholin-4-yl-propyl)-4-propoxy-benzol-sulfonsäureamid
R_{f}=0.33 (Dichlormethan/Methanol=9:1)

200 MHz ¹H-NMR (CDCl₃): 1.01, t, 3H, 1.15, t, 3H, 1.71, m, 2H; 1.93, m, 4H; 2.43, m, 6H; 2.62, s, 3H; 2.98, t, 2H; 3.12, t, 2H; 3.70, m, 4H; 4.21, t, 2H; 7.15, d, 1H; 7.96, dd, 1H; 8.55, d, 1H; 9.85, s, 1H.

### Beispiel 53

### N,N-Bis-(2-hydroxyethyl)-3-(5-Methyl-4-oxo-7-propyl-3,4-dihydro-imidazo[5,1-f][1,2,4]triazin-2-yl)-4-propoxy-benzolsulfonsäureamid

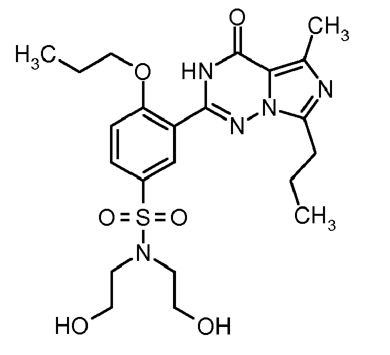

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 32 mg (0,3 mmol) Bishydroxyethylamin 34 mg (69 %) N,N-Bis-(2-hydroxyethyl)-3-(5-Methyl-4-oxo-7-propyl-3,4-dihydro-imidazo[5,1-f][1,2,4]triazin-2-yl)-4-propoxy-benzolsulfonsäureamid
R_{f}=0.36 (Dichlormethan/Methanol=9:1)

200 MHz ¹H-NMR (CDCl₃): 1.01, t, 3H; 1.15, t, 3H; 1.85, m, 2H; 1.97, m, 2H; 2.60, s, 3H; 2.98, t, 2H; 3.33, t, 4H; 3.87, t, 4H; 4.20, t, 2H; 7.15, d, 1H; 7.92, dd, 1H; 8.49, d, 1H; 9.85, s, 1H.

### Beispiel 54

### N-(3-Hydroxybenzyl)-3-(5-Methyl-4-oxo-7-propyl-3,4-dihydro-imidazo[5,1-f][1,2,4]triazin-2-yl)-4-propoxy-benzolsulfonsäureamid

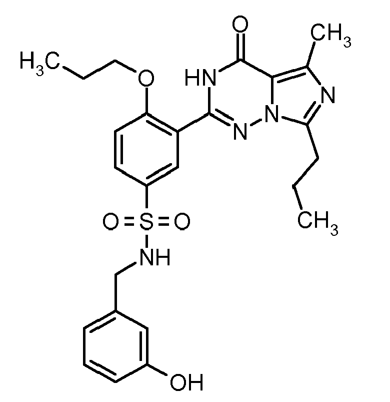

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 37 mg (0,3 mmo 1) 3-Hydroxybenzylamin 4 mg (8 %) N-(3-Hydroxybenzyl)-3-(5-Methyl-4-oxo-7-propyl-3,4-dihydro-imidazo[5,1-f][1,2,4]-triazin-2-yl)-4-propoxy-benzolsulfonsäureamid
R_{f}=0.43 (Dichlormethan/Methanol=9:1)

200 MHz ¹H-NMR (CDCl₃):1.01, t, 3H, 1.13, t, 3H; 1.83, m, 2H; 1.96, m, 2H; 2.59, s, 3H, 2.96, t, 2H, 4.16, m, 4H, 5.05, t, 1H; 6.52, s, 1H; 6.70, m, 2H; 7.06, m, 2H; 7.93, dd, 1H, 8.41, d, 1H, 9.77, s, 1H.

### Beispiel 55

### N-Ethyl-N-(2-hydroxyethyl)-3-(5-Methyl-4-oxo-7-propyl-3,4-dihydro-imidazo[5,1-f][1,2,4]triazin-2-yl)-4-propoxy-benzolsulfonsäureamid

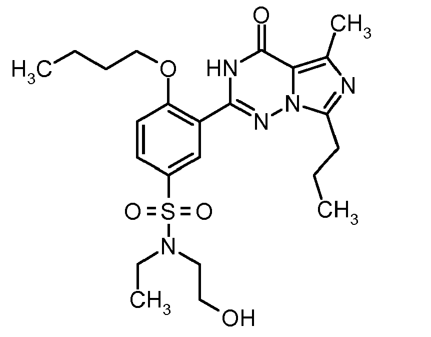

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 27 mg (0,3 mmol) Ethylhydroxyethylamin 18 mg (38 %) N-Ethyl-N-(2-hydroxyethyl)-3-(5-Methyl-4-oxo-7-propyl-3,4-dihydro-imidazo[5,1-f][1,2,4]-triazin-2-yl)-4-propoxy-benzolsulfonsäureamid
R_{f}=0.48 (Dichlormethan/Methanol=9:1)

200 MHz ¹H-NMR (CDCl₃):1.01, t, 3H; 1.15, 2t, 6H; 1.75, s, 2H; 1.85, m, 2H; 1.98, m, 2H; 2.40, s, 1H; 2.62, s, 3H; 2.99, t, 2H; 3.32, m, 4H; 3.90, quart., 2H, 4.21, quart., 2H; 7.15, d, 1H; 7.95, dd, 1H; 8.55, d, 1H, 9.73, s, 1H.

### Beispiel 56

### N-(3-Ethoxypropyl)-3-(5-Methyl-4-oxo-7-propyl-3,4-dihydro-imidazo[5,1-f][1,2,4]triazin-2-yl)-4-propoxy-benzolsulfonsäureamid

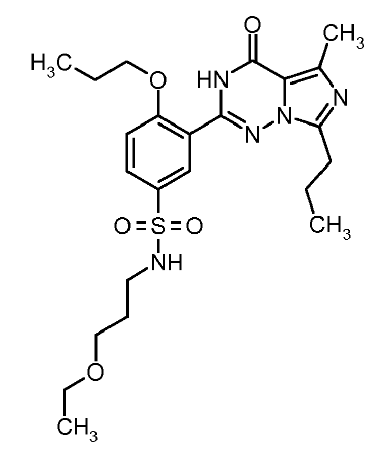

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 31 mg (0,3 mmol) 3-Ethoxypropylamin 47 mg (96 %) N-(3-Ethoxypropyl)-3-(5-Methyl-4-oxo-7-propyl-3,4-dihydro-imidazo[5,1-f][1,2,4]triazin-2-yl)-4-propoxy-benzolsulfonsäureamid
R_{f}=0.60 (Dichlormethan/Methanol=9:1)

200 MHz ¹H-NMR (CDCl₃): 1.01, t, 3H; 1.15, m, 6H; 1.89, m, 7H; 2.62, s, 3H; 3.00, t, 2H; 3.12, quart., 2H; 3.46, m, 4H; 4.20, t, 2H; 5.52, m, 1H; 7.15, d, 1H; 7.98, dd, 1H; 8.55, d, 1H, 9.85, s, 1H.

### Beispiel 57

### 2-[5(4-Hydroxypiperidin-1-sulfonyl)2-propoxy-phenyl]-5-methyl-7-propyl-3H-imidazo[5,1-f][1,2,4]triazin-4-on

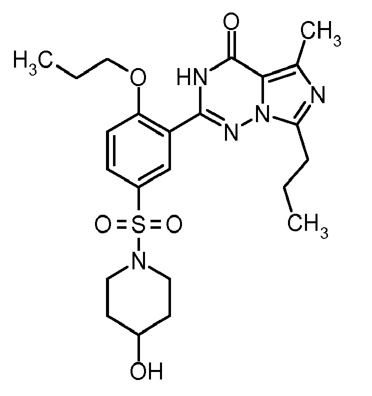

auf analoge Weise erhält man ausgehend von 212 mg (0,5 mmol) 4-Propoxy-3-(5-methyl-4-oxo-7-propyl-3,4-dihydro-imidazo[5,1-f][1,2,4]triazin-2-yl)-benzolsulfonsäurechlorid und 152 mg (1,5 mmol) 4-Hydroxypiperidin 125 mg (50 %) 2-[5(4-Hydroxypiperidin-1-sulfonyl)2-propoxy-phenyl]-5-methyl-7-propyl-3*H*-imidazo[5,1-*f*][1,2,4]triazin-4-on
R_{f}=0.07 (Dichlormethan/Methanol=19:1)

200 MHz ¹H-NMR (CDCl₃): 1.05, t, 3H; 1.18, t, 3H, 1.98, m, 8H, 2.71, s, 3H; 3.10, m, 2H; 3.28, m, 4H; 3.88, m, 1H; 4.28, t, 2H; 7.21, d, 1H; 7.97, dd, 1H, 8.45, d, 1H. 10.45, s, 1H.

### Beispiel 58

### 3-(5-Methyl-4-oxo-7-propyl-3,4-dihydro-imidazo[5,1-f][1,2,4]triazin-2-yl)-4-propoxy-N-pyridin-4-yl-benzolsulfonsäureamid

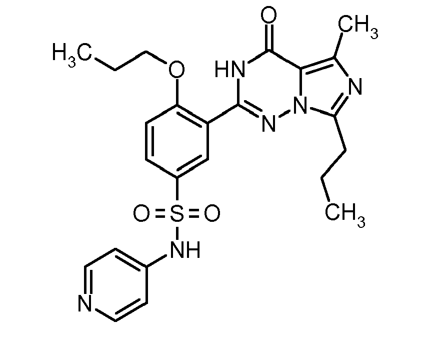

auf analoge Weise erhält man ausgehend von 85 mg (0,2 mmol) 4-Propoxy-3-(5-methyl-4-oxo-7-propyl-3,4-dihydro-imidazo[5,1-f][1,2,4]triazin-2-yl)-benzolsulfonsäurechlorid und 56 mg (0,6 mmol) 4-Aminopyridin nach 18 Stunden reflux in 1 ml THF 24 mg (25 %) 3-(5-Methyl-4-oxo-7-propyl-3,4-dihydro-imidazo[5,1-f][1,2,4]-triazin-2-yl)-4-propoxy-N-pyridin-4-yl-benzolsulfonsäureamid
R_{f}=0.13 (Dichlormethan/Methanol=9:1)

200 MHz ¹H-NMR (CDCl₃ + CD₃OD): 1.01, t, 3H; 1.09, t, 3H; 1.90, m, 4H; 2.60, s, 3H; 2.99, t, 2H; 4.16, t, 2H; 7.05, d, 2H; 7.15, d, 1H; 7.88, d, 2H; 8.05, dd, 1H; 8.41, d, 1H.

### Beispiel 59

### N,N-Diethyl-3-(5-methyl-4-oxo-7-propyl-3,4-dihydro-imidazo[5,1-f][1,2,4]triazin-2-yl)-4-propoxy-benzolsulfonsäureamid

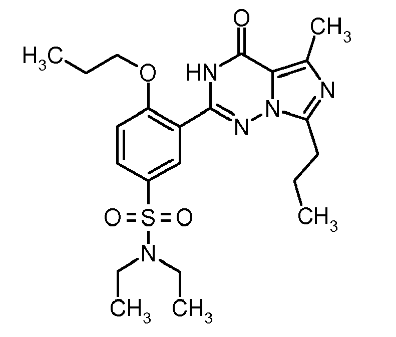

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 22 mg (0,6 mmol) Diethylamin 42 mg (92 %) N,N-Diethyl-3-(5-methyl-4-oxo-7-propyl-3,4-dihydro-imidazo[5,1-f][1,2,4]triazin-2-yl)-4-propoxy-benzolsulfonsäureamid.
R_{f}=0.64 (Dichlormethan/Methanol=9:1)

200 MHz ¹H-NMR (CDCl₃): 1.01, t, 3H; 1.18, 2t, 9H; 1.92, 2 hex., 4H; 2.62, s, 3H; 3.00, t, 2H, 3.29, quart., 4H; 4.21, t, 2H; 7.13, d, 1H; 7.93, dd, 1H, 8.51, d, 1H, 9.85, s, 1H.

### Beispiel 60

### 1-[3-(5-methyl-4-oxo-7-propyl-3,4-dihydro-imidazo[5,1-f][1,2,4]triazin-2-yl)-4-propoxy-benzolsulfonyl]-piperidin-4-carbonsäure

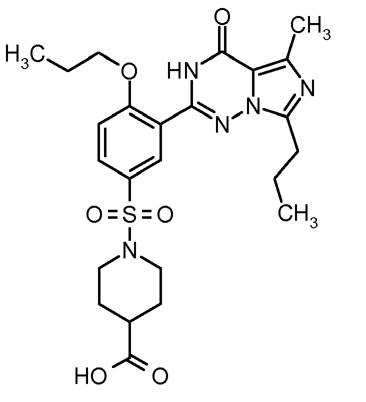

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 14 mg (0,6 mmol) Piperidincarbonsäure in 1 ml eines Gemisches aus THF und Wasser (1: 1) mit 26,5 mg Natriumcarbonat 21 mg (41 %) 1-[3-(5-methyl-4-oxo-7-propyl-3,4-dihydro-imidazo[5,1-f][1,2,4]triazin-2-yl)-4-propoxy-benzolsulfonyl]-piperidin-4-carbonsäure.
R_{f}=0.28 (Dichlormethan/Methanol=9:1)

200 MHz ¹H-NMR (CDCl₃): 0.90, t, 3H; 1.04, t, 3H; 1.80, m, 4H; 2.21, m, 2H, 2.51, s, 3H, 2.85, m, 2H, 3.56, m, 6H; 4.10, t, 2H; 7.12, d, 1H, 7.71, dd, 1H, 8.10, d, 1H, 10.72, s, breit, 1H.

### Beispiel 61

### 5-Methyl-2-[5-(morpholin-4-sulfonyl)-2-propoxy-phenyl]-7-propyl-3H-imidazo[5,1-f][1,2,4]triazin-4-on

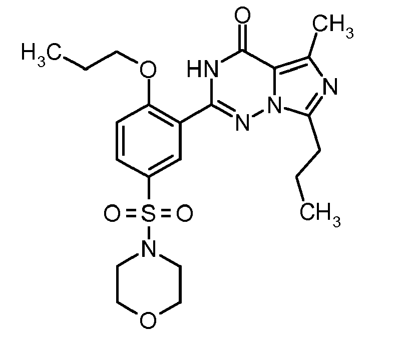

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 26 mg (0,3 mmol) Morpholin 34 mg (71 %) 5-Methyl-2-[5-(morpholin-4-sulfonyl)-2-propoxy-phenyl]-7-propyl-3*H*-imidazo[5,1-*f*][1,2,4]triazin-4-on.
R_{f}=0.64 (Dichlormethan/Methanol=9:1)

200 MHz ¹H-NMR (CDCl₃): 1.01, t, 3H; 1.16, t, 3H, 1.89, hex., 2H, 2.00, hex., 2H; 2.63, s, 3H; 3.02, m, 4H; 4.25, t, 2H, 7.19, d, 1H, 7.89, dd, 1H; 8.48, d, 1H; 9.78, s, 1H.

### Beispiel 62

### N-(2-Hydroxyethyl)-N-methyl-3-(5-methyl-4-oxo-7-propyl-3,4-dihydro-imidazo[5,1-f][1,2,4]triazin-2-yl)-4-propoxy-benzolsulfonsäureamid

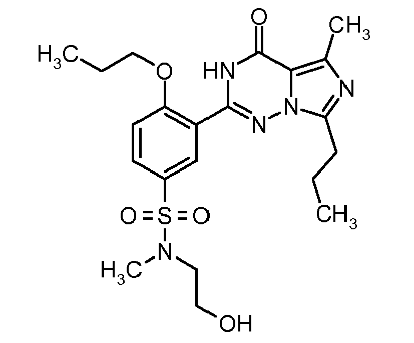

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 23 mg (0,63 mmol) Methylhydroxyethylamin 25 mg (54 %) N-(2-Hydroxyethyl)-N-methyl-3-(5-methyl-4-oxo-7-propyl-3,4-dihydro-imidazo[5,1-f][1,2,4]triazin-2-yl)-4-propoxy-benzolsulfonsäureamid.
R_{f}=0.53 (Dichlormethan/Methanol=9:1)

200 MHz ¹H-NMR (CDCl₃): 1.01, t, 3H; 1.15, t, 3H;1.82, m, 2H; 1.99, hex., 2H; 2.40, s, breit, 1H, 2.62, s, 3H, 2.89, s, 3H; 2.99, t, 2H; 3.21, t, 2H; 3.80, s, breit, 2H; 4.21, t, 2H, 7.16, d, 1H; 7.92, dd, 1H, 8.50, d, 1H, 9.79, s, 1H.

### Beispiel 63

### N-(2-Hydroxyethyl)-3-(5-methyl-4-oxo-7-propyl-3,4-dihydro-imidazo[5,1-f][1,2,4]triazin-2-yl)-4-propoxy-N-propyl-benzolsulfonsäureamid

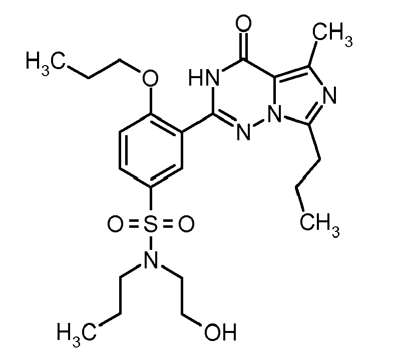

auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 31 mg (0,6 mmol) Propylhydroxyethylamin 20 mg (40 %) N-(2-Hydroxyethyl)-3-(5-methyl-4-oxo-7-propyl-3,4-dihydro-imidazo[5,1-f][1,2,4]triazin-2-yl)-4-propoxy-N-propyl-benzolsulfonsäureamid.
R_{f}=0.52 (Dichlormethan/Methanol=9:1)

200 MHz ¹H-NMR (CDCl₃): 0.90, t, ,3H; 1.01, t, 3H; 1.15, t, 3H; 1.52, m, 2H, 1.88, m, 2H, 2.00, m, 2H; 2.40, s, 1H; 2.63, s, 3H, 3.01, t, 2H, 3.22, m, 4H; 3.80, quart., 2H; 4.21, t, 2H, 7.15, d, 2H, 7.95, dd, 1H, 8.55, d, 1H; 9.75, s, 1H.

### Beispiel 64

### N-[2-(3,4-Dimethoxy-phenyl)ethyl]-N-methyl-3-(5-methyl-4-oxo-7-propyl-3,4-dihydro-imidazo[5,1-f][1,2,4]triazin-2-yl)-4-propoxy-benzolsulfonsäureamid

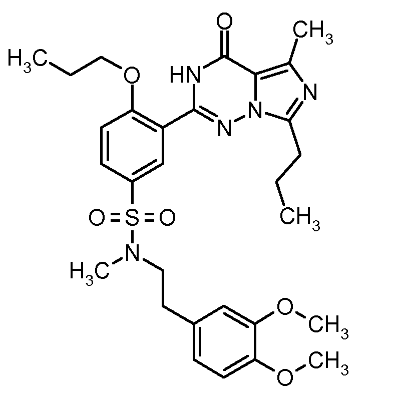

Auf analoge Weise erhält man ausgehend von 42 mg (0,1 mmol) 4-Propoxy-3-(5-methyl-4-oxo-7-propyl-3,4-dihydroimidazo[5,1-f][1,2,4]triazin-2-yl)-benzolsulfonsäurechlorid und 59 mg (0,3 mmol) N-Methyl-3,4-dimethoxyphenylethylamin 45 mg (78 % ) N-[2-(3,4-Dimethoxyphenyl)-ethyl]-N-methyl-3-(5-methyl-4-oxo-7-propyl-3,4-dihydro-imidazo[5,1-f][1,2,4]triazin-2-yl)-4-propoxybenzolsulfonsäureamid. R_{f}=0.35 (Dichlormethan/Methanol=19:1)

200 MHz ¹H-NMR (CDCl₃): 0.90, t, 3H; 1.07, t, 3H; 1.78, m, 2H; 1.92, m, 2H; 2.55, s, 3H; 2.73, s, 3H; 2.78, m, 2H; 2.89, t, 2H; 3.23, t, 2H, 3.80, s, 6H, 4.15, t, 2H, 6.65, m, 3H, 7.05, d, 1H, 7.75, dd, 1H, 8.41, d, 1H, 9.67, s, 1H.

### Beispiel 65

### N-Allyl-N-(2-hydroxyethyl)-3-(5-methyl-4-oxo-7-propyl-3,4-dihydroimidazo[5,1-f][1,2,4]triazin-2-yl)-4-propoxybenzolsulfonsäureamid

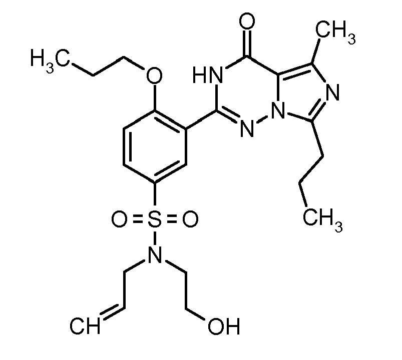

Auf analoge Weise erhält man ausgehend von 42 mg (0,1 mmol) 4-Propoxy-3-(5-methyl-4-oxo-7-propyl-3,4-dihydroimidazo[5,1-f][1,2,4]triazin-2-yl)-benzolsulfonsäurechlorid und 31 mg (0,3 mmol) Allylhydroxyethylamin 34 mg (70 %) N-Allyl-N-(2-hydroxyethyl)-3-(5-methyl-4-oxo-7-propyl-3,4-dihydroimidazo[5,1-f][1,2,4]-triazin-2-yl)-4-propoxybenzolsulfon-säureamid.
R_{f}=0.52 (Dichlormethan/Methanol=9:1)

200 MHz ¹H-NMR (CDCl₃):1.01, t, 3H; 1.15, t, 3H; 1.85, m, 2H; 1.99, m, 2H; 2.38, s, breit, 1H, 2.63, s, 3H; 3.00, t, 2H, 3.32, t, 2H, 3.86, t, 2H, 3.90, d, 2H; 4.25, t, 2H, 5.21, m, 2H, 5.71, m, 1H; 7.15, d, 1h, 7.95, dd, 1H; 8.55, d, 1H, 9.77, s, 1H.

### Beispiel 66

### N-Allyl-N-cyclopentyl-3-(5-methyl-4-oxo-7-propyl-3,4-dihydroimidazo[5,1-f][1,2,4]triazin-2-yl)-4-propoxybenzolsulfonsäureamid

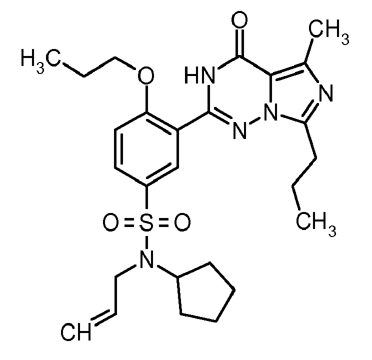

Auf analoge Weise erhält man ausgehend von 42 mg (0,1 mmol) 4-Propoxy-3-(5-methyl-4-oxo-7-propyl-3,4-dihydroimidazo[5,1-f][1,2,4]triazin-2-yl)-benzolsulfonsäurechlorid und 38 mg (0,3 mmol) Allylcyclopentylamin 33 mg (64 %) N-Allyl-N-cyclopentyl-3-(5-methyl-4-oxo-7-propyl-3,4-dihydro-imidazo[5,1-f][1,2,4]triazin-2-yl)-4-propoxybenzolsulfonsäureamid.
R_{f}=0.43 (Dichlormethan/Methanol=19:1)

200 MHz ¹H-NMR (CDCl₃):1.01, t, 3H;1.15, t, 3H; 1.53, m, 9H; 2.00, m, 4H, 2.63, s, 3H; 3.00, t, 2H; 3.80, m, 2H, 4.21, t, 2H, 5.20, m, 2H; 5.88, m, 1H, 7.12, d, 1H, 7.95, dd, 1H, 8.55, d, 1H, 9.75, s, 1H.

### Beispiel 67

### N-Allyl-N-ethyl-3-(5-methyl-4-oxo-7-propyl-3,4-dihydroimidazo[5,1-f][1,2,4]triazin-2-yl)-4-propoxybenzolsulfonsäureamid

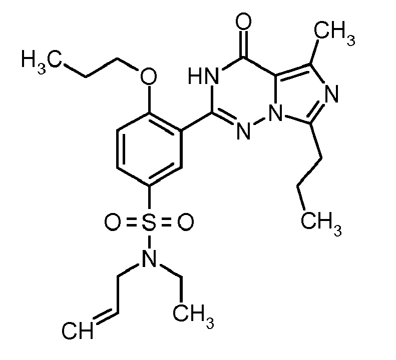

Auf analoge Weise erhält man ausgehend von 42 mg (0,1 mmol) 4-Propoxy-3-(5-methyl-4-oxo-7-propyl-3,4-dihydro-imidazo[5,1-f][1,2,4]triazin-2-yl)-benzolsulfonsäurechlorid und 26 mg (0,3 mmol) Allylethylamin 30 mg (64 %) N-Allyl-N-ethyl-3-(5-methyl-4-oxo-7-propyl-3,4-dihydroimidazo[5,1-f][1,2,4]triazin-2-yl)-4-propoxy-benzolsulfonsäureamid.
R_{f}=0.44 (Dichlormethan/Methanol=19:1)

200 MHz ¹H-NMR (CDCl₃):1.01, t, 3H;1.15, t, 6H;1.89, m, 2H, 2.01, m, 2H, 2.63, s, 3H, 3.00, t, 2H, 3.27, quart., 2H, 3.87, d, 2H, 4.23, t, 2H, 5.20, m, 2H, 5.72, m, 1H; 7.15, d, 1H, 7.95, dd, 1H, 8.55, d, 1H; 9.80, s, 1H.

### Beispiel 68

### 2-[2-Ethoxy-4-methoxy-5-(4-methylpiperazin-1-sulfonyl)-phenyl]-5-methyl-7-propyl-3H-imidazo[5,1-f][1,2,4]triazin-4-on

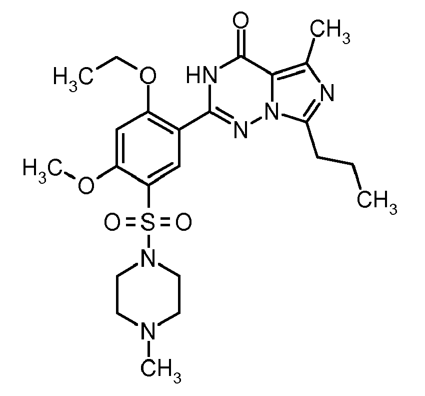

20 mg (0.045mmol) 4-Ethoxy-2-methoxy-5-(5-methyl-4-oxo-7-propyl-3,4-dihydro-imidazo-[5,1-f][1,2,4]triazin-2-yl)-benzolsulfonsäurechlorid werden in 0,5 ml Dichlormethan gelöst, mit einer Spatelspitze Dimethylaminopyridin und 14 mg (0,136 mmol) N-Methylpiperazin versetzt und die Reaktionsmischung über Nacht bei Raumtemperatur gerührt. Nach Reinigung über Kieselgel erhält man 12,8 mg (55 %) 2-[2-Ethoxy-4-methoxy-5-(4-methylpiperazin-1-sulfonyl)phenyl]-5-methyl-7-propyl-3H-imidazo[5,1-f][1,2,4]triazin-4-on.
R_{f}=0.22 (Dichlormethan/Methanol=20: 1).

200 MHz ¹H-NMR (CDCl₃): 0.94, t, 3H; 1.55, t, 3H; 1.80, m, 2H; 2.24, s, 3H; 2.42, t, 4H; 2.55, s, ,3H; 2.92, t, 2H; 3.19, t, 4H, 3.91, s, 3H; 4.25, quart., 2H; 6.48, s, 1H; 8.57, s, 1H; 9.54, s, 1H.

### Beispiel 69

### 2-{2-Ethoxy-5-[4-(2-hydroxyethyl)-piperazin-1-sulfonyl]-4-methoxy-phenyl}-5-methyl-7-propyl-3H-imidazo[5,1-f][1,2,4]triazin-4-on

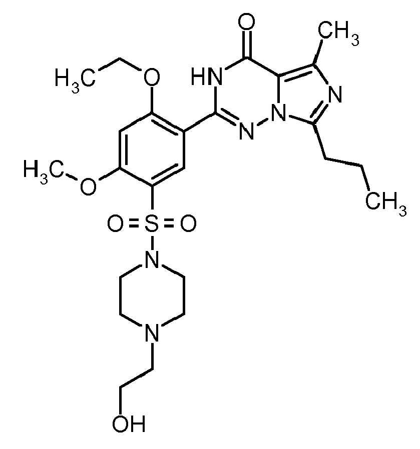

Auf analoge Weise erhält man ausgehend von 20 mg (0,045 mmol) 4-Ethoxy-2-methoxy-5-(5-methyl-4-oxo-7-propyl-3,4-dihydroimidazo[5,1-f][1,2,4]triazin-2-yl)-benzolsulfonsäure-chlorid und 18 mg (0,14 mmol) 4-Hydroxyethylpiperazin 11 mg (46 %) 2-{2-Ethoxy-5-[4-(2-hydroxyethyl)-piperazin-1-sulfonyl]-4-methoxyphenyl}-5-methyl-7-propyl-3H-imidazo[5,1-f][1,2,4]triazin-4-on.
R_{f}=0.34 (Dichlormethan/Methanol=15:1)

200 MHz ¹H-NMR (CDCl₃): 0.94, t, 3H; 1.55, t, 3H; 1.80, m, 3H; 2.52, m, 9H; 2.92, t, 2H; 3.20, t, 4H; 3.44, t, 2H; 3.92, s, 3H; 4.25, quart., 2H; 6.49, s, 1H; 8.56, s, 1H; 9.55, s, 1H.

### Beispiel 70

### 4-Ethoxy-N-ethyl-N-(2-hydroxyethyl)-2-methoxy-5-(5-methyl-4-oxo-7-propyl-3,4-dihydro-imidazo[5,1-f][1,2,4]triazin-2-yl)-benzolsulfonsäureamid

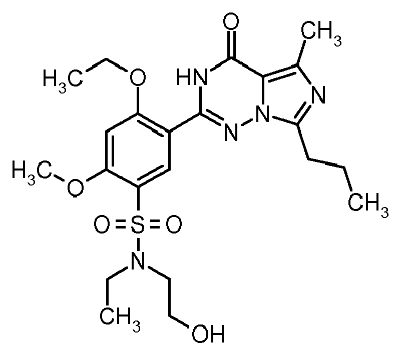

Auf analoge Weise erhält man ausgehend von 20 mg (0,045 mmol) 4-Ethoxy-2-methoxy-5-(5-methyl-4-oxo-7-propyl-3,4-dihydro-imidazo[5,1-f][1,2,4]triazin-2-yl)-benzolsulfonsäure-chlorid und 12 mg (0,14 mmol) Ethylhydroxyethylamin 8 mg (34 % ) 4-Ethoxy-N-ethyl-N-(2-hydroxyethyl)-2-methoxy-5-(5-methyl-4-oxo-7-propyl-3,4-dihydro-imidazo[5,1-f][1,2,4]triazin-2-yl)-benzolsulfonsäureamid.
R_{f}=0.45 (Dichlormethan/Methanol=15:1)

200 MHz ¹H-NMR (CDCl₃): 1.02, t, 3H; 1.18, t, 3H; 1.61, t, 2H; 1.88, m, 2H; 2.39, s, breit, 1H; 2.65, s, 3H; 3.00, t, 2H; 3.38, quart., 2H; 3.45, t, 2H; 3.78, m, 2H; 4.01, s, 3H; 4.20, quart., 2H; 6.58, s, 1H; 8.67, s, 1H; 9.61, s, 1H.

### Beispiel 71

### 4-Ethoxy-N-(4-ethoxyphenyl)-2-methoxy-5-(5-methyl-4-oxo-7-propyl-3,4-dihydro-imidazo[5,1-f][1,2,4]triazin-2-yl)-benzolsulfonsäureamid

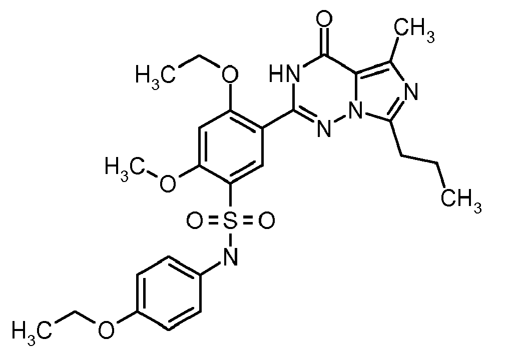

Auf analoge Weise erhält man ausgehend von 20 mg (0,045 mmol) 4-Ethoxy-2-methoxy-5-(5-methyl-4-oxo-7-propyl-3,4-dihydro-imidazo[5,1-f][1,2,4]triazin-2-yl)-benzolsulfonsäure-chlorid und 19 mg (0,14 mmol) 4-Ethoxyanilin 7 mg (34 %) 4-Ethoxy-N-(4-ethoxyphenyl)-2-methoxy-5-(5-methyl-4-oxo-7-propyl-3,4-dihydro-imidazo[5,1-f][,2,4]triazin-2-yl)-benzol-sulfonsäureamid.
R_{f}=0.36 (Dichlormethan/Methanol=20:1)

200 MHz H-NMR (CDCl₃): 1.02, t, 3H; 1.33, t, 3H, 1.59, t, 3H, 1.86, hex., 2H, 2.62, s, 3H; 3.02, t, 2H; 3.92, quart., 2H; 4.11, s, 3H; 4.31, quart., 2H; 6.58, s, 1H, 6.72, d, 2H; 6.88, s, breit, 1H; 6.99, d, 2H, 8.50, s, 1H; 9.59, s, 1H.

### Beispiel 72

### 4-Ethoxy-N-ethyl-N-(2-hydroxy-ethyl)-3-(5-ethyl-4-oxo-7-propyl-3,4-dihydroimidazo[5,1-f][1,2,4]triazin-2-yl)benzolsulfonsäureamid

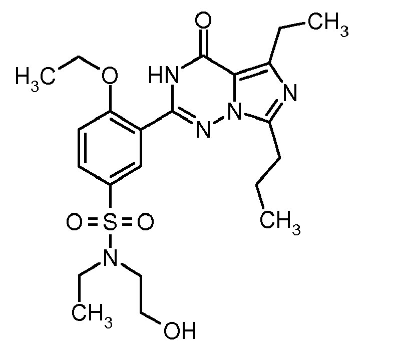

0,64 g ( 1 , 5 mmol) 4-Ethoxy-3-(5-ethyl-4-oxo-7-propyl-3,4-dihydroimidazo[5,1-f][1,2,4]tri-azin-2-yl)-benzolsulfonsäurechlorid werden in 20 ml Dichlormethan gelöst und auf 0°C gekühlt. Nach Zugabe einer Spatelspitze Dimethylaminopyridin werden 0,40 g (4,50 mmol) 2-(Ethylamino)-ethanol zugegeben und die Reaktionsmischung über Nacht bei Raumtemperatur gerührt. Es wird mit Dichlormethan verdünnt, die organische Phase mit Wasser gewaschen, über Natriumsulfat getrocknet und das Lösungsmittel im Vakuum entfernt. Chromatographie (Dichlormethan/Methanol=95:5) ergibt 0,454 g (63 %) farblosen Feststoff.

200 MHz H-NMR (CDCl₃):1.02, t, 3H; 1.20, t, 3H; 1.35, t, 3H; 1.61, t, 3H; 1.88, sex., 2H; 2.25, s, breit, 1H; 3.01, m, 4H; 3.32, m, 4H; 3.70, m, 2H; 3.80, m, 2H; 4.37, quart., 2H; 7.15, d, 1H; 7.98, dd, 1H; 8.56, d, 1H; 9.70, s, 1H.

### Beispiel 73

### N-(2-methoxyethyl)-3-(5-ethyl-4-oxo-7-propyl-3,4-dihydro-imidazo[5,1-f][1,2,4]triazin-2-yl)-4-ethoxybenzolsulfonsäureamid

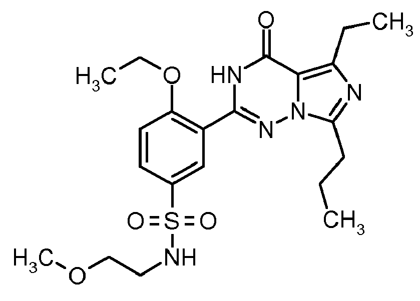

Auf analoge Weise erhält man ausgehend von 40 mg (0,094 mmol) 4-Ethoxy-3-(5-ethyl-4-oxo-7-propyl-3,4-dihydroimidazo[5,1-f][1,2,4]triazin-2-yl)-benzolsulfonsäurechlorid und 21 mg (0,282 mmol) 2-Methoxyethylamin 15 mg (34 %) N-(2-methoxyethyl)-3-(5-ethyl-4-oxo-7-propyl-3,4-dihydroimidazo[5,1-f][1,2,4]triazin-2-yl)-4-ethoxybenzolsulfonsäureamid.
R_{f}=0.2 (Ethylacetat/Cyclohexan=2: 1)

200 MHz H-NMR (CDCl₃): 0.97, t, 3H;1.25, t, 3H; 1.53, t, 3H; 1.82, sex., 2H; 2.97, m, 4H; 3.11 m, 2H; 3.22, s, 3H; 3.39, t, 2H; 4.37, quart., 2H; 5.00, t, 1H; 7.17, d, 1H, 7.97, dd, 1H, 8.53, d, 1H; 9.82, s, 1H.

### Beispiel 74

### N,N-Bis-(2-Methoxyethyl)-3-(5-ethyl-4-oxo-7-propyl-3,4-dihydroimidazo[5,1-f][1,2,4]tri-azin-2-yl)-4-ethoxybenzolsulfonsäureamid

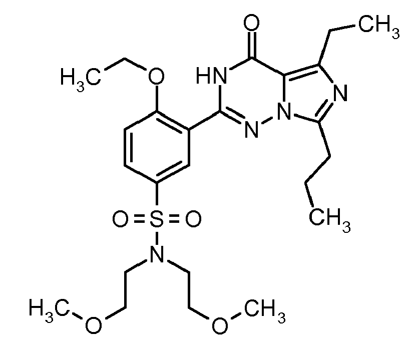

Auf analoge Weise erhält man ausgehend von 40 mg (0,094 mmol) 4-Ethoxy-3-(5-ethyl-4-oxo-7-propyl-3,4-dihydro-imidazo[5,1-f][1,2,4]triazin-2-yl)-benzolsulfonsäurechlorid und 38 mg (0,28 mmol) Bismethoxyethylamin 17 mg (34 %) N,N-Bis-(2-Methoxyethyl)-3-(5-ethyl-4-oxo-7-propyl-3,4-dihydroimidazo[5,1-f][1,2,4]triazin-2-yl)-4-ethoxybenzolsulfonsäureamid.
R_{f}=0.34 (Ethylacetat/Cyclohexan=2:1)

200 MHz ¹H-NMR (CDC1₃): 0.97, t, 3H;1.27, t, 3H; 1.53, t, 3H; 1.80, sex., 2H; 2.95, m, 4H; 3.22, s, 6H; 3.39, m, 4H; 3.49, m, 4H; 4.27, quart., 2H; 7.17, d, 1H, 7.97, dd, 1H, 8.53, d, 1H; 9.82, s, 1H.

### Beispiel 75

### 2-[5-(4-Hydroxypiperidin-l-sulfonyl)-2-ethoxyphenyl]-5-ethyl-7-propyl-3H-inidazo[5,1-f]-[1,2,4]triazin-4-on

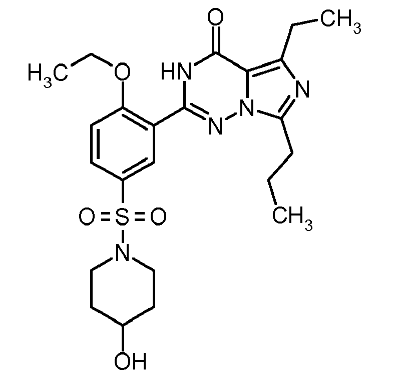

Auf analoge Weise erhält man ausgehend von 640 mg (1,5 mmol) 4-Ethoxy-3-(5-ethyl-4-oxo-7-propyl-3,4-dihydroimidazo [5,1-f][1,2,4]triazin-2-yl)-benzolsulfonsäurechlorid und 460 mg (4,5 mmol) 4-Hydroxypiperidin 485 mg (66 %) 2-[5-(4-Hydroxypiperidin-1-sulfnyl)-2-ethoxyphenyl]-5-ethyl-7-propyl-3*H-*imidazo[5,1-*f*][1,2,4]triazin-4-on.
R_{f}=0.37 (Dichlormethan/Methanol=19:1)

200 MHz ¹H-NMR (CDC1₃): 1.02, t, 3H; 1.32, t, 3H; 1.60, t, 3H; 1.80, m, 7H; 2.97, m, 6H; 3.30, m, 2H; 3.82, m, 1H; 4.34, quart., 2H; 7.17, d, 1H; 7.90, dd, 1H, 8.45, d, 1H. 9.75, s, 1H.

### Beispiel 76

### 2-[5-(4-Hydroxymethylpiperdin-1-sulfonyl)-2-ethoxy-phenyl]-5-ethyl-7-propyl-3H-imidazo[5,1-f](1,2,4]triazin-4-on

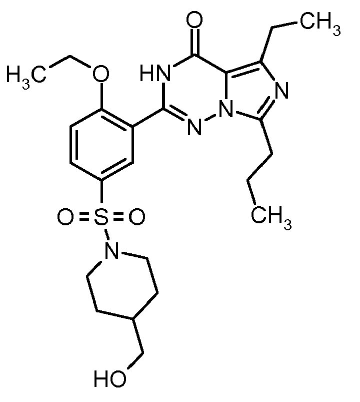

Auf analoge Weise erhält man ausgehend von 40 mg (0,094 mmol) 4-Ethoxy-3-(5-ethyl-4-oxo-7-propyl-3,4-dihydroimidazo[5,1-f][1,2,4]triazin-2-yl)-benzolsulfonsäurechlorid und 33 mg (0,28 mmol) 4-Hydroxymethylpiperidin 23 mg (48 %) 2-[5-(4-Hydroxymethylpiperidin-1-sulfonyl)-2-ethoxyphenyl]-5-ethyl-7-propyl-3*H-*imidazo[5,1-f][1,2,4]triazin-4-on.
R_{f}=0.38 (Dichlormethan/Methanol=10:1)

200 MHz ¹H-NMR (CDC1₃): 1.01, t, 3H; 1.33, t, 3H; 1.60, t, 3H; 1.80, m, 8H; 2.41, m, 2H, 3.00, m, 4H; 3.56, m, 4H; 4.35, quart, 2H; 7.,17, d, 1H; 7.88, dd, 1H, 8.45, d, 1H; 9. 71, s, 1H.

### Beispiel 77

### 2-{2-Ethoxy-5-[4-(2-hydroxyethyl)-piperazin-1-sulfonyl]-phenyl}-5-ethyl-7-propyl-3H-imidazo[5,1-f][1,2,4]triazin-4-on

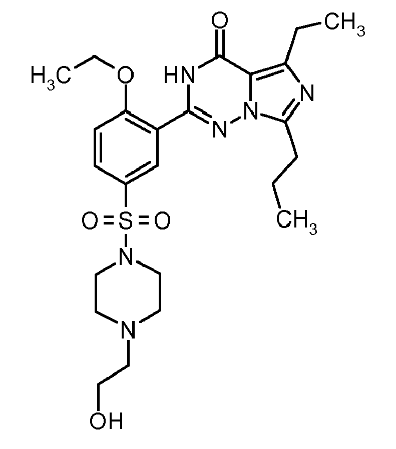

Auf analoge Weise erhält man ausgehend von 40 mg (0,094 mmol) 4-Ethoxy-3-(5-ethyl-4-oxo-7-propyl-3,4-dihydroimidazo[5,1-f][1,2,4]triazin-2-yl)-benzolsulfonsäurechlorid und 37 mg (0,28 mmol) 4-Hydroxyethylpiperazin 35 mg (71 %) 2-{2-Ethoxy-5-[4-(2-hydroxyethyl)-piperazin-1-sulfonyl]-phenyl}-5-ethyl-7-propyl-3*H-*imidazo[5,1-f][1,2,4]triazin-4-on.
R_{f}=0.65 (Dichlormethan/Methanol=10:1)

### Beispiel 78

### 2-[2-Ethoxy-5-(4-methylpiperazin-1-sulfonyl)-phenyl]-5-ethyl-7-propyl-3H-imidazo[5,1-f]-[1,2,4]triazin-4-on

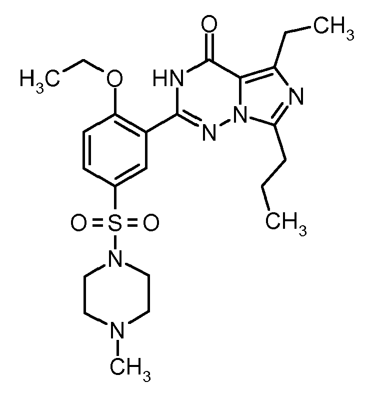

Auf analoge Weise erhält man ausgehend von 640 mg (1,50 mmol) 4-Ethoxy-3-(5-ethyl-4-oxo-7-propyl-3,4-dihydro-imidazo[5,1-f][1,2,4]triazin-2-yl)-benzolsulfonsäurechlorid und 450 mg (4,5 mmol) 4-Hydroxyethylpiperazin 495 mg (66 %) 2-[2-Ethoxy-5-(4-methylpiperazin-1-sulfonyl)-phenyl]-5-ethyl-7-propyl-3*H*-imidazo[5,1-f][1,2,4]triazin-4-on.
R_{f}=0. 30(Dichlormethan/Methanol= 19: 1)

200 MHz ¹H-NMR (CDCl₃):1.01, t, 3H; 1.35, t, 3H; 1.61, t, 3H; 1.89, sex., 2H; 2.31, s, 3H; 2.53, m, 4H; 3.05, m, 8H; 4.35, quart., 2H; 7.17, d, 1H; 7.89, dd, 1H; 8.48, d, 1H; 9.65, s, 1H.

### Beispiel 79

### 2-[2-Ethoxy-5-(4-methylpiperazin-1-sulfonyl)-phenyl]-5-ethyl-7-propyl-3H-imidazo[5,1-f][1,2,4]triazin-4-on Hydrochlorid

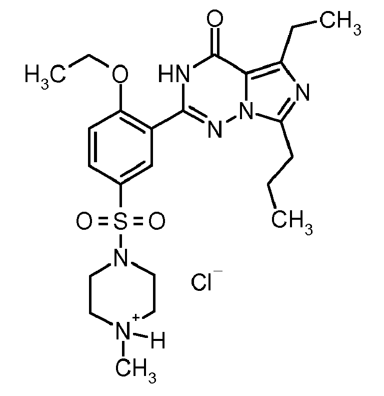

300 mg (0,61 mmol) 2-[2-Ethoxy-5-(4-methyl-piperazin-1-sulfonyl)-phenyl]-5-ethyl-7-propyl-3*H-*imidazo[5,1-f][1,2,4]triazin-4-on werden in einer Mischung aus Ether und Dichlormethan gelöst und mit 2 ml einer IM Lösung von HCI in Ether versetzt. Nach 20 Minuten wird der ausgefallene Feststoff abgesaugt und getrocknet.

200 MHz ¹H-NMR (DMSO-d₆): 0.95, t, 3H; 1.32, 2t, 6H; 1.80, sex., 2H; 2.76, m, 4H; 3.01, m, 4H; 3.15, m, 2H; 3.44, m, 2H; 3.81, m, 2H; 4.25, quart., 2H; 7.49, d, 1H; 7.95, m, 2H; 11.25, s, 1H; 12.30, s, 1H.

### Beispiel 80

### 3-(5-Ethyl-4-oxo-7-propyl-3,4-dihydroimidazo[5,1-f] [1,2,4]triazin-2-yl)-N-(3-morpholin-4-yl-propyl)-4-ethoxybenzolsulfonsäureamid

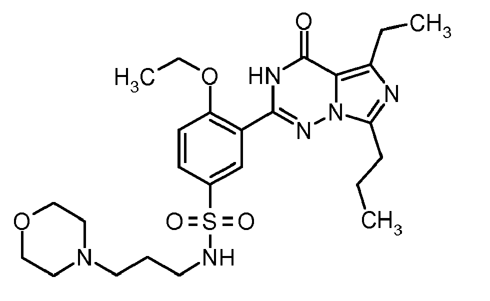

Auf analoge Weise erhält man ausgehend von 640 mg (1,5 mmol) 4-Ethoxy-3-(5-ethyl-4-oxo-7-propyl-3,4-dihydroimidazo[5,1-f][1,2,4]triazin-2-yl)-benzolsulfonsäurechlorid und 650 mg (4,5 mmol) 1-(3-Aminopropyl)-morpholin 476 mg (59 %) 3-(5-Ethyl-4-oxo-7-propyl-3,4-dihydroimidazo[5,1-f][1,2,4]triazin-2-yl)-N-(3-morpholin-4-yl-propyl)-4-ethoxy-benzol-sulfonsäureamid.
R_{f}=0.18 (Dichlormethan/Methanol=19:1)

200 MHz ¹H-NMR (CDCl₃): 1.01, t, 3H; 1.32, t, 3H; 1.60, t, 3H; 1.70, m, 3H; 1.89, sex., 2H; 2.43, m, 7H; 3.01, m, 4H; 3.15, t, 2H; 3.70, m, 4H; 4.35, quart., 2H; 7.15, d, 1H; 7.95, dd, 1H; 8.55, d, 1H; 9.82, s, 1H.

### Beispiel 81

### N-(2-Hydroxyethyl)-3-(5-ethyl-4-oxo-7-propyl-3,4-dihydro-imidazo[5,1-f][1,2,4]triazin-2-yl)-4-ethoxy-N-propyl-benzolsulfonsäureamid

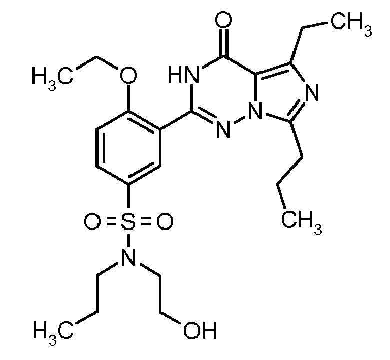

Auf analoge Weise erhält man ausgehend von 640 mg (1,5 mmol) 4-Ethoxy-3-(5-ethyl-4-oxo-7-propyl-3,4-dihydroimidazo[5,1-f][1,2,4]triazin-2-yl)-benzolsulfonsäurechlorid und 464 mg (4,5 mmol) Propylhydroxyethylamin 600 mg (81 %) N-(2-Hydroxyethyl)-3-(5-ethyl-4-oxo-7-propyl-3,4-dihydro-imidazo[5,1-f] [1,2,4]triazin-2-yl)-4-ethoxy-N-propylbenzolsulfonsäure-amid.
R_{f}=0.73 (Dichlormethan/Methanol=10:1)

200 MHz ¹H-NMR (CDCl₃): 0.91, t, ,3H; 1.01, t, 3H; 1.32, t, 3H; 1.62, m, 5H; 1.88, m, 2H; 2.32, s, 1H; 3.01, m, 4H; 3.22, m, 4H; 3.80, m, 2H; 4.35, t, 2H; 7.15, d, 2H, 7.95, dd, 1H, 8.55, d, 1H; 9.75, s, 1H.

Die in den folgenden Tabellen 1, 2, 3, 4 und 6 aufgeführten Sulfonamide wurden mittels automatisierter Parallelsynthese aus 4-Ethoxy-3-(5-methyl-4-oxo-7-propyl-3,4-dihydro-imidazo[5,1-f][1,2,4]triazin-2-yl)-benzolsulfonsäurechlorid und dem entsprechenden Amin nach einer der drei folgenden Standardvorschriften hergestellt.

Die in der Tabelle 5 aufgeführten Sulfonamide wurden in analoger Weise mittels automatisierter Parallelsynthese aus 4-Ethoxy-3-(5-ethyl-4-oxo-7-propyl-3,4-dihydro-imidazo[5,1-8][1,2,4]triazin-2-yl)-benzolsulfonsäure-chlorid und dem entspechenden Amin hergestellt.

Die Reinheit der Endprodukte wurde mittels HPLC bestimmt, ihre Charakterisierungen durch LC-MS Messung vorgenommen. Der Gehalt der gewünschten Verbindung nach HPLC-MS ist in den Tabellen in der Spalte "HPLC" in Prozent angegeben. Standardvorschrift A wurde angewendet bei Aminen mit aciden Funktionalitäten, Standardvorschrift B bei Aminen mit neutralen Funktionalitäten, Standardvorschrift C bei Aminen mit zusätzlichen basischen Funktionalitäten.

In den Strukturformeln der folgenden Tabellen 1, 2, 3, 4, 5 und 6 wurde gelegentlich auf die Abbildung der Wasserstoffatome verzichtet. Stickstoffatome mit einer freien Valenz sind daher als -NH-Rest zu verstehen.

### Standardvorschrift A: Umsetzung von Aminen mit aciden Funktionalitäten

Zunächst werden 0,05 mmol Amin, 0,042 mmol Sulfonsäurechlorid und 0,10 mmol Na₂CO₃ vorgelegt und 0,5 ml eines Gemisches aus THF/H₂O von Hand zupipettiert. Nach 24 h bei RT wird mit 0,5 ml 1 M H₂SO₄-Lösung versetzt und über eine zweiphasige Kartusche filtrier (500 mg Extrelut (Oberphase) und 500 mg SiO₂, Laufmittel Essigester). Nach dem Einengen des Filtrates im Vakuum erhält man das Produkt.

### Standardvorschrift B: Umsetzung von Aminen mit neutralen Funktionalitäten

Zunächst werden 0,125 mmol Amin vorgelegt und vom Synthesizer 0,03 mmol Sulfonsäurechlorid als Lösung in 1,2-Dichlorethan zupipettiert. Nach 24 h wird das Gemisch mit 0,5 ml 1 M H₂S0₄ versetzt und über eine zweiphasige Kartusche (500 mg Extrelut (Oberphase) und 500 mg SiO₂, Laufmittel: Essigester) filtriert. Das Filtrat wird im Vakuum eingeengt.

### Standardvorschrift C: Umsetzung von Aminen mit basischen Funktionalitäten

Zunächst werden 0,05 mmol Amin vorgelegt und vom Synthesizer 0,038 mmol Sulfonsäurechlorid als Lösung in 1,2-Dichlorethan und 0,05 mmol Triethylamin als Lösung in 1,2-Dichlorethan zupipettiert. Nach 24 h wird zunächst mit 3 ml gesättigter NaHCO₃-Lösung versetzt und das Reaktionsgemisch über eine zweiphasige Kartusche filtriert. Nach dem Einengen des Filtrates im Vakuum erhält man das Produkt.

Alle Reaktionen werden dünnschichtchromatographisch verfolgt. Für den Fall, daß nach 24 h bei RT keine vollständige Umsetzung erfolgt ist, wird für weitere 12 h auf 60°C erhitzt und im Anschluß der Versuch beendet.

| **Tabelle 1:** | | | | |
|---|---|---|---|---|
| **3 sp.-Ni** | **Struktur** | **MG** **[g/mol]** | **HPLC** | **MS + H** |
| 82 | 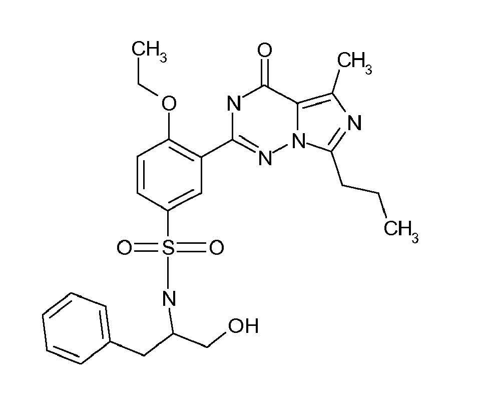 | 525,6315 | 83 | 526 |
| 83 | 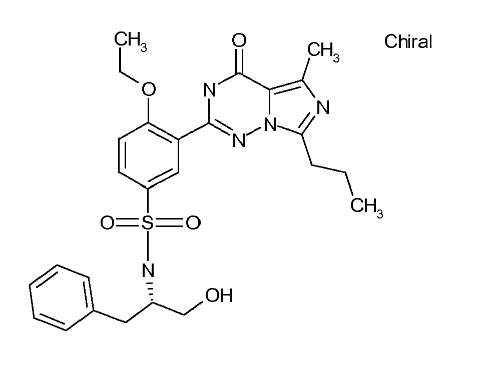 | 525,6315 | 71 | 526 |
| 84 | 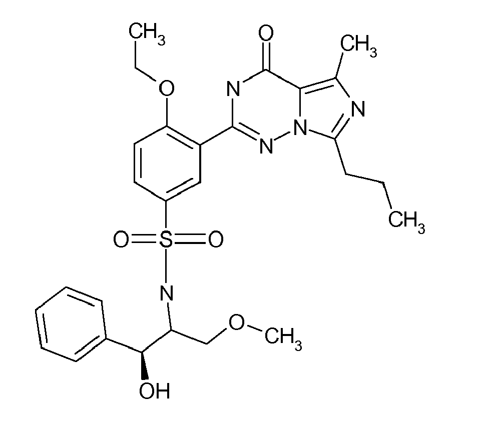 | 555,658 | 91 | 556 |
| 85 | 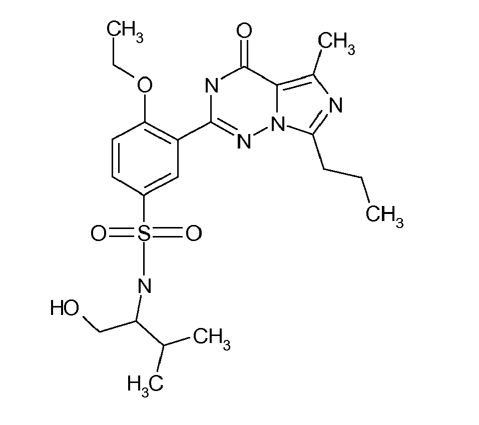 | 477,5869 | 76 | 478 |
| 86 | 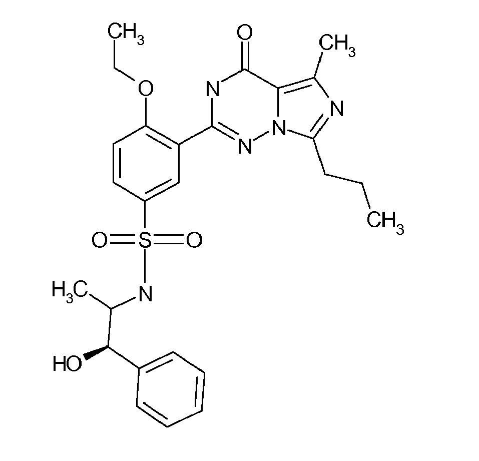 | 525,6315 | 81 | 526 |
| 87 | 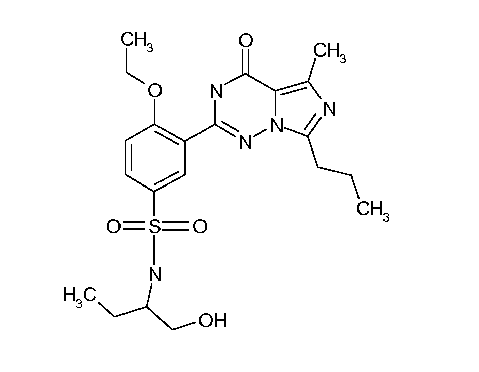 | 463,5598 | 65 | 464 |
| 88 | 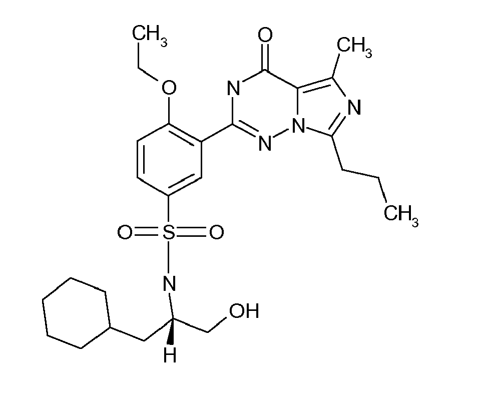 | 531,6793 | 83 | 532 |
| 89 | 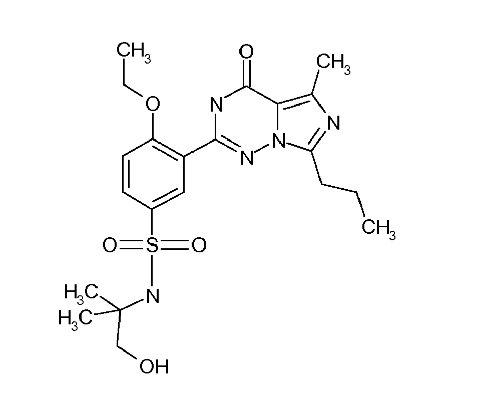 | 463,5598 | 40 | 464 |
| 90 | 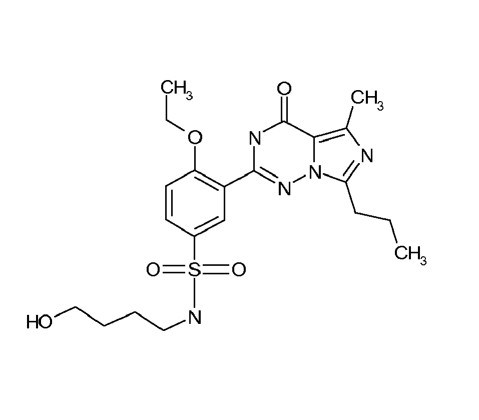 | 463,5598 | 44 | 464 |
| 91 | 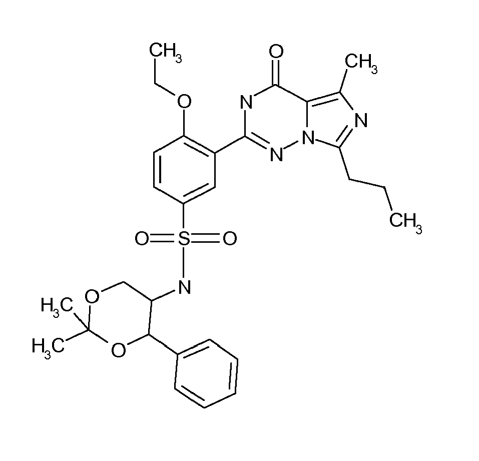 | 581,6962 | 76 | 582 |
| 92 | 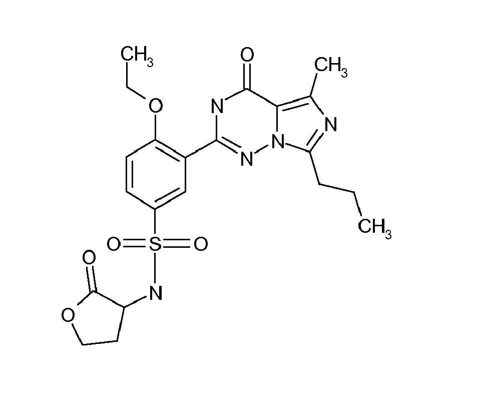 | 475,5273 | 61 | 476 |
| 93 | 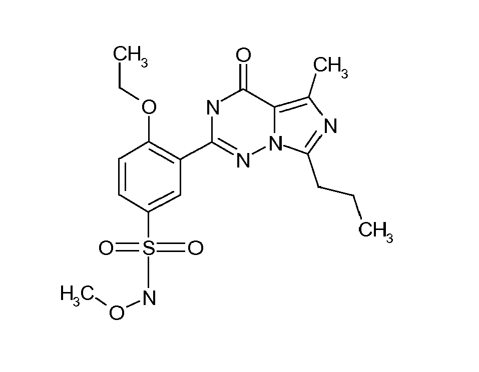 | 421,4785 | 80 | 422 |
| 94 | 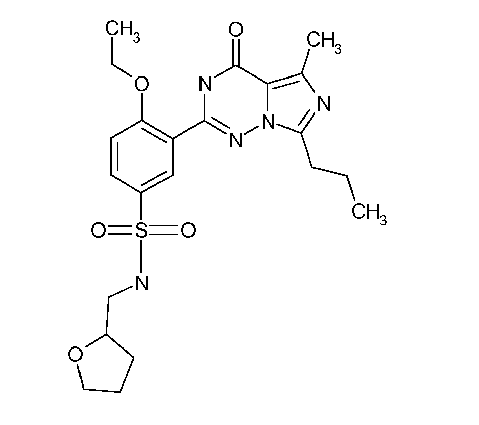 | 475,5709 | 81 | 476 |
| 95 | 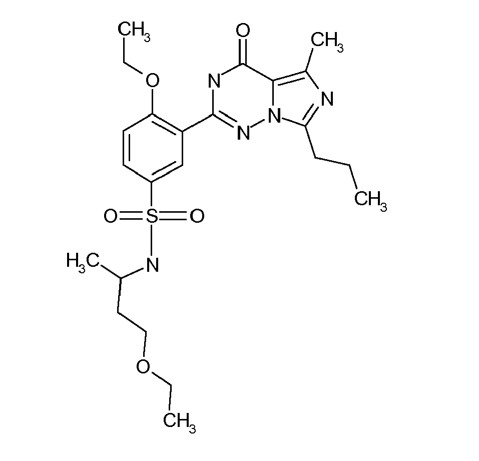 | 491,614 | 97 | 492 |
| 96 | 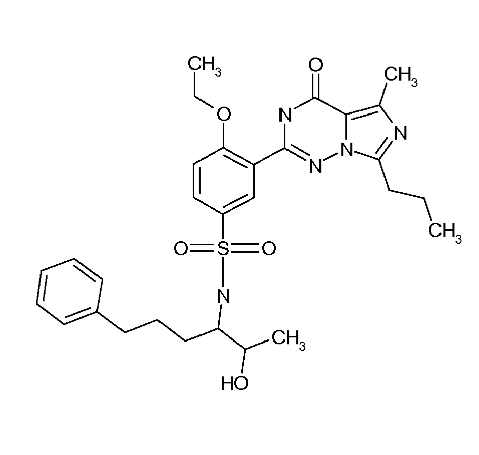 | 567,7127 | 80 | 568 |
| 97 | 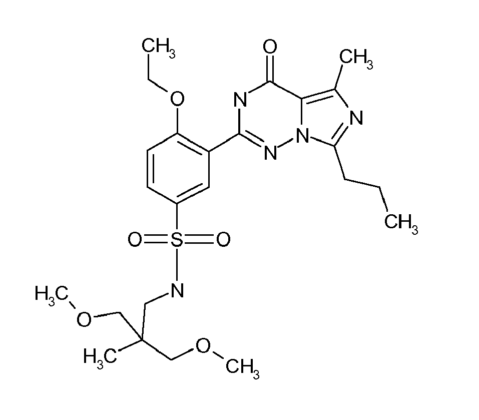 | 521,6405 | 94 | 522 |
| 98 | 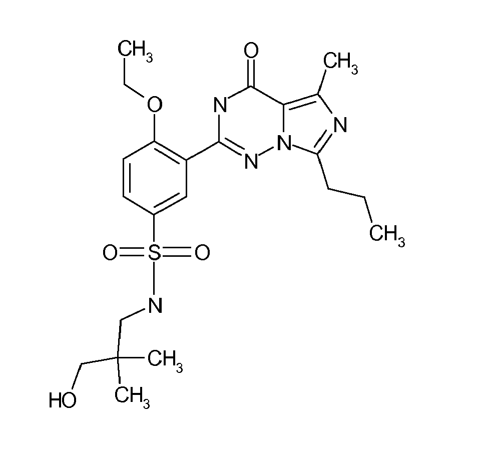 | 477,5869 | 70 | 478 |
| 99 | 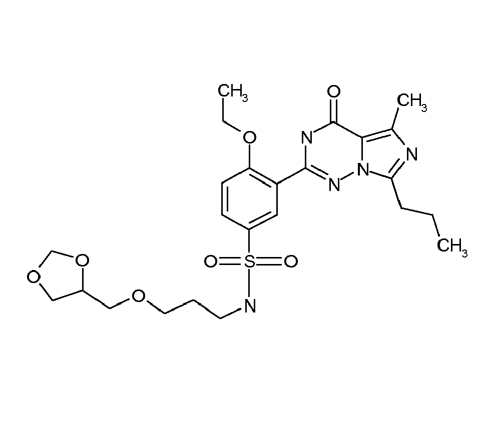 | 535,6239 | 88 | 536 |
| 100 | 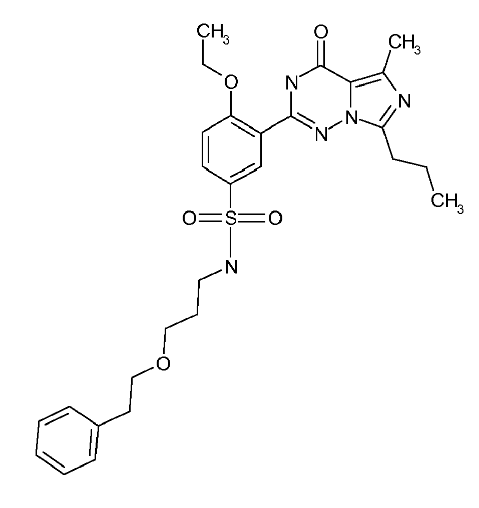 | 553,6857 | 88 | 554 |
| 101 | 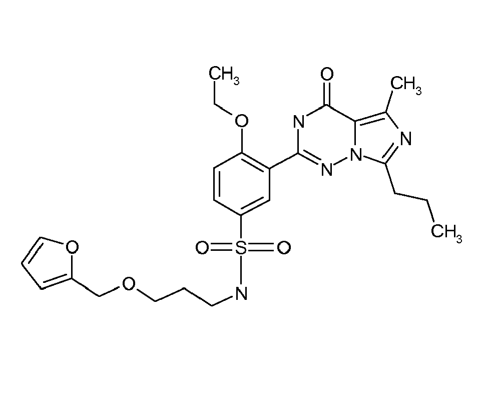 | 529,6197 | 85 | 530 |
| 102 | 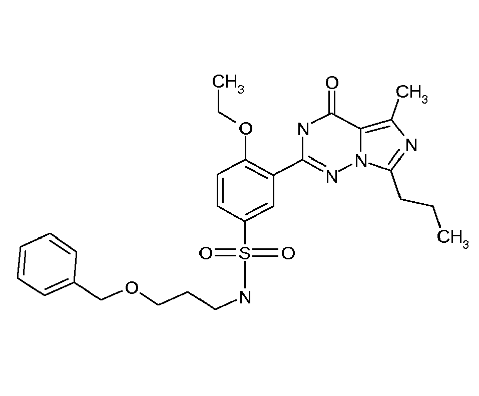 | 539,6586 | 91 | 540 |
| 103 | 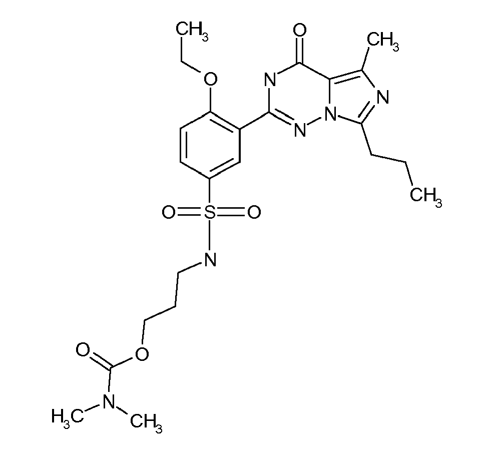 | 520,6121 | 55 | 521 |
| 104 | 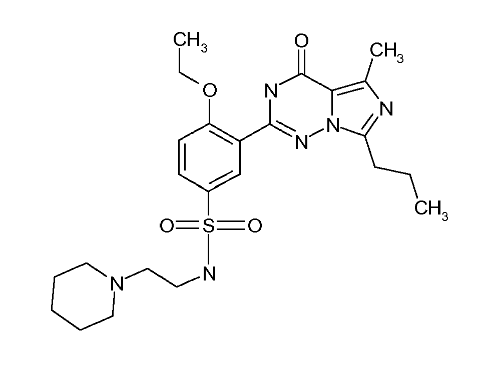 | 502,6404 | 82 | 503 |
| 105 | 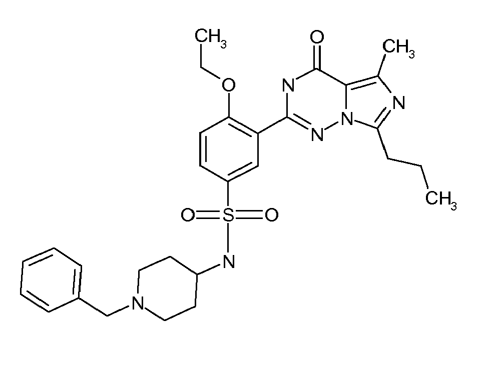 | 564,7121 | 86 | 565 |
| 106 | 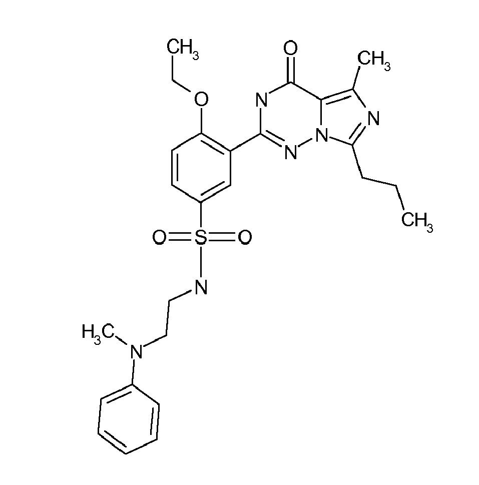 | 524,6467 | 85 | 525 |
| 107 | 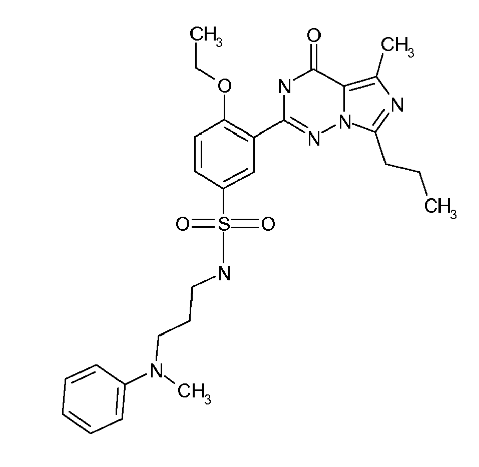 | 538,6738 | 85 | 539 |
| 108 | 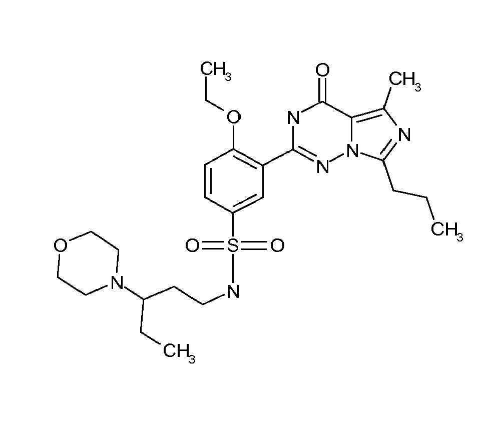 | 546,694 | 84 | 547 |
| 109 | 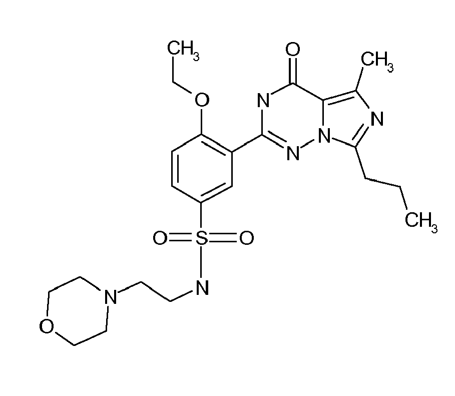 | 504,6127 | 90 | 505 |

| **Tabelle 2:** | | | | |
|---|---|---|---|---|
| **Bsp.-Nr.** | **Struktur** | **MG** **[g/mol]** | **HPLC** | **MZ+H** |
| 110 | 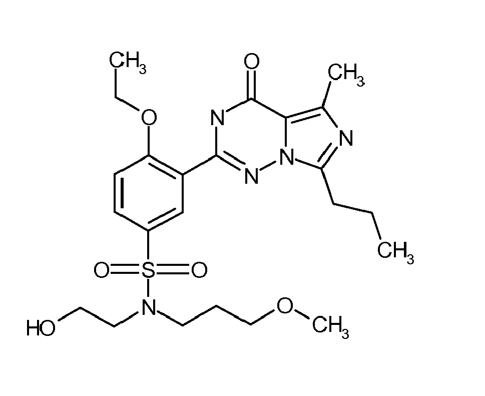 | 507,6134 | 74 | 508 |
| 111 | 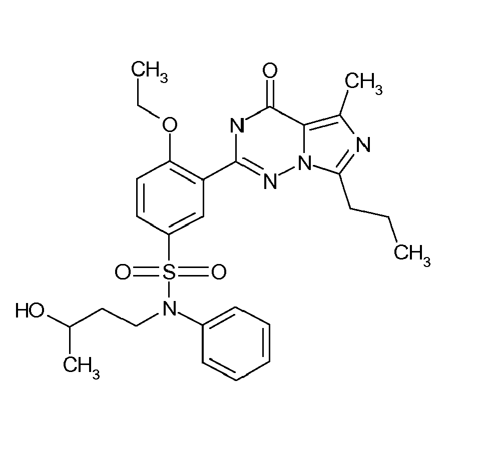 | 539,6586 | 75 | 540 |
| 112 | 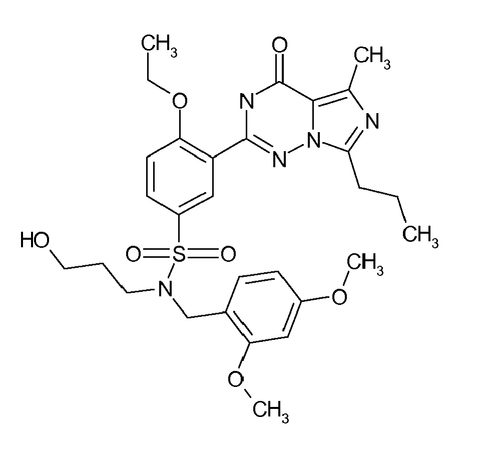 | 599,7115 | 83 | 600 |
| 113 | 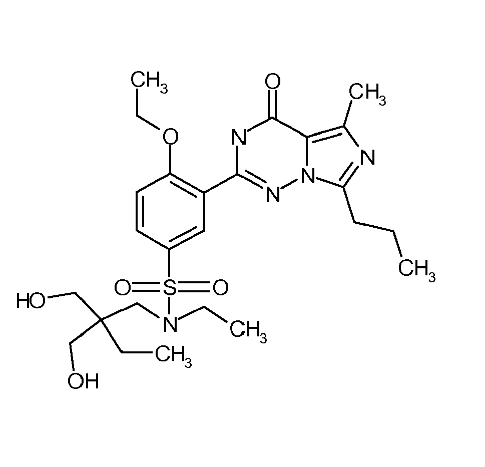 | 535,6675 | 60 | 536 |
| 114 | 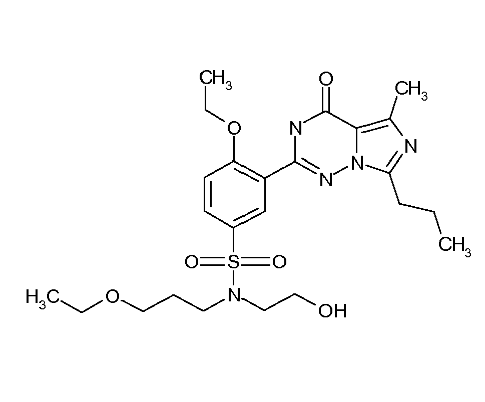 | 521,6405 | 95 | 522 |
| 115 | 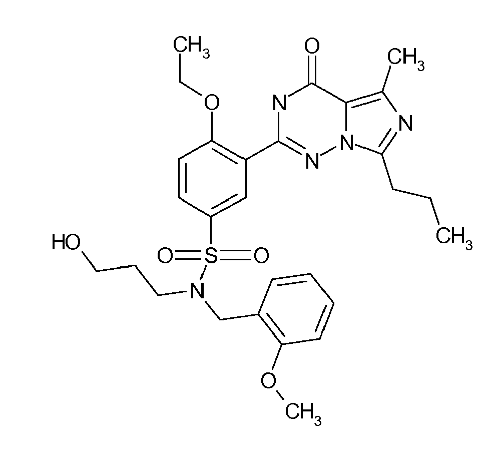 | 569,6851 | 84 | 570 |
| 116 | 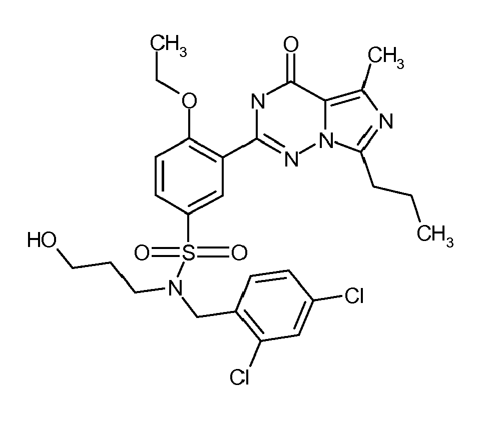 | 608,5486 | 85 | 608 |
| 117 | 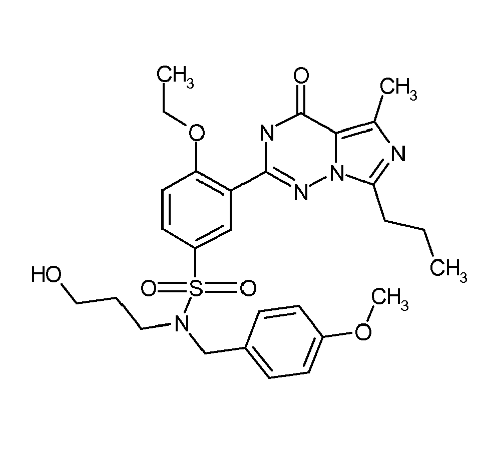 | 569,6851 | 88 | 570 |
| 118 | 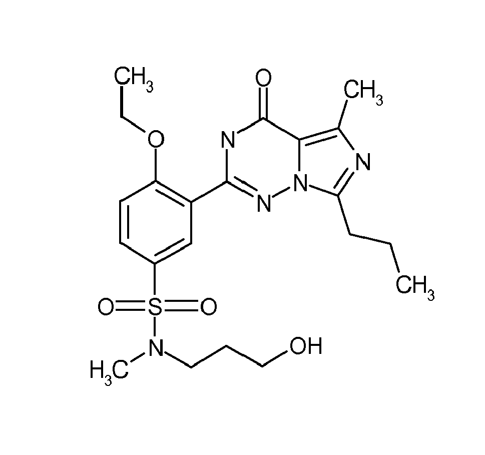 | 463,5598 | 94 | 464 |
| 119 | 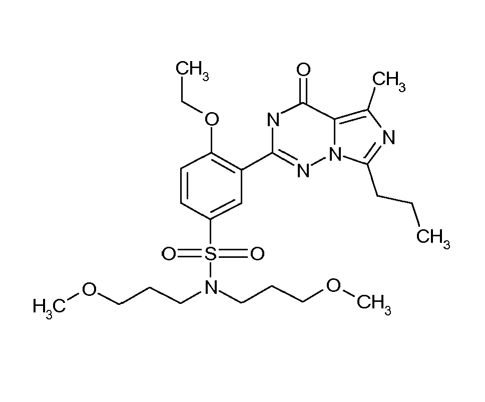 | 535,6675 | 93 | 536 |
| 120 | 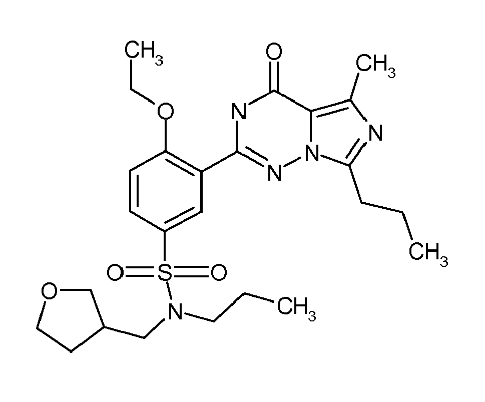 | 517,6522 | 71 | 518 |
| 121 | 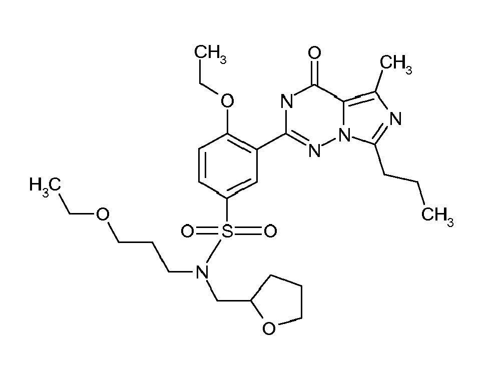 | 561,7058 | 92 | 562 |
| 122 | 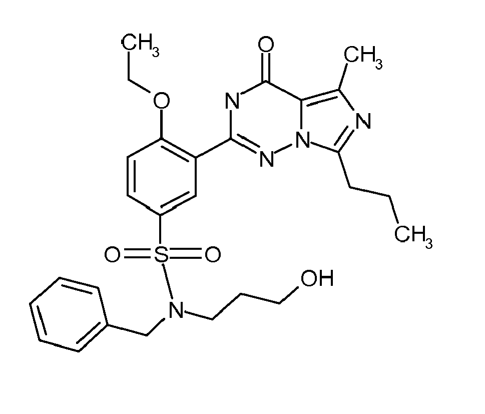 | 539,6586 | 85 | 540 |
| 123 | 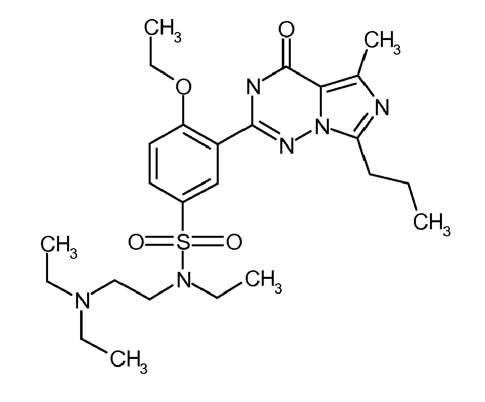 | 518,6834 | 87 | 519 |
| 124 | 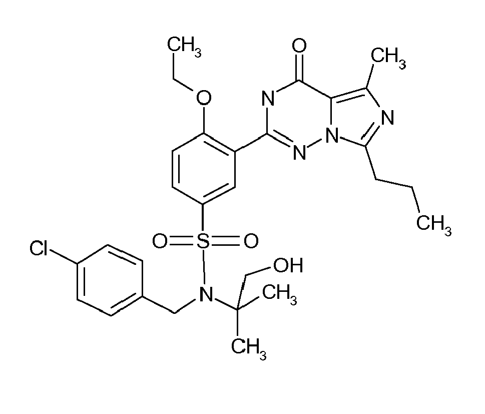 | 588,1307 | 30 | 588 |
| 125 | 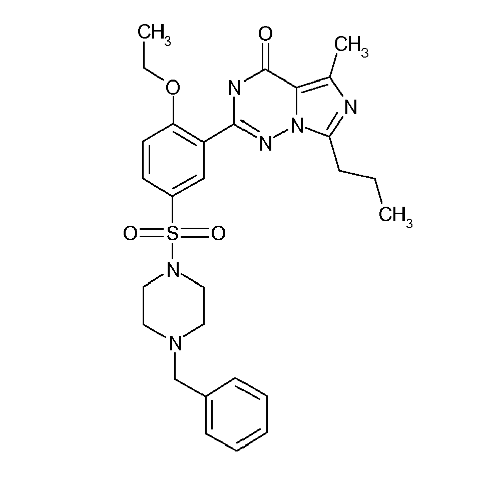 | 550,685 | 83 | 551 |
| 126 | 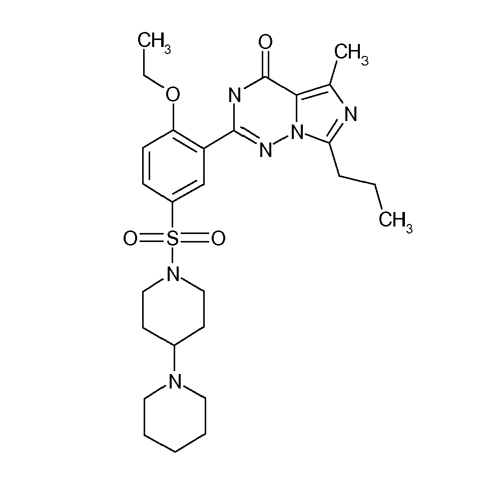 | 542,7057 | 77 | 543 |
| 127 | 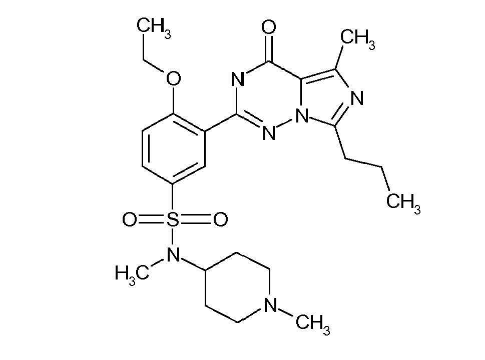 | 502,6404 | 91 | 503 |
| 128 | 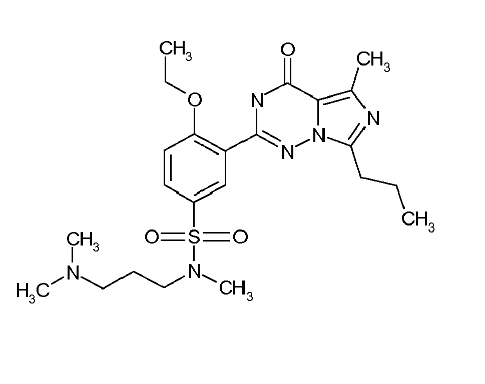 | 490,6292 | 45 | 491 |
| 129 | 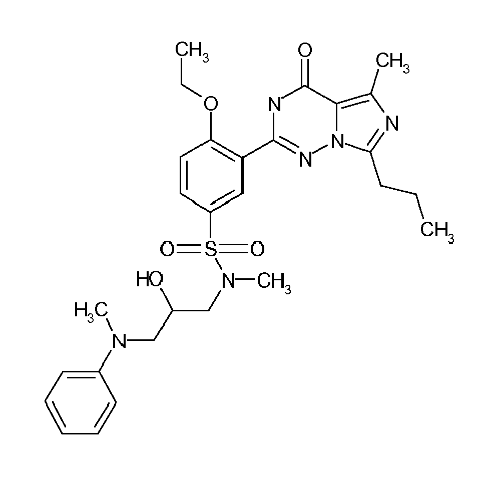 | 568,7003 | 66 | 569 |
| 130 | 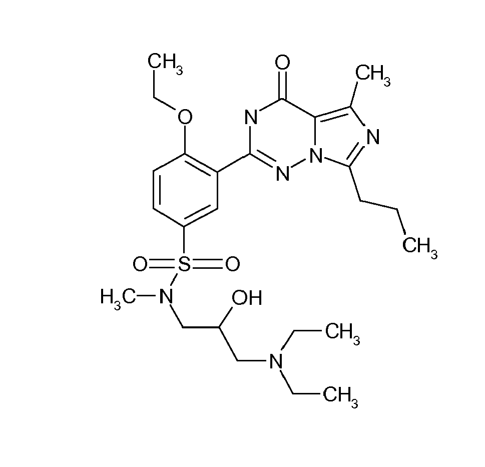 | 534,6828 | 86 | 535 |
| 131 | 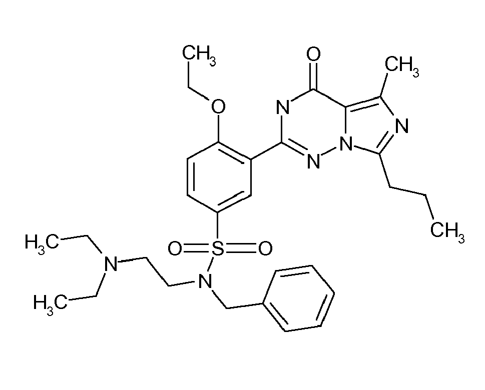 | 580,7551 | 95 | 581 |
| 132 | 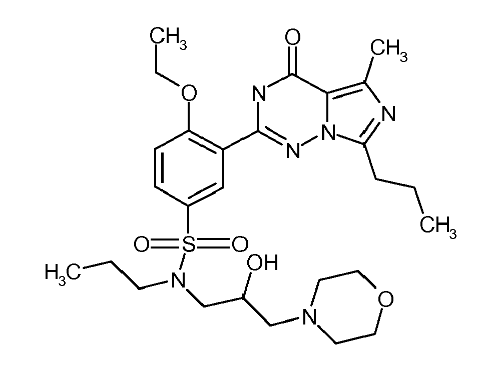 | 576,7205 | 87 | 577 |
| 133 | 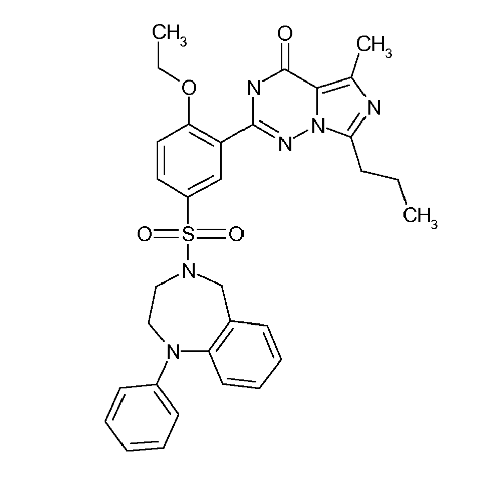 | 598,7296 | 60 | 599 |
| 134 | 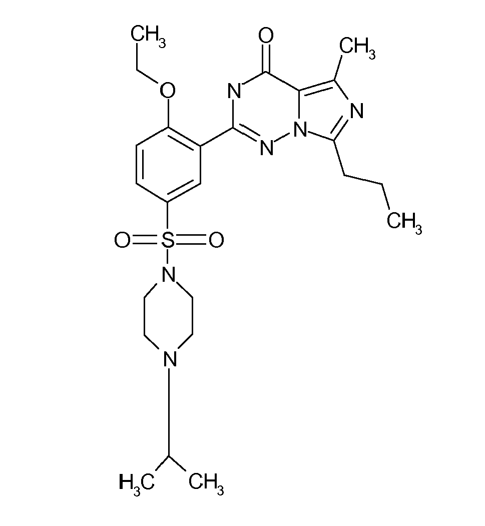 | 516,6675 | 95 | 517 |
| 135 | 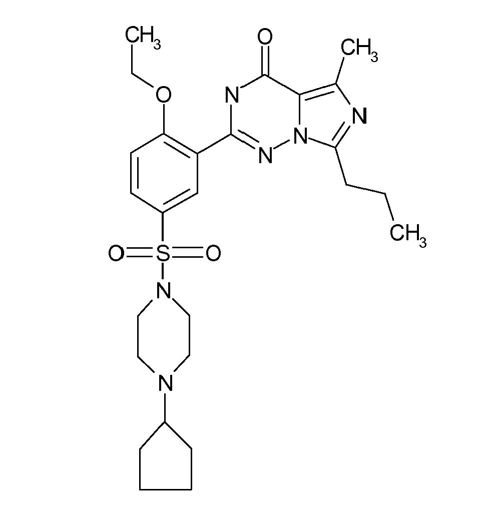 | 528,6786 | 80 | 529 |
| 136 | 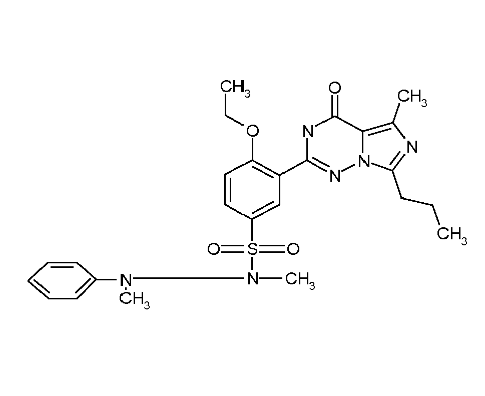 | 538,6738 | 85 | 539 |
| 137 | 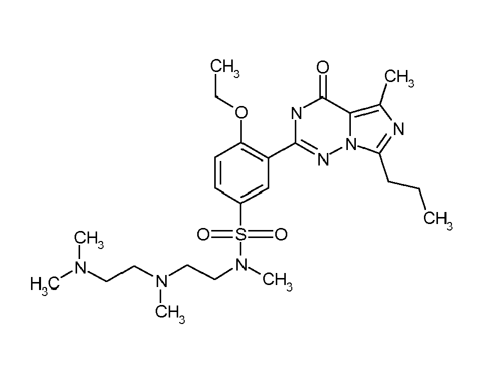 | 533,6981 | 68 | 534 |
| 138 | 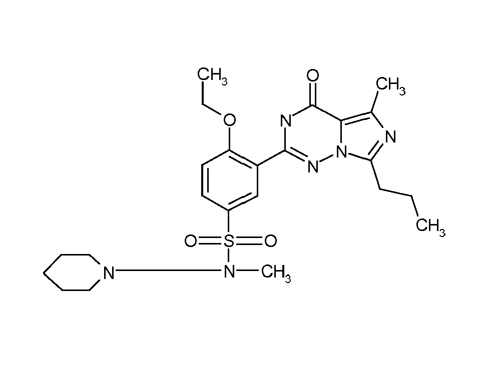 | 516,6675 | 91 | 517 |
| 139 | 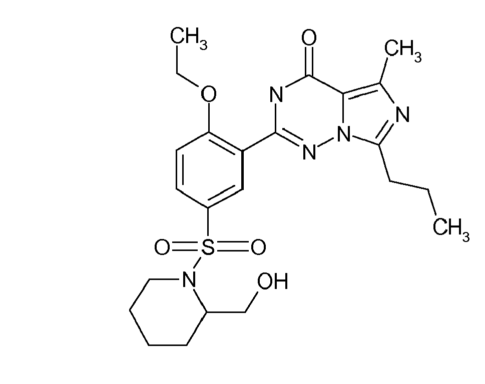 | 489,598 | 85 | 490 |
| 140 | 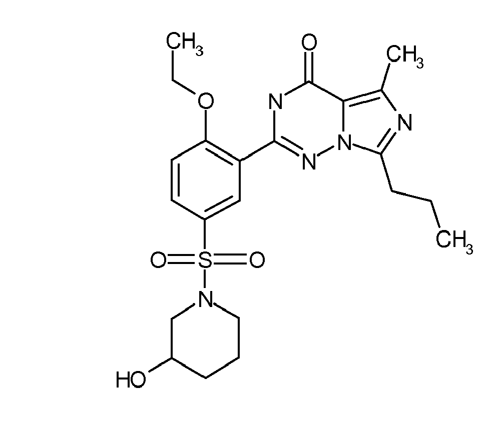 | 475,5709 | 83 | 476 |
| 141 | 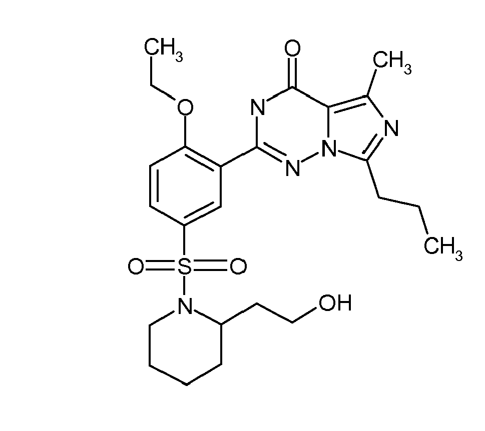 | 503,6251 | 85 | 504 |
| 142 | 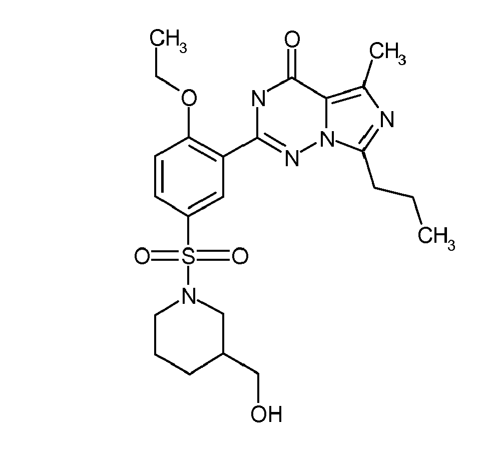 | 489,598 | 91 | 490 |
| 143 | 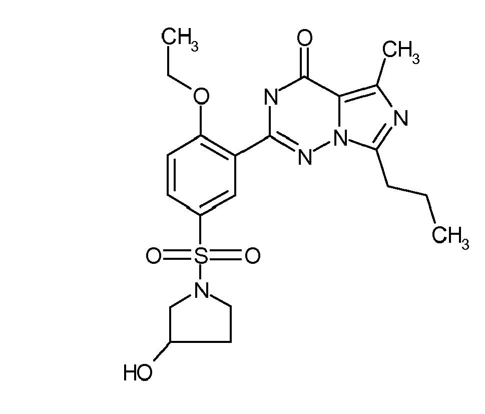 | 461,5438 | 78 | 462 |
| 144 | 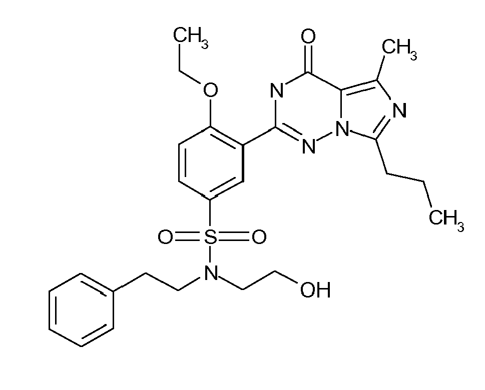 | 539,6586 | 88 | 540 |
| 145 | 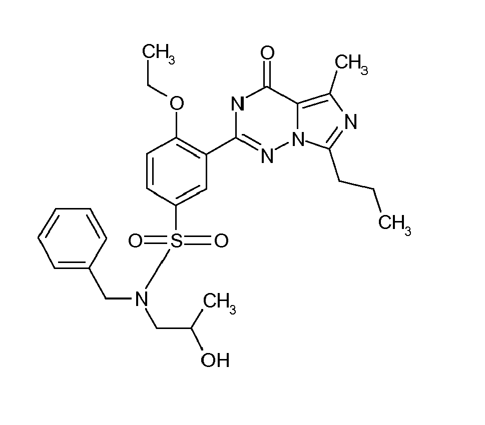 | 539,6586 | 58 | 538 |
| 146 | 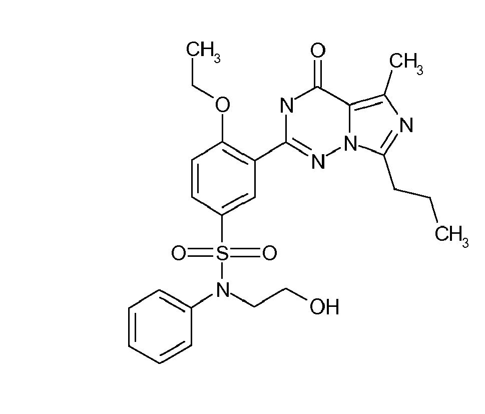 | 511,6044 | 80 | 512 |
| 147 | 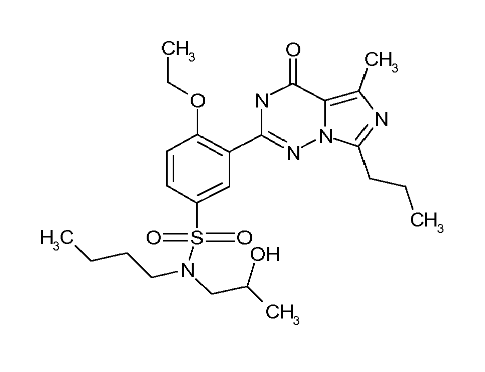 | 505,6411 | 90 | 506 |

| **Tabelle 3:** | | | | |
|---|---|---|---|---|
| **Bsp.-Nr.** | **Struktur** | **MG** **[g/mol]** | **HPLC** | **Mz + H** |
| 148 | 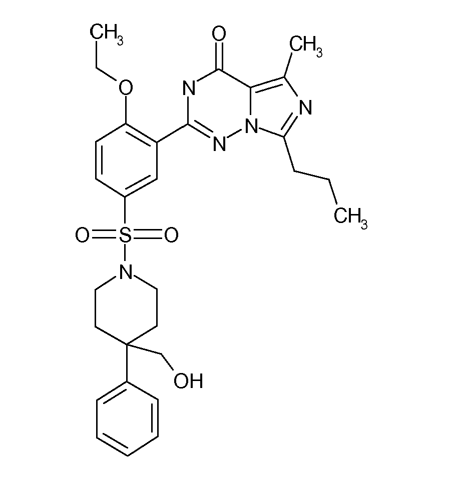 | 565,70 | 38 | 566 |
| 149 | 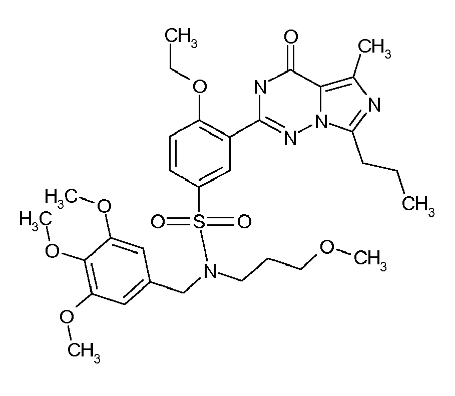 | 643,77 | 85 | 644 |
| 150 | 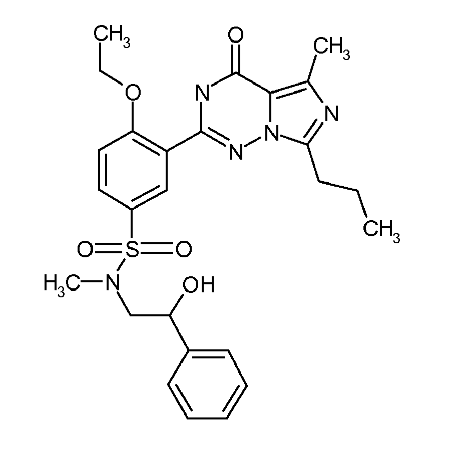 | 525,63 | 80 | 526 |
| 151 | 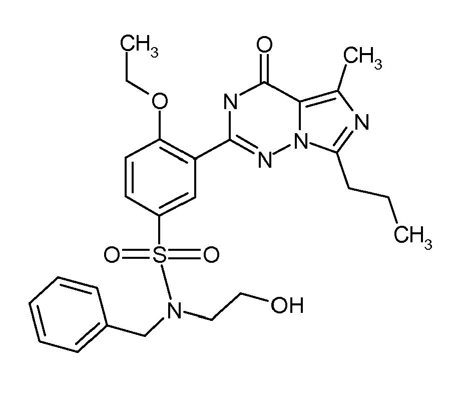 | 525,63 | 78 | 526 |
| 152 | 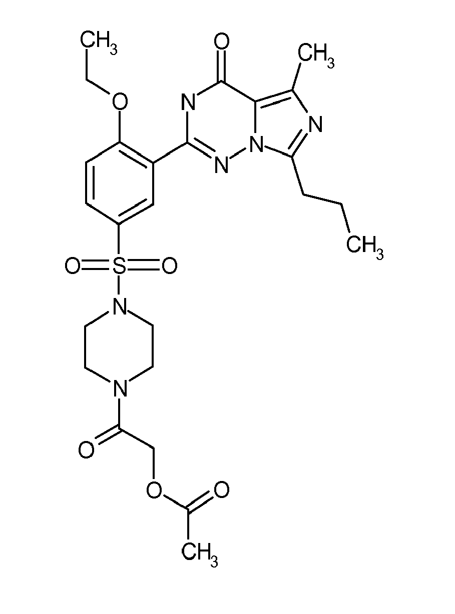 | 560,63 | 51 | 561 |
| 153 | 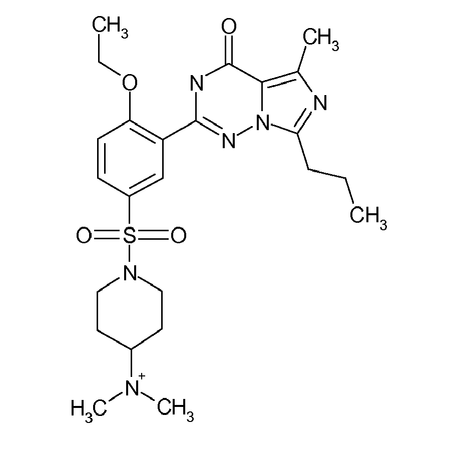 | 503,65 | 78 | 504 |
| 154 | 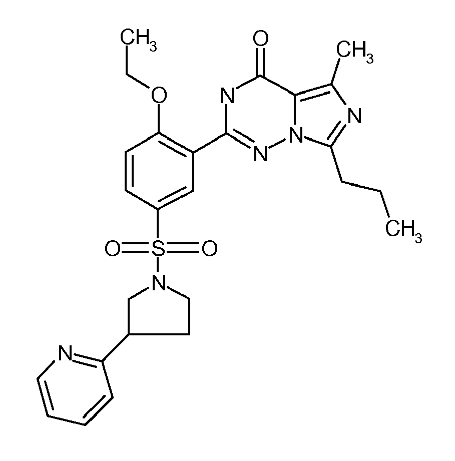 | 522,63 | 82 | 523 |
| 155 | 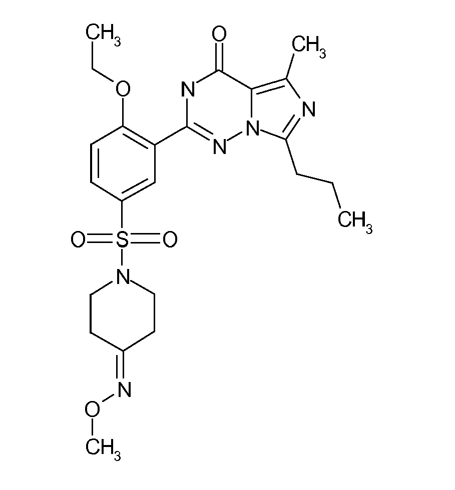 | 502,60 | 84 | 503 |
| 156 | 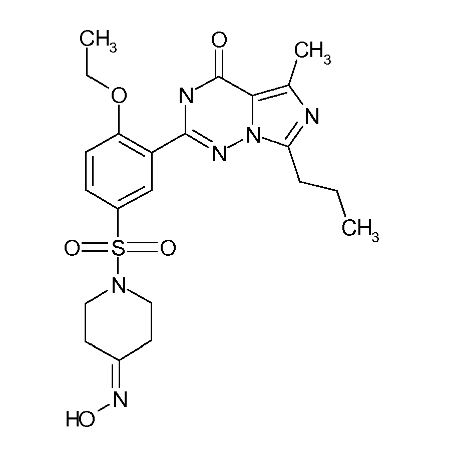 | 488,57 | 83 | 489 |
| 157 | 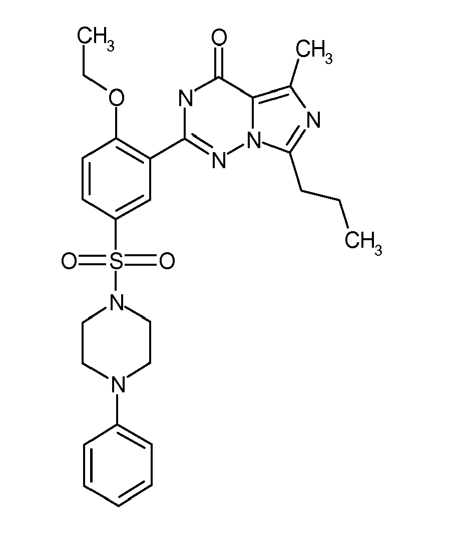 | 536,66 | 82 | 537 |
| 158 | 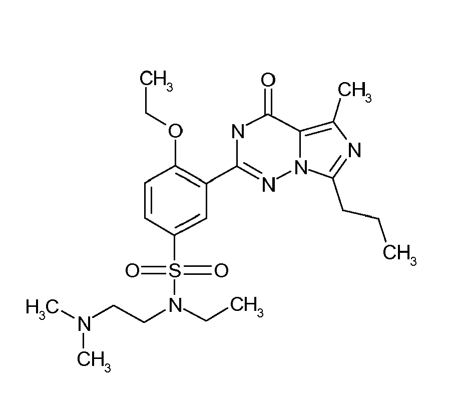 | 490,63 | 90 | 491 |
| 159 | 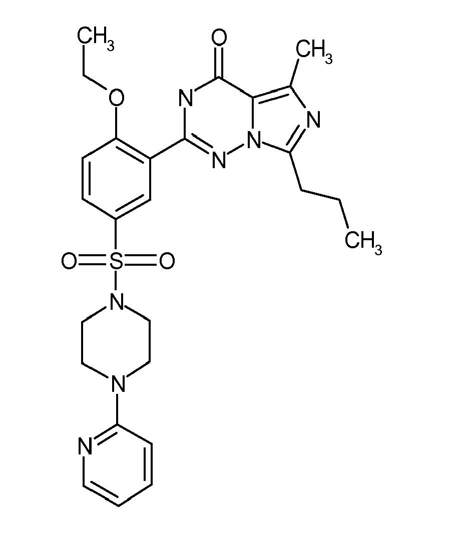 | 537,65 | 83 | 538 |
| 160 | 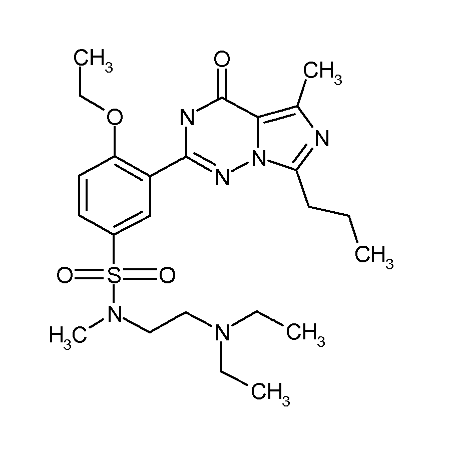 | 504,66 | 91 | 505 |
| 161 | 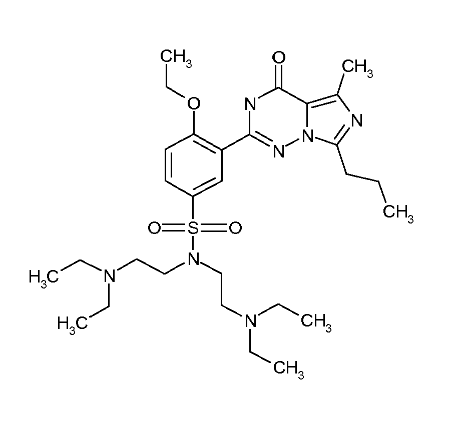 | 589,81 | 65 | 590 |
| 162 | 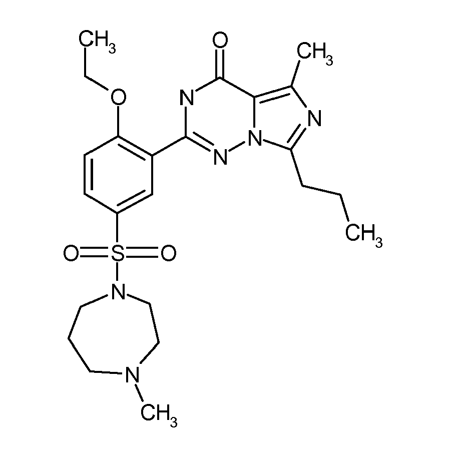 | 488,61 | 88 | 489 |
| 163 | 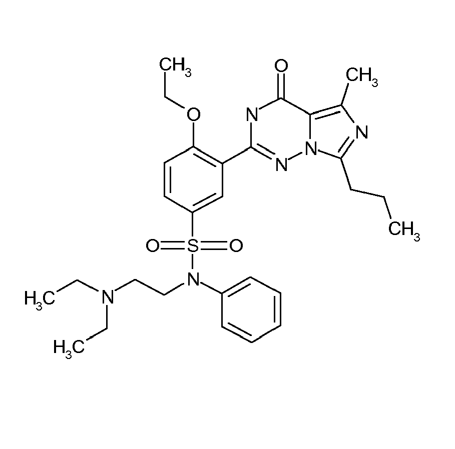 | 566,73 | 32 | 567 |
| 164 | 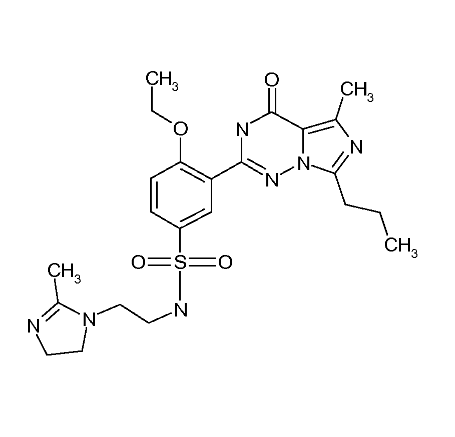 | 501,61 | 75 | 502 |
| 165 | 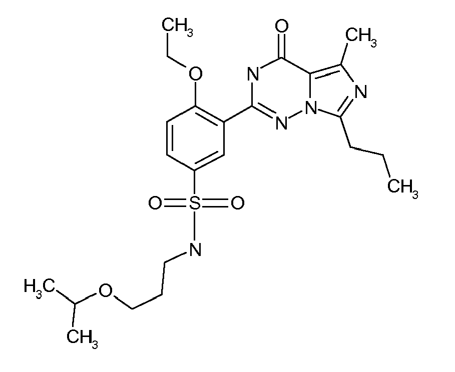 | 491,61 | 91 | 492 |
| 166 | 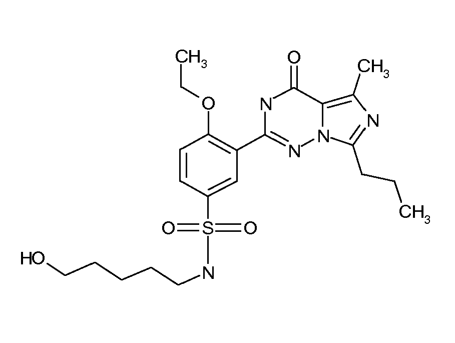 | 477,59 | 73 | 478 |
| 167 | 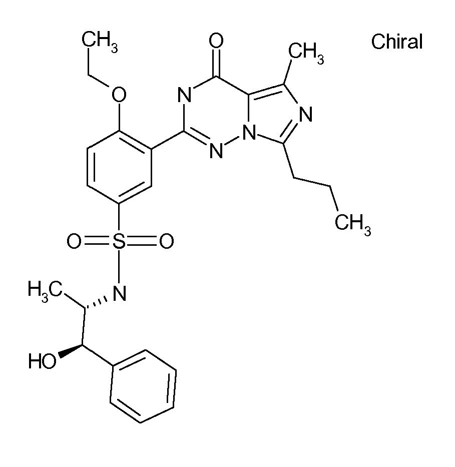 | 525,63 | 81 | 526 |
| 168 | 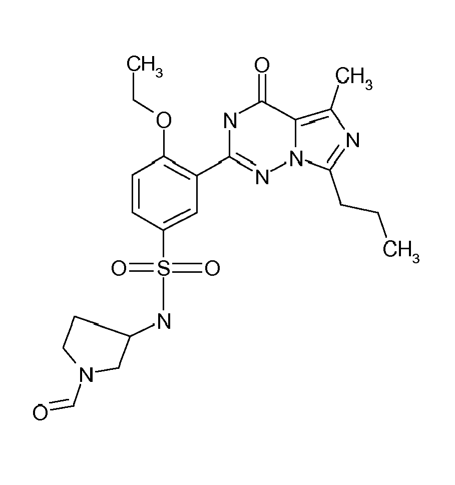 | 488,57 | 70 | 489 |
| 169 | 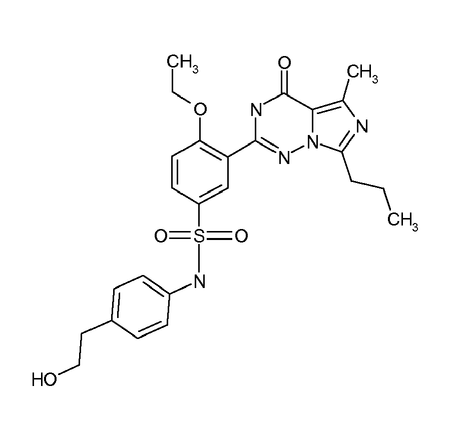 | 511,60 | 76 | 512 |
| 170 | 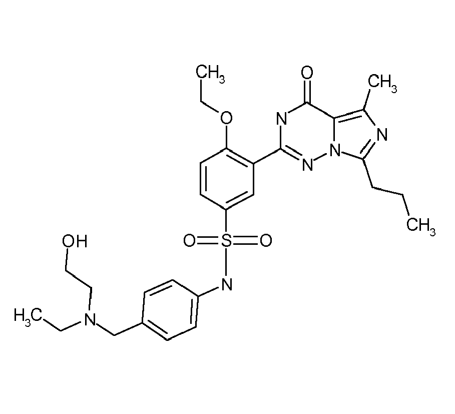 | 568,70 | 50 | 569 |
| 171 | 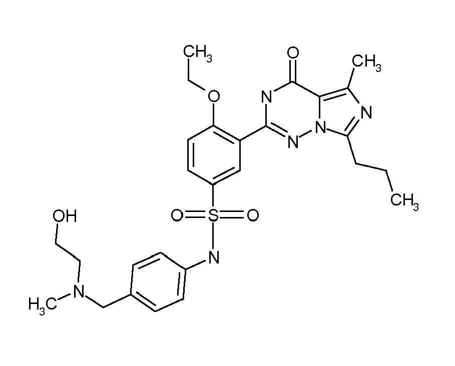 | 554,67 | 63 | 555 |
| 172 | 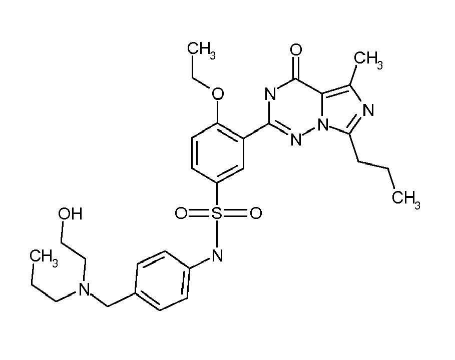 | 582,73 | 50 | 583 |
| 173 | 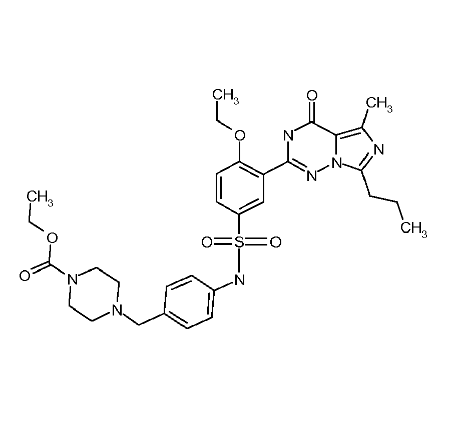 | 637,76 | 30 | 638 |
| 174 | 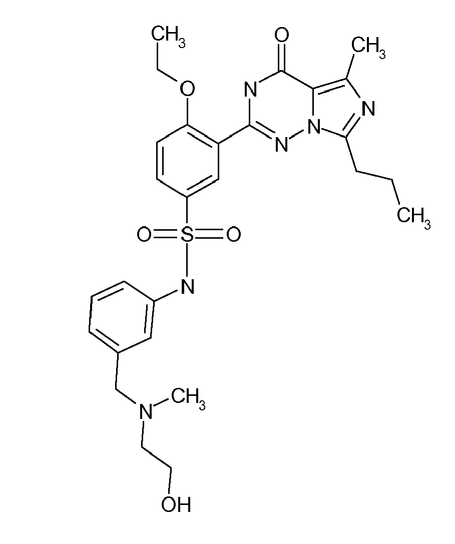 | 554,67 | 70 | 555 |
| 175 | 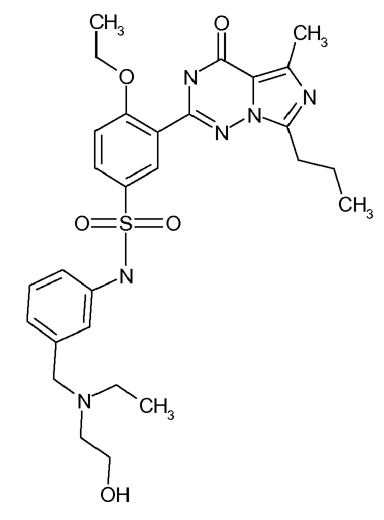 | 568,70 | 44 | 569 |

| **Tabelle 4:** | | | | |
|---|---|---|---|---|
| **Bsp.-Nr.** | **Struktur** | **MG** **[g/mol]** | **HPLC** | **Mz+H** |
| 176 | 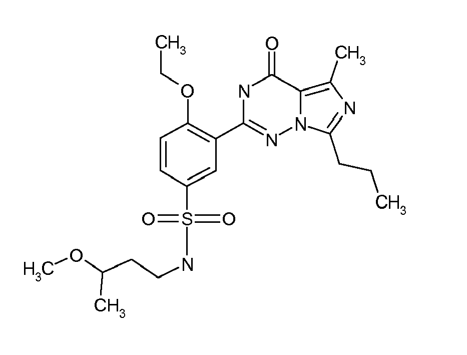 | 477,59 | 82 | 478 |
| 177 | 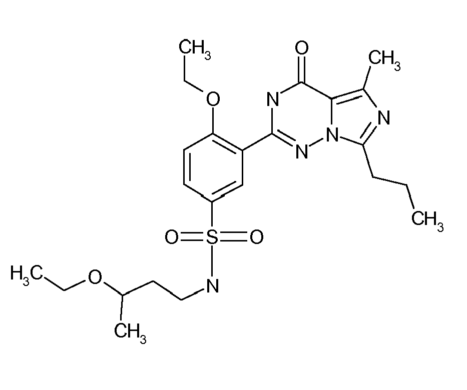 | 491,61 | 89 | 492 |
| 178 | 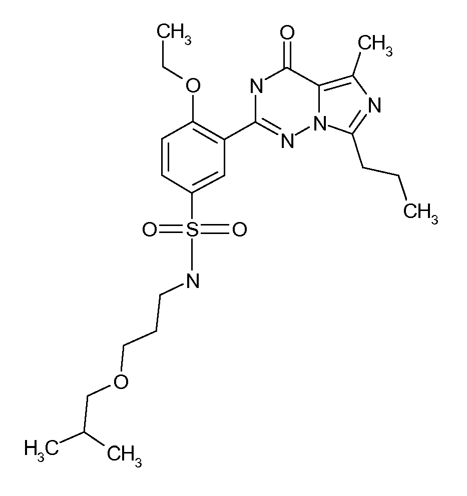 | 505,64 | 88 | 506 |
| 179 | 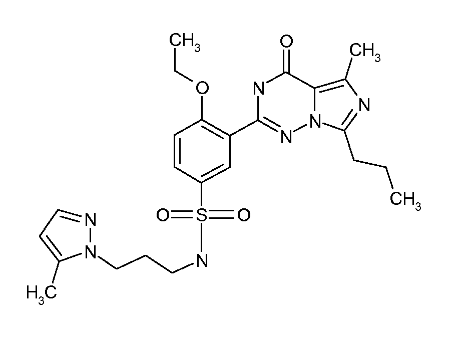 | 513,62 | 47 | 514 |
| 180 | 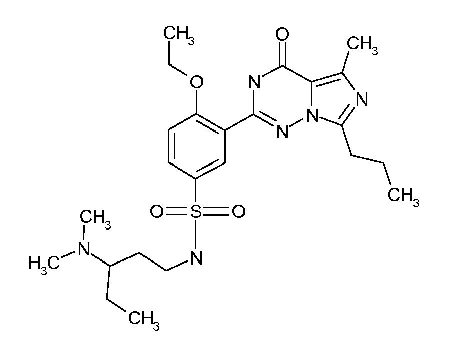 | 504,66 | 83 | 505 |
| 181 | 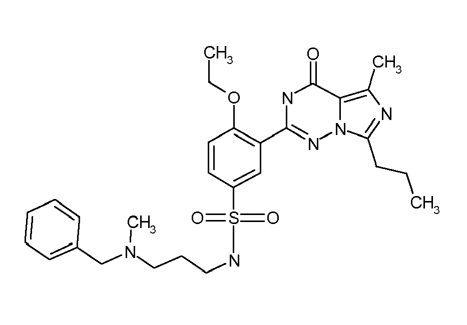 | 552,70 | 83 | 553 |
| 182 | 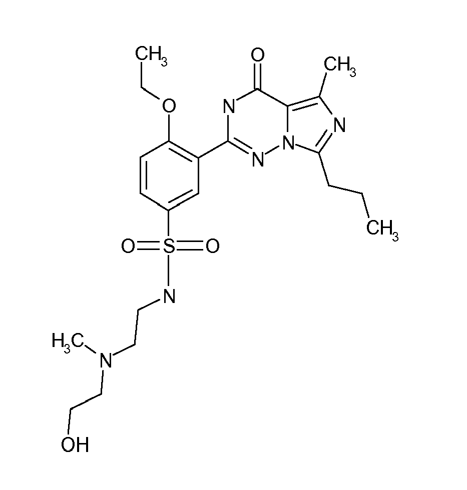 | 492,60 | 72 | 493 |
| 183 | 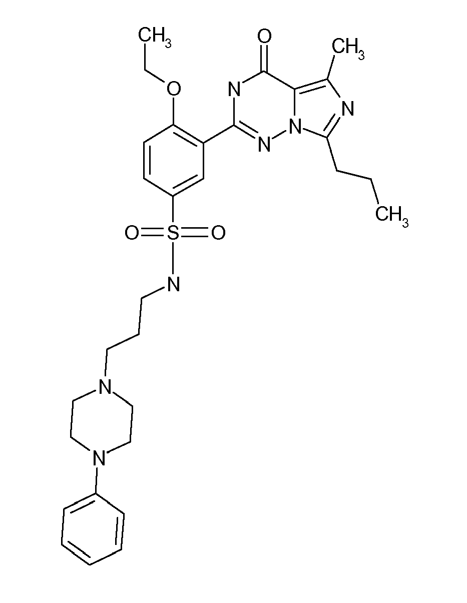 | 593,75 | 52 | 594 |
| 184 | 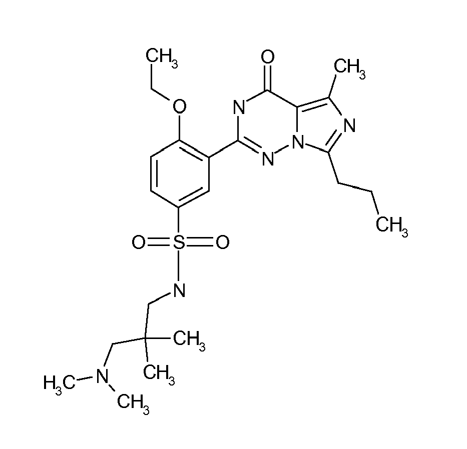 | 504,66 | 82 | 505 |
| 185 | 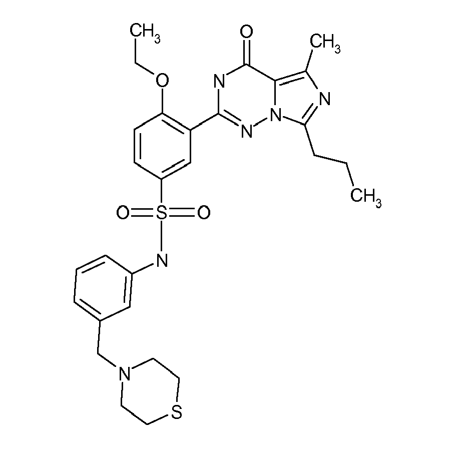 | 582,75 | 59 | 583 |
| 186 | 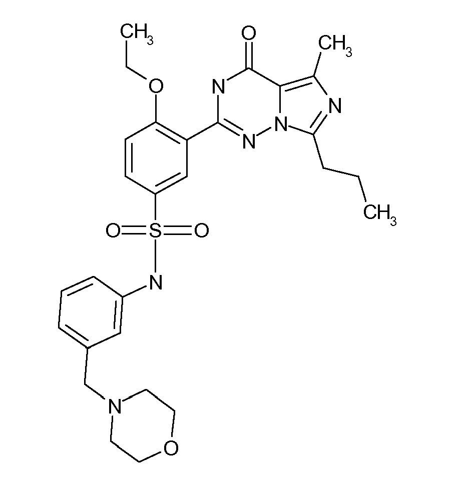 | 566,68 | 60 | 567 |
| 187 | 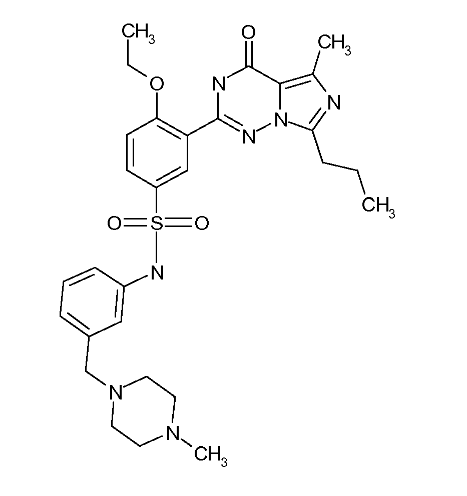 | 579,73 | 30 | 580 |
| 188 | 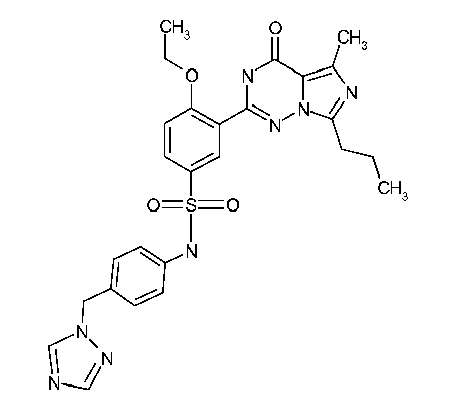 | 548,63 | 73 | 549 |
| 189 | 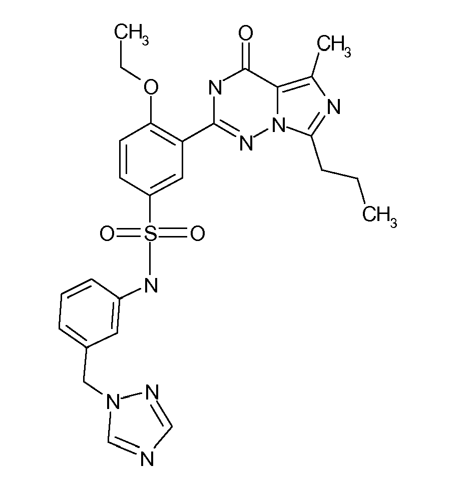 | 548,63 | 72 | 549 |
| 190 | 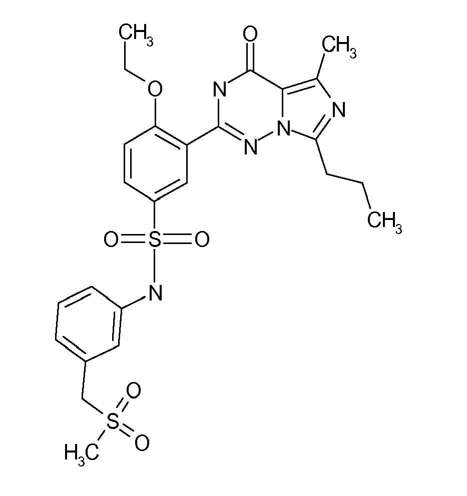 | 559,67 | 54 | 560 |
| 191 | 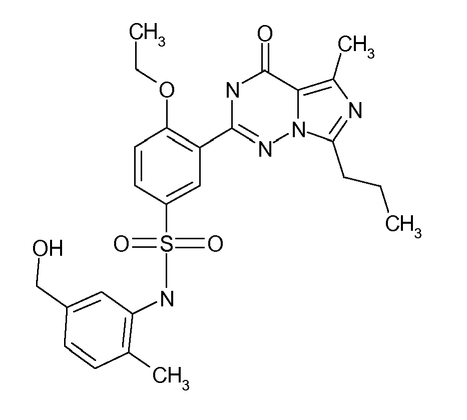 | 511,60 | 70 | 512 |
| 192 | 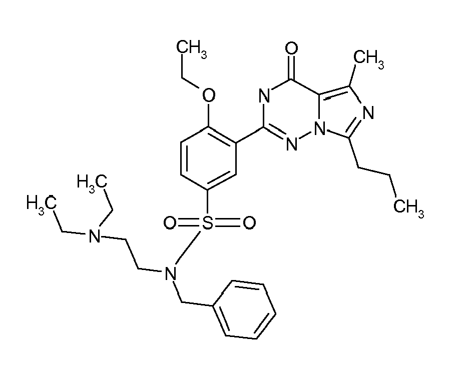 | 580,76 | 68 | 581 |
| 193 | 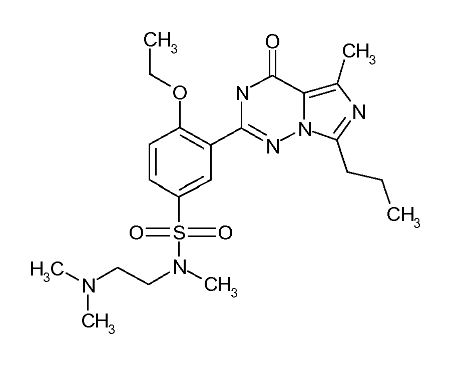 | 476,60 | 89 | 477 |
| 194 | 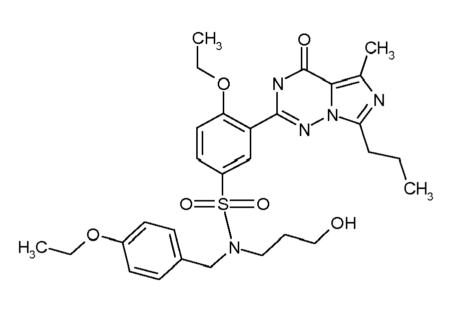 | 583,71 | 80 | 584 |
| 195 | 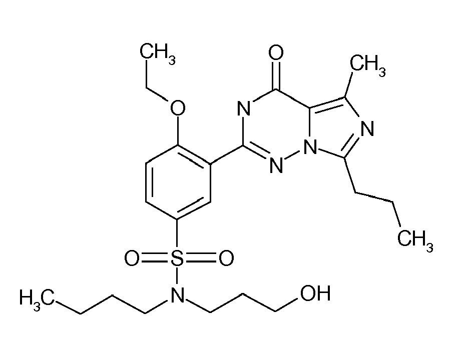 | 505,64 | 84 | 506 |
| 196 | 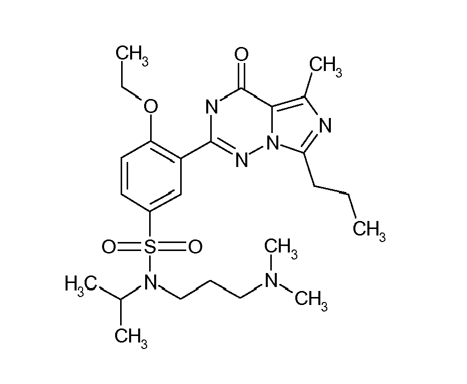 | 518,68 | 40 | 519 |
| 197 | 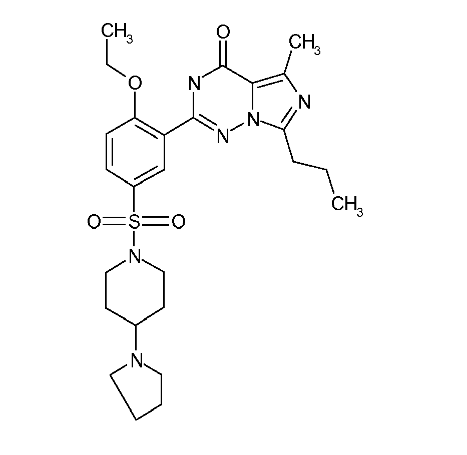 | 528,68 | 82 ? | 529 |
| 198 | 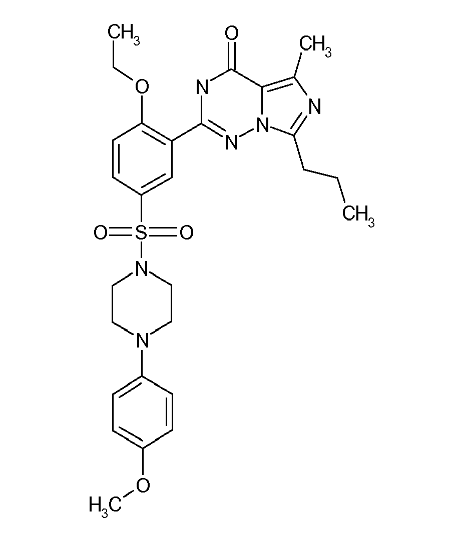 | 566,68 | 63 | 567 |
| 199 | 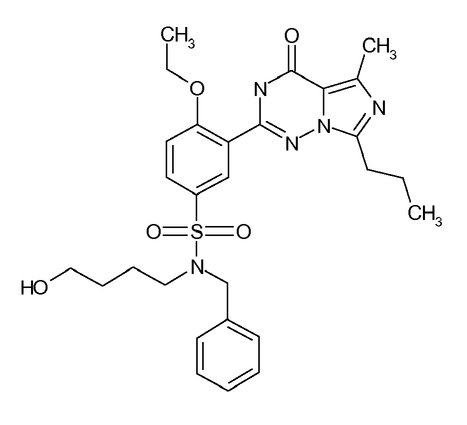 | 553,69 | 87 | 554 |

| **Tabelle 5** | | | | |
|---|---|---|---|---|
| **Bsp.-Nr.** | **Struktur** | **MW** | **HPLC** | **MZ+H** |
| 200 | 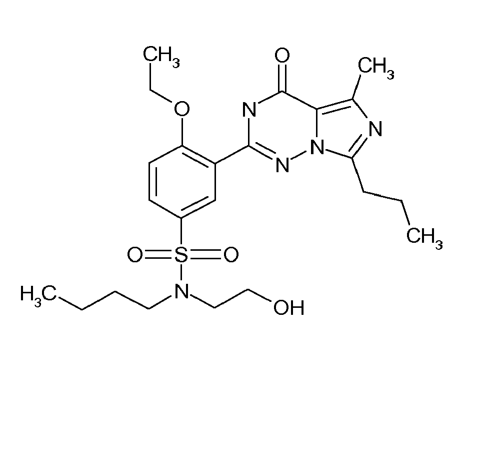 | 491,61 | 82 | 505 |
| 201 | 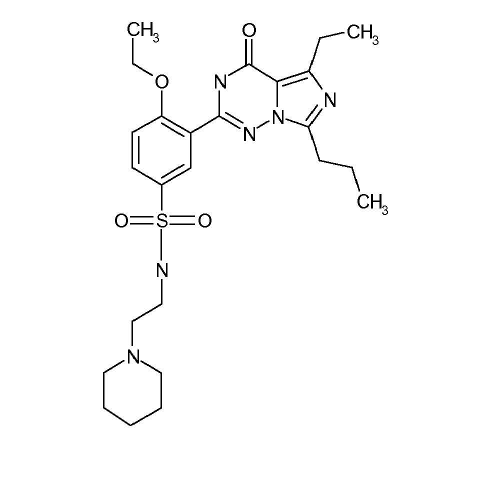 | 516,668 | 87 | 517 |
| 202 | 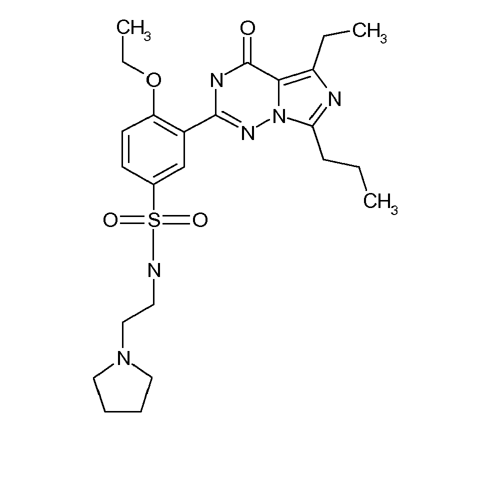 | 502,64 | 84 | 503 |
| 203 | 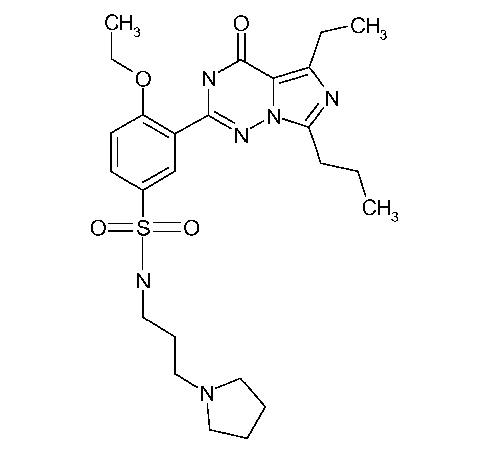 | 516,668 | 87 | 517 |
| 204 | 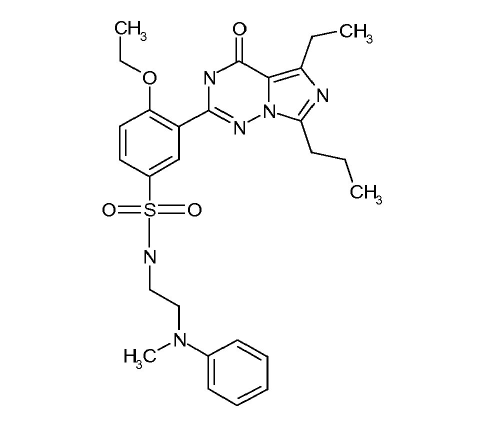 | 538,674 | 91 | 539 |
| 205 | 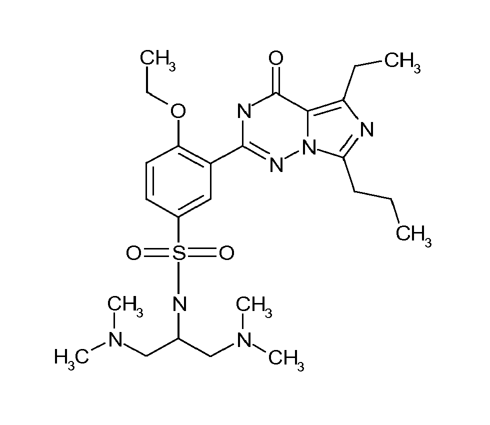 | 533,698 | 85 | 534 |
| 206 | 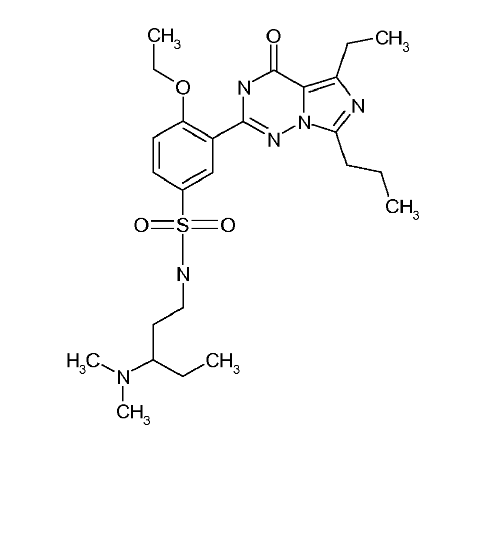 | 518,683 | 77 | 519 |
| 207 | 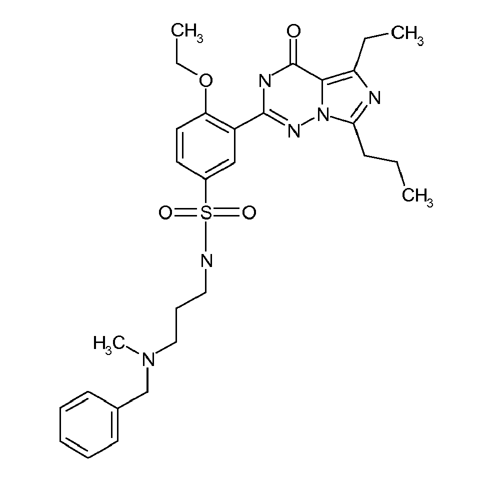 | 566,728 | 92 | 567 |
| 208 | 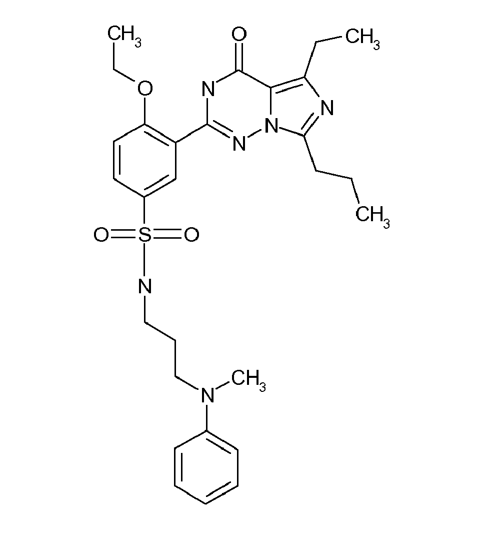 | 552,701 | 87 | 553 |
| 209 | 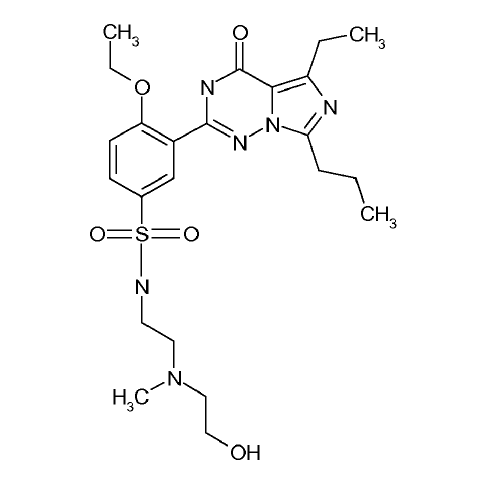 | 506,629 | 52 | 507 |
| 210 | 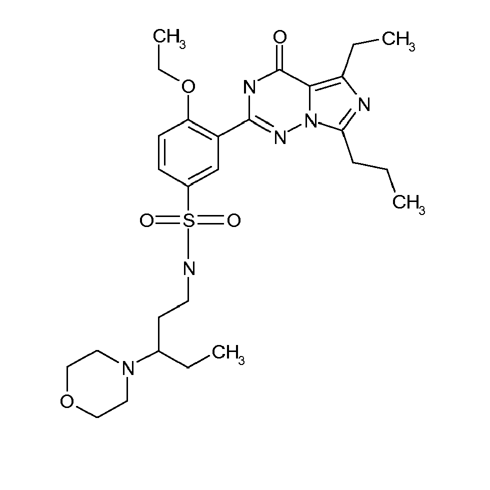 | 560,721 | 62 | 561 |
| 211 | 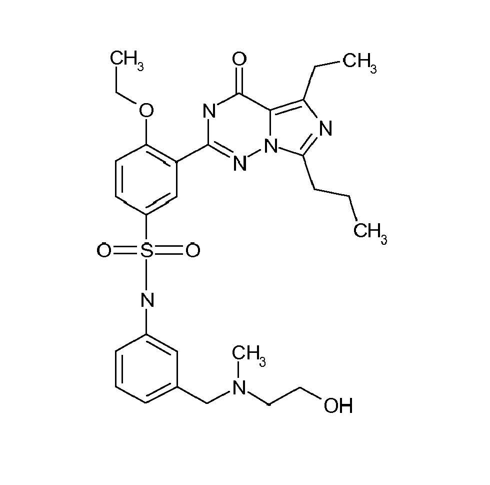 | 568,7 | 88 | 569 |
| 212 | 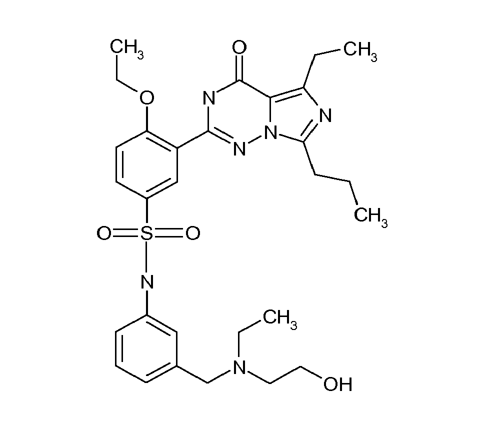 | 582,727 | 89 | 583 |
| 213 | 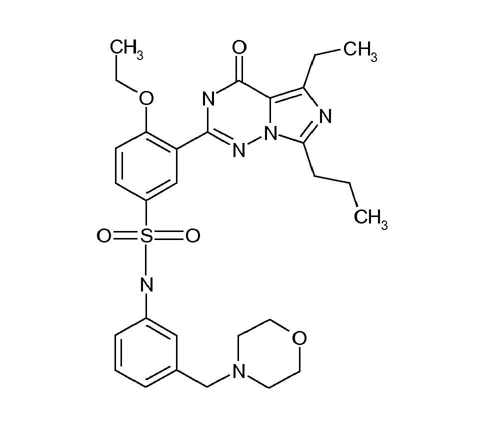 | 580,712 | 83 | 581 |
| 214 | 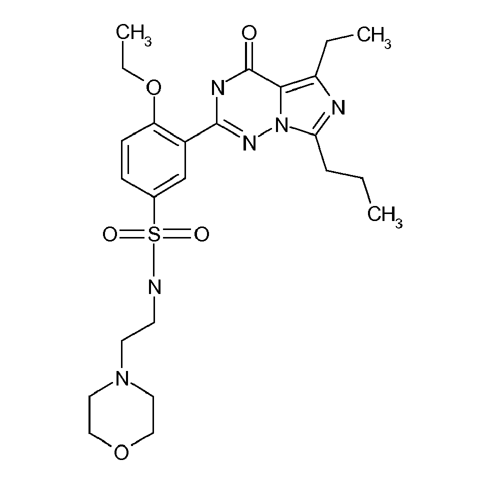 | 518,64 | 89 | 519 |
| 215 | 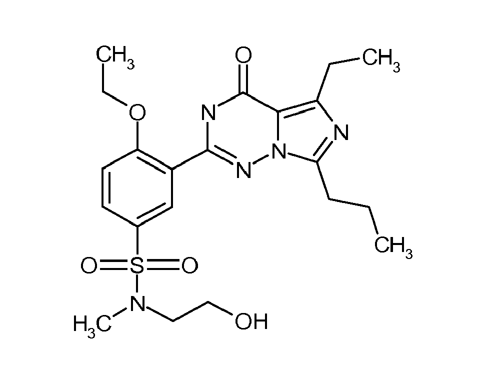 | 463,56 | 90 | 464 |
| 216 | 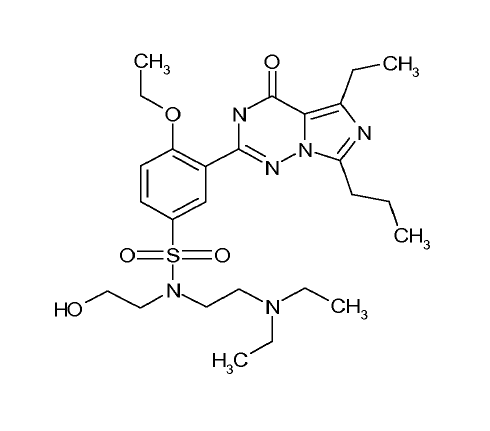 | 548,71 | 78 | 549 |
| 217 | 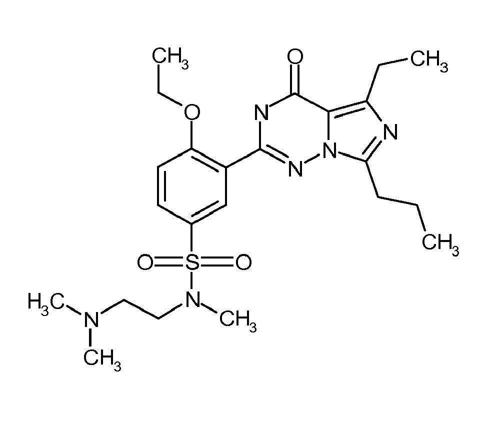 | 490,629 | 87 | 491 |
| 218 | 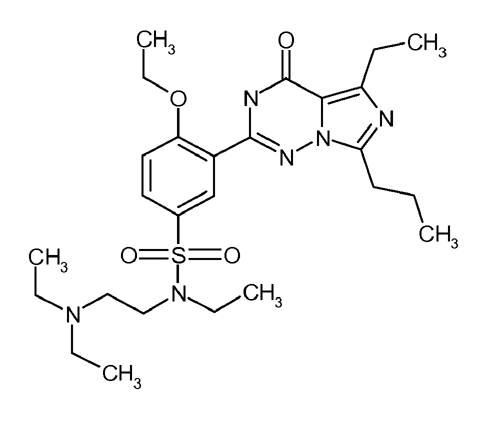 | 532,711 | 93 | 533 |
| 219 | 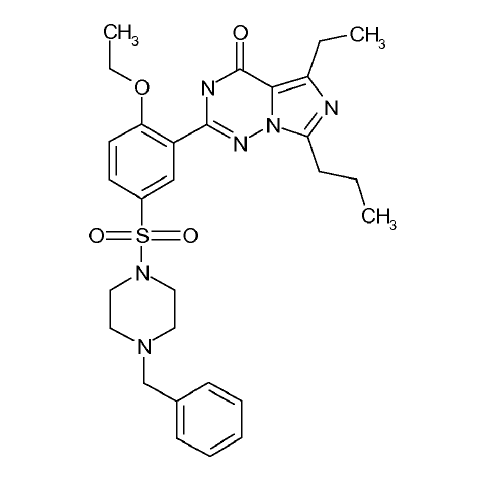 | 564,712 | 91 | 565 |
| 220 | 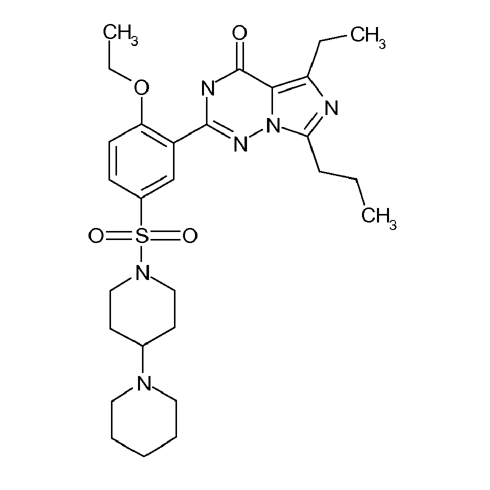 | 556,733 | 92 | 557 |
| 221 | 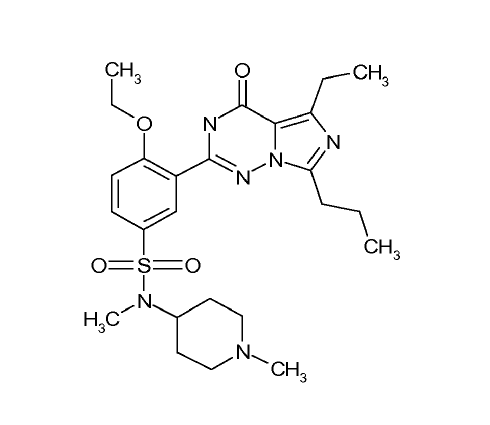 | 516,668 | 92 | 517 |
| 222 | 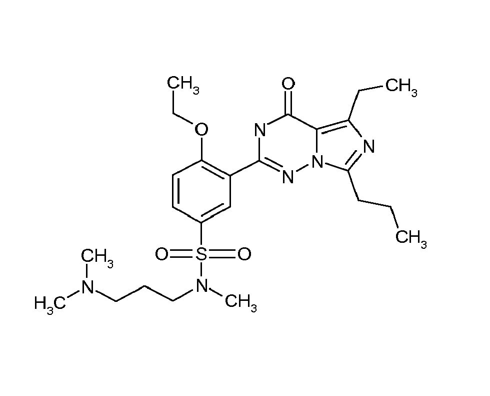 | 504,656 | 83 | 505 |
| 223 | 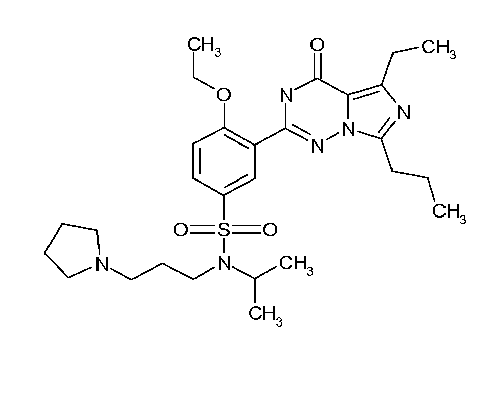 | 558,749 | 90 | 559 |
| 224 | 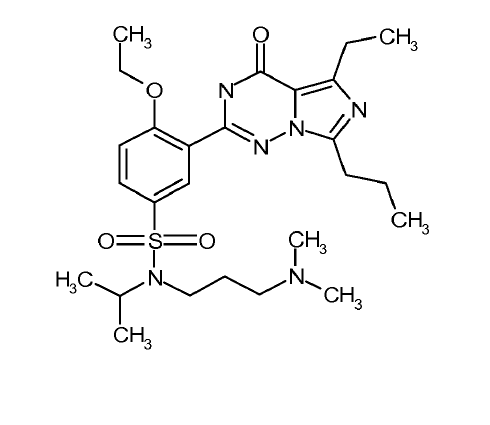 | 532,711 | 86 | 533 |
| 225 | 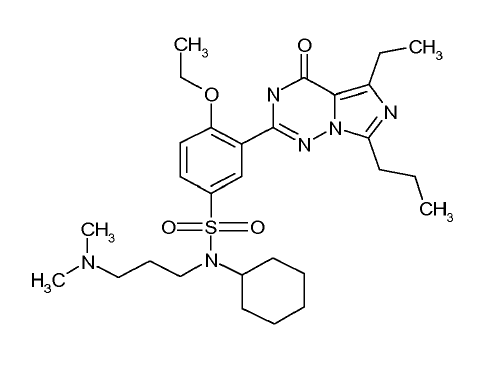 | 572,776 | 68 | 573 |
| 226 | 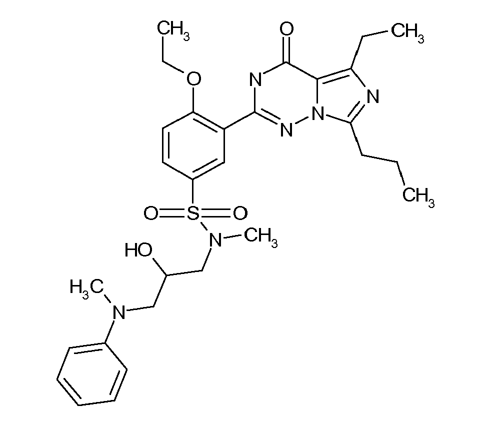 | 582,727 | 87 | 583 |
| 227 | 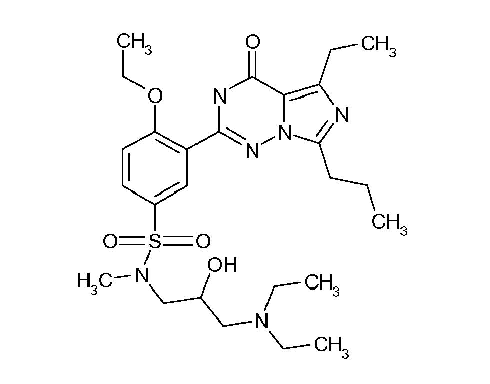 | 548,71 | 85 | 549 |
| 228 | 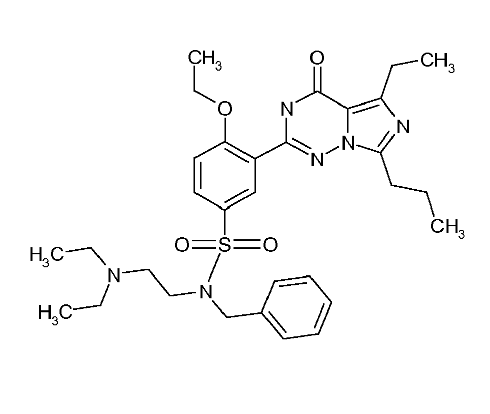 | 594,782 | 97 | 595 |
| 229 | 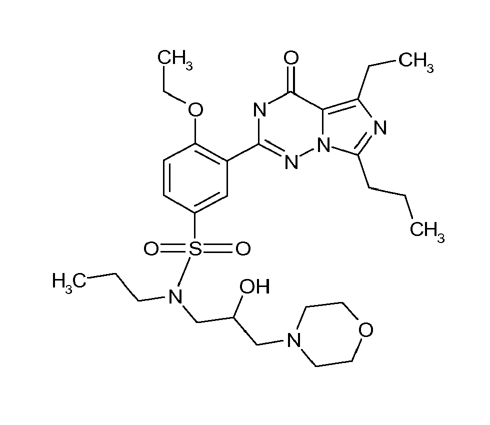 | 590,748 | 90 | 591 |
| 230 | 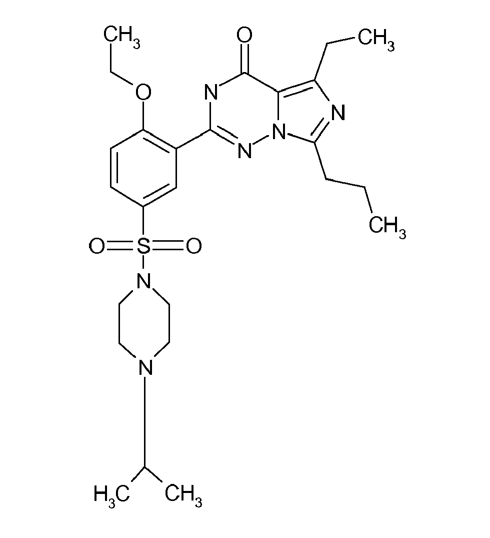 | 530,695 | 95 | 531 |
| 231 | 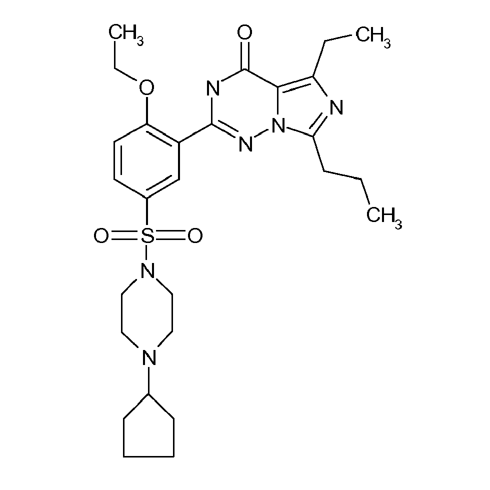 | 542,706 | 88 | 543 |
| 232 | 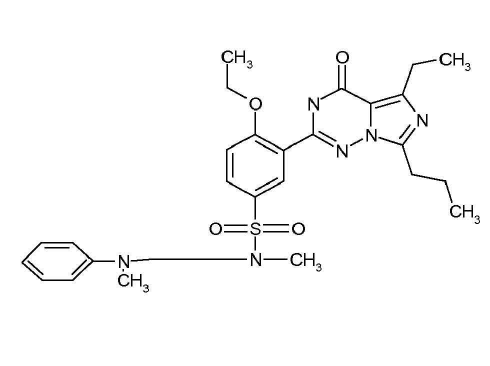 | 552,701 | 91 | 553 |
| 233 | 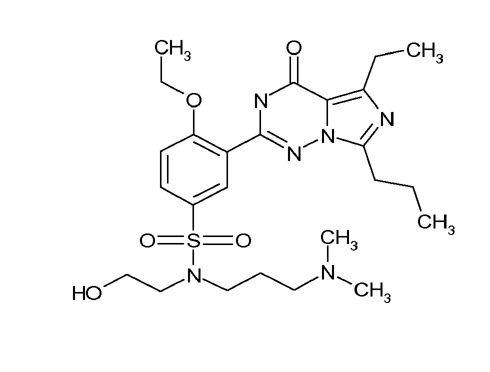 | 534,683 | 65 | 535 |
| 234 | 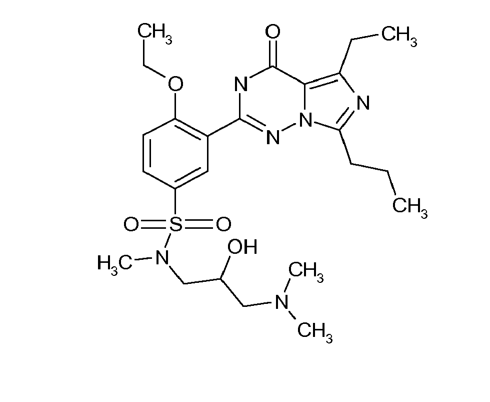 | 520,656 | 83 | 521 |
| 235 | 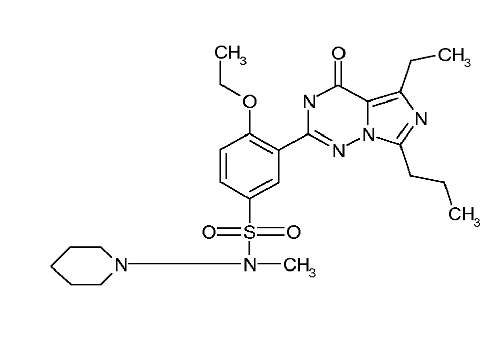 | 530,695 | 89 | 531 |
| 236 | 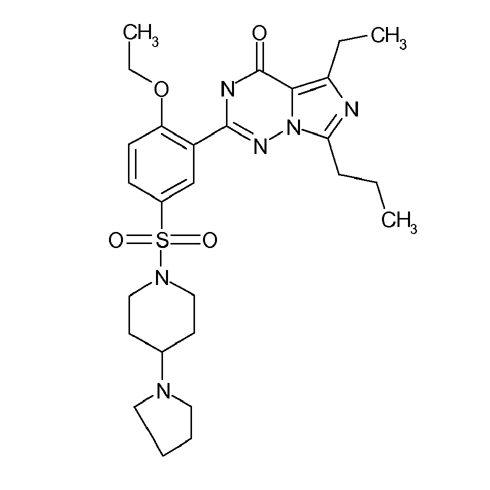 | 542,706 | 70 | 543 |
| 237 | 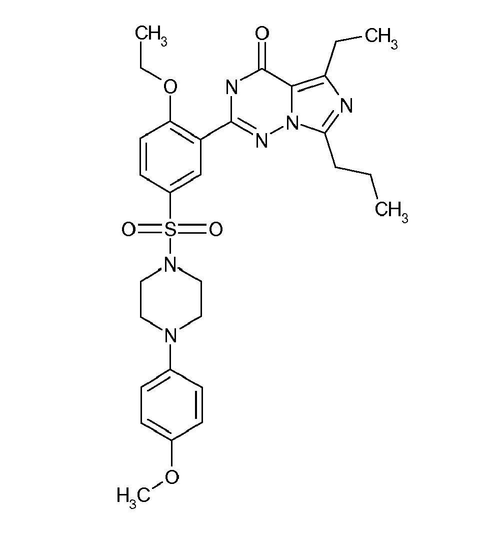 | 580,712 | 81 | 581 |
| 238 | 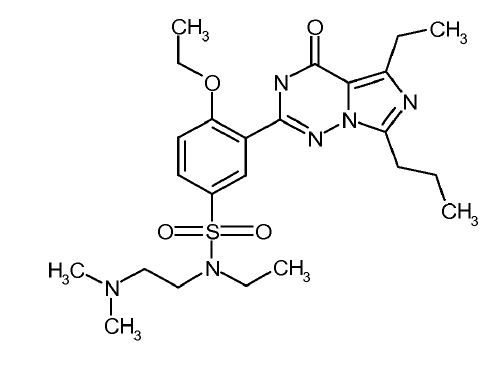 | 504,656 | 81 | 505 |
| 239 | 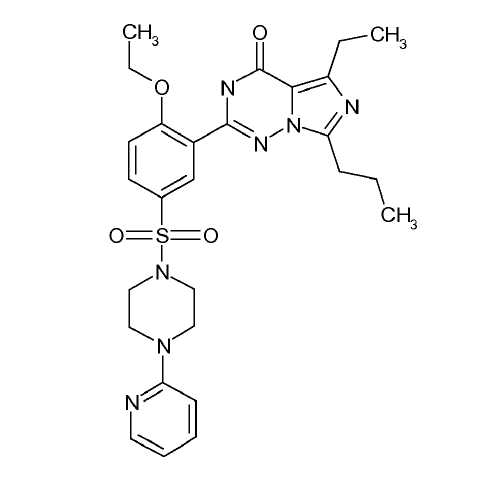 | 551,673 | 86 | 552 |
| 240 | 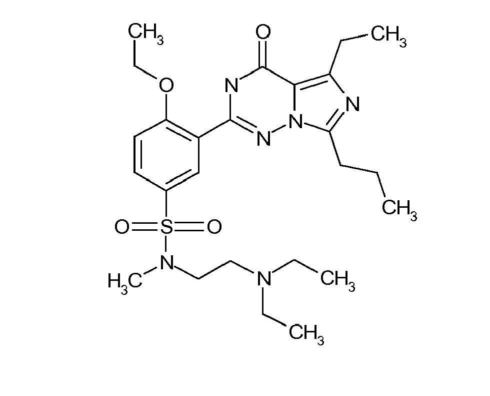 | 518,683 | 85 | 519 |
| 241 | 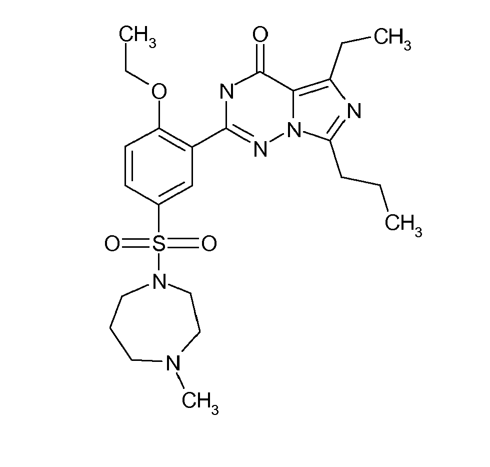 | 502,64 | 85 | 503 |
| 242 | 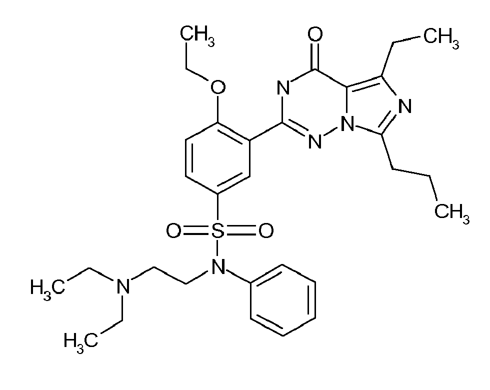 | 580,755 | 79 | 581 |

| **Tabelle 6** | | | | |
|---|---|---|---|---|
| **Bsp.-Nr.** | **Struktur** | **MW** | **HPLC** | **MZ+H** |
| 243 | 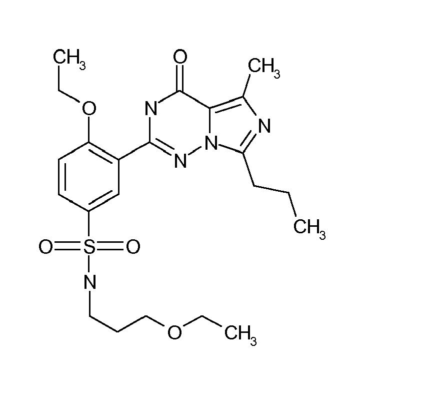 | 477,5869 | 86 | 478 |
| 244 | 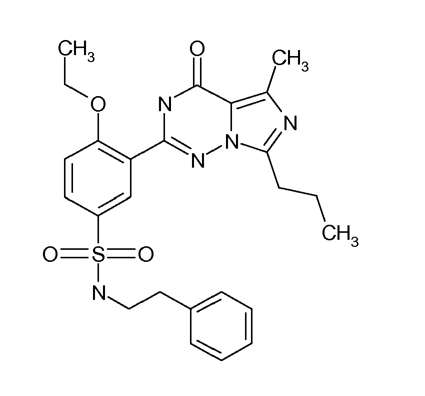 | 495,605 | 62 | 496 |
| 245 | 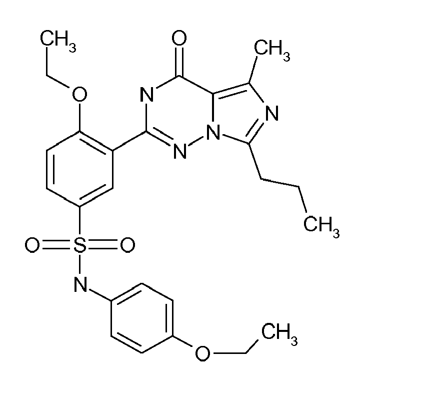 | 511,6044 | 50 | 512 |
| 246 | 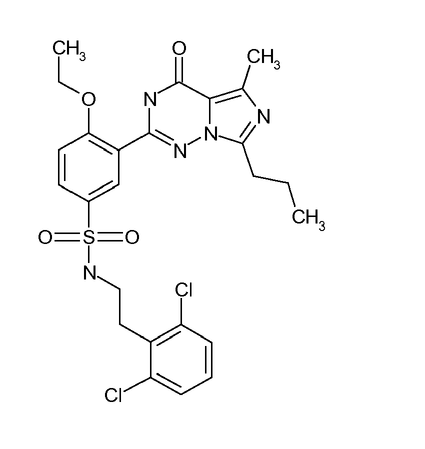 | 564,495 | 40 | 565 |
| 247 | 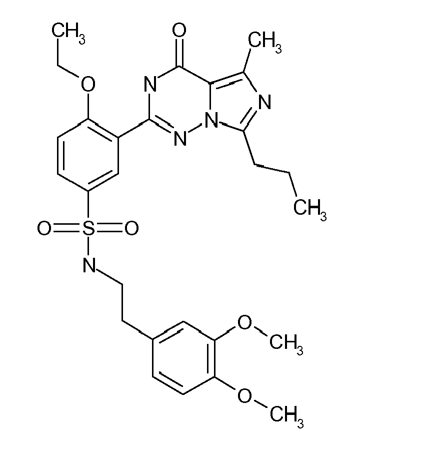 | 555,658 | 61 | 556 |
| 248 | 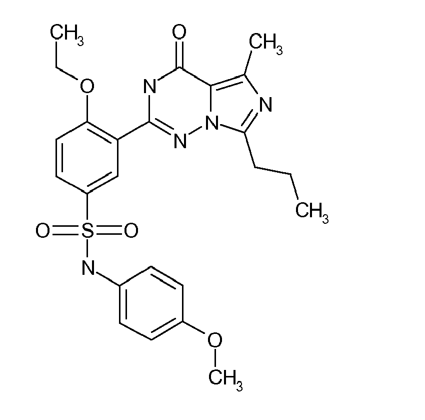 | 497,5773 | 60 | 498 |
| 249 | 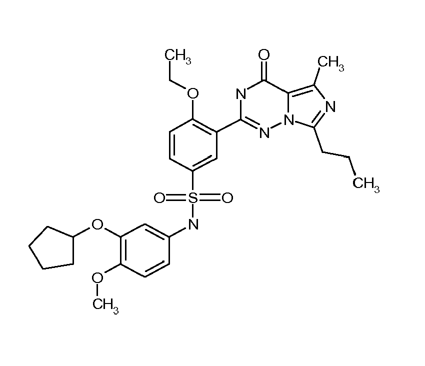 | 581,6963 | 77 | 582 |
| 250 | 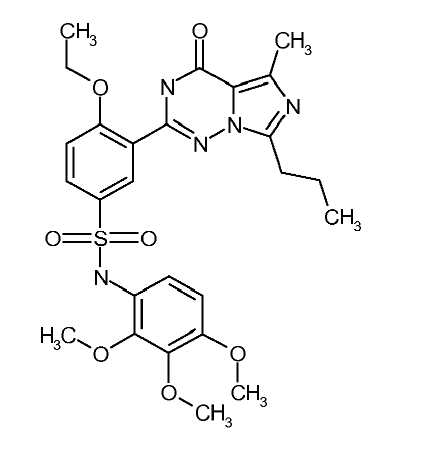 | 557,6303 | 76 | 558 |
| 251 | 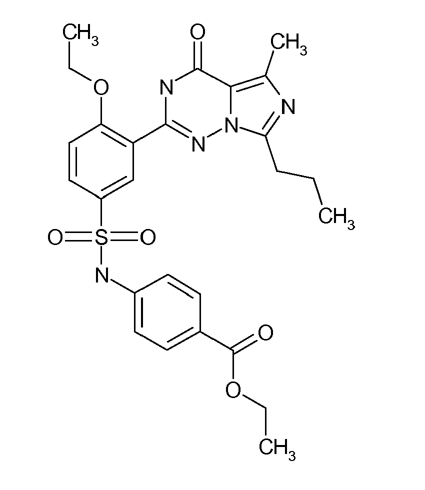 | 539,615 | 74 | 540 |
| 252 | 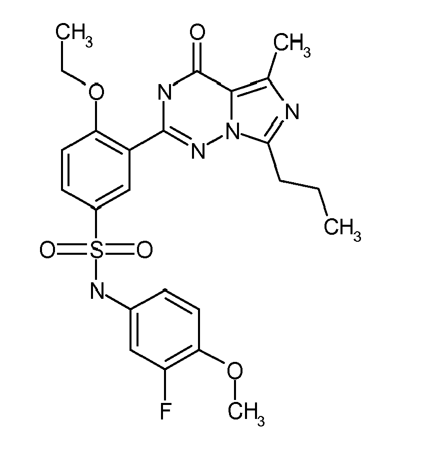 | 515,5677 | 64 | 516 |
| 253 | 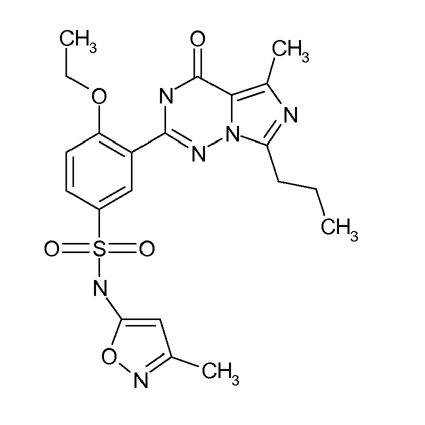 | 472,5266 | 38 | 473 |
| 254 | 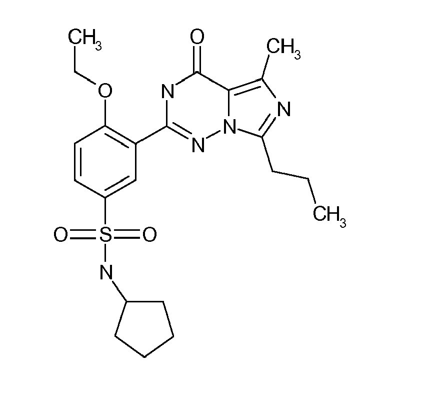 | 459,5715 | 88 | 460 |
| 255 | 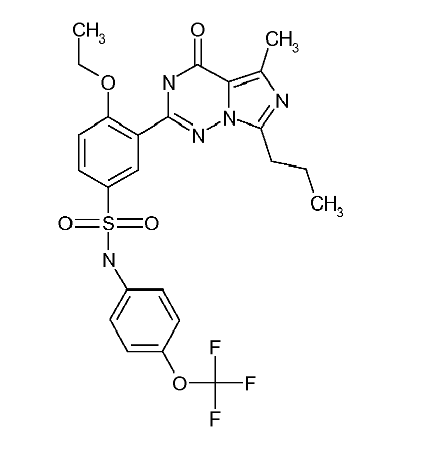 | 551,5486 | 78 | 552 |
| 256 | 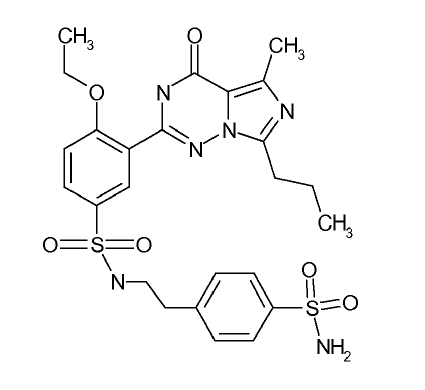 | 574,6824 | 59 | 575 |
| 257 | 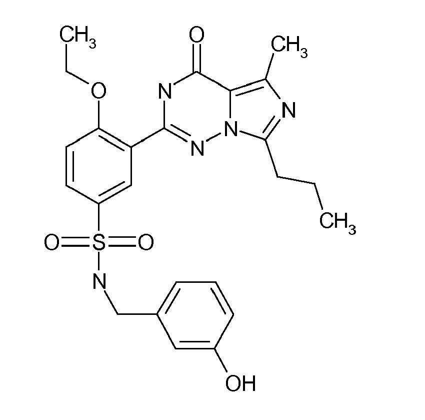 | 497,5773 | 40 | 498 |
| 258 | 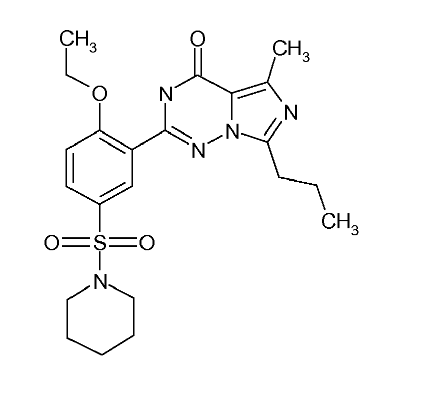 | 459,5715 | 90 | 460 |
| 259 | 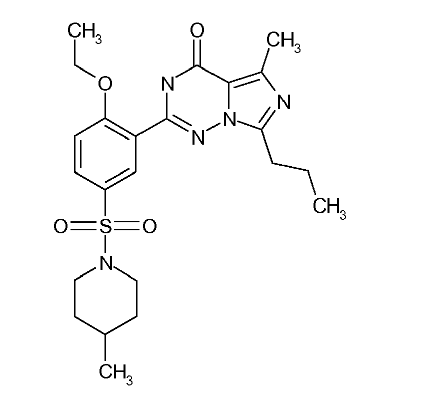 | 473,5986 | 80 | 474 |
| 260 | 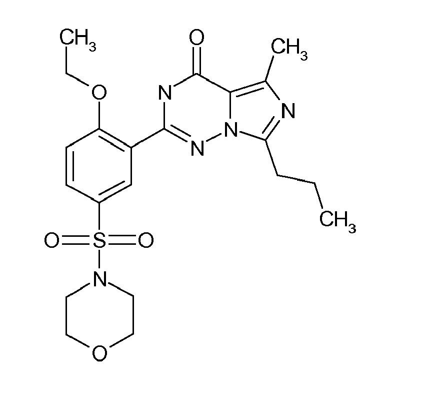 | 461,5439 | 83 | 462 |
| 261 | 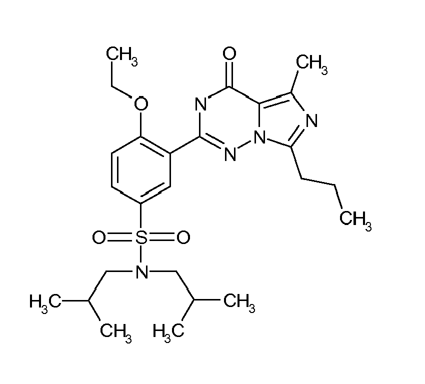 | 503,6687 | 71 | 504 |
| 262 | 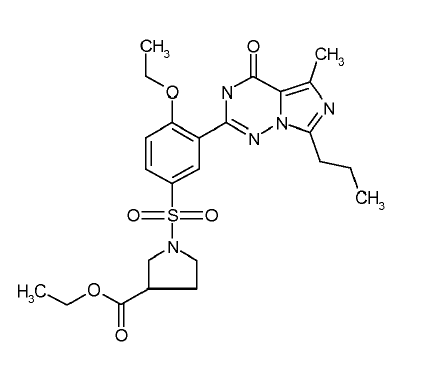 | 517,6086 | 71 | 518 |
| 263 | 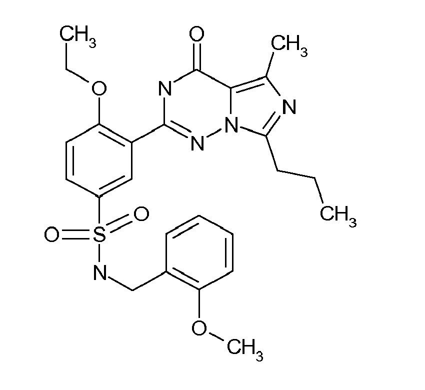 | 511,6044 | 76 | 512 |
| 264 | 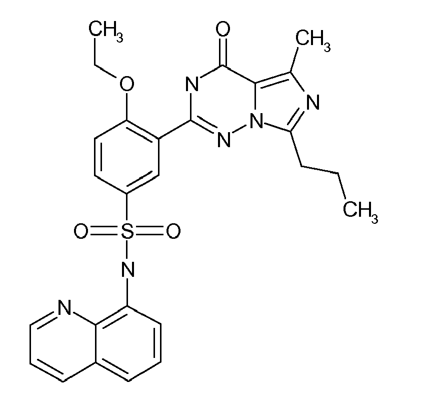 | 518,5989 | 74 | 519 |
| 265 | 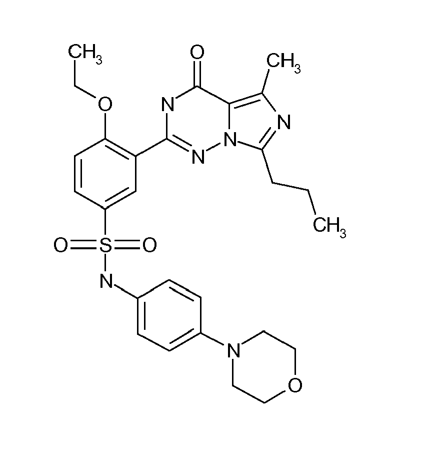 | 552,6573 | 91 | 553 |
| 266 | 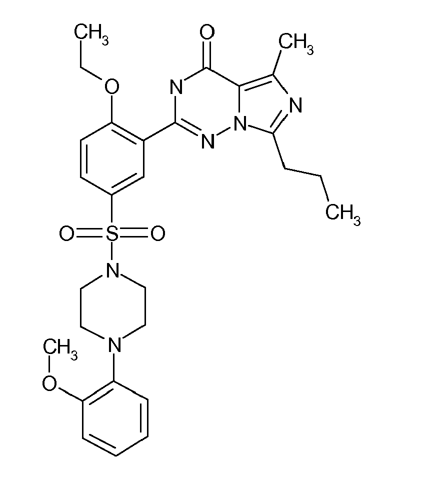 | 566,6844 | 71 | 567 |
| 267 | 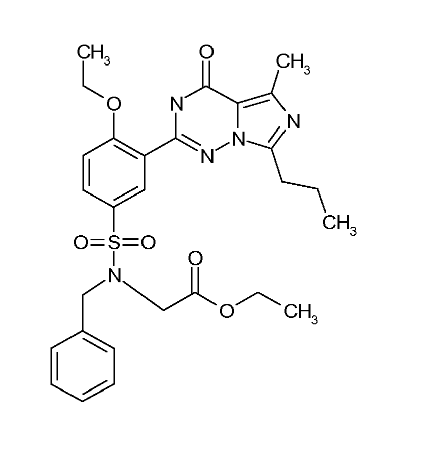 | 567,6692 | 48 | 568 |
| 268 | 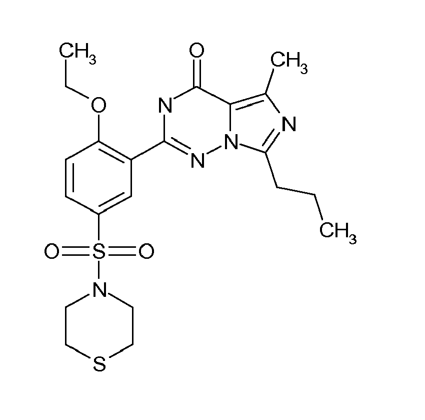 | 477,6084 | 90 | 478 |
| 269 | 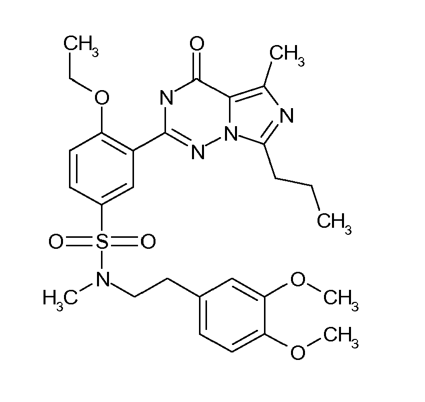 | 569,6851 | 73 | 570 |
| 270 | 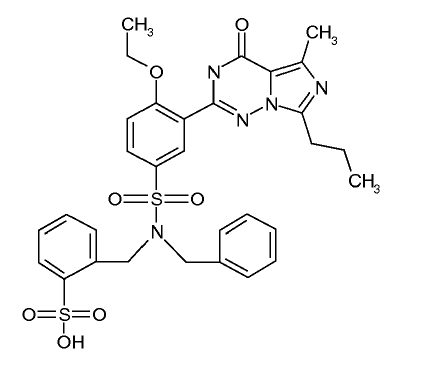 | 651,766 | 65 | 652 |
| 271 | 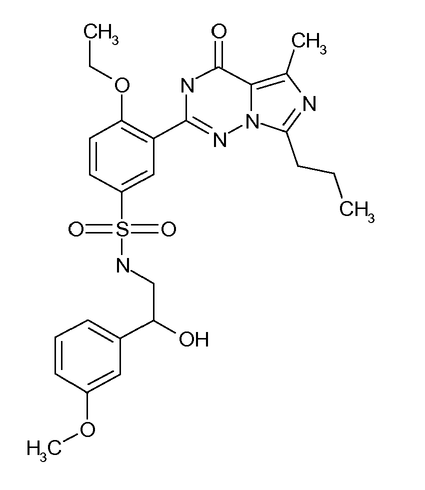 | 541,6309 | 71 | 542 |
| 272 | 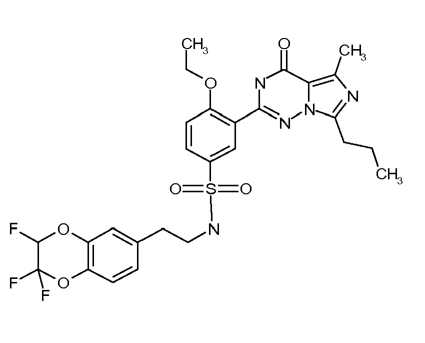 | 607,6133 | 39 | 608 |
| 273 | 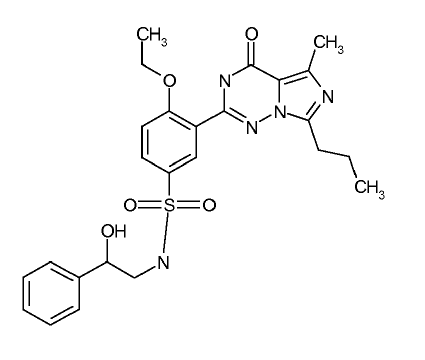 | 511,6044 | 92 | 512 |
| 274 | 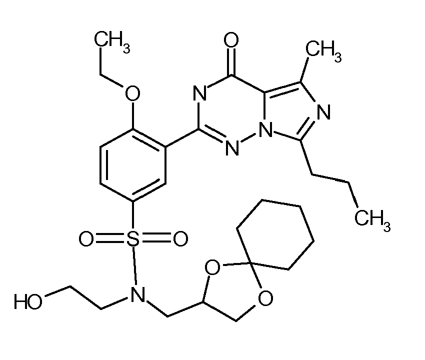 | 589,7164 | >95 | 590 |
| 275 | 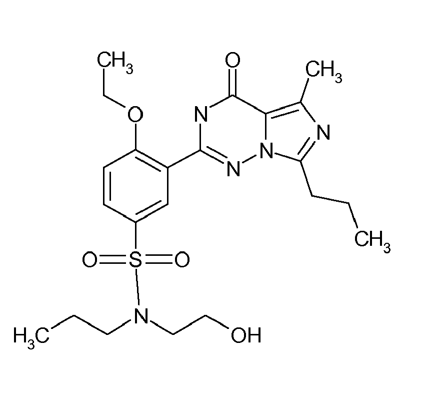 | 477,5869 | >95 | 478 |
| 276 | 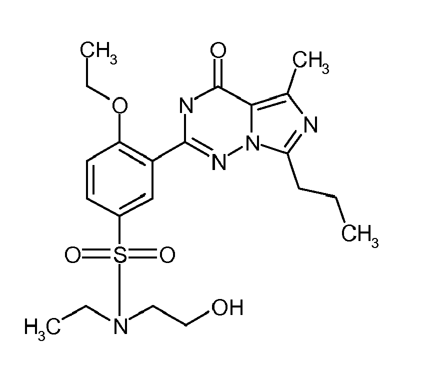 | 463,5598 | 64 | 464 |
| 277 | 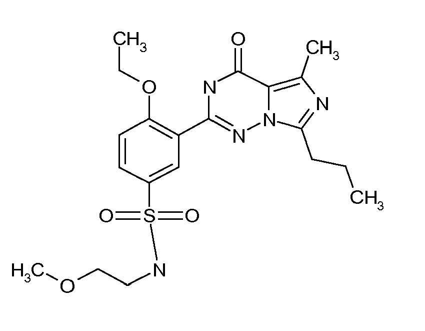 | 449,5327 | >95 | 450 |
| 278 | 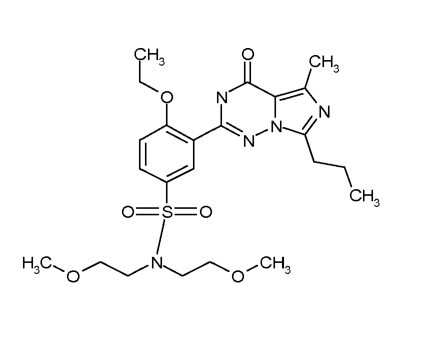 | 507,6134 | >95 | 508 |
| 279 | 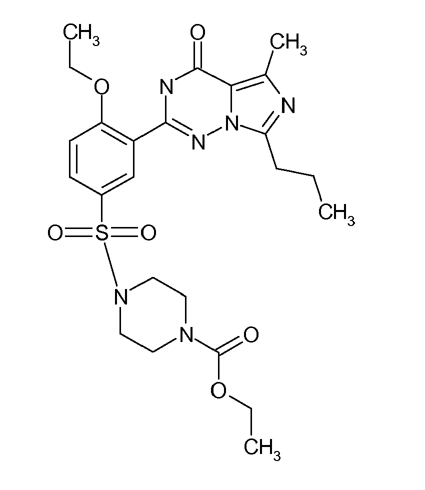 | 532,6232 | >95 | 533 |
| 280 | 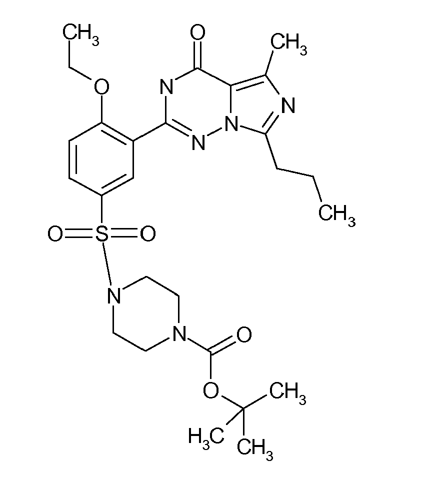 | 560,6775 | 89 | 561 |
| 281 | 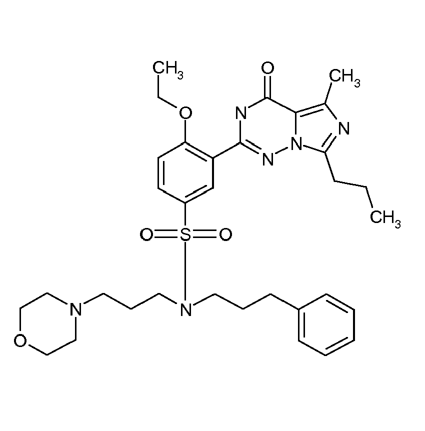 | 636,8199 | 88 | 637 |
| 282 | 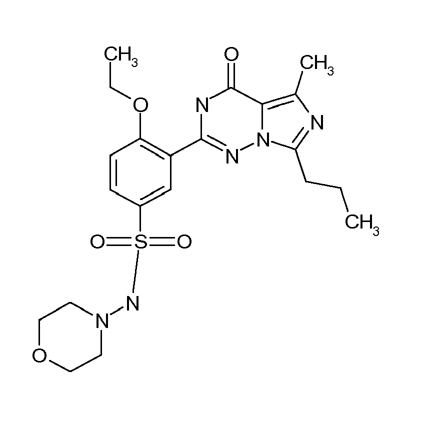 | 476,5585 | 50 | 477 |
| 283 | 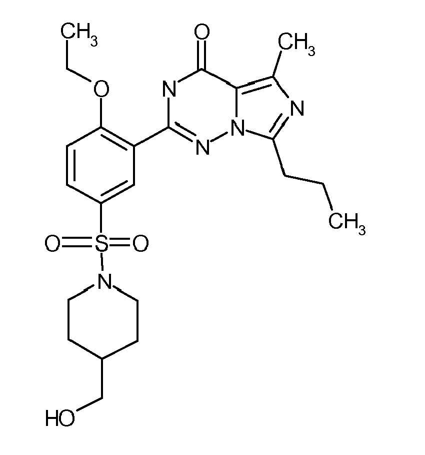 | 489,5981 | 93 | 490 |
| 284 | 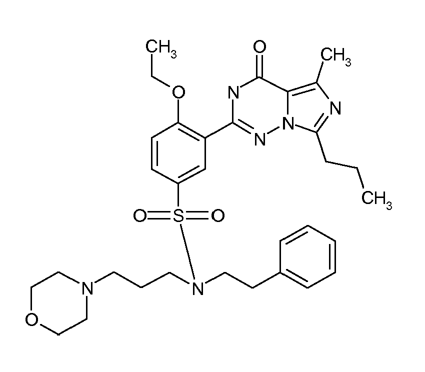 | 622,7928 | 68 | 623 |
| 285 | 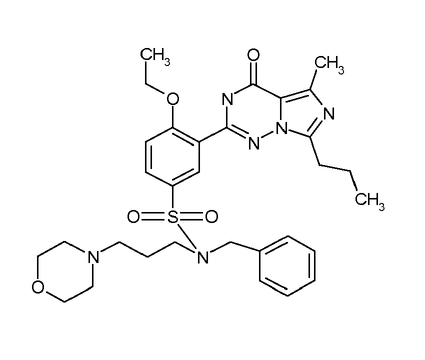 | 608,7657 | >95 | 609 |
| 286 | 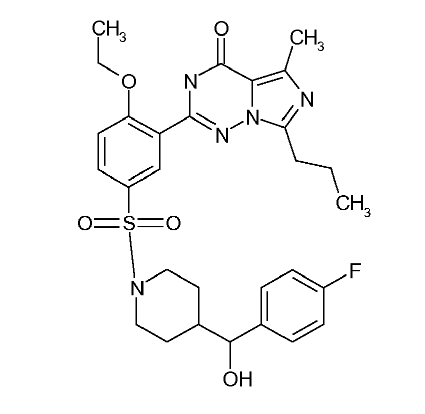 | 583,6873 | 85 | 584 |
| 287 | 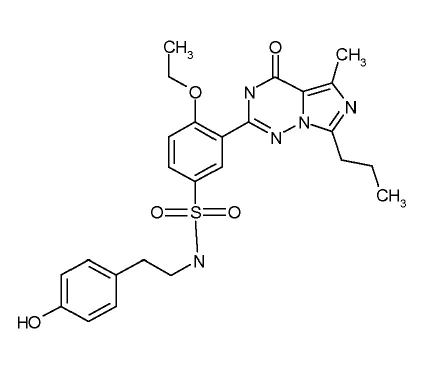 | 511,6044 | >95 | 512 |
| 288 | 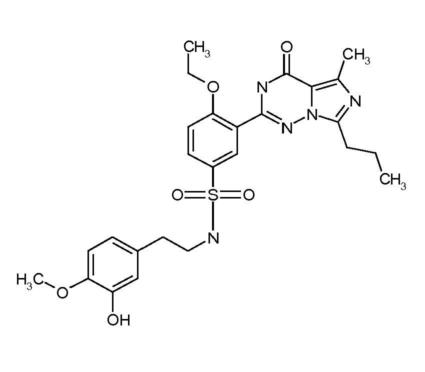 | 541,6309 | >95 | 542 |
| 289 | 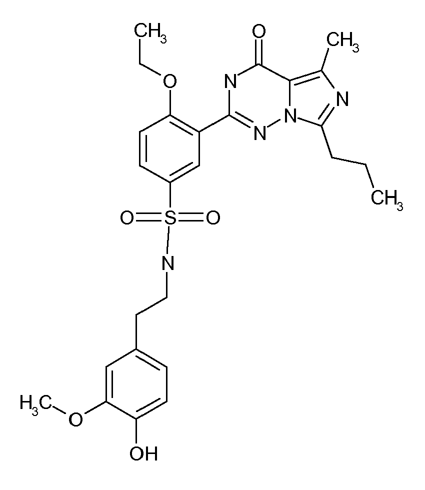 | 541,6309 | >95 | 542 |
| 290 | 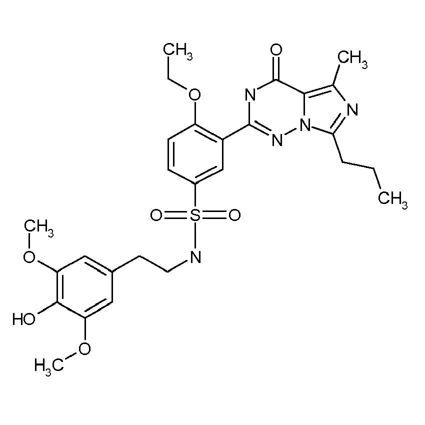 | 571,6574 | 73 | 572 |
| 291 | 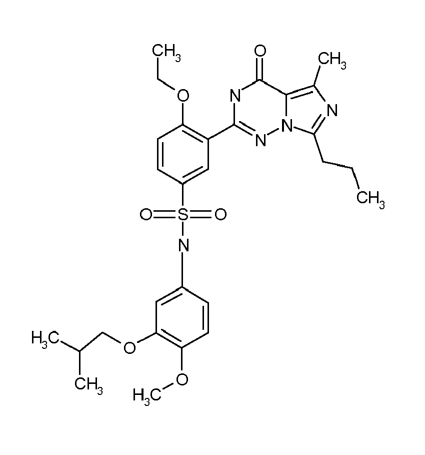 | 569,6851 | 83 | 570 |
| 292 | 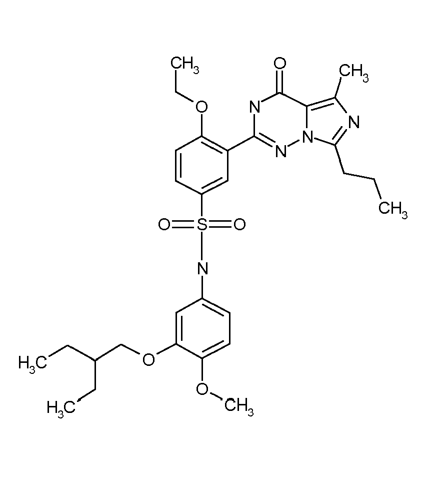 | 597,7393 | 89 | 598 |
| 293 | 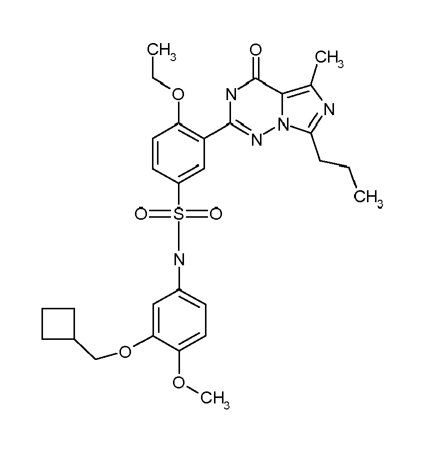 | 581,6963 | 76 | 582 |
| 294 | 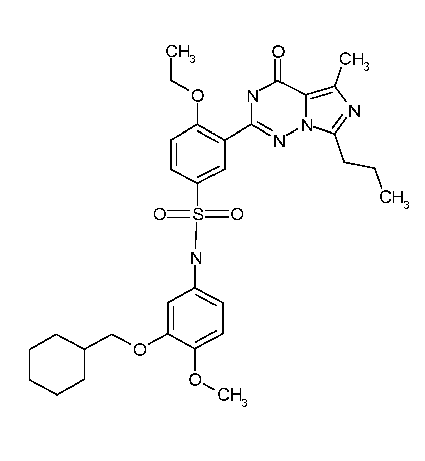 | 609,7504 | 83 | 610 |
| 295 | 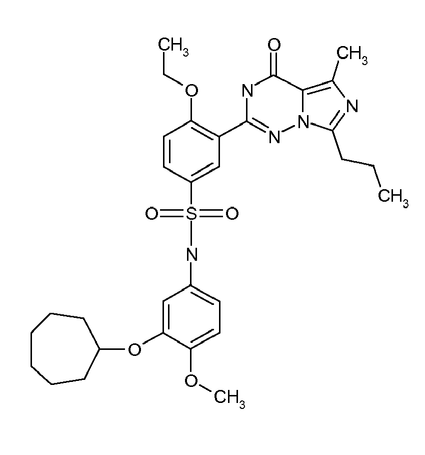 | 609,7504 | 77 | 610 |
| 296 | 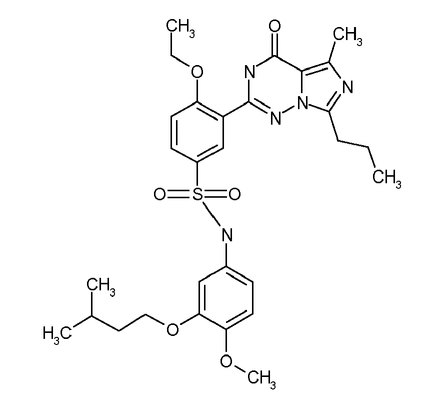 | 583,7122 | 82 | 584 |
| 297 | 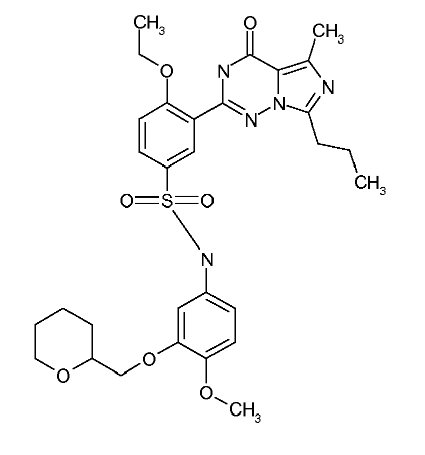 | 611,7227 | 88 | 612 |
| 298 | 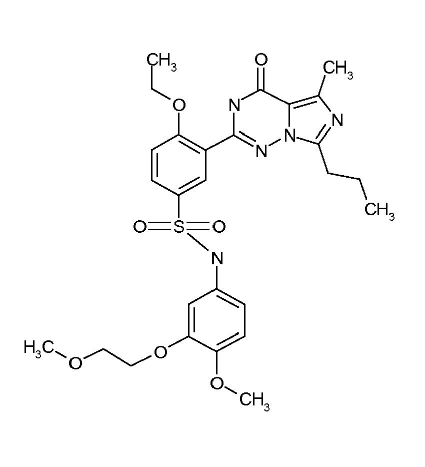 | 571,6574 | 89 | 572 |
| 299 | 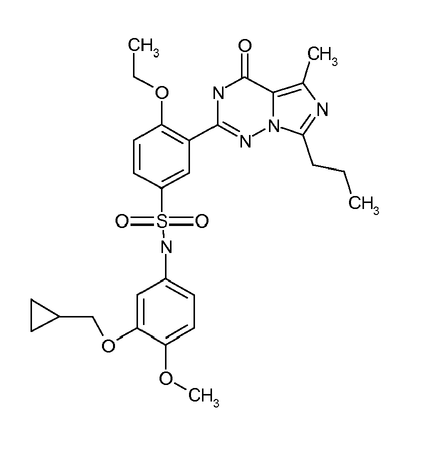 | 567,6692 | 81 | 568 |
| 300 | 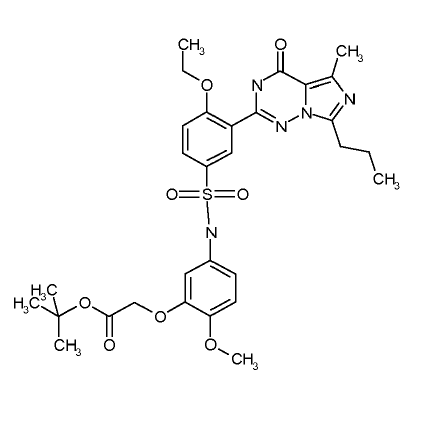 | 627,7221 | 82 | 628 |
| 301 | 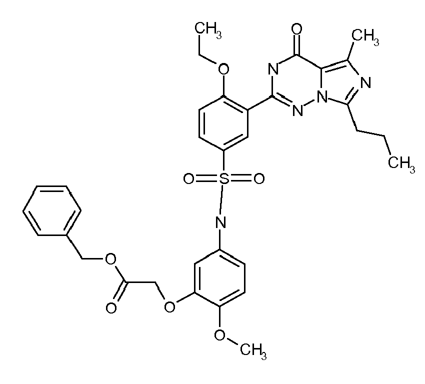 | 661,7396 | 64 | 662 |
| 302 | 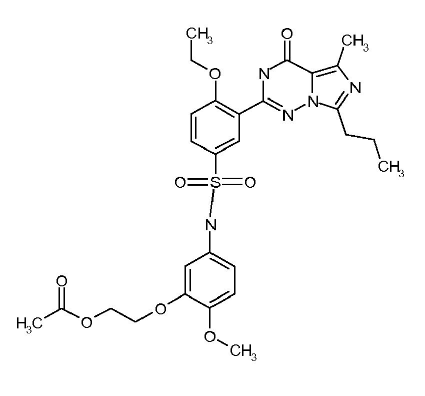 | 599,668 | 77 | 600 |
| 303 | 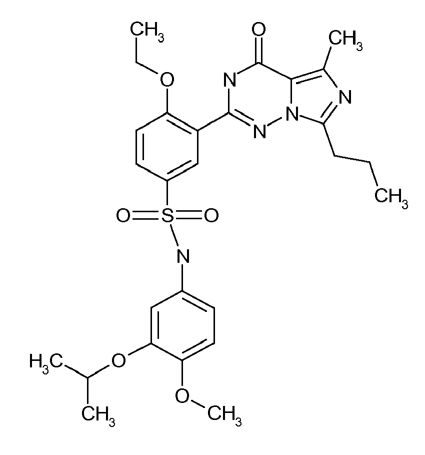 | 555,658 | 83 | 556 |
| 304 | 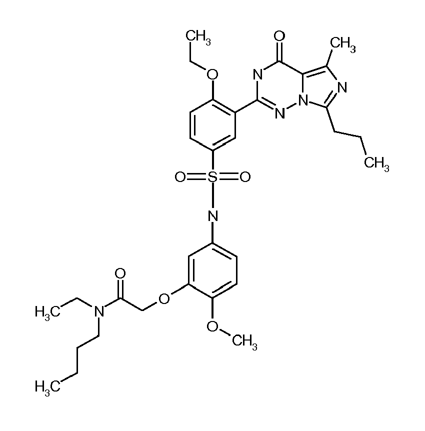 | 654,7916 | 60 | 655 |
| 305 | 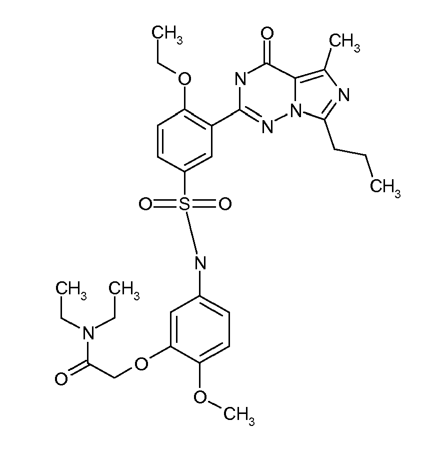 | 626,7374 | 86 | 627 |
| 306 | 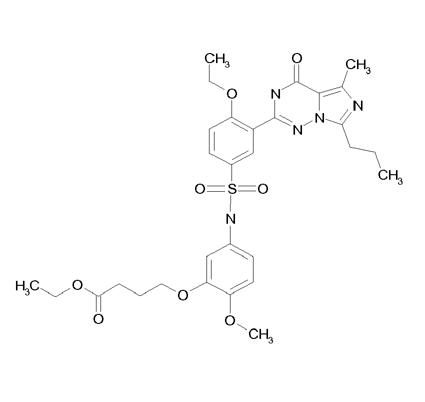 | 627,7221 | 82 | 628 |
| 307 | 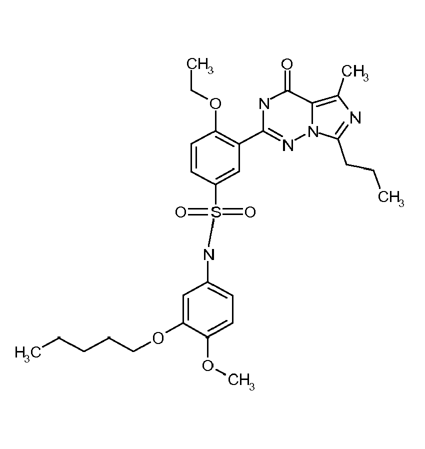 | 583,7122 | 81 | 584 |
| 308 | 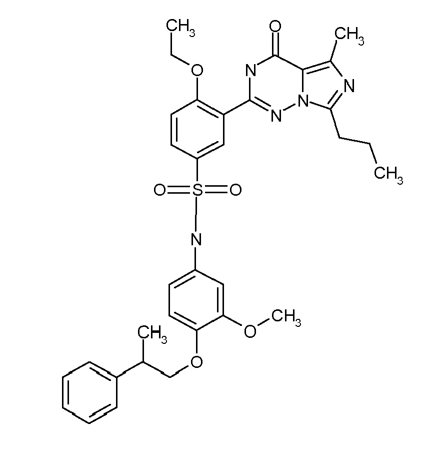 | 631,7568 | 29 | 632 |
| 309 | 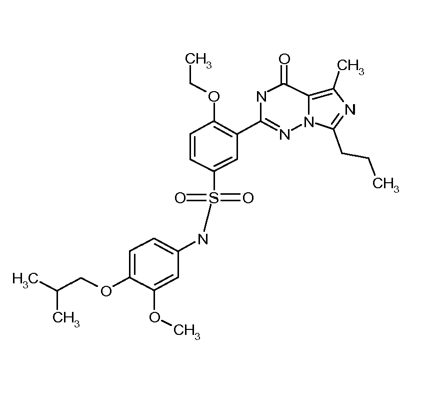 | 569,6851 | 60 | 570 |
| 310 | 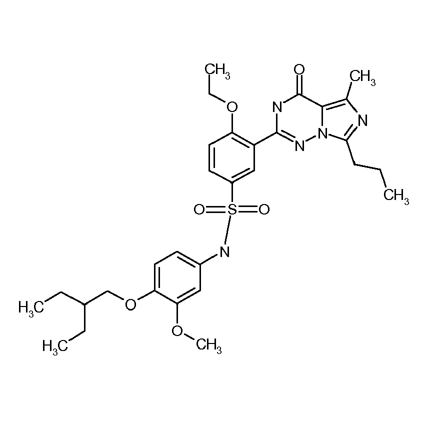 | 597,7393 | 62 | 598 |
| 311 | 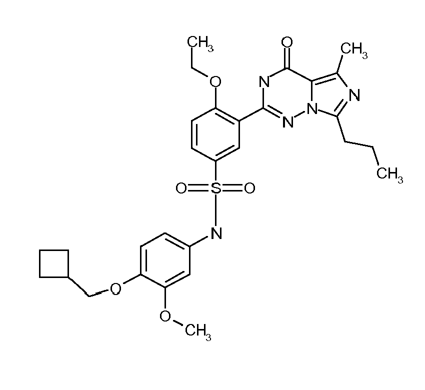 | 581,6963 | 87 | 582 |
| 312 | 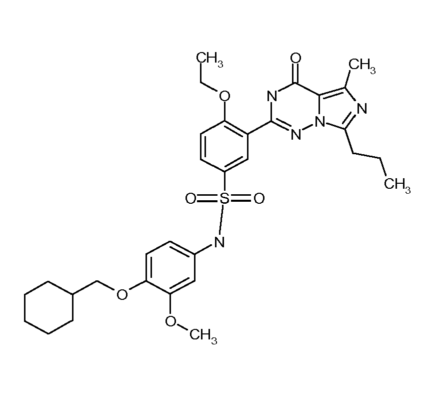 | 609,7504 | 71 | 610 |
| 313 | 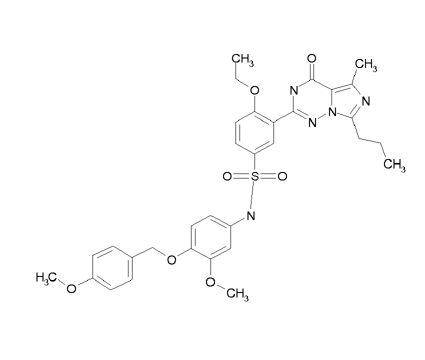 | 633,7291 | 47 | 634 |
| 314 | 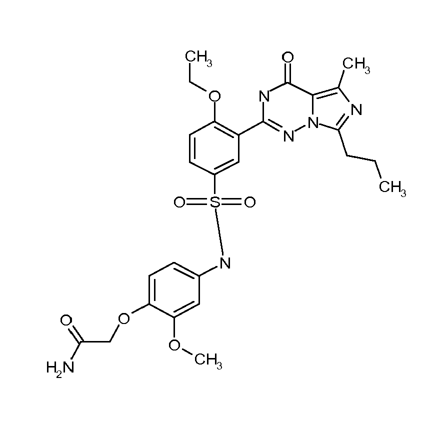 | 570,629 | 59 | 571 |
| 315 | 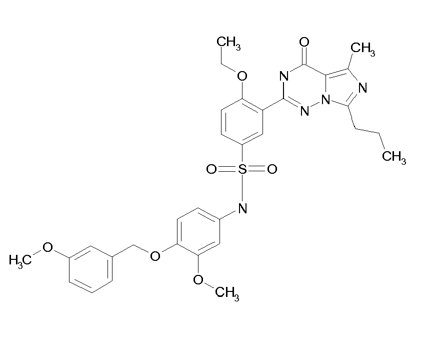 | 633,7291 | 35 | 634 |
| 316 | 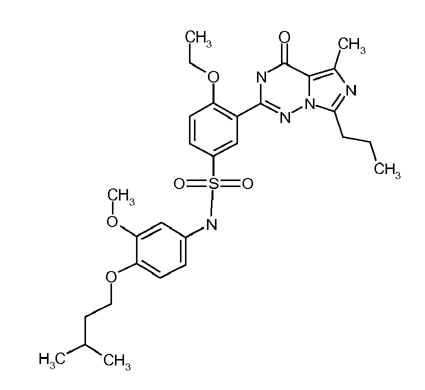 | 583,7122 | 51 | 584 |
| 317 | 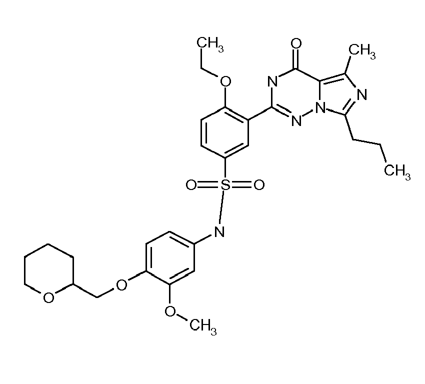 | 611,7227 | 51 | 612 |
| 318 | 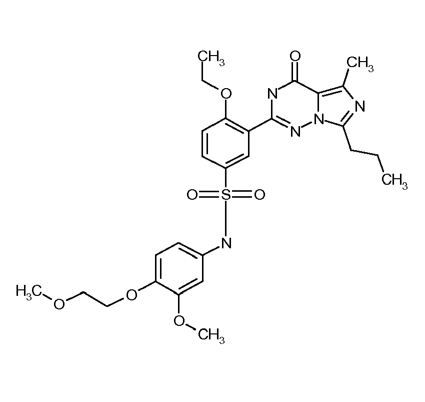 | 571,6574 | 75 | 572 |
| 319 | 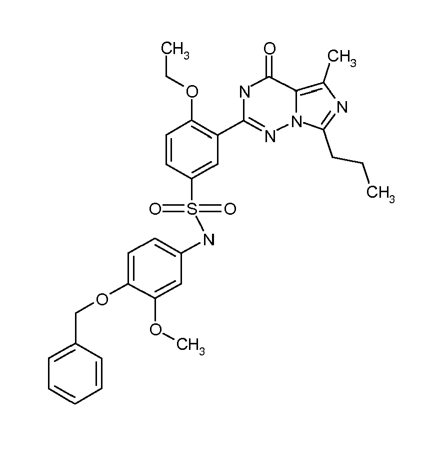 | 603,7026 | 64 | 604 |
| 320 | 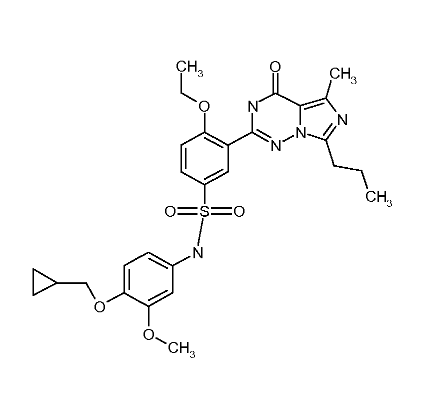 | 567,6692 | 74 | 568 |
| 321 | 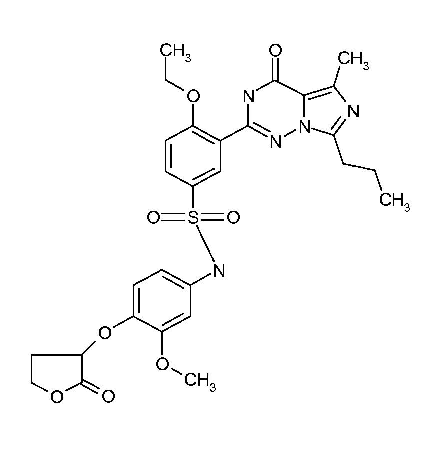 | 597,652 | 88 | 598 |
| 322 | 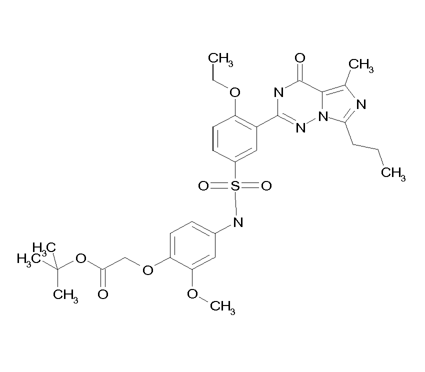 | 627,7221 | 80 | 628 |
| 323 | 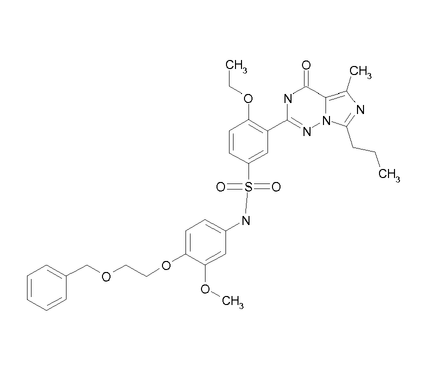 | 647,7562 | 47 | 648 |
| 324 | 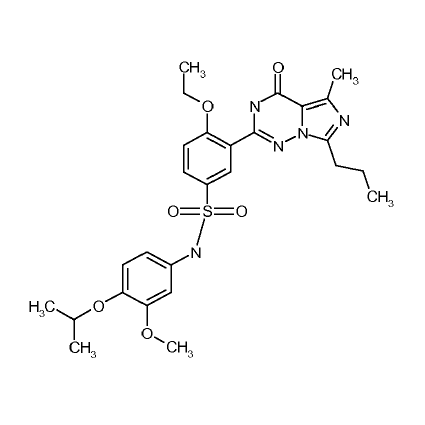 | 555,658 | 43 | 556 |
| 325 | 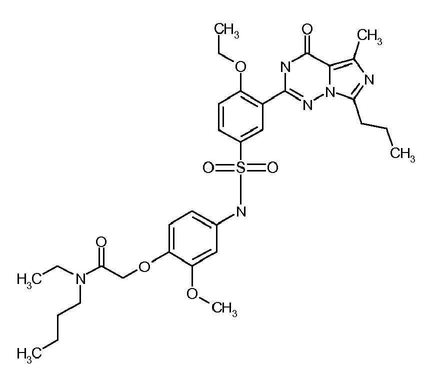 | 654,7916 | 54 | 655 |
| 326 | 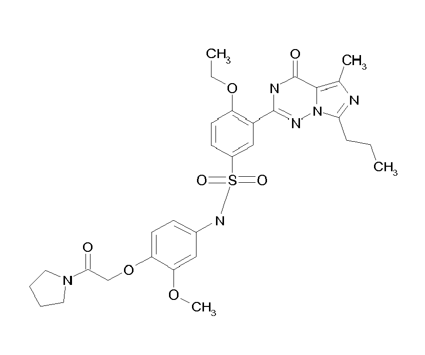 | 624,7214 | 71 | 625 |
| 327 | 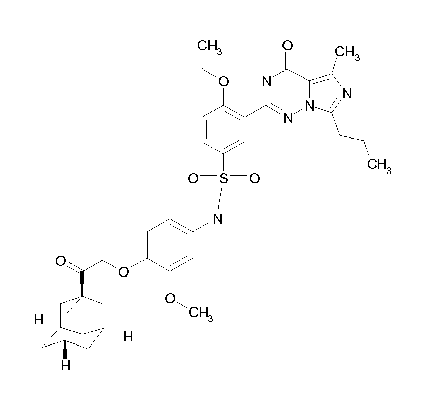 | 689,8375 | 42 | 690 |
| 328 | 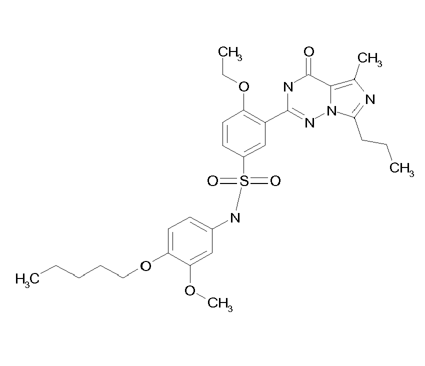 | 583,7122 | 40 | 584 |
| 329 | 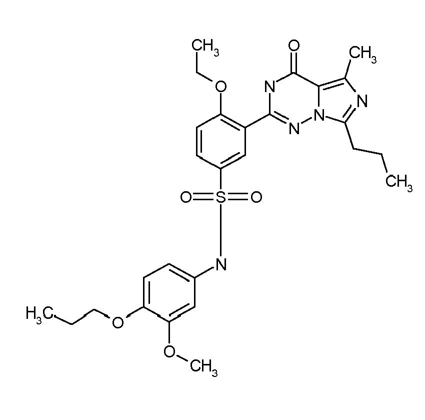 | 555,658 | 49 | 556 |
| 330 | 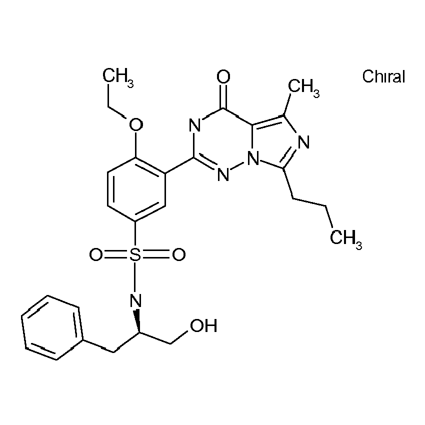 | 525,6315 | 83 | 526 |
| 331 | 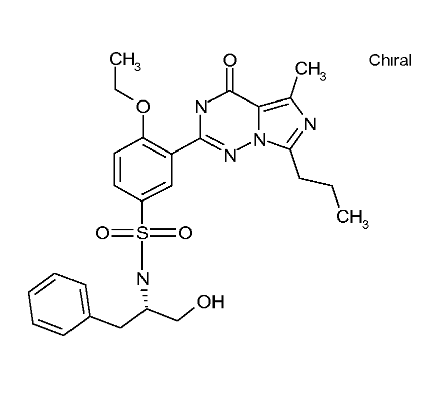 | 525,6315 | 71 | 526 |
| 332 | 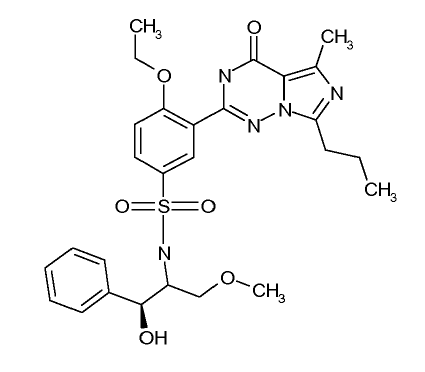 | 555,658 | 91 | 556 |
| 333 | 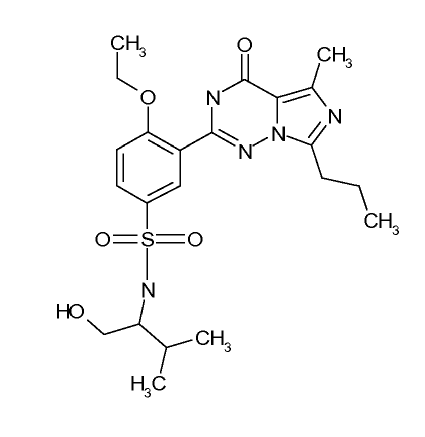 | 477,5869 | 76 | 478 |
| 334 | 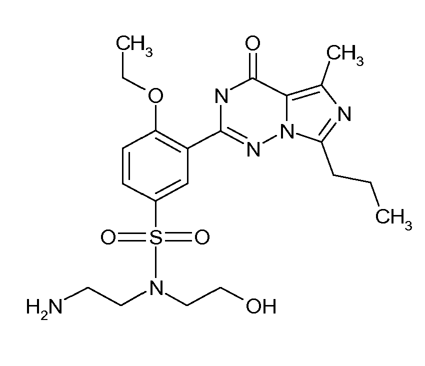 | 478,5745 | 62 | 479 |
| 335 | 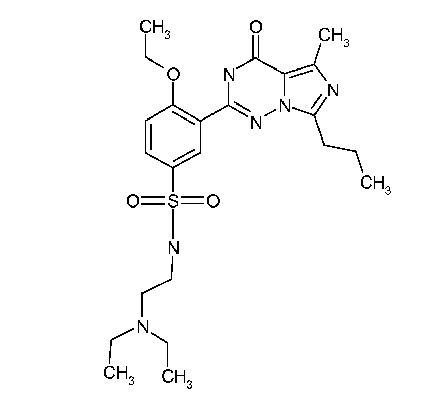 | 490,6292 | 42 | 491 |

### Beispiel 336

### 2-[2-Ethoxy-5-(4-ethyl-piperazin-1-sulfonyl)-phenyl]-5-methyl-7-propyl-3H-imidazol[5,1-f][1,2,4]triazin-4-on Hydrochlorid-Trihydrat

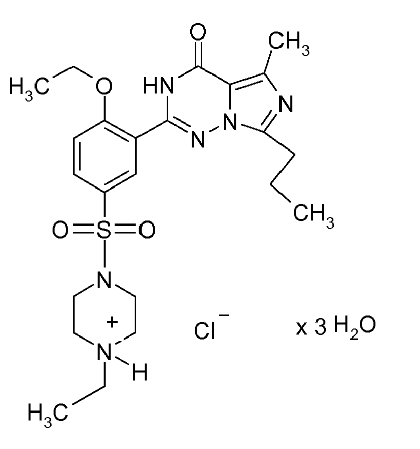

Kristallisiert man die freie Base aus Beispiel 19 aus einem Gemisch eines organischen Lösungsmittels und verdünnter wäßriger Salzsäure um, so erhält man ein Hydrochlorid Trihydrat.
Fp.: 218°C
Wassergehalt: 9,4 % (K. Fischer)
Chloridgehalt: 6,1 %

### Beispiel 337

### 2-[2-Ethoxy-5-(4-ethyl-piperazin-1-sulfonyl)-phenyl]-5-methyl-7-propyl-3H-imidazo[5,1-f][1,2,4]triazin-4-on Dihydrochlorid

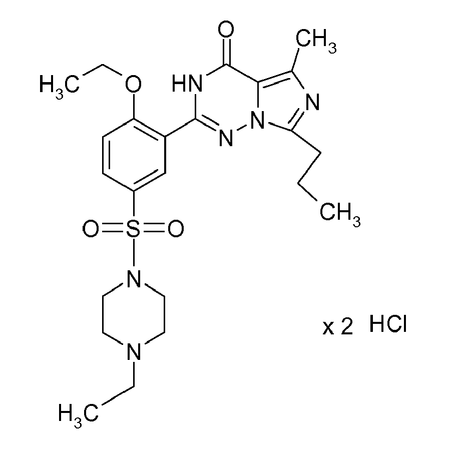

0,35 g (0,712 mmol) 2-[2-Ethoxy-5-(4-ethyl-piperazin-1-sulfonyl)-phenyl]-5-methyl-7-propyl-3H-imidazo[5,1-f][1,2,4]triazin-4-on werden in 8 ml Ether suspendiert und soviel Dichlormethan zugegeben, bis eine homogene Lösung entsteht. Man gibt 2,4 ml einer 1M Lösung von HCl in Ether zu, rührt 20 Minuten bei Raumtemperatur und saugt ab. Man erhält 372 mg (99 %) 2-[2-Ethoxy-5-(4-ethyl-piperazin-1-sulfonyl)-phenyl]-5-methyl-7-propyl-3H-imidazo[5,1-f][1,2,4]triazin-4-on Dihydrochlorid. 200 Mhz ¹H-NMR (DMSO-d₆): 0,96, t, 3H; 1,22, t, 3H; 1,36, t, 3H; 1,82, sex., 2H; 2,61, s, 3H; 2,88, m, 2H; 3,08, m, 6H; 3,50, m, 2H; 3,70, m, 2H; 4,25, quart., 2H; 7,48, d, 1H; 7,95, m, 2H; 11,42, s, 1H; 12,45, s, 1H.

## Patentansprüche

1. 2-Phenyl-substituierte Imidazotriazinone der allgemeinen Formel (I) 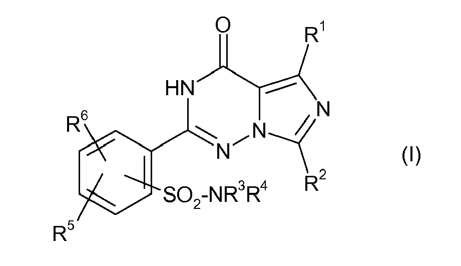 in welcher
R¹ für Wasserstoff oder geradkettiges oder verzweigtes Alkyl mit bis zu 4 Kohlenstoffatomen steht,
R² für geradkettiges Alkyl mit bis zu 4 Kohlenstoffatomen steht,
R³ und R⁴ gleich oder verschieden sind und für Wasserstoff oder für geradkettiges oder verzweigtes Alkenyl oder Alkoxy mit jeweils bis zu 8 Kohlenstoffatomen stehen, oder für eine geradkettige oder verzweigte Alkylkette mit bis zu 10 Kohlenstoffatomen stehen, die gegebenenfalls durch ein Sauerstoffatom unterbrochen ist, und die gegebenenfalls ein- bis mehrfach, gleich oder verschieden durch Trifluormethyl, Trifluormethoxy, Hydroxy, Halogen, Carboxyl, Benzyloxycarbonyl, geradkettiges oder verzweigtes Alkoxycarbonyl mit bis zu 6 Kohlenstoffatomen und/oder durch Reste der Formeln -SO₃H, -(A)ₐ-NR⁷R⁸, -O-CO-NR^{7'}R^{8'}, -S(O)_{b}-R⁹, -P(O)(OR¹⁰)(OR¹¹), 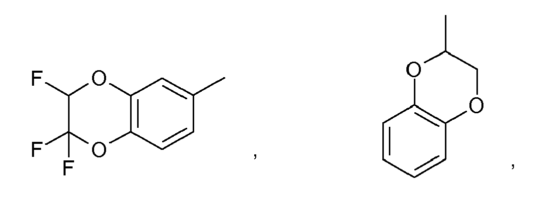 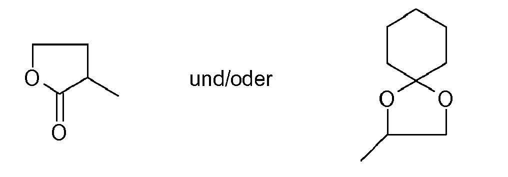 substituiert ist,
worin
a und b gleich oder verschieden sind und eine Zahl 0 oder 1 bedeuten,
A einen Rest CO oder SO₂ bedeutet,
R⁷, R^{7'}, R⁸ und R^{8'} gleich oder verschieden sind und Wasserstoff bedeuten, oder
Cycloalkyl mit 3 bis 8 Kohlenstoffatomen, Aryl mit 6 bis 10 Kohlenstoffatomen, einen 5- bis 6-gliedrigen ungesättigten, partiell ungesättigten oder gesättigten, gegebenenfalls benzokondensierten Heterocyclus, mit bis zu 3 Heteroatomen aus der Reihe S, N und/oder O bedeuten, wobei die oben aufgeführten Ringsysteme gegebenenfalls ein- bis mehrfach, gleich oder verschieden durch Hydroxy, Nitro, Trifluormethyl, Trifluormethoxy, Carboxyl, Halogen, geradkettiges oder verzweigtes Alkoxy oder Alkoxycarbonyl mit jeweils bis zu 6 Kohlenstoffatomen oder durch eine Gruppe der Formel -(SO₂)_{c}-NR¹²R¹³ substituiert sind,
worin
c eine Zahl 0 oder 1 bedeutet,
R¹² und R¹³ gleich oder verschieden sind und Wasserstoff oder geradkettiges oder verzweigtes Alkyl mit bis zu 5 Kohlenstoffatomen bedeuten,
oder
R⁷, R^{7'}, R⁸ und R^{8'} geradkettiges oder verzweigtes Alkoxy mit bis zu 6 Kohlenstoffatomen bedeuten, oder geradkettiges oder verzweigtes Alkyl mit bis zu 8 Kohlenstoffatomen bedeuten, das gegebenenfalls ein- oder mehrfach, gleich oder verschieden durch Hydroxy, Halogen, Aryl mit 6 bis 10 Kohlenstoffatomen, geradkettiges oder verzweigtes Alkoxy oder Alkoxycarbonyl mit jeweils bis zu 6 Kohlenstoffatomen oder durch eine Gruppe der Formel -(CO)_{d}-NR¹⁴R¹⁵ substituiert ist,
worin
R¹⁴ und R¹⁵ gleich oder verschieden sind und Wasserstoff oder geradkettiges oder verzweigtes Alkyl mit bis zu 4 Kohlenstoffatomen bedeuten,
und
d eine Zahl 0 oder 1 bedeutet,
oder
R⁷ und R⁸ und/oder R^{7'} und R^{8'} gemeinsam mit dem Stickstoffatom einen 5-bis 7-gliedrigen, gesättigten Heterocyclus bilden, der gegebenenfalls noch ein weiteres Heteroatom aus der Reihe S oder O oder einen Rest der Formel -NR¹⁶ enthalten kann,
worin
R¹⁶ Wasserstoff, Aryl mit 6 bis 10 Kohlenstoffatomen, Benzyl, einen 5- bis 7-gliedrigen aromatischen oder gesättigten Heterocyclus mit bis zu 3 Heteroatomen aus der Reihe S, N und/oder O bedeutet, der gegebenenfalls durch Methyl substituiert ist, oder geradkettiges oder verzweigtes Alkyl mit bis zu 6 Kohlenstoffatomen bedeutet, das gegebenenfalls durch Hydroxy substituiert ist,
R⁹ Aryl mit 6 bis 10 Kohlenstoffatomen bedeutet, oder geradkettiges oder verzweigtes Alkyl mit bis zu 4 Kohlenstoffatomen bedeutet,
R¹⁰ und R¹¹ gleich oder verschieden sind und Wasserstoff oder geradkettiges oder verzweigtes Alkyl mit bis zu 4 Kohlenstoffatomen bedeuten, und/oder die oben unter R³/R⁴ aufgeführte Alkylkette gegebenenfalls durch Cycloalkyl mit 3 bis 8 Kohlenstoffatomen, Aryl mit 6 bis 10 Kohlenstoffatomen oder durch einen 5- bis 7-gliedrigen, partiell ungesättigten, gesättigten oder ungesättigten, gegebenenfalls benzokondensierten Heterocyclus, der bis zu 4 Heteroatome aus der Reihe S, N und O oder einen Rest der Formel -NR¹⁷ enthalten kann, substituiert ist,
worin
R¹⁷ Wasserstoff, Hydroxy, Formyl, Trifluormethyl, geradkettiges oder verzweigtes Acyl oder Alkoxy mit jeweils bis zu 4 Kohlenstoffatomen bedeutet, oder geradkettiges oder verzweigtes Alkyl mit bis zu 6 Kohlenstoffatomen bedeutet, das gegebenenfalls ein- bis mehrfach, gleich oder verschieden durch Hydroxy, oder geradkettiges oder verzweigtes Alkoxy mit bis zu 6 Kohlenstoffatomen substituiert ist,
und wobei Aryl und der Heterocyclus gegebenenfalls ein- bis mehrfach, gleich oder verschieden durch Nitro, Halogen, -SO₃H, geradkettiges oder verzweigtes Alkyl oder Alkoxy mit jeweils bis zu 6 Kohlenstoffatomen, Hydroxy, Trifluormethyl, Trifluormethoxy und/oder durch einen Rest der Formel -SO₂-NR¹⁸R¹⁹ substituiert sind,
worin
R¹⁸ und R¹⁹ gleich oder verschieden sind und Wasserstoff oder geradkettiges oder verzweigtes Alkyl mit bis zu 6 Kohlenstoffatomen bedeuten,
und/oder
R³ oder R⁴ für eine Gruppe der Formel -NR²⁰R²¹ steht,
worin
R²⁰ und R²¹ die oben angegebene Bedeutung von R¹⁸ und R¹⁹ haben und mit dieser gleich oder verschieden sind,
und/oder
R³ oder R⁴ für Adamantyl stehen, oder für Reste der Formeln 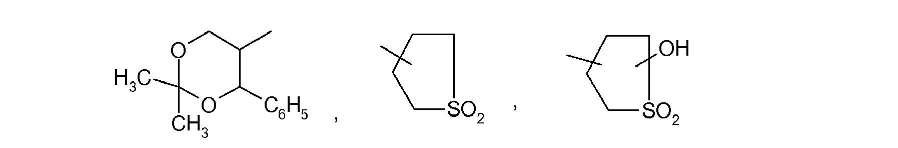 oder 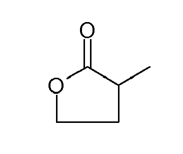 stehen,
oder für Cycloalkyl mit 3 bis 8 Kohlenstoffatomen, Aryl mit 6 bis 10 Kohlenstoffatomen oder für einen 5- bis 7-gliedrigen partiell ungesättigten, gesättigten oder ungesättigten, gegebenenfalls benzokondensierten Heterocyclus stehen, der bis zu 4 Heteroatome aus der Reihe S, N, O oder einen Rest der Formel -NR²² enthalten kann,
worin
R²² die oben angegebene Bedeutung von R¹⁶ hat und mit dieser gleich oder verschieden ist, oder Carboxyl, Formyl oder geradkettiges oder verzweigtes Acyl mit bis zu 5 Kohlenstoffatomen bedeutet,
und wobei Cycloalkyl, Aryl und/oder der Heterocyclus gegebenenfalls ein- bis mehrfach, gleich oder verschieden durch Halogen, Triazolyl, Trifluormethyl, Trifluormethoxy, Carboxyl, geradkettiges oder verzweigtes Acyl oder Alkoxycarbonyl mit jeweils bis zu 6 Kohlenstoffatomen, Nitro und/oder durch Gruppen der Formeln -SO₃H, -OR²³, (SO₂)ₑNR²⁴R²⁵, -P(O)(OR²⁶)(OR²⁷) substituiert sind,
worin
e eine Zahl 0 oder 1 bedeutet,
R²³ einen Rest der Formel 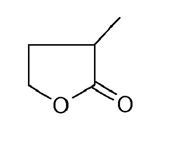 bedeutet, oder
Cycloalkyl mit 3 bis 7 Kohlenstoffatomen bedeutet, oder Wasserstoff oder geradkettiges oder verzweigtes Alkyl mit bis zu 4 Kohlenstoffatomen bedeutet, das gegebenenfalls durch Cycloalkyl mit 3 bis 7 Kohlenstoffatomen, Benzyloxy, Tetrahydropyranyl, Tetrahydrofuranyl, geradkettiges oder verzweigtes Alkoxy oder Alkoxycarbonyl mit jeweils bis zu 6 Kohlenstoffatomen, Carboxyl, Benzyloxycarbonyl oder Phenyl substituiert ist, das seinerseits ein- bis mehrfach, gleich oder verschieden durch geradkettiges oder verzweigtes Alkoxy mit bis zu 4 Kohlenstoffatomen, Hydroxy oder Halogen substituiert sein kann,
und/oder Alkyl gegebenenfalls durch Reste der Formeln -CO-NR²⁸R²⁹ oder -CO-R³⁰ substituiert ist,
worin
R²⁸ und R²⁹ gleich oder verschieden sind und Wasserstoff oder geradkettiges oder verzweigtes Alkyl mit bis zu 8 Kohlenstoffatomen bedeuten, oder
R²⁸ und R²⁹ gemeinsam mit dem Stickstoffatom einen 5- bis 7-gliedrigen gesättigten Heterocyclus bilden, der gegebenenfalls ein weiteres Heteroatom aus der Reihe S oder O enthalten kann,
und
R³⁰ Phenyl oder Adamantyl bedeutet,
R²⁴ und R²⁵ die oben angegebene Bedeutung von R¹⁸ und R¹⁹ haben und mit dieser gleich oder verschieden sind,
R²⁶ und R²⁷ die oben angegebene Bedeutung von R¹⁰ und R¹¹ haben und mit dieser gleich oder verschieden sind
und/oder Cycloalkyl, Aryl und/oder der Heterocyclus gegebenenfalls durch geradkettiges oder verzweigtes Alkyl mit bis zu 6 Kohlenstoffatomen substituiert sind, das gegebenenfalls durch Hydroxy, Carboxyl, durch einen 5- bis 7-gliedrigen Heterocyclus mit bis zu 3 Heteroatomen aus der Reihe S, N und/oder O, oder durch Gruppen der Formel -SO₂-R³¹, P(O)(OR³²)(OR³³) oder NR³⁴R³⁵ substituiert ist,
worin
R³¹ Wasserstoff bedeutet oder die oben angegebene Bedeutung von R⁹ hat und mit dieser gleich oder verschieden ist,
R³² und R³³ die oben angegebene Bedeutung von R¹⁰ und R¹¹ haben und mit dieser gleich oder verschieden sind,
R³⁴ und R³⁵ gleich oder verschieden sind und Wasserstoff oder geradkettiges oder verzweigtes Alkyl mit bis zu 6 Kohlenstoffatomen bedeuten, das gegebenenfalls durch Hydroxy oder geradkettiges oder verzweigtes Alkoxy mit bis zu 4 Kohlenstoffatomen substituiert ist, oder
R³⁴ und R³⁵ gemeinsam mit dem Stickstoffatom einen 5- bis 6-gliedrigen gesättigten Heterocyclus bilden, der ein weiteres Heteroatom aus der Reihe S oder O oder einen Rest der Formel -NR³⁶ enthalten kann,
worin
R³⁶ Wasserstoff, Hydroxy, geradkettiges oder verzweigtes Alkoxycarbonyl mit bis zu 7 Kohlenstoffatomen oder geradkettiges oder verzweigtes Alkyl mit bis zu 5 Kohlenstoffatomen bedeutet, das gegebenenfalls durch Hydroxy substituiert ist,
oder
R³ und R⁴ gemeinsam mit dem Stickstoffatom einen 5- bis 7-gliedrigen, ungesättigten oder gesättigten oder partiell ungesättigten, gegebenenfalls benzokondensierten Heterocyclus bilden, der gegebenenfalls bis zu 3 Heteroatome aus der Reihe S, N, O, oder einen Rest der Formel -NR³⁷ enthalten kann,
worin
R³⁷ Wasserstoff, Hydroxy, Formyl, Trifluormethyl, geradkettiges oder verzweigtes Acyl, Alkoxy oder Alkoxycarbonyl mit jeweils bis zu 4 Kohlenstoffatomen bedeutet, oder geradkettiges oder verzweigtes Alkyl mit bis zu 6 Kohlenstoffatomen bedeutet, das gegebenenfalls ein- bis mehrfach, gleich oder verschieden durch Hydroxy, Trifluormethyl, Carboxyl, geradkettiges oder verzweigtes Alkoxy oder Alkoxycarbonyl mit jeweils bis zu 6 Kohlenstoffatomen oder durch Gruppen der Formel -(D)_{f-}NR³⁸R³⁹, -CO-(CH₂)_{g}-O-CO-R⁴⁰, -CO-(CH₂)ₕ-OR⁴¹ oder -P(O)(OR⁴²)(OR⁴³) substituiert ist,
worin
g und h gleich oder verschieden sind und eine Zahl 1, 2, 3 oder 4 bedeuten,
und
f eine Zahl 0 oder 1 bedeutet,
D eine Gruppe der Formel -CO oder -SO₂ bedeutet,
R³⁸ und R³⁹ gleich oder verschieden sind und die oben angegebene Bedeutung von R⁷ und R⁸ haben,
**R⁴⁰** geradkettiges oder verzweigtes Alkyl mit bis zu 6 Kohlenstoffatomen bedeutet,
R⁴¹ geradkettiges oder verzweigtes Alkyl mit bis zu 6 Kohlenstoffatomen bedeutet,
R⁴² und R⁴³ gleich oder verschieden sind und Wasserstoff oder geradkettiges oder verzweigtes Alkyl mit bis zu 4 Kohlenstoffatomen bedeuten,
oder
R³⁷ einen Rest der Formel -(CO)ᵢ-E bedeutet,
worin
i eine Zahl 0 oder 1 bedeutet,
E Cycloalkyl mit 3 bis 7 Kohlenstoffatomen oder Benzyl bedeutet, Aryl mit 6 bis 10 Kohlenstoffatomen oder einen 5- bis 6-gliedrigen aromatischen Heterocyclus mit bis zu 4 Heteroatomen aus der Reihe S, N und/oder O bedeutet, wobei die oben aufgeführten Ringsysteme gegebenenfalls ein- bis mehrfach, gleich oder verschieden durch Nitro, Halogen, -SO₃H, geradkettiges oder verzweigtes Alkoxy mit bis zu 6 Kohlenstoffatomen, Hydroxy, Trifluormethyl, Trifluormethoxy oder durch einen Rest der Formel -SO₂-NR⁴⁴R⁴⁵, substituiert sind,
worin
R⁴⁴ und R⁴⁵ die oben angegebene Bedeutung von R¹⁸ und R¹⁹ haben und mit dieser gleich oder verschieden sind,
oder
E Reste der Formeln 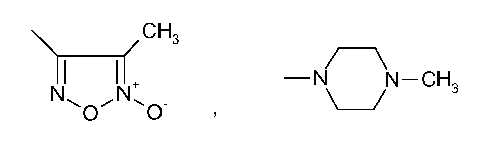 oder 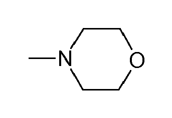 bedeutet,
und der unter R³ und R⁴ aufgeführte, gemeinsam mit dem Stickstoffatom gebildete Heterocyclus, gegebenenfalls ein- bis mehrfach, gleich oder verschieden, gegebenenfalls auch geminal, durch Hydroxy, Formyl, Carboxyl, geradkettiges oder verzweigtes Acyl oder Alkoxycarbonyl mit bis jeweils zu 6 Kohlenstoffatomen, Nitro und Gruppen der Formeln -P(O)(OR⁴⁶)(OR⁴⁷), 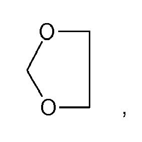 =NR⁴⁸ oder -(CO)ⱼNR⁴⁹R⁵⁰ substituiert ist, worin
R⁴⁶ und R⁴⁷ die oben angegebene Bedeutung von R¹⁰ und R¹¹ haben und mit dieser gleich oder verschieden sind,
R⁴⁸ Hydroxy oder geradkettiges oder verzweigtes Alkoxy mit bis zu 4 Kohlenstoffatomen bedeutet,
j eine Zahl 0 oder 1 bedeutet,
und
R⁴⁹ und R⁵⁰ gleich oder verschieden sind und die oben angegebene Bedeutung von R¹⁴ und R¹⁵ haben,
und/oder der unter R³ und R⁴ aufgeführte, gemeinsam mit dem Stickstoffatom gebildete Heterocyclus, gegebenenfalls durch geradkettiges oder verzweigtes Alkyl mit bis zu 6 Kohlenstoffatomen substituiert ist, das gegebenenfalls ein- bis mehrfach, gleich oder verschieden durch Hydroxy, Halogen, Carboxyl, Cycloalkyl oder Cycloalkyloxy mit jeweils 3 bis 8 Kohlenstoffatomen, geradkettiges oder verzweigtes Alkoxy oder Alkoxycarbonyl mit jeweils bis zu 6 Kohlenstoffatomen oder durch einen Rest der Formel -SO₃H, -NR⁵¹R⁵² oder P(O)OR⁵³OR⁵⁴ substituiert ist,
worin
R⁵¹ und R⁵² gleich oder verschieden sind und Wasserstoff, Phenyl, Carboxyl, Benzyl oder geradkettiges oder verzweigtes Alkyl oder Alkoxy mit jeweils bis zu 6 Kohlenstoffatomen bedeuten,
R⁵³ und R⁵⁴ gleich oder verschieden sind und die oben angegebene Bedeutung von R¹⁰ und R¹¹ haben,
und/oder das Alkyl gegebenenfalls durch Aryl mit 6 bis 10 Kohlenstoffatomen substituiert ist, das seinerseits ein- bis mehrfach, gleich oder verschieden durch Halogen, Hydroxy, geradkettiges oder verzweigtes Alkoxy mit bis zu 6 Kohlenstoffatomen, oder durch eine Gruppe der Formel -NR^{51'}R^{52'} substituiert sein kann,
worin
R^{51'} und R^{52'} die oben angegebene Bedeutung von R⁵¹ und R⁵² haben und mit dieser gleich oder verschieden sind,
und/oder der unter R³ und R⁴ aufgeführte, gemeinsam mit dem Stickstoffatom gebildete Heterocyclus, gegebenenfalls durch Aryl mit 6 bis 10 Kohlenstoffatomen oder durch einen 5- bis 7-gliedrigen, gesättigen, partiell ungesättigten oder ungesättigten Heterocyclus mit bis zu 3 Heteratomen aus der Reihe S, N und/oder O , gegebenenfalls auch über eine N-Funktion verknüpft, substituiert ist, wobei die Ringsysteme ihrerseits durch Hydroxy oder durch geradkettiges oder verzweigtes Alkyl oder Alkoxy mit jeweils bis zu 6 Kohlenstoffatomen substituiert sein können,
oder
R³ und R⁴ gemeinsam mit dem Stickstoffatom Reste der Formeln 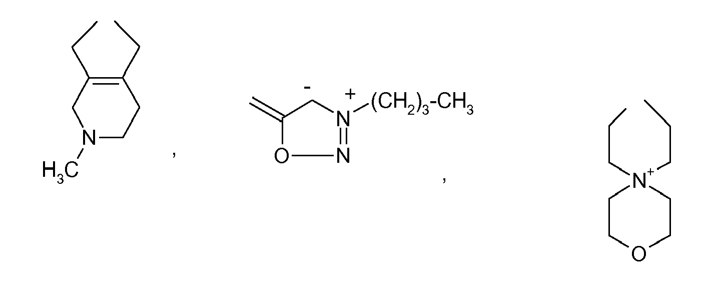 oder 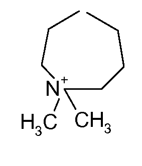 bilden,
R⁵ und R⁶ gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen,
und deren Salze, Hydrate, N-Oxide und isomere Formen.

2. 2-Phenyl-substituierte Imidazotriazinone der allgemeinen Formel (I) gemäß Anspruch 1, in welcher
R¹ für geradkettiges oder verzweigtes Alkyl mit bis zu 3 Kohlenstoffatomen steht,
R² für geradkettiges Alkyl mit bis zu 3 Kohlenstoffatomen steht,
R³ und R⁴ gleich oder verschieden sind und für Wasserstoff oder für geradkettiges oder verzweigtes Alkenyl oder Alkoxy mit jeweils bis zu 6 Kohlenstoffatomen stehen, oder für eine geradkettige oder verzweigte Alkylkette mit bis zu 8 Kohlenstoffatomen stehen, die gegebenenfalls durch ein Sauerstoffatom unterbrochen ist, und die gegebenenfalls ein- bis dreifach, gleich oder verschieden durch Hydroxy, Fluor, Chlor, Carboxyl, Benzyloxycarbonyl, geradkettiges oder verzweigtes Alkoxycarbonyl mit bis zu 5 Kohlenstoffatomen und/oder durch Reste der Formeln -SO₃H, -(A)ₐ-NR⁷R⁸, -O-CO-NR^{7'}R^{8'}, -S(O)_{b}-R⁹, -P(O)(OR¹⁰)(OR¹¹), 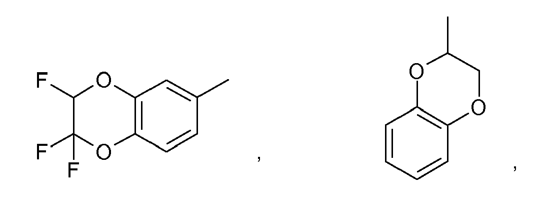 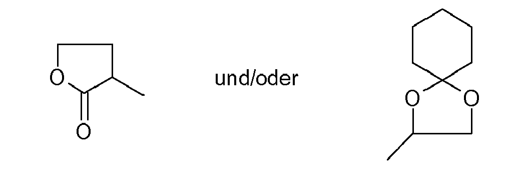 substituiert ist,
worin
a und b gleich oder verschieden sind und eine Zahl 0 oder 1 bedeuten,
A einen Rest CO oder SO₂ bedeutet,
R⁷, R^{7'}, R⁸ und R^{8'} gleich oder verschieden sind und Wasserstoff bedeuten, oder
Cyclopropyl, Cyclopentyl, Cyclohexyl, Cycloheptyl, Phenyl, Piperidinyl und Pyridyl bedeuten, wobei die oben aufgeführten Ringsysteme gegebenenfalls ein- bis dreifach, gleich oder verschieden durch Hydroxy, Nitro, Trifluormethyl, Trifluormethoxy, Carboxyl, Fluor, Chlor, geradkettiges oder verzweigtes Alkoxy oder Alkoxycarbonyl mit jeweils bis zu 4 Kohlenstoffatomen oder durch eine Gruppe der Formel -(SO₂)_{c}-NR¹²R¹³ substituiert sind,
worin
c eine Zahl 0 oder 1 bedeutet,
R¹² und R¹³ gleich oder verschieden sind und Wasserstoff oder geradkettiges oder verzweigtes Alkyl mit bis zu 4 Kohlenstoffatomen bedeuten,
oder
R⁷, R^{7'}, R⁸ und R^{8'} geradkettiges oder verzweigtes Alkoxy mit bis zu 3 Kohlenstoffatomen bedeuten, oder
geradkettiges oder verzweigtes Alkyl mit bis zu 7 Kohlenstoffatomen bedeuten, das gegebenenfalls ein- oder mehrfach, gleich oder verschieden durch Hydroxy, Fluor, Chlor, Phenyl, geradkettiges oder verzweigtes Alkoxy oder Alkoxycarbonyl mit jeweils bis zu 4 Kohlenstoffatomen oder durch eine Gruppe der Formel -(CO)_{d}-NR¹⁴R¹⁵ substituiert ist,
worin
R¹⁴ und R¹⁵ leich oder verschieden sind und Wasserstoff oder geradkettiges oder verzweigtes Alkyl mit bis zu 3 Kohlenstoffatomen bedeuten,
und
d eine Zahl 0 oder 1 bedeutet,
oder
R⁷ und R⁸ und/oder R^{7'} und R^{8'} gemeinsam mit dem Stickstoffatom einen Pyrrolidinyl-, Morpholinyl-, Piperidinyl- oder Triazolylring oder Reste der Formeln 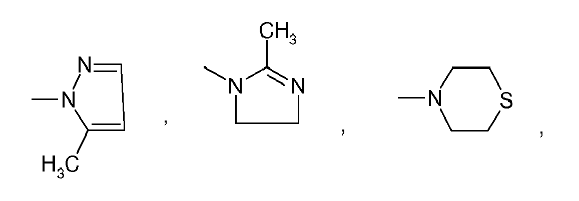 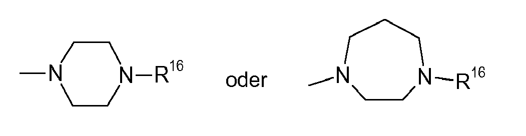 bilden,
worin
R¹⁶ Wasserstoff, Phenyl, Benzyl, Morpholinyl, Pyrrolidinyl, Piperidinyl, Piperazinyl oder N-Methylpiperazinyl bedeutet, oder geradkettiges oder verzweigtes Alkyl mit bis zu 5 Kohlenstoffatomen bedeutet, das gegebenenfalls durch Hydroxy substituiert ist,
R⁹ geradkettiges oder verzweigtes Alkyl mit bis zu 3 Kohlenstoffatomen bedeutet,
R¹⁰ und R¹¹ gleich oder verschieden sind und Wasserstoff oder geradkettiges oder verzweigtes Alkyl mit bis zu 3 Kohlenstoffatomen bedeuten, und/oder die unter R³/R⁴ aufgeführte Alkylkette gegebenenfalls durch Cyclopropyl, Cyclopentyl, Cyclohexyl, Cycloheptyl, Phenyl, Pyridyl, Chinolyl, Pyrrolidinyl, Pyrimidyl, Morpholinyl, Furyl, Piperidinyl, Tetrahydrofuranyl oder durch Reste der Formeln 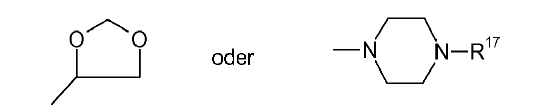 substituiert ist,
worin
R¹⁷ Wasserstoff, Hydroxy, Formyl, Trifluormethyl, geradkettiges oder verzweigtes Acyl oder Alkoxy mit jeweils bis zu 3 Kohlenstoffatomen bedeutet, oder geradkettiges oder verzweigtes Alkyl mit bis zu 4 Kohlenstoffatomen bedeutet, das gegebenenfalls ein- bis dreifach gleich oder verschieden durch Hydroxy, oder geradkettiges oder verzweigtes Alkoxy mit bis zu 4 Kohlenstoffatomen substituiert ist,
und wobei Phenyl und die Heterocyclen gegebenenfalls ein- bis dreifach, gleich oder verschieden durch Nitro, Fluor, Chlor, -SO₃H, geradkettiges oder verzweigtes Alkyl oder Alkoxy mit jeweils bis zu 4 Kohlenstoffatomen, Hydroxy und/oder durch einen Rest der Formel -SO₂₋NR¹⁸R¹⁹ substituiert sind,
worin
R¹⁸ und R¹⁹ gleich oder verschieden sind und Wasserstoff oder geradkettiges oder verzweigtes Alkyl mit bis zu 4 Kohlenstoffatomen bedeuten,
und/oder
R³ oder R⁴ für eine Gruppe der Formel -NR²⁰R²¹ steht,
worin
R²⁰ und R²¹ die oben angegebene Bedeutung von R¹⁸ und R¹⁹ haben und mit dieser gleich oder verschieden sind,
und/oder
R³ oder R⁴ für Adamantyl stehen, oder für Reste der Formeln 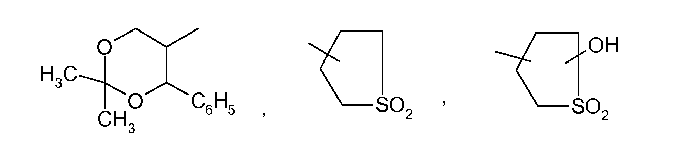 oder 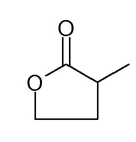 stehen,
oder für Cyclopentyl, Cyclohexyl, Cycloheptyl, Phenyl, Morpholinyl, Oxazolyl, Thiazolyl, Chinolyl, Isoxazolyl, Pyridyl, Tetrahydrofuranyl, Tetrahydropyranyl oder für Reste der Formeln 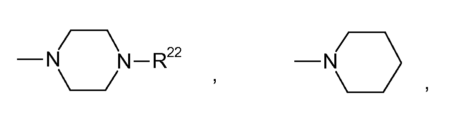 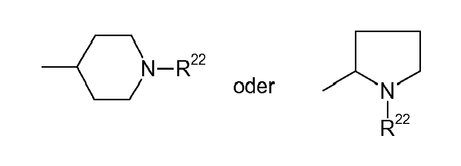 stehen,
worin
R²² die oben angegbene Bedeutung von R¹⁶ hat und mit dieser gleich oder verschieden ist, oder Carboxyl, Formyl oder geradkettiges oder verzweigtes Acyl mit bis zu 3 Kohlenstoffatomen bedeutet,
und wobei Cycloalkyl, Phenyl und/oder die Heterocyclen gegebenenfalls einbis dreifach, gleich oder verschieden durch Fluor, Chlor, Triazolyl, Trifluormethyl, Trifluormethoxy, Carboxyl, geradkettiges oder verzweigtes Acyl oder Alkoxycarbonyl mit jeweils bis zu 5 Kohlenstoffatomen, Nitro und/oder durch Gruppen der Formeln -SO₃H, -OR²³, (SO₂)ₑNR²⁴R²⁵, -P(O)(OR²⁶)(OR²⁷) substituiert sind,
worin
e eine Zahl 0 oder 1 bedeutet,
R²³ einen Rest der Formel 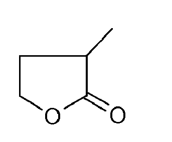 bedeutet, oder
Cyclopropyl, Cyclopentyl, Cyclobutyl, Cyclohexyl oder Cycloheptyl bedeutet,
Wasserstoff oder geradkettiges oder verzweigtes Alkyl mit bis zu 4 Kohlenstoffatomen bedeutet, das gegebenenfalls durch Cyclopropyl, Cyclopentyl, Cyclohexyl, Benzyloxy, Tetrahydropyranyl, Tetrahydrofuranyl, geradkettiges oder verzweigtes Alkoxy oder Alkoxycarbonyl mit jeweils bis zu 4 Kohlenstoffatomen, Benzyloxycarbonyl oder Phenyl substituiert ist, das seinerseits ein- bis mehrfach, gleich oder verschieden durch geradkettiges oder verzweigtes Alkoxy mit bis zu 3 Kohlenstoffatomen, Hydroxy, Fluor oder Chlor substituiert sein kann,
und/oder Alkyl gegebenenfalls durch Reste der Formeln -CO-NR²⁸R²⁹ oder -CO-R³⁰ substituiert ist,
worin
R²⁸ und R²⁹ gleich oder verschieden sind und Wasserstoff oder geradkettiges oder verzweigtes Alkyl mit bis zu 5 Kohlenstoffatomen bedeuten, oder
R²⁸ und R²⁹ gemeinsam mit dem Stickstoffatom einen Morpholinyl-, Pyrrolidinyl- oder Piperidinylring bilden,
und
R³⁰ Phenyl oder Adamantyl bedeutet,
R²⁴ und R²⁵ die oben angegebene Bedeutung von R¹⁸ und R¹⁹ haben und mit dieser gleich oder verschieden sind,
R²⁶ und R²⁷ die oben angegebene Bedeutung von R¹⁰ und R¹¹ haben und mit dieser gleich oder verschieden sind
und/oder Cycloalkyl, Phenyl und/oder die Heterocyclen gegebenenfalls durch geradkettiges oder verzweigtes Alkyl mit bis zu 4 Kohlenstoffatomen substituiert sind, das gegebenenfalls durch Hydroxy, Carboxyl, Pyridyl, Pyrimidyl, Pyrrolidinyl, Piperidinyl, Tetrahydrofuranyl, Triazolyl oder durch Gruppen der Formel -SO₂-R³¹, -P(O)(OR³²)(OR³³) oder -NR³⁴R³⁵ substituiert ist,
worin
R³¹ die oben angegebene Bedeutung von R⁹ hat und mit dieser gleich oder verschieden ist,
R³² und R³³ die oben angegebene Bedeutung von R¹⁰ und R¹¹ haben und mit dieser gleich oder verschieden sind,
R³⁴ und R³⁵ gleich oder verschieden sind und Wasserstoff oder geradkettiges oder verzweigtes Alkyl mit bis zu 5 Kohlenstoffatomen bedeuten, das gegebenenfalls durch Hydroxy oder geradkettiges oder verzweigtes Alkoxy mit bis zu 3 Kohlenstoffatomen substituiert ist, oder
R³⁴ und R³⁵ gemeinsam mit dem Stickstoffatom einen Morpholinyl-, Triazolyl- oder Thiomorpholinylring oder einen Rest der Formel 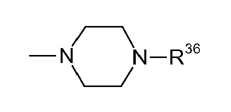 bilden,
worin
R³⁶ Wasserstoff, Hydroxy, geradkettiges oder verzweigtes Alkoxycarbonyl mit bis zu 5 Kohlenstoffatomen oder geradkettiges oder verzweigtes Alkyl mit bis zu 4 Kohlenstoffatomen bedeutet, das gegebenenfalls durch Hydroxy substituiert ist,
oder
R³ und R⁴ gemeinsam mit dem Stickstoffatom einen Morpholinyl-, Thiomorpholinyl-, Pyrrolidinyl-, Piperidinylring oder einen einen Rest der Formel 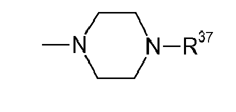 bilden,
worin
R³⁷ Wasserstoff, Hydroxy, Formyl, Trifluormethyl, geradkettiges oder verzweigtes Acyl, Alkoxy oder Alkoxycarbonyl mit jeweils bis zu 4 Kohlenstoffatomen bedeutet, oder geradkettiges oder verzweigtes Alkyl mit bis zu 5 Kohlenstoffatomen bedeutet, das gegebenenfalls ein- bis dreifach, gleich oder verschieden durch Hydroxy, Trifluormethyl, Carboxyl, geradkettiges oder verzweigtes Alkoxy oder Alkoxycarbonyl mit jeweils bis zu 4 Kohlenstoffatomen oder durch Gruppen der Formel -(D)_{f-}NR³⁸R³⁹, -CO-(CH₂)_{g}-O-CO-R⁴⁰, -CO-(CH₂)ₕ-OR⁴¹ oder -P(O)(OR⁴²)(OR⁴³) substituiert ist,
worin
g und h gleich oder verschieden sind und eine Zahl 1, 2 oder 3 bedeuten,
und
f eine Zahl 0 oder 1 bedeutet,
D eine Gruppe der Formel -CO oder -SO₂ bedeutet,
R³⁸ und R³⁹ gleich oder verschieden sind und die oben angegebene Bedeutung von R⁷ und R⁸ haben,
R⁴⁰ geradkettiges oder verzweigtes Alkyl mit bis zu 4 Kohlenstoffatomen bedeuten,
R⁴¹ geradkettiges oder verzweigtes Alkyl mit bis zu 4 Kohlenstoffatomen bedeutet,
R⁴² und R⁴³ gleich oder verschieden sind und Wasserstoff oder geradkettiges oder verzweigtes Alkyl mit bis zu 3 Kohlenstoffatomen bedeuten,
oder
R³⁷ einen Rest der Formel -(CO)ᵢ-E bedeutet,
worin
i eine Zahl 0 oder 1 bedeutet,
E Cyclopentyl, Cyclohexyl, Cycloheptyl, Benzyl, Phenyl, Pyridyl, Pyrimidyl oder Furyl bedeutet, wobei die oben aufgeführten Ringsysteme gegebenenfalls ein- bis zweifach, gleich oder verschieden durch Nitro, Fluor, Chlor, -SO₃H, geradkettiges oder verzweigtes Alkoxy mit bis zu 4 Kohlenstoffatomen, Hydroxy, Trifluormethyl, Trifluormethoxy oder durch einen Rest der Formel -SO₂-NR⁴⁴R⁴⁵, substituiert sind,
worin
R⁴⁴ und R⁴⁵ die oben angegebene Bedeutung von R¹⁸ und R¹⁹ haben und mit dieser gleich oder verschieden sind,
oder
E Reste der Formeln 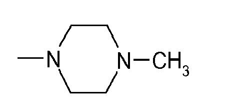 oder 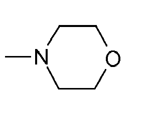 bedeutet, und die unter R³ und R⁴ aufgeführten, gemeinsam mit dem Stickstoffatom gebildeten Heterocyclen, gegebenenfalls ein- bis dreifach, gleich oder verschieden, gegebenenfalls auch geminal, durch Hydroxy, Formyl, Carboxyl, geradkettiges oder verzweigtes Acyl oder Alkoxycarbonyl mit bis jeweils zu 5 Kohlenstoffatomen, Nitro und Gruppen der Formeln -P(O)(OR⁴⁶)(OR⁴⁷), 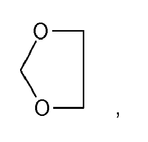 =NR⁴⁸ oder -(CO)ⱼNR⁴⁹R⁵⁰
substituiert sind,
worin
R⁴⁶ und R⁴⁷ die oben angegebene Bedeutung von R¹⁰ und R¹¹ haben und mit dieser gleich oder verschieden sind,
R⁴⁸ Hydroxy oder geradkettiges oder verzweigtes Alkoxy mit bis zu 3 Kohlenstoffatomen bedeutet,
j eine Zahl 0 oder 1 bedeutet,
und
R⁴⁹ und R⁵⁰ gleich oder verschieden sind und die oben angegebene Bedeutung von R¹⁴ und R¹⁵ haben,
und/oder die unter R³ und R⁴ aufgeführten, gemeinsam mit dem Stickstoffatom gebildeten Heterocyclen, gegebenenfalls durch geradkettiges oder verzweigtes Alkyl mit bis zu 5 Kohlenstoffatomen substituiert sind, das gegebenenfalls ein- bis mehrfach, gleich oder verschieden durch Hydroxy, Fluor, Chlor, Carboxyl, Cyclopropyl, Cyclopentyl, Cyclohexyl, Cycloheptyl, geradkettiges oder verzweigtes Alkoxy oder Alkoxycarbonyl mit jeweils bis zu 4 Kohlenstoffatomen oder durch einen Rest der Formel -SO₃H, -NR⁵¹R⁵² oder -P(O)OR⁵³OR⁵⁴ substituiert ist,
worin
R⁵¹ und R⁵² gleich oder verschieden sind und Wasserstoff, Phenyl, Carboxyl, Benzyl oder geradkettiges oder verzweigtes Alkyl oder Alkoxy mit jeweils bis zu 4 Kohlenstoffatomen bedeuten,
R⁵³ und R⁵⁴ gleich oder verschieden sind und die oben angegebene Bedeutung von R¹⁰ und R¹¹ haben,
und/oder das Alkyl gegebenenfalls durch Phenyl substituiert ist, das seinerseits ein- bis dreifach, gleich oder verschieden durch Fluor, Chlor, Hydroxy, geradkettiges oder verzweigtes Alkoxy mit bis zu 4 Kohlenstoffatomen, oder durch eine Gruppe der Formel -NR^{51'}R^{52'} substituiert sein kann,
worin
R^{51'} und R^{52'} die oben angegebene Bedeutung von R⁵¹ und R⁵² haben und mit dieser gleich oder verschieden sind,
und/oder die unter R³ und R⁴ aufgeführten, gemeinsam mit dem Stickstoffatom gebildeten Heterocyclen, gegebenenfalls durch Phenyl, Pyridyl, Piperidinyl, Pyrrolidinyl oder Tetrazolyl, gegebenenfalls auch über eine N-Funktion verknüpft, substituiert sind, wobei die Ringsysteme ihrerseits durch Hydroxy oder durch geradkettiges oder verzweigtes Alkyl oder Alkoxy mit jeweils bis zu 5 Kohlenstoffatomen substituiert sein können,
oder
R³ und R⁴ gemeinsam mit dem Stickstoffatom Reste der Formeln 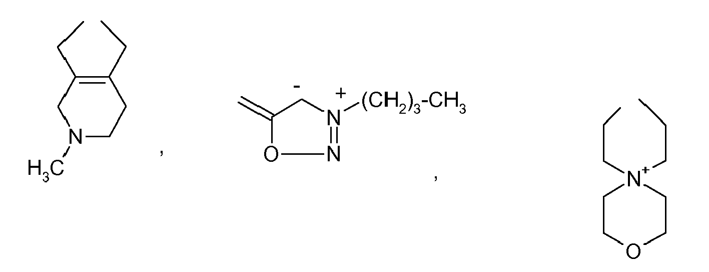 oder 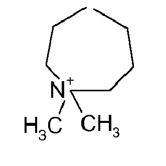 bilden,
R⁵ und R⁶ gleich oder verschieden sind und für Wasserstoff, Hydroxy oder für geradkettiges oder verzweigtes Alkoxy mit bis zu 4 Kohlenstoffatomen stehen,
und deren Salze, Hydrate, N-Oxide und isomere Formen.

3. 2-Phenyl-substituierte Imidazotriazinone der allgemeinen Formel (I) gemäß Anspruch 1, in welcher
R¹ für geradkettiges oder verzweigtes Alkyl mit bis zu 3 Kohlenstoffatomen steht,
R² für geradkettiges Alkyl mit bis zu 3 Kohlenstoffatomen steht,
R³ und R⁴ gleich oder verschieden sind und für Wasserstoff oder für geradkettiges oder verzweigtes Alkenyl oder Alkoxy mit jeweils bis zu 4 Kohlenstoffatomen stehen, oder
für eine geradkettige oder verzweigte Alkylkette mit bis zu 6 Kohlenstoffatomen stehen, die gegebenenfalls durch ein Sauerstoffatom unterbrochen ist, und die gegebenenfalls ein- bis dreifach, gleich oder verschieden durch Hydroxy, Fluor, Chlor, Carboxyl, geradkettiges oder verzweigtes Alkoxycarbonyl mit bis zu 4 Kohlenstoffatomen und/oder durch Reste der Formeln -SO₃H, -(A)ₐ-NR⁷R⁸, -O-CO-NR^{7'}R^{8'}, -S(O)_{b}-R⁹, -P(O)(OR¹⁰)(OR¹¹), 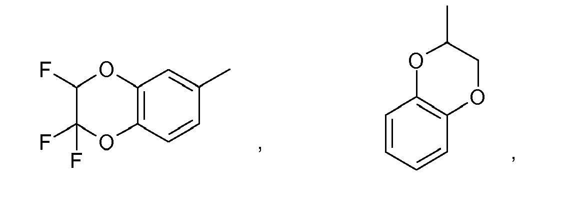 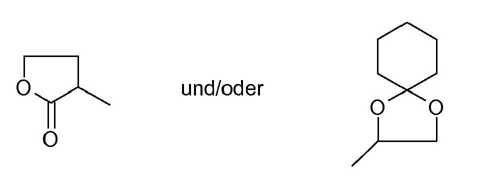 substituiert ist,
worin
a und b gleich oder verschieden sind und eine Zahl 0 oder 1 bedeuten,
A einen Rest CO oder SO₂ bedeutet,
R⁷, R^{7'}, R⁸ und R^{8'} gleich oder verschieden sind und Wasserstoff bedeuten, oder Cyclopentyl, Cyclohexyl, Cycloheptyl, Phenyl, Piperidinyl und Pyridyl bedeuten, wobei die oben aufgeführten Ringsysteme gegebenenfalls ein- bis zweifach, gleich oder verschieden durch Hydroxy, Nitro, Carboxyl, Fluor, Chlor, geradkettiges oder verzweigtes Alkoxy oder Alkoxycarbonyl mit jeweils bis zu 3 Kohlenstoffatomen oder durch eine Gruppe der Formel -(SO₂)_{c}-NR¹²R¹³ substituiert sind,
worin
c eine Zahl 0 oder 1 bedeutet,
R¹² und R¹³ leich oder verschieden sind und Wasserstoff oder geradkettiges oder verzweigtes Alkyl mit bis zu 3 Kohlenstoffatomen bedeuten,
oder
R⁷, R^{7'}, R⁸ und R^{8'} Methoxy bedeuten, oder geradkettiges oder verzweigtes Alkyl mit bis zu 6 Kohlenstoffatomen bedeuten, das gegebenenfalls ein- oder zweifach, gleich oder verschieden durch Hydroxy, Fluor, Chlor, Phenyl, geradkettiges oder verzweigtes Alkoxy oder Alkoxycarbonyl mit jeweils bis zu 3 Kohlenstoffatomen oder durch eine Gruppe der Formel -(CO)_{d}-NR¹⁴R¹⁵ substituiert ist,
worin
R¹⁴ und R¹⁵ gleich oder verschieden sind und Wasserstoff, Methyl oder Ethyl bedeuten,
und
d eine Zahl 0 oder 1 bedeutet,
oder
R⁷ und R⁸ und/oder R^{7'} und R^{8'} gemeinsam mit dem Stickstoffatom einen Morpholinyl-, Piperidinyl- oder Triazolylring oder Reste der Formeln 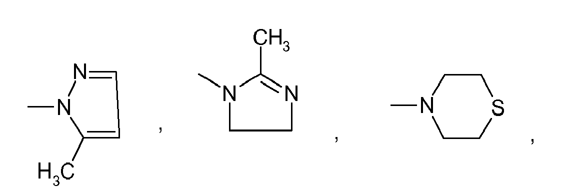 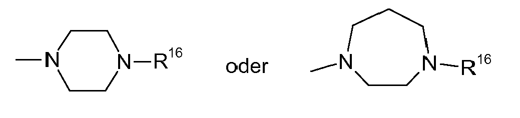 bilden,
worin
R¹⁶ Wasserstoff, Phenyl, Benzyl, Morpholinyl, Pyrrolidinyl, Piperidinyl, Piperazinyl oder N-Methylpiperazinyl bedeutet, oder geradkettiges oder verzweigtes Alkyl mit bis zu 3 Kohlenstoffatomen bedeutet, das gegebenenfalls durch Hydroxy substituiert ist,
R⁹ Methyl bedeutet,
R¹⁰ und R¹¹ gleich oder verschieden sind und Wasserstoff, Methyl oder Ethyl bedeuten,
und/oder die unter R³/R⁴ aufgeführte Alkylkette gegebenenfalls durch Cyclopropyl, Cyclopentyl, Cyclohexyl, Cycloheptyl, Morpholinyl, Furyl, Tetrahydrofuranyl oder durch Reste der Formeln 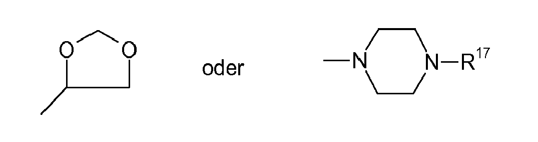 substituiert ist,
worin
R¹⁷ Wasserstoff, Hydroxy, Formyl, Acetyl oder Alkoxy mit bis zu 3 Kohlenstoffatomen bedeutet, oder geradkettiges oder verzweigtes Alkyl mit bis zu 3 Kohlenstoffatomen bedeutet, das gegebenenfalls ein- bis zweifach gleich oder verschieden durch Hydroxy oder geradkettiges oder verzweigtes Alkoxy mit bis zu 3 Kohlenstoffatomen substituiert ist,
und wobei Phenyl und die Heterocyclen gegebenenfalls ein- bis dreifach, gleich oder verschieden durch Fluor, Chlor, -SO₃H, geradkettiges oder verzweigtes Alkyl oder Alkoxy mit jeweils bis zu 3 Kohlenstoffatomen, Hydroxy und/oder durch einen Rest der Formel -SO₂₋NR¹⁸R¹⁹ substituiert sind,
worin
R¹⁸ und R¹⁹ gleich oder verschieden sind und Wasserstoff oder geradkettiges oder verzweigtes Alkyl mit bis zu 3 Kohlenstoffatomen bedeuten,
und/oder
R³ oder R⁴ für eine Gruppe der Formel -NR²⁰R²¹ steht,
worin
R²⁰ und R²¹ die oben angegebene Bedeutung von R¹⁸ und R¹⁹ haben und mit dieser gleich oder verschieden sind,
und/oder
R³ oder R⁴ für Adamantyl stehen, oder für Reste der Formeln 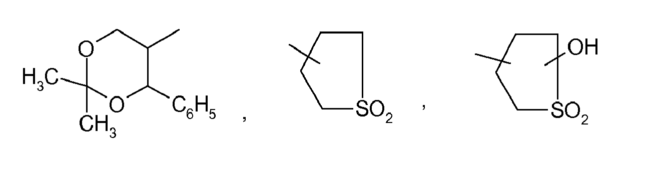 oder 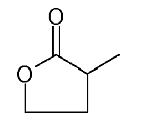 stehen,
oder für Cyclopentyl, Cyclohexyl, Cycloheptyl, Phenyl, Morpholinyl, Oxazolyl, Thiazolyl, Chinolyl, Isoxazolyl, Pyridyl, Tetrahydrofuranyl, Tetrahydropyranyl oder für Reste der Formeln 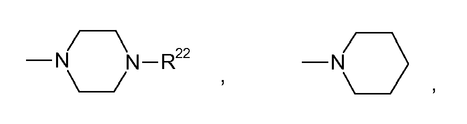 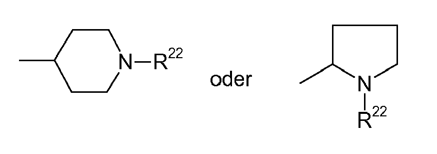 stehen,
worin
R²² die oben angegebene Bedeutung von R¹⁶ hat und mit dieser gleich oder verschieden ist, oder Formyl oder Acetyl bedeutet,
und wobei Cycloalkyl, Phenyl und/oder die Heterocyclen gegebenenfalls ein- bis zweifach, gleich oder verschieden durch Fluor, Chlor, Triazolyl, Carboxyl, geradkettiges oder verzweigtes Acyl oder Alkoxycarbonyl mit jeweils bis zu 4 Kohlenstoffatomen, Nitro und/oder durch Gruppen der Formeln -SO₃H, -OR²³, (SO₂)ₑNR²⁴R²⁵, -P(O)(OR²⁶)(OR²⁷) substituiert sind,
worin
e eine Zahl 0 oder 1 bedeutet,
R²³ einen Rest der Formel 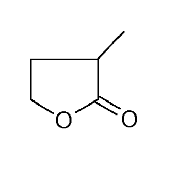 bedeutet, oder Cyclopropyl, Cyclopentyl, Cyclobutyl oder Cyclohexyl bedeutet, Wasserstoff oder geradkettiges oder verzweigtes Alkyl mit bis zu 3 Kohlenstoffatomen bedeutet, das gegebenenfalls durch Cyclopropyl, Cyclohexyl, Benzyloxy, Tetrahydropyranyl, geradkettiges oder verzweigtes Alkoxy oder Alkoxycarbonyl mit jeweils bis zu 3 Kohlenstoffatomen, Benzyloxycarbonyl oder Phenyl substituiert ist, das seinerseits ein- bis zweifach, gleich oder verschieden durch Methoxy, Hydroxy, Fluor oder Chlor substituiert sein kann,
und/oder Alkyl gegebenenfalls durch Reste der Formeln -CO-NR²⁸R²⁹ oder -CO-R³⁰ substituiert ist,
worin
R²⁸ und R²⁹ gleich oder verschieden sind und Wasserstoff oder geradkettiges oder verzweigtes Alkyl mit bis zu 4 Kohlenstoffatomen bedeuten, oder
R²⁸ und R²⁹ gemeinsam mit dem Stickstoffatom einen Morpholinyl-, Pyrrolidinyl- oder Piperidinylring bilden,
und
R³⁰ Phenyl oder Adamantyl bedeutet,
R²⁴ und R²⁵ die oben angegebene Bedeutung von R¹⁸ und R¹⁹ haben und mit dieser gleich oder verschieden sind,
R²⁶ und R²⁷ die oben angegebene Bedeutung von R¹⁰ und R¹¹ haben und mit dieser gleich oder verschieden sind und/oder Cycloalkyl, Phenyl und/oder die Heterocyclen gegebenenfalls durch geradkettiges oder verzweigtes Alkyl mit bis zu 3 Kohlenstoffatomen substituiert sind, das gegebenenfalls durch Hydroxy, Carboxyl, Pyridyl, Pyrimidyl, Pyrrolidinyl, Piperidinyl, Tetrahydrofuranyl, Triazolyl oder durch Gruppen der Formel -SO₂-R³¹, P(O)(OR³²)(OR³³) oder -NR³⁴R³⁵ substituiert ist,
worin
R³¹ Methyl bedeutet,
R³² und R³³ die oben angegebene Bedeutung von R¹⁰ und R¹¹ haben und mit dieser gleich oder verschieden sind,
R³⁴ und R³⁵ gleich oder verschieden sind und Wasserstoff oder geradkettiges oder verzweigtes Alkyl mit bis zu 3 Kohlenstoffatomen bedeuten, das gegebenenfalls durch Hydroxy oder Methoxy substituiert ist, oder
R³⁴ und R³⁵ gemeinsam mit dem Stickstoffatom einen Morpholinyl-, Triazolyl- oder Thiomorpholinylring oder einen Rest der Formel 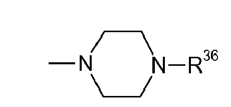 bilden, worin
R³⁶ Wasserstoff, Hydroxy, geradkettiges oder verzweigtes Alkoxycarbonyl mit bis zu 3 Kohlenstoffatomen oder geradkettiges oder verzweigtes Alkyl mit bis zu 3 Koh- lenstoffatomen bedeutet, das gegebenenfalls durch Hydroxy substituiert ist,
oder
R³ und R⁴ gemeinsam mit dem Stickstoffatom einen Morpholinyl-, Thiomorpholinyl-, Pyrrolidinyl-, Piperidinylring oder einen einen Rest der Formel 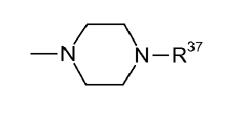 bilden,
worin
R³⁷ Wasserstoff, Hydroxy, Formyl, geradkettiges oder verzweigtes Acyl, Alkoxy oder Alkoxycarbonyl mit jeweils bis zu 3 Kohlenstoffatomen bedeutet, oder geradkettiges oder verzweigtes Alkyl mit bis zu 4 Kohlenstoffatomen bedeutet, das gegebenenfalls ein- bis zweifach, gleich oder verschieden durch Hydroxy, geradkettiges oder verzweigtes Alkoxy oder Alkoxycarbonyl mit jeweils bis zu 3 Kohlenstoffatomen oder durch Gruppen der Formel -(D)_{f-}NR³⁸R³⁹, -CO-(CH₂)_{g}-O-CO-R⁴⁰, -CO-(CH₂)ₕ-OR⁴¹ oder -P(O)(OR⁴²)(OR⁴³) substituiert ist,
worin
g und h gleich oder verschieden sind und eine Zahl 1 oder 2 bedeuten,
und
f eine Zahl 0 oder 1 bedeutet,
D eine Gruppe der Formel -CO oder-SO₂ bedeutet,
R³⁸ und R³⁹ gleich oder verschieden sind und die oben angegebene Bedeutung von R⁷ und R⁸ haben,
R⁴⁰ geradkettiges oder verzweigtes Alkyl mit bis zu 3 Kohlenstoffatomen bedeutet,
R⁴¹ geradkettiges oder verzweigtes Alkyl mit bis zu 3 Kohlenstoffatomen bedeutet,
R⁴² und R⁴³ gleich oder verschieden sind und Wasserstoff, Methyl oder Ethyl bedeuten,
oder
R³⁷ einen Rest der Formel -(CO)ᵢ-E bedeutet,
worin
i eine Zahl 0 oder 1 bedeutet,
E Cyclopentyl, Benzyl, Phenyl, Pyridyl, Pyrimidyl oder Furyl bedeutet, wobei die oben aufgeführten Ringsysteme gegebenenfalls ein- bis zweifach, gleich oder verschieden durch Nitro, Fluor, Chlor, -SO₃H, geradkettiges oder verzweigtes Alkoxy mit bis zu 3 Kohlenstoffatomen, Hydroxy oder durch einen Rest der Formel -SO₂-NR⁴⁴R⁴⁵, substituiert sind,
worin
R⁴⁴ und R⁴⁵ die oben angegebene Bedeutung von R¹⁸ und R¹⁹ haben und mit dieser gleich oder verschieden sind,
oder
E Reste der Formeln 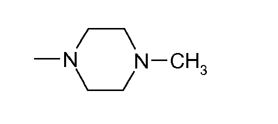 oder 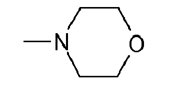 bedeutet, und die unter R³ und R⁴ aufgeführten, gemeinsam mit dem Stickstoffatom gebildeten Heterocyclen, gegebenenfalls ein- bis dreifach, gleich oder verschieden, gegebenenfalls auch geminal, durch Hydroxy, Formyl, Carboxyl, geradkettiges oder verzweigtes Acyl oder Alkoxycarbonyl mit bis jeweils zu 3 Kohlenstoffatomen oder Gruppen der Formeln -P(O)(OR⁴⁶)(OR⁴⁷), 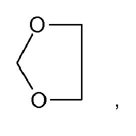 =NR⁴⁸ oder -(CO)ⱼNR⁴⁹R⁵⁰
substituiert sind, worin
R⁴⁶ und R⁴⁷ die oben angegebene Bedeutung von R¹⁰ und R¹¹ haben und mit dieser gleich oder verschieden sind,
R⁴⁸ Hydroxy oder Methoxy bedeutet,
j eine Zahl 0 oder 1 bedeutet,
und
R⁴⁹ und R⁵⁰ gleich oder verschieden sind und die oben angegebene Bedeutung von R¹⁴ und R¹⁵ haben,
und/oder die unter R³ und R⁴ aufgeführten, gemeinsam mit dem Stickstoffatom gebildeten Heterocyclen, gegebenenfalls durch geradkettiges oder verzweigtes Alkyl mit bis zu 4 Kohlenstoffatomen substituiert sind, das gegebenenfalls ein- bis dreifach, gleich oder verschieden durch Hydroxy, Fluor, Chlor, Carboxyl, Cyclopropyl, Cycloheptyl, geradkettiges oder verzweigtes Alkoxy oder Alkoxycarbonyl mit jeweils bis zu 3 Kohlenstoffatomen oder durch einen Rest der Formel -SO₃H, -NR⁵¹R⁵² oder P(O)OR⁵³OR⁵⁴ substituiert ist,
worin
R⁵¹ und R⁵² gleich oder verschieden sind und Wasserstoff, Phenyl, Carboxyl, Benzyl oder geradkettiges oder verzweigtes Alkyl oder Alkoxy mit jeweils bis zu 3 Kohlenstoffatomen bedeuten,
R⁵³ und R⁵⁴ gleich oder verschieden sind und die oben angegebene Bedeutung von R¹⁰ und R¹¹ haben,
und/oder das Alkyl gegebenenfalls durch Phenyl substituiert ist, das seinerseits ein- bis zweifach, gleich oder verschieden durch Fluor, Chlor, Hydroxy, Methoxy oder durch eine Gruppe der Formel - NR^{51'}R^{52'} substituiert sein kann,
worin
R^{51'} und R^{52'} die oben angegebene Bedeutung von R⁵¹ und R⁵² haben und mit dieser gleich oder verschieden sind,
und/oder die unter R³ und R⁴ aufgeführten, gemeinsam mit dem Stickstoffatom gebildeten Heterocyclen, gegebenenfalls durch Phenyl, Pyridyl, Piperidinyl, Pyrrolidinyl oder Tetrazolyl, gegebenenfalls auch über eine N-Funktion verknüpft, substituiert sind, wobei die Ringsysteme ihrerseits durch Hydroxy oder durch geradkettiges oder verzweigtes Alkyl oder Alkoxy mit jeweils bis zu 3 Kohlenstoffatomen substituiert sein können,
oder
R³ und R⁴ gemeinsam mit dem Stickstoffatom Reste der Formeln 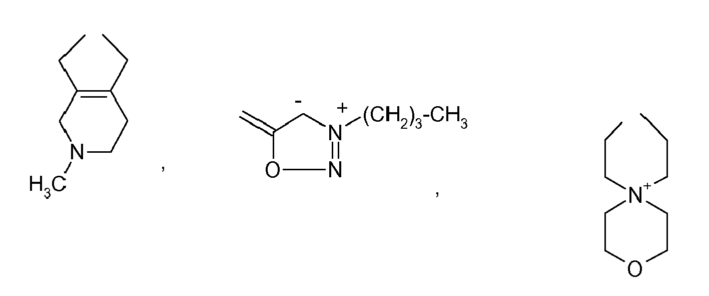 oder 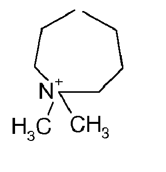 bilden,
R⁵ und R⁶ gleich oder verschieden sind und für Wasserstoff, Hydroxy oder für geradkettiges oder verzweigtes Alkoxy mit bis zu 3 Kohlenstoffatomen stehen,
und deren Salze, Hydrate, N-Oxide und isomere Formen.

4. 2-Phenyl-substituierte Imidazotriazinone der allgemeinen Formel (I) gemäß Anspruch 1, in welcher
R¹ für Methyl oder Ethyl steht,
R² für Ethyl oder Propyl steht,
R³ und R⁴ gleich oder verschieden sind und für eine geradkettige oder verzweigte Alkylkette mit bis zu 5 Kohlenstoffatomen stehen, die gegebenenfalls bis zu zweifach gleich oder verschieden durch Hydroxy oder Methoxy substituiert ist,
oder
R³ und R⁴ gemeinsam mit dem Stickstoffatom einen Piperidinyl-, Morpholinyl-, Thiomorpholinylring oder einen Rest der Formel 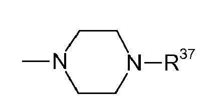 bilden,
worin
R³⁷ Wasserstoff, Formyl, geradkettiges oder verzweigtes Acyl oder Alkoxycarbonyl mit jeweils bis zu 3 Kohlenstoffatomen bedeutet,
oder geradkettiges oder verzweigtes Alkyl mit bis zu 3 Kohlenstoffatomen bedeutet, das gegebenenfalls ein- bis zweifach, gleich oder verschieden durch Hydroxy, Carboxyl, geradkettiges oder verzweigtes Alkoxy oder Alkoxycarbonyl mit jeweils bis zu 3 Kohlenstoffatomen oder durch Gruppen der Formeln -(D)_{f}-NR³⁸R³⁹ oder -P(O)(OR⁴²)(OR⁴³) substituiert ist,
worin
f eine Zahl 0 oder 1 bedeutet,
D eine Gruppe der Formel -CO bedeutet,
R³⁸ und R³⁹ gleich oder verschieden sind und Wasserstoff oder Methyl bedeuten,
R⁴² und R⁴³ gleich oder verschieden sind und Wasserstoff, Methyl oder Ethyl bedeuten,
oder
R³⁷ Cyclopentyl bedeutet,
und die unter R³ und R⁴ aufgeführten, gemeinsam mit dem Stickstoffatom gebildeten Heterocyclen, gegebenenfalls ein- bis zweifach, gleich oder verschieden, gegebenenfalls auch geminal, durch Hydroxy, Formyl, Carboxyl, geradkettiges oder verzweigtes Acyl oder Alkoxycarbonyl mit bis jeweils zu 3 Kohlenstoffatomen oder Gruppen der Formeln -P(O)(OR⁴⁶)(OR⁴⁷) oder -(CO)ᵢNR⁴⁹R⁵⁰ substituiert sind,
worin
R⁴⁶ und R⁴⁷ gleich oder verschieden sind und Wasserstoff, Methyl oder Ethyl bedeuten,
j eine Zahl 0 oder 1 bedeutet,
und
R⁴⁹ und R⁵⁰ gleich oder verschieden sind und Wasserstoff oder Methyl bedeutenund/oder die unter R³ und R⁴ aufgeführten, gemeinsam mit dem Stickstoffatom gebildeten Heterocyclen, gegebenenfalls durch geradkettiges oder verzweigtes Alkyl mit bis zu 3 Kohlenstoffatomen substituiert sind, das gegebenenfalls ein- bis zweifach, gleich oder verschieden durch Hydroxy, Carboxyl oder durch einen Rest der Formel P(O)OR⁵³OR⁵⁴ substituiert ist,
worin
R⁵³ und R⁵⁴ gleich oder verschieden sind und Wasserstoff, Methyl oder Ethyl bedeuten,
und/oder die unter R³ und R⁴ aufgeführten, gemeinsam mit dem Stickstoffatom gebildeten Heterocyclen, gegebenenfalls durch über N-verknüpftes Piperidinyl oder Pyrrolidinyl substituiert sind,
R⁵ für Wasserstoff steht,
und
R⁶ für Ethoxy oder Propoxy steht,
und deren Salze, Hydrate, N-Oxide und isomere Formen.

5. 2-Phenyl-substituierte Imidazotriazinone gemäß Ansprüchen 1 bis 4 mit folgenden Strukturen:

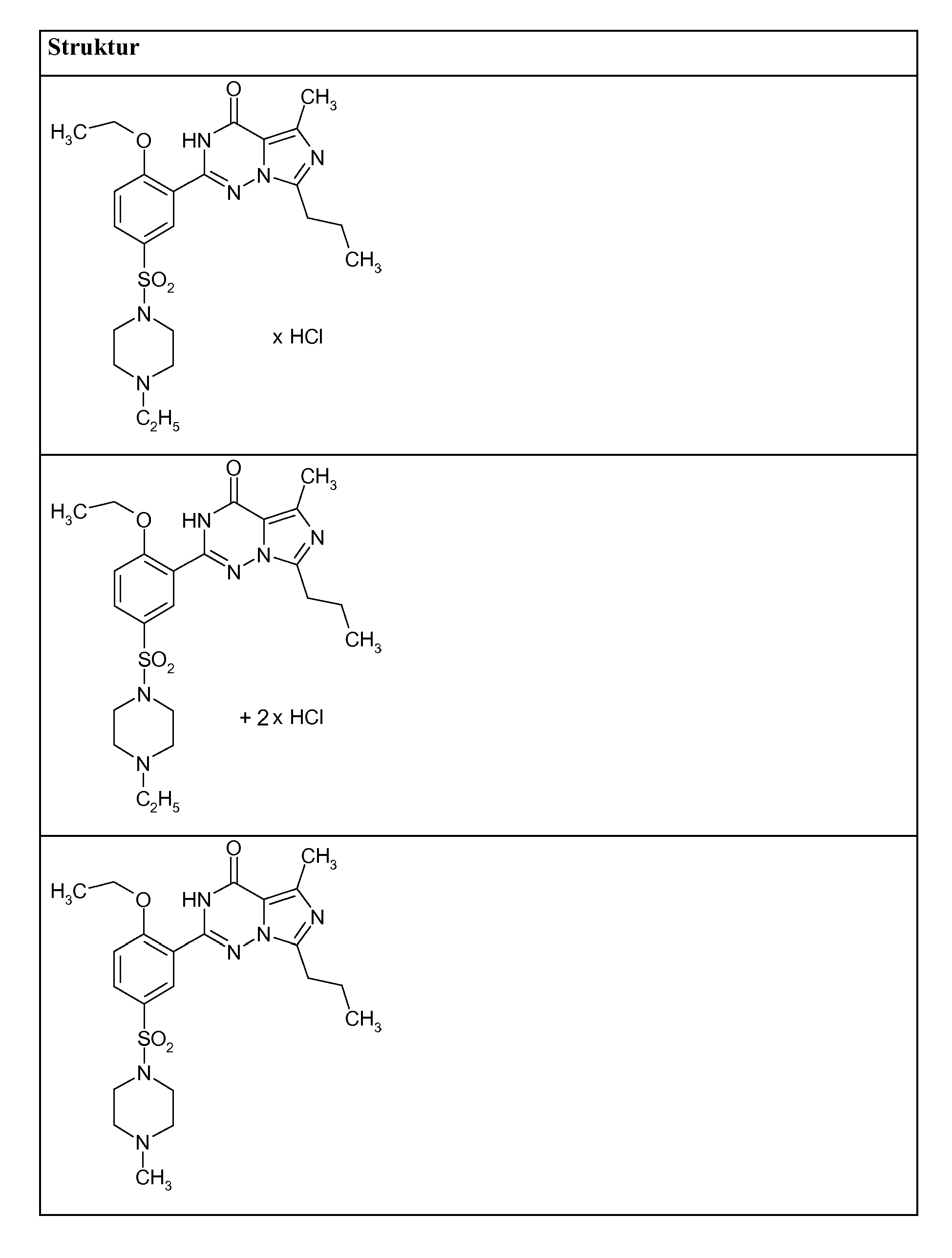

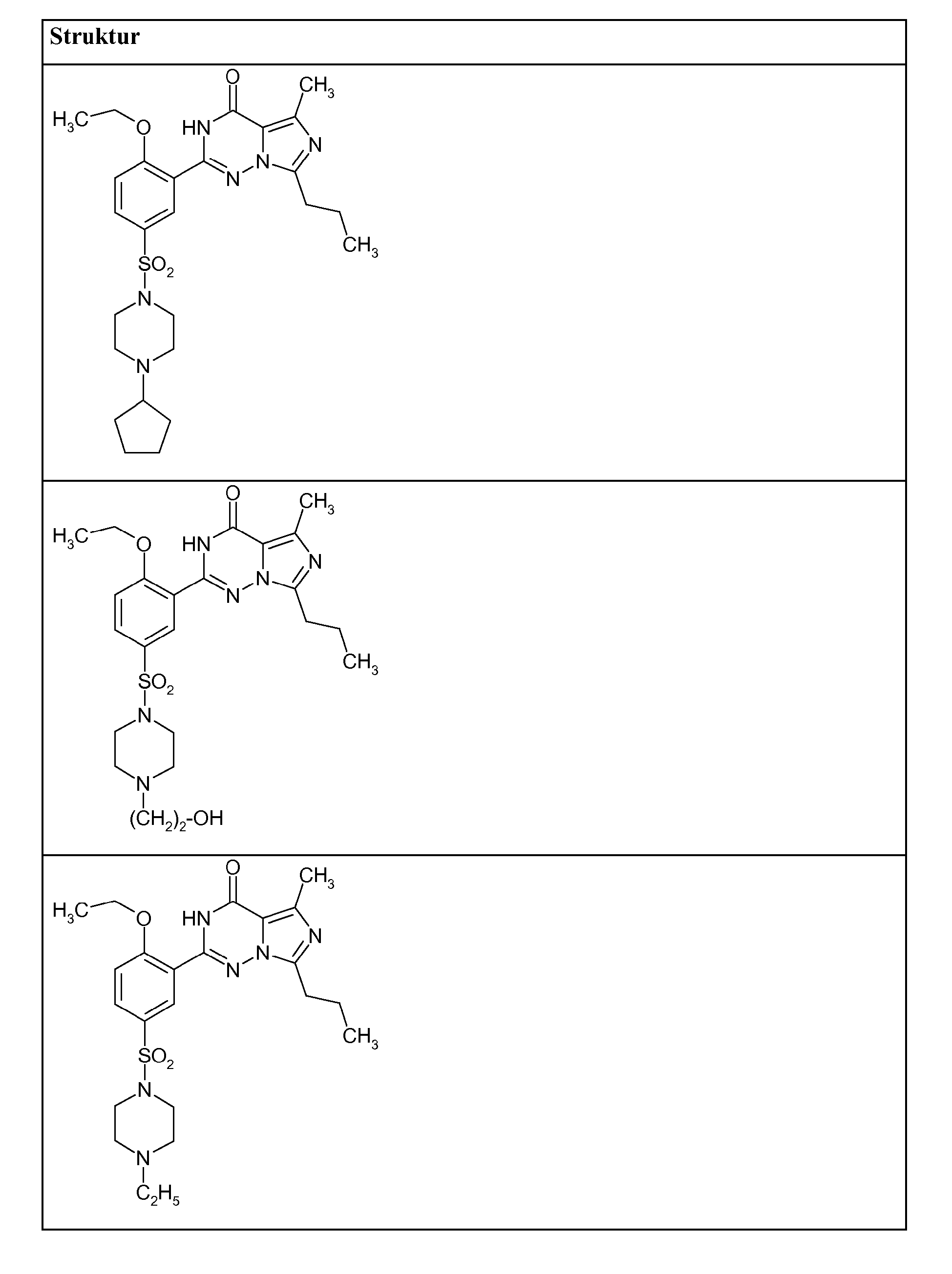

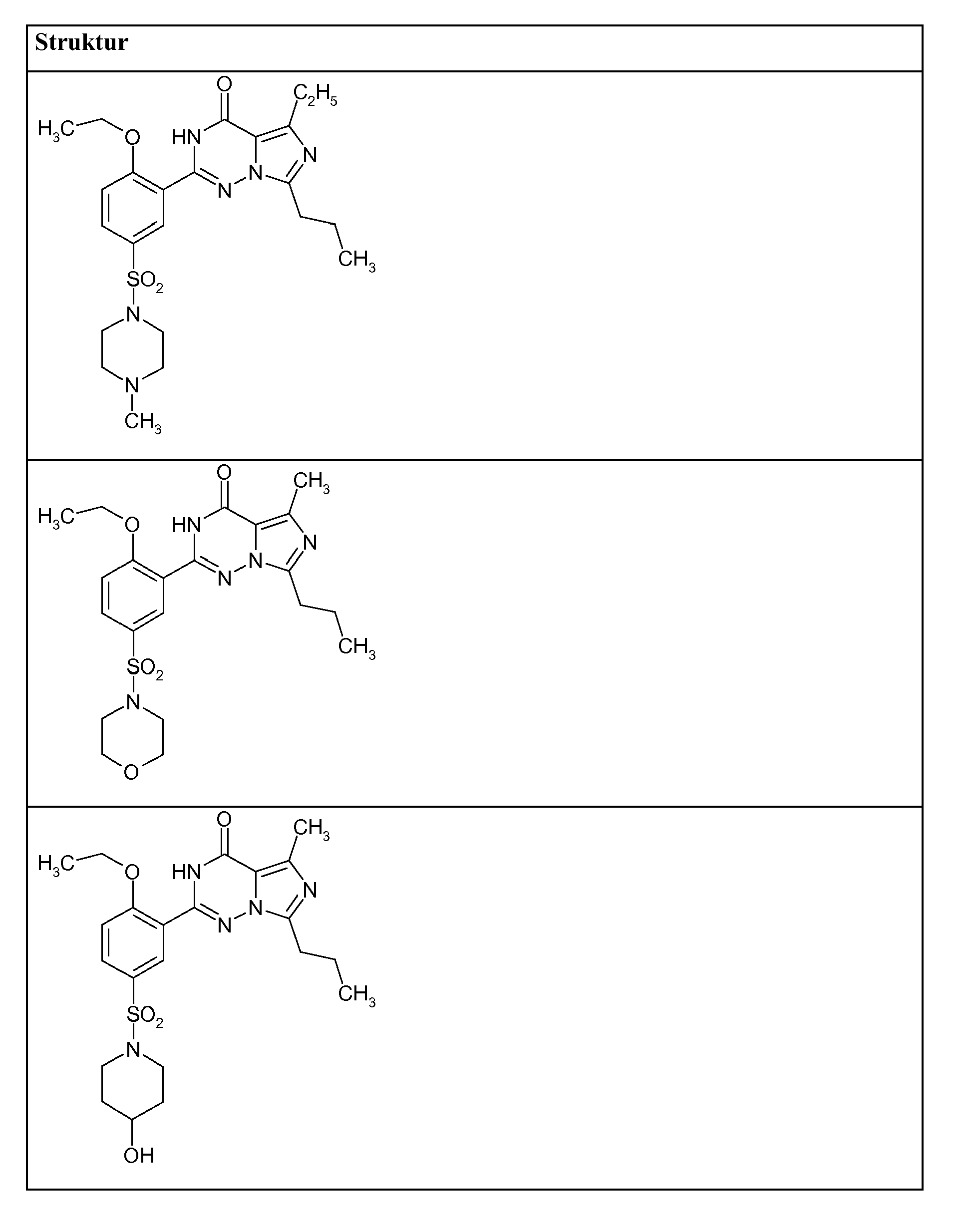

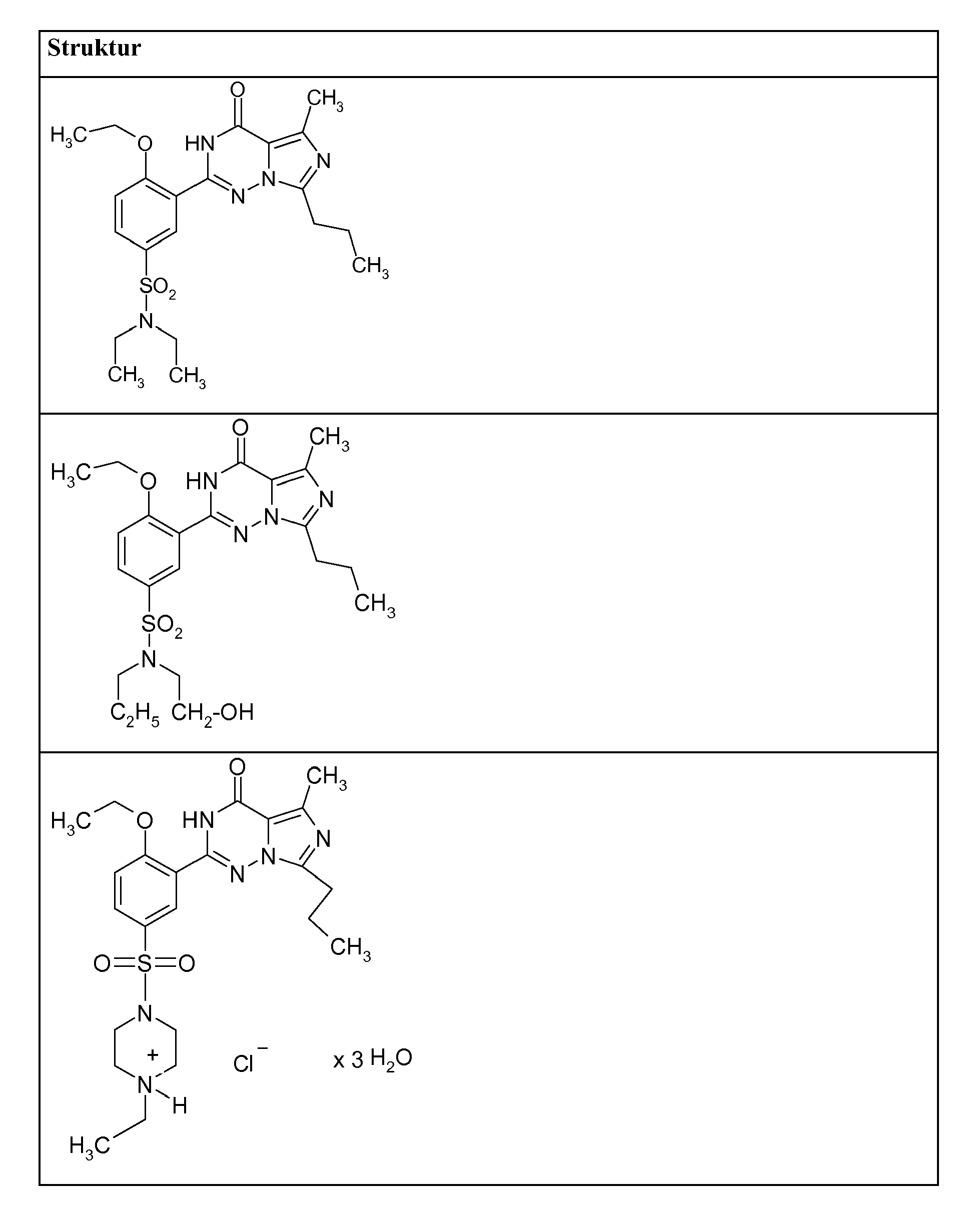

6. 2-Phenyl-substituierte Imidazotriazinone der allgemeinen Formel (I) gemäß Anspruch 1 zur Behandlung von Erkrankungen.

7. Verfahren zur Herstellung von 2-Phenyl-substituierten Imidazotriazinonen gemäß Anspruch 1, **dadurch gekennzeichnet, daß** man zunächst Verbindungen der allgemeinen Formel (II) 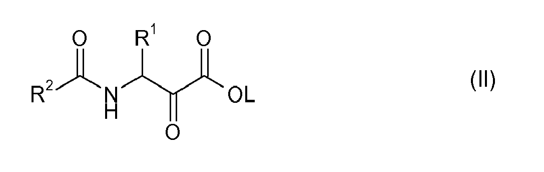 in welcher
R¹ und R² die oben angegebene Bedeutung haben und
L für geradkettiges oder verzweigtes Alkyl mit bis zu 4 Kohlenstoffatomen steht,
mit Verbindungen der allgemeinen Formel (III) 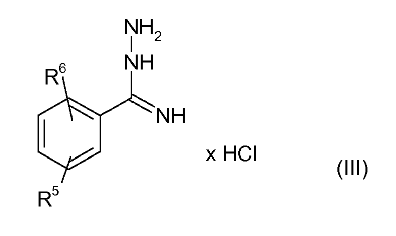 in welcher
R⁵ und R⁶ die oben angegebene Bedeutung haben,
in einer Zweistufenreaktion in den Systemen Ethanol und Phosphoroxytrichlorid / Dichlorethan in die Verbindungen der allgemeinen Formel (IV) 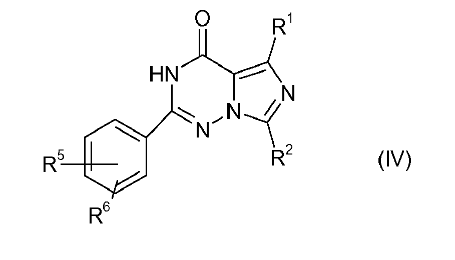 in welcher
R¹, R², R⁵ und R⁶ die oben angegebene Bedeutung haben,
überführt, in einem weiteren Schritt mit Chlorsulfonsäure zu den Verbindungen der allgemeinen Formel (V) 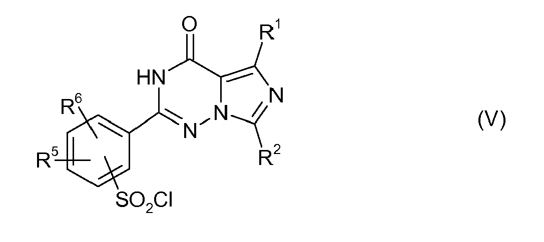 in welcher
R¹, R², R⁵ und R⁶ die oben angegebene Bedeutung haben,
umsetzt und abschließend mit Aminen der allgemeinen Formel (VI)
HN³R⁴ (VI)
in welcher
R³ und R⁴ die oben angegebene Bedeutung haben,
in inerten Lösemitteln umsetzt.

8. Arzneimittel enthaltend mindestens ein 2-Phenyl-substituiertes Imidazotriazinon gemäß Anspruch 1 sowie pharmakologisch unbedenkliche Formulierungsmittel.

9. Arzneimittel gemäß Anspruch 8 zur Behandlung von cardiovaskulären, cerebrovaskulären Erkrankungen und/oder Erkrankungen des Urogenitaltraktes.

10. Arzneimittel gemäß Anspruch 9 zur Behandlung von erektiler Dysfunktion.

11. Verwendung von 2-Phenyl-substituierten Imidazotriazinonen gemäß Anspruch 1 zur Herstellung von Arzneimitteln.
